# EUROPEAN PATENT SPECIFICATION

(11) **EP 3 373 933 B1**
(45) Date of publication and mention of the grant of the patent: **26.08.2020**
(21) Application number: 16864812.9
(22) Date of filing: 07.11.2016
(51) Int. Cl.: C07D 211/06, C07D 401/04, A61K 31/451, A61P 25/28

(54) **BISPIPERIDINYL DERIVATIVES AS LIVER X RECEPTOR BETA AGONISTS, COMPOSITIONS, AND THEIR USE**
BISPIPERIDINYLDERIVATE ALS LEBER-X-REZEPTOR-BETAAGONISTEN, ZUSAMMENSETZUNGEN UND DEREN VERWENDUNG
DÉRIVÉS DE BISPIPÉRIDINYLE UTILISÉS COMME AGONISTES DES RÉCEPTEURS BÊTA X DU FOIE, COMPOSITIONS ASSOCIÉES ET LEUR UTILISATION

(30) Priority: 12.11.2015 US 201562254465 P
(43) Date of publication of application: 19.09.2018
(73) Proprietor: Merck Sharp & Dohme Corp., Rahway, NJ 07065-0907 (US)
(72) Inventor: STACHEL, Shawn, J., West Point, Pennsylvania 19486 (US); BENNETT, David Jonathan, Boston, Massachusetts 02115-5727 (US); BRNARDIC, Edward, J., Lansdale, Pennsylvania 19446 (US); LIVERTON, Nigel, J., Harleysville, Pennsylvania 19438 (US); MANLEY, Peter, J., West Point, Pennsylvania 19486 (US); MENG, Zhaoyang, Ambler, Pennsylvania 19002 (US); NANDA, Kausik, K., West Point, Pennsylvania 19486 (US); RUDD, Michael, T., West Point, Pennsylvania 19486 (US); WAI, Jenny, Harleysville, Pennsylvania 19438 (US)
(74) Representative: Hussain, Deeba
(86) International application number: PCT/US2016/060768
(87) International publication number: WO 2017/083219

(56) References cited:
- WO-A1-2015/090496
- WO-A1-2015/091420
- US-A1- 2009 203 710
- US-A1- 2010 075 987
- SZEWCZYK ET AL.: "Design of potent and selective GPR119 agonists for type II diabetes", BIOORG. MED. CHEM. LETT., vol. 21, no. 9, 1 May 2011 (2011-05-01), pages 2665-2669, XP055137695, ISSN: 0960-894X, DOI: 10.1016/j.bmcl.2010.12.086
- LI ET AL.: "Liver X receptor modulators: a review of recently patented compounds (2007 - 2009)", EXPERT OPIN. THER. PATENTS, vol. 20, no. 4, 20 March 2010 (2010-03-20) , pages 535-562, XP055294659, ISSN: 1354-3776, DOI: 10.1517/13543771003621269
- LOREN ET AL.: "Liver X receptor modulators: a review of recently patented compounds (2009 - 2012)", EXPERT OPIN. THER. PATENTS, vol. 23, no. 10, 5 July 2013 (2013-07-05), pages 1317-1335, XP055294658, ISSN: 1354-3776, DOI: 10.1517/13543776.2013.814640
- STACHEL ET AL.: "Identification and in Vivo Evaluation of Liver X Receptor [beta]-Selective Agonists for the Potential Treatment of Alzheimer's Disease", J. MED. CHEM., vol. 59, no. 7, 24 March 2016 (2016-03-24) , pages 3489-3498, XP055572398, US ISSN: 0022-2623, DOI: 10.1021/acs.jmedchem.6b00176

## Description

### FIELD OF THE INVENTION

The present invention provides certain compounds of formula (I), and compositions comprising these compounds, as Liver X-β receptor (LXRβ) agonists, which may be useful for treating or preventing pathologies related thereto. Such pathologies include, but are not limited to, inflammatory diseases and diseases characterized by defects in cholesterol and lipid metabolism, such as Alzheimer's disease.

### BACKGROUND OF THE INVENTION

Alzheimer's disease is a common neurodegenerative disease affecting the elderly, resulting in progressive memory impairment, loss of language and visuospatial skills, and behavior deficits. Clinical, genetic, epidemiological and biochemical evidence suggest that dysfunctional cholesterol metabolism is implicated in the pathogenesis of Alzheimer's Disease. Hypercholesterolemia and low levels of high density lipoprotein are well-established risk factors for Alzheimer's Disease. It has been suggested that vascular, genetic and amyloid factors, in combination with diet and lifestyle, contribute to the cause and progression of Alzheimer's Disease. Hooijmans et al, Eur J Pharmacol 585 (2008), 176-196.

The liver X receptor (LXR) is a member of the nuclear receptor family of transcription factors, and is a part of the cholesterol regulation pathway. There are two identified isoforms of LXRs. LXRα is found in liver, intestine and in macrophages, while LXRβ is widely expressed in many tissues and is considered a ubiquitous receptor. Typically, the activity of nuclear receptors is controlled by small lipophilic moieties, such as hormones, fatty acids, bile acids, cholesterol precursors and oxysterols. Lala, Curr Opinions Invest Drugs 2005, 6:934-943. Cholesterol precursors such as desmosterol and oxysterols are known to bind and activate LXRs.

LXRs have demonstrated a role in the physiological metabolism of lipid and cholesterol, and thus are believed to have an important role in metabolic disorders such as hyperlipidemia and atherosclerosis. Activation of LXRs reduces cholesterol absorption, thereby reducing the ability of the body to take up cholesterol. Consistently, deletion of LXRs in mice leads to impaired cholesterol and bile acid metabolism. *See* Peet et al, Cell 1998, 93(5): 693-704. Activation of LXRs also increase peripheral cholesterol efflux systems, and impact the elimination of cholesterol by regulating cholesterol excretion into bile. *See* Cao et al, Drug News Perspect 20004, 17(1), 35-41.

LXRs also regulate lipid homeostasis in the brain. The connection between metabolic disorders and Alzheimer's Disease suggests that LXRs may have a role in the Alzheimer's disease pathway. Activation of LXRs also inhibit inflammation and pro-inflammatory expression in the body. Zelcer et al, J Clin Invest 2006, 116:3 (607-614). Thus, LXRs may serve as targets for the treatment of inflammatory diseases. However, activation of hepatic LXRα is believed to be the underlying cause of liver steatosis and hyperlipidemia associated with dual LXRα/β small agonist molecules developed to date.

LXRs have also been proposed as possible therapeutics to treat a number of cancers e.g. prostate, breast, ovarian, melanoma, pancreas, lung, colon and hematological malignancy (Lin, C-Y and Gustafsson, J-A, (2015) Nature Reviews Cancer 15, 216-224).

LXRβ is the predominant brain isoform. *See* Song et al, Ann NY Acad Sci 195, 761:38-49. LXRβ knockout male mice demonstrated adult-onset motor neuron degeneration. (Andersson et al, Proc Nat'lAcad Sci USA 2005, 8;1902(1)):3857-3862), and the LXRα and LXRβ double knockout mice develop neurodegenerative changes in brain tissue. (Wang et al, Proc Natl Acad Sci U S A. 2002, 99(21):13878-83). Therefore development of selective LXRβ agonists could be a therapeutic approach to neurodegenerative diseases such as AD and avoid the peripheral adverse lipid effects that have been linked to LXRα.

LXR modulators have been reviewed in Li et al, Expert Opin. Ther. Patents 2010, 20(4), 535-562 and Loren et al, Expert Opin. Ther. Patents 2013, 23(10), 1317-1335. Applicants have now discovered a series of LXRβ selective agonists. Thus, the compounds of the invention, which are selective LXRβ agonists, may be useful in the treatment of Alzheimer's disease, inflammatory diseases, and diseases characterized by defects in cholesterol and lipid metabolism.

### SUMMARY OF THE INVENTION

The present invention provides certain compounds, which are collectively or individually referred to herein as "compound(s) of the invention", as described herein. The compounds of the invention are selective agonists of LXRβ, and may be useful for treating or preventing diseases or pathologies related thereto.

In one embodiment, the compounds of the invention have the structural Formula (I): 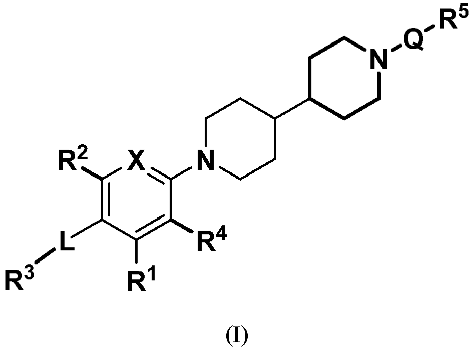 or a pharmaceutically acceptable salt thereof, wherein:
X is selected from the group consisting of -N- and -CH-;
R¹ is selected from the group consisting of H, methyl, and halogen;
R² is selected from the group consisting of H and halogen;
R⁴ is selected from the group consisting of H and methyl;
L is a bond and R³ is selected from the group consisting of -(C₁-C₆)alkyl and -(C₁-C₆)alkyl-OH;
or, alternatively, L is a divalent moiety selected from the group consisting of -C(O)- and -S(O)₂-; and R³ is -N(R^{N1})(R^{N2}), wherein:
   R^{N1} is selected from the group consisting of H and -(C₁-C₆)alkyl; and
   R^{N2} is selected from the group consisting of: H, -(C₁-C₆)alkyl, -O-(C₁-C₆)alkyl, -OH, halogen, -CN, and -(C₁-C₆)alkyl which is substituted with 1 or 2 groups independently selected from:
      -OH, halogen, -CN,
      optionally substituted phenyl, (wherein said optional substitutents on said phenyl are 1 to 3 groups independently selected from OH, CN, -(C₁-C₄)alkyl, -(C₁-C₄)alkoxyl),
      optionally substituted heteroaryl, (wherein said optional substituents on said heteroaryl are 1 to 3 groups independently selected from -(C₁-C₆)alkyl, -(C₁-C₄)alkoxyl, and cyclopropyl),
      optionally substituted cyclopropyl (wherein said optional substituents on said cyclopropyl are 1 to 3 groups independently selected from -(C₁-C₆)alkyl), and optionally substituted heterocycloalkyl (wherein said optional substitutents on said heterocycloalkyl are 1 to 3 groups independently selected from halogen, - OH, oxo, CN, and -(C₁-C₆)alkyl,
   or, alternatively, R^{N1} and R^{N2} are taken together with the nitrogen atom to which they are shown attached to form a 4-, 5-, or 6-membered fully saturated heterocyclic ring comprising (including said nitrogen atom) 1, 2, or 3 ring heteroatoms selected from the group consisting of N, N-oxide, O, S, and S-oxide,
      wherein said heterocyclic ring is unsubstituted or substituted with 1, 2, or 3 substituents independently selected from the group consisting of halogen, -OH, oxo, CN, -(C₁-C₆)alkyl, amino-substituted -(C₁-C₆)alkyl (wherein said amino is 1, 2, or 3 groups independently selected from the group consisting of -NH₂, -N(C₁-C₄alkyl)₂,and -NH(C₁-C₄alkyl)), -O-(C₁-C₆)alkyl, -(C₁-C₆)alkyl-OH, -(C₁-C₆)haloalkyl, -C(O)O-(C₁-C₆)alkyl, cyclopropyl, spirocyclopropyl, -CH₂-NHC(O)O-(C₁-C₆)alkyl, -CH₂-N(CH₃)C(O)O-(C₁-C₆)alkyl, phenyl, benzyl, - NHC(O)-phenyl, heteroaryl, and -(C₁-C₄)alkylheteroaryl, heterocycloalkyl;
Q is a bond or a divalent moiety selected from the group consisting of -C(O)-, and -S(O)₂-; and
R⁵ is selected from the group consisting of:
   -C(R^{5A})(R^{5B})(R^{5C}), wherein:
   each of R^{5A}, R^{5B} and R^{5C} is independently selected from the group consisting of: H, halogen, OH, -(C₁-C₆)alkyl, -(C₁-C₆)haloalkyl, -(C₁-C₆)alkenyl, -(C₁-C₆)alkynyl, -(C₃-C₆)cycloalkyl, -(C₃-C₆)cycloalkyl substituted with -(C₁-C₆)alkyl, -(C₁-C₆)alkenyl, -(C₁-C₆)alkynyl, phenyl, phenyl substituted with from 1 to 3 groups independently selected from halogen, -(C₁-C₆)alkyl, -O-(C₁-C₆)alkyl, and -C(O)O-(C₁-C₆)alkyl, 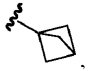 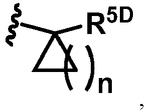 wherein:
   n is an integer from 1 to 4; and R^{5D} is selected from the group consisting of H, - (C₁-C₆)alkyl, -(C₁-C₆)haloalkyl, phenyl, and phenyl substituted with from 1 to 3 groups independently selected from the group consisting of OH, halogen, -(C₁-C₆)alkyl, and -O-(C₁-C₆)alkyl, and
   phenyl, wherein:
   said phenyl is unsubstituted or substituted with 1, 2, or 3 groups independently selected from the group consisting of halogen, CN, -(C₁-C₆)alkyl, and -(C₁-C₆)haloalkyl.

In other embodiments, the invention provides compositions, including pharmaceutical compositions, comprising one or more compounds of the invention (e.g., one compound of the invention) or a pharmaceutically acceptable salt thereof, optionally together with one or more additional therapeutic agents, optionally in an acceptable (e.g., pharmaceutically acceptable) carrier or diluent.

In another embodiment, the description is directed to methods of treating an inflammatory diseases or a disease characterized by defects in cholesterol or lipid metabolism, in a patient in need thereof by administering to the patient a compound of the invention or a pharmaceutically acceptable salt thereof. Non-limiting examples of such inflammatory diseases and diseases characterized by defects in cholesterol or lipid metabolism are described below.

In another embodiment, the description provides for the use of a compound of the invention in the manufacture of a medicament for treating an inflammatory disease or a disease characterized by defects in cholesterol or lipid metabolism in a patient in need thereof. Non-limiting examples of such inflammatory diseases and diseases characterized by defects in cholesterol or lipid metabolism are described below.

In another embodiment, the description is directed to a method of treating cancer in a patient in need thereof comprising administering to the patient a compound of the invention or a pharmaceutically acceptable salt thereof. Such cancers include prostate, breast, ovarian, melanoma, pancreas, lung, colon and hematological malignancy.

In another embodiment, the description provides for the use of a compound of the invention in the manufacture of a medicament for treating cancer in a patient in need thereof comprising administering to the patient a compound of the invention or a pharmaceutically acceptable salt thereof. Such cancers include prostate, breast, ovarian, melanoma, pancreas, lung, colon and hematological malignancy.

In another embodiment, the invention provides for any of the compounds of the invention for use as a medicament, or in medicine, or in therapy.

### DETAILED DESCRIPTION

For each of the following embodiments, any variable not explicitly defined in the embodiment is as defined in Formula (I). In each of the embodiments described herein, each variable is selected independently of the other unless otherwise noted.

In one embodiment, the compounds of the invention have the structural Formula (I) as described above, and pharmaceutically acceptable salts thereof.

In another embodiment, in Formula (I), X is N.

In another embodiment, in Formula (I), X is CH.

In another embodiment, in Formula (I), R¹ is selected from the group consisting of H, methyl, F, and Cl.

In another embodiment, in Formula (I), R² is selected from the group consisting of H, F, and Cl.

In another embodiment, in Formula (I), R⁴ is H.

In another embodiment, in Formula (I), R⁴ is methyl.

In another embodiment, in Formula (I), R¹ is H and R² is Cl.

In another embodiment, in Formula (I), R¹ is H and R² is H.

In another embodiment, in Formula (I), R¹ is methyl and R² is H.

In another embodiment, in Formula (I), R¹ is Cl and R² is Cl.

In another embodiment, in Formula (I), X is -CH-; R¹ is H; R² is Cl; and R⁴ is H.

In another embodiment, in Formula (I), X is -N-; R¹ is H; R² is Cl; and R⁴ is H.

In another embodiment, in Formula (I), X is -CH-; R¹ is H; R² is H; and R⁴ is H.

In another embodiment, in Formula (I), X is -N-; R¹ is H; R² is H; and R⁴ is H.

In another embodiment, in Formula (I), X is -CH-; R¹ is Cl; R² is Cl; and R⁴ is H.

In another embodiment, in Formula (I), X is -N-; R¹ is Cl; R² is Cl; and R⁴ is H.

The following alternative embodiments of L and R³ apply to Formula (I) and also to each of the embodiments and alternative embodiments of described hereinabove.

In another embodiment, in Formula (I):
L is a bond and R³ is selected from the group consisting of -(C₁-C₆)alkyl and -(C₁-C₆)alky l-OH;

In another embodiment, in Formula (I):
L is a divalent moiety selected from the group consisting of -C(O)- and -S(O)₂-; and
R³ is -N(R^{N1})(R^{N2}), wherein R^{N1} and R^{N2} are each independently selected from the group consisting of H and -(C₁-C₆)alkyl.

In another embodiment, in Formula (I):
L is -C(O)-; and
R³ is -N(R^{N1})(R^{N2}), wherein R^{N1} and R^{N2} are each independently selected from the group consisting of H and -(C₁-C₆)alkyl.

In another embodiment, in Formula (I), L is -C(O)-; and R³ is -N(CH₃)₂.

In another embodiment, in Formula (I):
L is -S(O)₂-; and
R³ is -N(R^{N1})(R^{N2}), wherein R^{N1} and R^{N2} are each independently selected from the group consisting of H and -(C₁-C₆)alkyl.

In another embodiment, in Formula (I):
L is -C(O)-; and R³ is -N(R^{N1})(R^{N2}), wherein:
R^{N1} is selected from the group consisting of H and -(C₁-C₆)alkyl; and
R^{N2} is -(C₁-C₆)alkyl which is optionally substituted with 1 or 2 groups independently selected from:
   optionally substituted phenyl, (wherein said optional substitutents on said phenyl are 1 to 3 groups independently selected from OH, CN, -(C₁-C₄)alkyl, -(C₁-C₄)alkoxyl),
   optionally substituted heteroaryl, (wherein said optional substituents on said heteroaryl are 1 to 3 groups independently selected from -(C₁-C₆)alkyl, -(C₁-C₄)alkoxyl, and cyclopropyl),
   optionally substituted cyclopropyl (wherein said optional substituents on said cyclopropyl are 1 to 3 groups independently selected from -(C₁-C₆)alkyl),
   optionally substituted heterocycloalkyl (wherein said optional substitutents on said heterocycloalkyl are 1 to 3 groups independently selected from halogen, - OH, oxo, CN, and -(C₁-C₆)alkyl, and -O-(C₁-C₆)alkyl, -OH, F, Cl, and -CN.

In each of the above embodiments, non-limiting examples of said optionally substituted heteroaryl include: pyridyl, pyrazinyl, furanyl, thienyl, pyrimidinyl, isoxazolyl, isothiazolyl, oxazolyl, oxadiazolyl, thiazolyl, thiadiazolyl, pyrazolyl, furazanyl, pyrrolyl, pyrazolyl, triazolyl, 1,2,4-thiadiazolyl, pyrazinyl, pyridazinyl, oxindolyl, indolyl, azaindolyl, imidazolyl, thienopyridyl, imidazopyridyl, isoquinolinyl, benzoazaindolyl, and triazinyl. In one embodiment, said optionally substituted heteroaryl is isoxazolyl, oxadiazolyl, or thiazolyl.

In each of the above embodiments, non-limiting examples of said optionally substituted heterocycloalkyl include: tetrahydrofuranyl and morpholinyl.

In each of the above embodiments, non-limiting examples of said optionally substituted cycloalkyl include cyclopropyl, cyclobutyl, cyclopentyl and cyclohexyl.

In another embodiment, in Formula (I):
L is -C(O)-; and R³ is -N(R^{N1})(R^{N2}), wherein:
R^{N1} and R^{N2} are taken together with the nitrogen atom to which they are shown attached to form a 4-, 5-, or 6-membered fully saturated heterocyclic ring comprising (including said nitrogen atom) 1, 2, or 3 ring heteroatoms selected from the group consisting of N, N-oxide, O, S, and S-oxide,
wherein said heterocyclic ring is unsubstituted or substituted with 1, 2, or 3 substituents independently selected from the group consisting of halogen, -OH, oxo, CN, -(C₁-C₆)alkyl, amino-substituted -(C₁-C₆)alkyl (wherein said amino is 1, 2, or 3 groups independently selected from the group consisting of -NH₂, -N(C₁-C₄alkyl)₂,and -NH(C₁-C₄alkyl)), -O-(C₁-C₆)alkyl, -(C₁-C₆)alkyl-OH, -(C₁-C₆)haloalkyl, -C(O)O-(C₁-C₆)alkyl, cyclopropyl, spirocyclopropyl, -CH₂-NHC(O)O-(C₁-C₆)alkyl, -CH₂-N(CH₃)C(O)O-(C₁-C₆)alkyl, phenyl, benzyl, - NHC(O)-phenyl, heteroaryl, and -(C₁-C₄)alkylheteroaryl, heterocycloalkyl. In the immediately preceding embodiment, non-limiting examples of said unsubstituted or substituted heterocyclic ring include azetidinyl, pyrrolidinyl, piperidinyl, and morpholinyl.

In another embodiment, in Formula (I), L is a bond and R³ is -(C₁-C₆)alkyl which is optionally substituted with OH.

In an alternative of the immediately preceding embodiment, R³ is

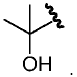

In another embodiment, in Formula (I), L is a-C(O) -; and R³ is -O-(C₁-C₆)alkyl.

The following alternative embodiments of Q and R⁵ apply to Formula (I) and also to each of the embodiments and alternative embodiments of described hereinabove.

In one embodiment, in Formula (I):
Q is a bond or a divalent moiety selected from the group consisting of -C(O)-, and S(O)₂; and
R⁵ is-C(R^{5A})(R^{5B})(R^{5C}),
   wherein each of R^{5A}, R^{5B} and R^{5C} is independently selected from the group consisting of: H, F, Cl, OH, -(C₁-C₆)alkyl, -(C₁-C₆)haloalkyl, -(C₁-C₆)alkenyl, - (C₁-C₆)alkynyl, -(C₃-C₆)cycloalkyl, -(C₃-C₆)cycloalkyl substituted with -(C₁-C₆)alkyl, -(C₁-C₆)alkenyl, -(C₁-C₆)alkynyl, phenyl, phenyl substituted with from 1 to 3 groups independently selected from F, Cl, -(C₁-C₆)alkyl, -O-(C₁-C₆)alkyl, and -C(O)O-(C₁-C₆)alkyl.

In an alternative of the immediately preceding embodiment, Q is -C(O)-.

In another alternative of the immediately preceding embodiment, Q is -C(O)-;
R^{5A} is OH;
R^{5B} is -(C₁-C₃)fluoroalkyl; and
R^{5C} is selected from the group consisiting of -(C₁-C₆)alkyl, -(C₁-C₄)fluoroalkyl, phenyl, (wherein said phenyl substituted with from 1 - 3 groups independently selected from halogen - (C₁-C₆)alkyl, and -(C₁-C₆)alkoxy), cyclopropyl (wherein said cyclopropyl is optionally substituted with -(C₁-C₆)alkyl), cyclobutyl (wherein said cyclobutyl is optionally substituted with -(C₁-C₆)alkyl), ethenyl, and ethynyl.

In one embodiment, in Formula (I):
Q is a bond or a divalent moiety selected from the group consisting of -C(O)-, and S(O)₂; and R⁵ is selected from the group consisting of 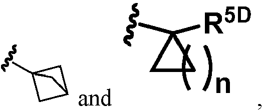 wherein n is an integer from 1 to 4; and R^{5D} is selected from the group consisting of H, -(C₁-C₆)alkyl, -(C₁-C₆)haloalkyl, phenyl, and phenyl substituted with from 1 to 3 groups independently selected from the group consisting of OH, F, Cl, -(C₁-C₆)alkyl, and -O-(C₁-C₆)alkyl.

In an alternative of the immediately preceding embodiment, Q is -C(O)-.

In one embodiment, in Formula (I):
Q is a bond or a divalent moiety selected from the group consisting of -C(O)-, -S(O)₂-, and -C(O)O-; and
R⁵ is phenyl,
   wherein said phenyl is unsubstituted or substituted with 1, 2, or 3 groups independently selected from the group consisting of F, Cl, CN, -(C₁-C₆)alkyl, and -(C₁-C₆)haloalkyl.

In an alternative of the immediately preceding embodiment, Q is a bond. In another alternative of the immediately preceding embodiment, Q is -C(O)-. In another alternative of the immediately preceding embodiment, Q is -S(O)₂-. In another alternative of the immediately preceding embodiment, Q is -C(O)-.

Specific non-limiting examples of compounds of the invention are shown in the examples below. Examples wherein Q corresponds with -C(O)O- are not comprised in the appendant claims and do not form part of the invention. both the structures and the IUPAC names of the example compounds are provided, if there are any discrepancies between the structure and the name provided, the structure shall control unless otherwise apparent in context.

In another embodiment, there is provided a composition comprising a compound of the invention and a pharmaceutically acceptable carrier or diluent.

In one embodiment, the description is directed to methods of treating a patient (preferably a human) for inflammatory diseases and diseases characterized by defects in cholesterol or lipid metabolism, by administering to the patient a compound of the invention or a pharmaceutically acceptable salt thereof.

The description is also directed to the use of a compound of the invention for treating inflammatory diseases and diseases characterized by defects in cholesterol or lipid metabolism, by administering to a patient (preferably a human) a compound of the invention or a pharmaceutically acceptable salt thereof.

The invention is also directed to medicaments or pharmaceutical compositions for treating inflammatory diseases and diseases characterized by defects in cholesterol or lipid metabolism, by administering to a patient (preferably a human) a compound of the invention, or a pharmaceutically acceptable salt thereof, and a pharmaceutically acceptable carrier.

The description is further directed to a the manufacture of a medicament or a composition for inflammatory diseases and diseases characterized by defects in cholesterol or lipid metabolism, by administering to a patient (preferably a human) a compound of the invention or a pharmaceutically acceptable salt thereof, with one or more pharmaceutically acceptable carriers.

Exemplary inflammatory diseases and diseases characterized by defects in cholesterol or lipid metabolism for which the compounds of the invention are useful include neurodegenerative and neurological diseases, such as Alzheimer's Disease, Neimann-Pick disease type Cl, Parkinson's Disease, amyotrophic lateral sclerosis, stroke, age-related macular degeneration, psychiatric disorders such as schizophrenia and depression, and metabolic disorders such as cardiovascular disease, obesity and diabetes.

The present description is directed to the use of the compounds of the invention as LXRβ agonists in a patient or subject such as a mammal in need of such activity, comprising the administration of an effective amount of the compound. In addition to humans, a variety of other mammals can be treated according to the method of the present invention.

The compounds of the invention may have utility in treating or ameliorating Alzheimer's disease. The compounds may also be useful in treating or ameliorating other inflammatory diseases and diseases characterized by defects in cholesterol and lipid metabolism, such as Neimann-Pick disease type Cl, Parkinson's Disease, amyotrophic lateral sclerosis, stroke, age-related macular degeneration, psychiatric disorders such as schizophrenia and depression, and metabolic disorders such as cardiovascular disease, obesity and diabetes.

For example, the compounds of the invention may be useful for the prevention of dementia of the Alzheimer's type, as well as for the treatment of early stage, intermediate stage or late stage dementia of the Alzheimer's type.

Potential cardiovascular conditions or disorders for which the compounds of the invention may be useful include atherosclerosis, hypertension, hyperlipidemia, coronary heart disease, peripheral vascular disease, dyslipidemia, hyperbetalipoproteinemia, hypoalphalipoproteinemia, hypercholesterolemia, hypertriglyceridemia, familial-hypercholesterolemia, angina, ischemia, cardiac ischemia, stroke, myocardial infarction, reperfusion injury, angioplastic restenosis, vascular complications of diabetes, obesity (including abdominal obesity) and endotoxemia.

The compounds of the invention may also be useful in the treatment of the metabolic syndrome. According to one widely used definition, a patient having metabolic syndrome is characterized as having three or more symptoms selected from the following group of five symptoms: (1) abdominal obesity; (2) hypertriglyceridemia; (3) low high-density lipoprotein cholesterol (HDL); (4) high blood pressure; and (5) elevated fasting glucose, which may be in the range characteristic of Type 2 diabetes if the patient is also diabetic. Each of these symptoms is defined clinically in the recently released Third Report of the National Cholesterol Education Program Expert Panel on Detection, Evaluation and Treatment of High Blood Cholesterol in Adults (Adult Treatment Panel III, or ATP III), National Institutes of Health, 2001, NIH Publication No. 01-3670. Patients with metabolic syndrome have an increased risk of developing the macrovascular and microvascular complications that are listed above, including atherosclerosis and coronary heart disease.

The compounds of the invention may also be useful for the treatment of Type 2 diabetes, and conditions and disorders related to Type 2 diabetes, such as (1) hyperglycemia, (2) low glucose tolerance, (3) insulin resistance, (4) obesity, (5) lipid disorders, (6) dyslipidemia, (7) hyperlipidemia, (8) hypertriglyceridemia, (9) hypercholesterolemia, (10) low HDL levels, (11) high LDL levels, (12) atherosclerosis and its sequelae, (13) vascular restenosis, (14) irritable bowel syndrome, (15) inflammatory bowel disease, including Crohn's disease and ulcerative colitis, (16) other inflammatory conditions, (17) pancreatitis, (18) abdominal obesity, (19) neurodegenerative disease, (20) retinopathy, (21) nephropathy, (22) neuropathy, (23) Syndrome X, (24) ovarian hyperandrogenism (polycystic ovarian syndrome), and other disorders where insulin resistance is a component.

The compounds of the invention may also have utility in treating certain kinds of cancers which are affected by the LXR mechanism. Such cancers include, but are not limited to, prostate, breast, ovarian, melanoma, pancreas, lung, colon and hematological malignancy. (Lin, C-Y and Gustafsson, J-A, (2015) Nature Reviews Cancer 15, 216-224).

The compounds of the invention may be used in combination with one or more other drugs in the treatment of diseases or conditions for which the compounds of the invention have utility, where the combination of the drugs together are safer or more effective than either drug alone. Additionally, the compounds of the invention may be used in combination with one or more other drugs that treat, prevent, control, ameliorate, or reduce the risk of side effects or toxicity of the compounds of the invention. Such other drugs may be administered, by a route and in an amount commonly used contemporaneously or sequentially with the compounds of the invention. Accordingly, the pharmaceutical compositions of the present invention include those that contain one or more other active ingredients, in addition to the compounds of the invention. The combinations may be administered as part of a unit dosage form combination product, or as a kit or treatment protocol wherein one or more additional drugs are administered in separate dosage forms as part of a treatment regimen.

Examples of combinations of the compounds of the invention include combinations with anti-Alzheimer's Disease agents, for example: other LXRβ agonists; beta-secretase inhibitors including verubecestat (N-[3-[(5R)-3-amino-2,5-dimethyl-1,1-dioxo-6H-1,2,4-thiadiazin-5-yl]-4-fluorophenyl]-5-fluoropyridine-2-carboxamide); alpha 7 nicotinic agonists; ADAM 10 ligands or activators; gamma-secretase inhibitors, such as LY450139 and TAK 070; gamma secretase modulators; tau phosphorylation inhibitors; glycine transport inhibitors; ApoE4 conformational modulators; NR2B antagonists; androgen receptor modulators; blockers of Aβ 13orticotr formation; 5-HT4 agonists, such as PRX-03140; 5-HT6 antagonists, such as xaliproden; 5-HT1a antagonists, such as lecozotan; p25/CDK5 inhibitors; NK1/NK3 receptor antagonists; COX-2 inhibitors; HMG-CoA reductase inhibitors; NSAIDs including ibuprofen; vitamin E; anti-amyloid antibodies (including anti-amyloid humanized monoclonal antibodies), such as bapineuzumab; anti-inflammatory compounds such as I-flurbiprofen, nitroflurbiprofen; PPAR gamma agonists, such as pioglitazone and rosiglitazone; CB-1 receptor antagonists or CB-1 receptor inverse agonists; antibiotics such as doxycycline and rifampin; N-methyl-D-aspartate (NMDA) receptor antagonists, such as memantine, neramexane and EVT101; cholinesterase inhibitors such as galantamine, rivastigmine, donepezil, tacrine, phenserine and ladostigil; growth hormone secretagogues such as ibutamoren, ibutamoren mesylate, and capromorelin; histamine H₃ receptor antagonists; AMPA agonists or AMPA modulators; PDE IV inhibitors; GABA_{A} inverse agonists; GSK3β inhibitors; neuronal nicotinic agonists; selective M1 agonists; HDAC inhibitors; and microtubule affinity regulating kinase (MARK) ligands; dimebon; or other drugs that affect receptors or enzymes that either increase the efficacy, safety, convenience, or reduce unwanted side effects or toxicity of the compounds of the invention.

Other examples of combinations of the compounds of the invention include combinations with anti-obesity agents such as apolipoprotein-B secretion/microsomal triglyceride transfer protein (apo-B/MTP) inhibitors, 11β-hydroxy steroid dehydrogenase-1 (11 β -HSD type 1) inhibitors, peptide YY₃₋₃₆ or analogs thereof, MCR-4 agonists, cholecystokinin-A (CCK-A) agonists, monoamine reuptake inhibitors (such as sibutramine), sympathomimetic agents, β₃ adrenergic receptor agonists, dopamine agonists (such as bromocriptine), melanocyte-stimulating hormone receptor analogs, 5HT2c agonists, melanin concentrating hormone antagonists, leptin (the OB protein), leptin analogs, leptin receptor agonists, galanin antagonists, lipase inhibitors (such as tetrahydrolipstatin, i.e. orlistat), anorectic agents (such as a bombesin agonist), neuropeptide-Y antagonists (e.g., NPY Y5 receptor antagonists) thyromimetic agents, dehydroepiandrosterone or an analog thereof, glucocorticoid receptor agonists or antagonists, orexin receptor antagonists, glucagon-like peptide-1 receptor agonists, ciliary neurotrophic factors (such as Axokine™, available from Regeneron Pharmaceuticals, Inc., Tarrytown, N.Y. and Procter & Gamble Company, Cincinnati, Ohio), human agouti-related proteins (AGRP), ghrelin receptor antagonists, histamine 3 receptor antagonists or inverse agonists, neuromedin U receptor agonists and the like.

Other examples of combinations of the compounds of the invention include combinations with antihypertensive agents; anti-inflammatory agents (e.g., COX-2 inhibitors); antidepressants (e.g., fluoxetine hydrochloride (Prozac™)); cognitive improvement agents (e.g., donepezil hydrochloride (Aircept™) and other acetylcholinesterase inhibitors); neuroprotective agents (e.g., memantine); antipsychotic medications (e.g., ziprasidone (Geodon™), risperidone and olanzapine); insulin and insulin analogs (e.g., LysPro insulin); GLP-1 (7-37) (insulinotropin) and GLP-1 (7-36)-NH₂; sulfonylureas and analogs thereof: chlorpropamide, glibenclamide, tolbutamide, tolazamide, acetohexamide, Glypizide™, glimepiride, repaglinide, meglitinide; biguanides: metformin, phenformin, buformin; *alpha*2-antagonists and imidazolines: midaglizole, isaglidole, deriglidole, idazoxan, efaroxan, fluparoxan; other insulin secretagogues: linogliride, A-4166; glitazones: ciglitazone, pioglitazone, englitazone, troglitazone, darglitazone, Avandia™; fatty acid oxidation inhibitors: clomoxir, etomoxir; *alpha*-glucosidase inhibitors: acarbose, miglitol, emiglitate, voglibose, MDL-25,637, camiglibose, MDL-73,945; (β-agonists: BRL 35135, BRL 37344, RO 16-8714, ICI D7114, CL 316,243; phosphodiesterase inhibitors: L-386,398; lipid-lowering agents: benfluorex: fenfluramine; vanadate and vanadium complexes (e.g., Naglivan™) and peroxovanadium complexes; amylin antagonists; glucagon antagonists; gluconeogenesis inhibitors; somatostatin analogs; antilipolytic agents: nicotinic acid, acipimox, WAG 994, pramlintide (Symlin™), AC 2993, nateglinide, aldose reductase inhibitors (e.g., zopolrestat), glycogen phosphorylase inhibitors, sorbitol dehydrogenase inhibitors, sodium-hydrogen exchanger type 1 (NHE-1) inhibitors and/or cholesterol biosynthesis inhibitors or cholesterol absorption inhibitors, especially a HMG-CoA reductase inhibitor, or a HMG-CoA synthase inhibitor, or a HMG-CoA reductase or synthase gene expression inhibitor, a CETP inhibitor, a bile acid sequesterant, a fibrate, an ACAT inhibitor, a squalene synthetase inhibitor, an anti-oxidant or niacin. Non-limiting examples of HMG-CoA reductase inhibitors include statins in their lactonized or dihydroxy open acid forms and pharmaceutically acceptable salts and esters thereof, including but not limited to lovastatin; simvastatin; dihydroxy open-acid simvastatin, particularly the ammonium or calcium salts thereof; pravastatin, particularly the sodium salt thereof; fluvastatin, particularly the sodium salt thereof; atorvastatin, particularly the calcium salt thereof; cerivastatin, particularly the sodium salt thereof, and nisvastatin.

The compounds of the invention may also be administered in combination with a naturally occurring compound that acts to lower plasma cholesterol levels. Such naturally occurring compounds are commonly called nutraceuticals and include, for example, garlic extract, Hoodia plant extracts, and niacin.

Other examples of combinations of the compounds of the invention include combinations with agents for the treatment of schizophrenia, for example in combination with sedatives, hypnotics, anxiolytics, antipsychotics, antianxiety agents, cyclopyrrolones, imidazopyridines, pyrazolopyrimidines, minor tranquilizers, melatonin agonists and antagonists, melatonergic agents, benzodiazepines, barbiturates, 5HT-2 antagonists, and the like, such as: adinazolam, allobarbital, alonimid, aiprazolam, amisulpride, amitriptyline, amobarbital, amoxapine, aripiprazole, bentazepam, benzoctamine, brotizolam, bupropion, busprione, butabarbital, butalbital, capuride, carbocloral, chloral betaine, chloral hydrate, clomipramine, clonazepam, cloperidone, clorazepate, chlordiazepoxide, clorethate, chlorpromazine, clozapine, cyprazepam, desipramine, dexclamol, diazepam, dichloralphenazone, divalproex, diphenhydramine, doxepin, estazolam, ethchlorvynol, etomidate, fenobam, flunitrazepam, flupentixol, fluphenazine, flurazepam, fluvoxamine, fluoxetine, fosazepam, glutethimide, halazepam, haloperidol, hydroxyzine, imipramine, lithium, lorazepam, lormetazepam, maprotiline, mecloqualone, melatonin, mephobarbital, meprobamate, methaqualone, midaflur, midazolam, nefazodone, nisobamate, nitrazepam, nortriptyline, olanzapine, oxazepam, paraldehyde, paroxetine, pentobarbital, perlapine, perphenazine, phenelzine, corticotrophi, prazepam, promethazine, propofol, protriptyline, quazepam, quetiapine, reclazepam, risperidone, roletamide, secobarbital, sertraline, suproelone, temazepam, thioridazine, thiothixene, tracazolate, tranylcypromaine, trazodone, triazolam, trepipam, tricetamide, triclofos, trifluoperazine, trimetozine, trimipramine, uldazepam, venlafaxine, zaleplon, ziprasidone, zolazepam, zolpidem, and salts thereof, and combinations thereof, and the like, or the subject compound may be administered in conjunction with the use of physical methods such as with light therapy or electrical stimulation.

In another embodiment, the subject compound may be used in combination with levodopa (with or without a selective extracerebral decarboxylase inhibitor such as carbidopa or benserazide), anticholinergics such as biperiden (optionally as its hydrochloride or lactate salt) and trihexyphenidyl (benzhexol) hydrochloride, COMT inhibitors such as entacapone, MOA-B inhibitors, antioxidants, A2a adenosine receptor antagonists, cholinergic agonists, NMDA receptor antagonists, serotonin receptor antagonists and dopamine receptor agonists such as alentemol, bromocriptine, fenoldopam, lisuride, naxagolide, pergolide and pramipexole. It will be appreciated that the dopamine agonist may be in the form of a pharmaceutically acceptable salt, for example, alentemol hydrobromide, bromocriptine mesylate, fenoldopam mesylate, naxagolide hydrochloride and pergolide mesylate.

In another embodiment, the compounds of the invention may be used in combination with a compound from the phenothiazine, thioxanthene, heterocyclic dibenzazepine, butyrophenone, diphenylbutylpiperidine and indolone classes of neuroleptic agent. Suitable examples of phenothiazines include chlorpromazine, mesoridazine, thioridazine, acetophenazine, fluphenazine, perphenazine and trifluoperazine. Suitable examples of thioxanthenes include chlorprothixene and thiothixene. An example of a dibenzazepine is clozapine. An example of a butyrophenone is haloperidol. An example of a diphenylbutylpiperidine is pimozide. An example of an indolone is molindolone. Other neuroleptic agents include loxapine, sulpiride and risperidone. It will be appreciated that the neuroleptic agents when used in combination with the subject compound may be in the form of a pharmaceutically acceptable salt, for example, chlorpromazine hydrochloride, mesoridazine besylate, thioridazine hydrochloride, acetophenazine maleate, fluphenazine hydrochloride, flurphenazine enathate, fluphenazine decanoate, trifluoperazine hydrochloride, thiothixene hydrochloride, haloperidol decanoate, loxapine succinate and molindone hydrochloride. Perphenazine, chlorprothixene, clozapine, haloperidol, pimozide and risperidone are commonly used in a non-salt form. Thus, the subject compound may be employed in combination with acetophenazine, alentemol, aripiprazole, amisuipride, benzhexol, bromocriptine, biperiden, chlorpromazine, chlorprothixene, clozapine, diazepam, fenoldopam, fluphenazine, haloperidol, levodopa, levodopa with benserazide, levodopa with carbidopa, lisuride, loxapine, mesoridazine, molindolone, naxagolide, olanzapine, pergolide, perphenazine, pimozide, pramipexole, quetiapine, risperidone, sulpiride, tetrabenazine, frihexyphenidyl, thioridazine, thiothixene, trifluoperazine or ziprasidone.

Other examples of combinations of the compounds of the invention include combinations with agents for the treatment of stroke or stroke recovery. Examples of such second agents for treatment of stroke include, but are not limited to, aspirin, intercellular adhesion molecule (ICAM)-I and LFA-I antagonists including antibodies such as enlimomab (an anti-ICAM- 1 monoclonal antibody), and anti- CD18 and anti-CD 1Ia antibodies, human anti-leukocytic antibodies such as Hu23F2G, glycoprotein lib Ilia antagonists such as eptifibatide (INTEGRELIN™), direct thrombin inhibitors, external or local ultrasound, mechanical clot retrieval or inaceration, fibrinolytic agents, neuronal wound healing agents such as basic fibroblast growth factor (e.g., FIBLAST™), neuroprotective agents such as citicoline, magnesium, nalmefene, dizocilpine, nimodipine, lamotrigine, sipatrigine, lubeluzole, mexiletine, clomethiazole, calcium and sodium channel blocking agents, beta-amino-3-hydroxy-5-methylisoxazole-4- proprionic acid antagonist, a serotonin agonist, a transmembrane potassium channel modulator, agents that inhibit astrocyte activation (e.g., ONO 2506), antioxidants (e.g., MCI- 186), anti-adhesion monoclonal antibodies and antagonists and antibodies inhibiting platelet aggregation such as argatroban and abciximab (REOPRO™), phenytoin, nitrogen oxides, CNS-protective therapies, free-radical scavengers such as tirilazad, reactive oxygen metabolites, and antioxidants, and other thrombolytic agents than tenecteplase, as defined below, such as, for example, acylated plasminogen-streptokinase activator complex (APSAC), single-chain urokinase-plasminogen activator (scu-PA), thrombin-like enzymes from snake venoms such as ancrod, streptokinase (e.g., SAKSTAR™), urokinase, anistreplase, alteplase, saruplase, reteplase, lanoteplase (SUN-9216; Genetics Institute Inc.), plasmin, a truncated form of plasmin (microplasmin; ThromboGenics Ltd), a direct-acting thrombolytic with non-thrombolytic - related neuroprotective activities, recombinant desmodus rotundus salivary plasminogen activator (rDSPA) alpha- 1 (Schering/Teijin Pharmaceuticals), a mutant fibrin-activated human plasminogen (BB 101 53; British Biotech Inc.), staphylokinase, fibrolase, prourokinase (intraarterial administration directly into M1 or M2 arterial thrombus), monteplase (modified rtPA), pamiteplase, tisokinase, and vampire bat plasminogen activator, a spin-trap agent such as NXY-059 (cerovive), clopidogrel, n- methyl-dextro-aspartic acid receptor blocking agent, an anticonvulsive agent, a caspase 3 inhibitor, ((tert butylimino)methyl) 1,3 (benzenedisulfonate disodium n oxide), ebselen, glutathione peroxidase, norphenazone, rovelizumab, lactacystin beta-lactone, tsukubaenolide, 4 phosphonomethylpipecolic acid, eliprodil, antibodies to ganglioside GM1; and thrombolytic agents, including streptokinase, acylated plasminogen-streptokinase activator complex (APSAC), urokinase, single-chain urokinase-plasminogen activator (scu- PA), thrombin-like enzymes from snake venoms such as ancrod (Bell, W. "Defibrinogenating enzymes" In Colman et al (eds), Hemostasis and Thrombosis Lippincott, Philadelphia (1987) p. 886), tPA, and biologically active variants of each of the above.

Other examples of combinations of the compounds of the invention include combinations with agents for the treatment of depression or anxiety, such as norepinephrine reuptake inhibitors, selective serotonin reuptake inhibitors (SSRIs), monoamine oxidase inhibitors (MAOIs), reversible inhibitors of monoamine oxidase (RIMAs), serotonin and noradrenaline reuptake inhibitors (SNRIs), *alpha*-adrenoreceptor antagonists, atypical anti-depressants, benzodiazepines, 5-HT_{1A} agonists or antagonists, especially 5-HT_{1A} partial agonists, 18orticotrophin releasing factor (CRF) antagonists, and pharmaceutically acceptable salts thereof.

Other examples of combinations of the compounds of the invention include combinations with agents for the treatment of diabetes or diabetes conditions, including dipeptidyl peptidase IV (DPP-IV) inhibitors (including isoleucine thiazolidide vildagliptin and saxagliptin); insulin sensitizers, including (i) PPARγ agonists, such as the glitazones (e.g. troglitazone, pioglitazone, englitazone, MCC-555, rosiglitazone, balaglitazone, and the like) and other PPAR ligands, including PPARα./γ dual agonists, such as KRP-297, muraglitazar, naveglitazar, tesaglitazar, TAK-559, PPARα agonists, such as fenofibric acid derivatives (gemfibrozil, clofibrate, fenofibrate and bezafibrate), and selective PPAR *gamma* modulators (SPPARγM's); (ii) biguanides such as metformin and phenformin, and (iii) protein tyrosine phosphatase-1B (PTP-1B) inhibitors; insulin or insulin mimetics; sulfonylureas and other insulin secretagogues, such as tolbutamide, glyburide, glipizide, glimepiride, and meglitinides, such as nateglinide and repaglinide; α-glucosidase inhibitors (such as acarbose and miglitol); glucagon receptor antagonists; GLP-1, GLP-1 analogues or mimetics, and GLP-1 receptor agonists, such as exendin-4 (exenatide), liraglutide; GIP and GIP mimetics and GIP receptor agonists; PACAP, PACAP mimetics, and PACAP receptor agonists; cholesterol lowering agents; PPAR *delta* agonists; antiobesity agents; ileal bile acid transporter inhibitors; agents intended for use in inflammatory conditions such as aspirin, non-steroidal anti-inflammatory drugs (NSAIDs), glucocorticoids, azulfidine, and selective cyclooxygenase-2 (COX-2) inhibitors; antihypertensive agents, such as ACE inhibitors (enalapril, lisinopril, captopril, quinapril, tandolapril), A-II receptor blockers (losartan, candesartan, irbesartan, valsartan, telmisartan, and eprosartan), beta blockers and calcium channel blockers; glucokinase activators (GKAs); inhibitors of 11-β-hydroxysteroid dehydrogenase type 1; inhibitors of cholesteryl ester transfer protein (CETP), such as torcetrapib; and inhibitors of fructose 1,6-bisphosphatase.

The subject or patient to whom the compounds of the invention is administered is generally a human being, male or female, in whom LXRβ agonism is desired, but may also encompass other mammals, such as dogs, cats, mice, rats, cattle, horses, sheep, rabbits, monkeys, chimpanzees or other apes or primates, for which treatment of the above noted disorders is desired.

### DEFINITIONS

The terms used herein have their ordinary meaning and the meaning of such terms is independent at each occurrence thereof. That notwithstanding and except where stated otherwise, the following definitions apply throughout the specification and claims. Chemical names, common names and chemical structures may be used interchangeably to describe that same structure. These definitions apply regardless of whether a term is used by itself or in combination with other terms, unless otherwise indicated. Hence the definition of "alkyl" applies to "alkyl" as well as the "alkyl" protion of "hydroxyalkyl", "haloalkyl", arylalkyl-, alkylaryl-, "alkoxy" etc.

It shall be understood that, in the various embodiments of the invention described herein, any variable not explicitly defined in the context of the embodiment is as defined in Formula (I). All valences not explicitly filled are assumed to be filled by hydrogen.
"Patient" includes both human and non-human animals. Non-human animals include those research animals and companion animals such as mice, primates, monkeys, great apes, canine (*e.g.,* dogs), and feline (*e.g.,* house cats).

"Pharmaceutical composition" (or "pharmaceutically acceptable composition") means a composition suitable for administration to a patient. Such compositions may contain the neat compound (or compounds) of the invention or mixtures thereof, or salts, solvates, prodrugs, isomers, or tautomers thereof, and they may contain one or more pharmaceutically acceptable carriers or diluents. The term "pharmaceutical composition" is also intended to encompass both the bulk composition and individual dosage units comprised of more than one (e.g., two) pharmaceutically active agents such as, for example, a compound of the present invention and an additional agent selected from the lists of the additional agents described herein, along with any pharmaceutically inactive excipients. The bulk composition and each individual dosage unit can contain fixed amounts of the afore-said "more than one pharmaceutically active agents". The bulk composition is material that has not yet been formed into individual dosage units. An illustrative dosage unit is an oral dosage unit such as tablets, pills and the like. Similarly, the herein-described method of treating a patient by administering a pharmaceutical composition of the present invention is also intended to encompass the administration of the afore-said bulk composition and individual dosage units.

"Halogen" (or "halo") means fluorine, chlorine, bromine, or iodine. Preferred are fluorine, chlorine and bromine.

"Alkyl" means an aliphatic hydrocarbon group, which may be straight or branched, comprising 1 to about 10 carbon atoms. "Lower alkyl" means a straight or branched alkyl group comprising 1 to about 4 carbon atoms. Branched means that one or more lower alkyl groups such as methyl, ethyl or propyl, are attached to a linear alkyl chain or to the same methyl group. Non-limiting examples of suitable alkyl groups include methyl, ethyl, n-propyl, isopropyl, n-butyl, i-butyl, and t-butyl.

"Haloalkyl" means an alkyl as defined above wherein one or more hydrogen atoms on the alkyl is replaced by a halo group defined above.

"Alkenyl" means an aliphatic hydrocarbon group containing at least one carbon-carbon double bond and which may be straight or branched and comprising about 2 to about 10 carbon atoms in the straight or branched chain. Branched means that one or more lower alkyl groups such as methyl, ethyl propyl, ethenyl or propenyl are attached to a linear or branched_alkenyl chain. "Lower alkenyl" means about 2 to about 4 carbon atoms in the chain which may be straight or branched. Non-limiting examples of suitable alkenyl groups include ethenyl, propenyl, n-butenyl, 3-methylbut-2-enyl, n-pentenyl, octenyl and decenyl.

"Alkynyl" means an aliphatic hydrocarbon group containing at least one carbon-carbon triple bond and which may be straight or branched and comprising about 2 to about 10 carbon atoms in the chain. Branched means that one or more lower alkyl groups such as methyl, ethyl or propyl, or lower alkenyl or lower alkynyl groups, are attached to a linear alkynyl chain. "Lower alkynyl" means about 2 to about 4 carbon atoms in the chain which may be straight or branched. Non-limiting examples of suitable alkynyl groups include ethynyl, propynyl, 2-butynyl and 3-methylbutynyl.

"Aryl" means an aromatic monocyclic or multicyclic ring system comprising about 6 to about 14 carbon atoms, preferably about 6 to about 10 carbon atoms. The aryl group can be optionally substituted with one or more "ring system substituents" which may be the same or different, and are as defined herein. Non-limiting examples of suitable aryl groups include phenyl and naphthyl.

"Heteroaryl" means an aromatic monocyclic or multicyclic ring system comprising about 5 to about 14 ring atoms, preferably about 5 to about 10 ring atoms, in which one or more of the ring atoms is an element other than carbon, for example nitrogen, oxygen or sulfur, alone or in combination. Preferred heteroaryls contain about 5 to about 6 ring atoms. The "heteroaryl" can be optionally substituted by one or more substituents, which may be the same or different, as defined herein. The prefix aza, oxa or thia before the heteroaryl root name means that at least a nitrogen, oxygen or sulfur atom respectively, is present as a ring atom. A nitrogen atom of a heteroaryl can be optionally oxidized to the corresponding N-oxide. "Heteroaryl" may also include a heteroaryl as defined above fused to an aryl as defined above. Non-limiting examples of suitable heteroaryls include pyridyl, pyrazinyl, furanyl, thienyl (which alternatively may be referred to as thiophenyl), pyrimidinyl, pyridone (including N-substituted pyridones), isoxazolyl, isothiazolyl, oxazolyl, oxadiazolyl, thiazolyl, thiadiazolyl, pyrazolyl, furazanyl, pyrrolyl, pyrazolyl, triazolyl, 1,2,4-thiadiazolyl, pyrazinyl, pyridazinyl, quinoxalinyl, phthalazinyl, oxindolyl, imidazo[1,2-a]pyridinyl, imidazo[2,1-b]thiazolyl, benzofurazanyl, indolyl, azaindolyl, benzimidazolyl, benzothienyl, quinolinyl, imidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl and the like. The term "heteroaryl" also refers to partially saturated heteroaryl moieties such as, for example, tetrahydroisoquinolyl, tetrahydroquinolyl and the like. The term "monocyclic heteroaryl" refers to monocyclic versions of heteroaryl as described above and includes 4- to 7-membered monocyclic heteroaryl groups comprising from 1 to 4 ring heteroatoms, said ring heteroatoms being independently selected from the group consisting of N, O, and S, and oxides thereof. The point of attachment to the parent moiety is to any available ring carbon or ring heteroatom. Non-limiting examples of monocyclic heteroaryl moities include pyridyl, pyrazinyl, furanyl, thienyl, pyrimidinyl, pyridazinyl, pyridoneyl, thiazolyl, isothiazolyl, oxazolyl, oxadiazolyl, isoxazolyl, pyrazolyl, furazanyl, pyrrolyl, pyrazolyl, triazolyl, thiadiazolyl (e.g., 1,2,4-thiadiazolyl), imidazolyl, and triazinyl (e.g., 1,2,4-triazinyl), and oxides thereof.

"Cycloalkyl" means a non-aromatic monocyclic or multicyclic ring system comprising about 3 to about 10 carbon atoms, preferably about 3 to about 6 carbon atoms. The cycloalkyl can be optionally substituted with one or more substituents, which may be the same or different, as described herein. Monocyclic cycloalkyl refers to monocyclic versions of the cycloalkyl moieties described herein. Non-limiting examples of suitable monocyclic cycloalkyls include cyclopropyl, cyclopentyl, cyclohexyl, cycloheptyl and the like. Non-limiting examples of multicyclic cycloalkyls include [1.1.1]-bicyclopentane, 1-decalinyl, norbornyl, adamantyl and the like.

"Heterocycloalkyl" (or "heterocyclyl") means a non-aromatic saturated monocyclic or multicyclic ring system comprising about 3 to about 10 ring atoms, preferably about 5 to about 10 ring atoms, in which one or more of the atoms in the ring system is an element other than carbon, for example nitrogen, oxygen or sulfur, alone or in combination. There are no adjacent oxygen and/or sulfur atoms present in the ring system. Preferred heterocyclyls contain about 5 to about 6 ring atoms. The prefix aza, oxa or thia before the heterocyclyl root name means that at least a nitrogen, oxygen or sulfur atom respectively is present as a ring atom. Any -NH in a heterocyclyl ring may exist protected such as, for example, as an -N(Boc), -N(CBz), -N(Tos) group and the like; such protections are also considered part of this invention. The heterocyclyl can be optionally substituted by one or more substituents, which may be the same or different, as described herein. The nitrogen or sulfur atom of the heterocyclyl can be optionally oxidized to the corresponding N-oxide, S-oxide or S,S-dioxide. Thus, the term "oxide," when it appears in a definition of a variable in a general structure described herein, refers to the corresponding N-oxide, S-oxide, or S,S-dioxide. "Heterocyclyl" also includes rings wherein =O replaces two available hydrogens on the same carbon atom (i.e., heterocyclyl includes rings having a carbonyl group in the ring). Such =O groups may be referred to herein as "oxo." An example of such a moiety is pyrrolidinone (or pyrrolidone): 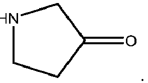 As used herein, the term "monocyclic heterocycloalkyl" refers monocyclic versions of the heterocycloalkyl moities decribed herein and include a 4- to 7-membered monocyclic heterocycloalkyl groups comprising from 1 to 4 ring heteroatoms, said ring heteroatoms being independently selected from the group consisting of N, N-oxide, O, S, S-oxide, S(O), and S(O)₂ The point of attachment to the parent moiety is to any available ring carbon or ring heteroatom. Non-limiting examples of monocyclic heterocycloalkyl groups include piperidyl, oxetanyl, pyrrolyl, piperazinyl, morpholinyl, thiomorpholinyl, thiazolidinyl, 1,4-dioxanyl, tetrahydrofuranyl, tetrahydrothiophenyl, *beta* lactam, *gamma* lactam, *delta* lactam, *beta* lactone, *gamma* lactone, *delta lactone,* and pyrrolidinone, and oxides thereof. Non-limiting examples of lower alkyl-substituted oxetanyl include the moiety:

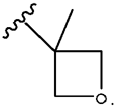

It should be noted that in hetero-atom containing ring systems of this invention, there are no hydroxyl groups on carbon atoms adjacent to a N, O or S, as well as there are no N or S groups on carbon adjacent to another heteroatom. 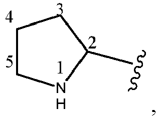 there is no -OH attached directly to carbons marked 2 and 5.

"Alkoxy" means an -O-alkyl group in which the alkyl group is as previously described. Non-limiting examples of alkoxy groups include methoxy, ethoxy, *n*-propoxy, isopropoxy and *n-*butoxy. The bond to the parent moiety is through the oxygen.

Any of the foregoing functional groups may be unsubstituted or substituted as described herein. The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

The term "optionally substituted" means optional substitution with the specified groups, radicals or moieties.

Substitution on a cycloalkylalkyl, heterocycloalkylalkyl, arylalkyl, heteroarylalkyl, arylfused cycloalkylalkyl- moiety or the like includes substitution on any ring portion and/or on the alkyl portion of the group.

When a variable appears more than once in a group, e.g., R⁶ in -N(R⁶)₂, or a variable appears more than once in a structure presented herein, the variables can be the same or different.

The line -, as a bond generally indicates a mixture of, or either of, the possible isomers, e.g., containing (R)- and (S)- stereochemistry. For example: 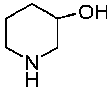 means containing both

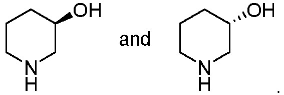

The wavy line 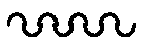 , as used herein, indicates a point of attachment to the rest of the compound. Lines drawn into the ring systems, such as, for example: 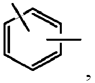 indicate that the indicated line (bond) may be attached to any of the substitutable ring carbon atoms.

"Oxo" is defined as a oxygen atom that is double bonded to a ring carbon in a cycloalkyl, cycloalkenyl, heterocyclyl, heterocyclenyl, or other ring described herein, e.g.,

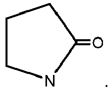

In this specification, where there are multiple oxygen and/or sulfur atoms in a ring system, there cannot be any adjacent oxygen and/or sulfur present in said ring system.

As well known in the art, a bond drawn from a particular atom wherein no moiety is depicted at the terminal end of the bond indicates a methyl group bound through that bond to the atom, unless stated otherwise. For example: 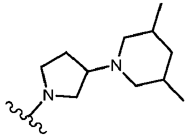 represents

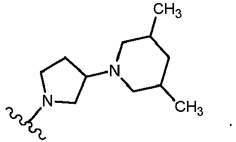

In another embodiment, the compounds of the invention, and/or compositions comprising them, are present in isolated and/or purified form. The term "purified", "in purified form" or "in isolated and purified form" for a compound refers to the physical state of said compound after being isolated from a synthetic process (e.g. from a reaction mixture), or natural source or combination thereof. Thus, the term "purified", "in purified form" or "in isolated and purified form" for a compound refers to the physical state of said compound (or a tautomer thereof, or pharmaceutically acceptable salt of said compound or said tautomer) after being obtained from a purification process or processes described herein or well known to the skilled artisan (e.g., chromatography, recrystallization and the like), in sufficient purity to be suitable for in vivo or medicinal use and/or characterizable by standard analytical techniques described herein or well known to the skilled artisan.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in organic Synthesis (1991), Wiley, New York.

Those skilled in the art will recognize those instances in which the compounds of the invention may be converted to prodrugs and/or solvates, another embodiment of the present description. A discussion of prodrugs is provided in T. Higuchi and V. Stella, Pro-drugs as Novel Delivery Systems (1987) 14 of the A.C.S. Symposium Series, and in Bioreversible Carriers in Drug Design, (1987) Edward B. Roche, ed., American Pharmaceutical Association and Pergamon Press. The term "prodrug" means a compound (e.g, a drug precursor) that is transformed *in vivo* to yield a compound of the invention or a pharmaceutically acceptable salt, hydrate or solvate of the compound. The transformation may occur by various mechanisms (e.g., by metabolic or chemical processes), such as, for example, through hydrolysis in blood. A discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

One or more compounds of the invention may exist in unsolvated as well as solvated forms with pharmaceutically acceptable solvents such as water, ethanol, and the like, and it is intended that the description embrace both solvated and unsolvated forms where they exist. "Solvate" means a physical association of a compound of the invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is H₂O.

"Effective amount" or "therapeutically effective amount" is meant to describe an amount of compound or a composition of the present invention effective in inhibiting the above-noted diseases and thus producing the desired therapeutic, ameliorative, inhibitory or preventative effect.

Those skilled in the art will recognize those instances in which the compounds of the invention may form salts. In such instances, another embodiment provides pharmaceutically acceptable salts of the compounds of the invention. Thus, reference to a compound of the invention herein is understood to include reference to salts thereof, unless otherwise indicated. The term "salt(s)", as employed herein, denotes any of the following: acidic salts formed with inorganic and/or organic acids, as well as basic salts formed with inorganic and/or organic bases. In addition, when a compound of the invention contains both a basic moiety, such as, but not limited to a pyridine or imidazole, and an acidic moiety, such as, but not limited to a carboxylic acid, zwitterions ("inner salts") may be formed and are included within the term "salt(s)" as used herein. Pharmaceutically acceptable (i.e., non-toxic, physiologically acceptable) salts are preferred, although other salts are also potentially useful. Salts of the compounds of the invention may be formed by methods known to those of ordinary skill in the art, for example, by reacting a compound of the invention with an amount of acid or base, such as an equivalent amount, in a medium such as one in which the salt precipitates or in an aqueous medium followed by lyophilization.

Exemplary acid addition salts which may be useful include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates,) and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website). These disclosures are incorporated herein by reference thereto.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, lysine and the like. Basic nitrogen-containing groups may be quarternized with agents such as lower alkyl halides (e.g. methyl, ethyl, and butyl chlorides, bromides and iodides), dialkyl sulfates (e.g. dimethyl, diethyl, and dibutyl sulfates), long chain halides (e.g. decyl, lauryl, and stearyl chlorides, bromides and iodides), aralkyl halides (e.g. benzyl and phenethyl bromides), and others.

All such acid salts and base salts are intended to be pharmaceutically acceptable salts within the scope of the invention and all acid and base salts are considered as potentially useful alternatives to the free forms of the corresponding compounds for purposes of the invention.

Another embodiment of the description which may be useful includes pharmaceutically acceptable esters of the compounds of the invention. Such esters do not form part of the invention; they may include the following groups: (1) carboxylic acid esters obtained by esterification of the hydroxy groups, in which the non-carbonyl moiety of the carboxylic acid portion of the ester grouping is selected from straight or branched chain alkyl (for example, acetyl, n-propyl, t-butyl, or n-butyl), alkoxyalkyl (for example, methoxymethyl), aralkyl (for example, benzyl), aryloxyalkyl (for example, phenoxymethyl), aryl (for example, phenyl optionally substituted with, for example, halogen, C₁₋₄alkyl, or C₁₋₄alkoxy or amino); (2) sulfonate esters, such as alkyl- or aralkylsulfonyl (for example, methanesulfonyl); (3) amino acid esters (for example, L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di (C₆₋₂₄)acyl glycerol.

As mentioned herein, under certain conditions the compounds of the invention may form tautomers. Such tautomers, when present, comprise another embodiment of the invention. It shall be understood that all tautomeric forms of such compounds are within the scope of the compounds of the invention. For example, all keto-enol and imine-enamine forms of the compounds, when present, are included in the invention.

The compounds of the invention may contain asymmetric or chiral centers, and, therefore, exist in different stereoisomeric forms. It is intended that all stereoisomeric forms of the compounds of the invention as well as mixtures thereof, including racemic mixtures, form part of the present invention. In addition, the present invention embraces all geometric and positional isomers. For example, if a compound of the invention incorporates a double bond or a fused ring, both the cis- and trans-forms, as well as mixtures, are embraced within the scope of the invention.

Where various stereoisomers of the compounds of the invention are possible, another embodiment provides for diastereomeric mixtures and individual enantiomers of the compounds of the invention. Diastereomeric mixtures can be separated into their individual diastereomers on the basis of their physical chemical differences by methods well known to those skilled in the art, such as, for example, by chromatography and/or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Also, some of the compounds of the invention may be atropisomers (e.g., substituted biaryls) and are considered as part of this invention. Enantiomers can also be separated by use of chiral HPLC column.

All stereoisomers (for example, geometric isomers, optical isomers and the like) of the compounds of the invention (including those of the salts, solvates, esters and prodrugs of the compounds as well as the salts, solvates and esters of the prodrugs), such as those which may exist due to asymmetric carbons on various substituents, including enantiomeric forms (which may exist even in the absence of asymmetric carbons), rotameric forms, atropisomers, and diastereomeric forms, are contemplated as embodiments within the scope of this invention, as are positional isomers (such as, for example, 4-pyridyl and 3-pyridyl). (For example, if a compound of the invention incorporates a double bond or a fused ring, both the cis- and trans-forms, as well as mixtures, are embraced within the scope of the invention. Also, for example, all keto-enol and imine-enamine forms of the compounds are included in the invention.).

Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate", "ester", "prodrug" and the like, is intended to equally apply to the salt, solvate, ester and prodrug of enantiomers, stereoisomers, rotamers, tautomers, positional isomers, racemates or prodrugs of the compounds of this description.

Another embodiment which may be useful include isotopically-labelled compounds of the invention. Such compounds are identical to those recited herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorus, fluorine and chlorine, such as ²H, ³H, ¹¹C, ¹³C, ¹⁴C, ¹⁵N, ¹⁸ O, ¹⁷O, ³¹P, ³²P, ³⁵S, ¹⁸F, and ³⁶Cl, respectively.

In the compounds of the invention, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of the invention. For example, different isotopic forms of hydrogen (H) include protium (¹H) and deuterium (²H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds of the invention can be prepared without undue experimentation by conventional techniques well known to those skilled in the art or by processes analogous to those described in the schemes and examples herein using appropriate isotopically-enriched reagents and/or intermediates.

Polymorphic forms of the compounds of the invention, and of the pharmaceutically acceptable salts of the compounds of the invention, are intended to be included in the present invention.

Another embodiment provides suitable dosages and dosage forms of the compounds of the invention. Suitable doses for administering compounds of the invention to patients may readily be determined by those skilled in the art, e.g., by an attending physician, pharmacist, or other skilled worker, and may vary according to patient health, age, weight, frequency of administration, use with other active ingredients, and/or indication for which the compounds are administered. Doses may range from about 0.001 to 500 mg/kg of body weight/day of the compound of the invention. In one embodiment, the dosage is from about 0.01 to about 25 mg/kg of body weight/day of a compound of the invention, or a pharmaceutically acceptable salt or solvate of said compound. In another embodiment, the quantity of active compound in a unit dose of preparation may be varied or adjusted from about 1 mg to about 100 mg, preferably from about 1 mg to about 50 mg, more preferably from about 1 mg to about 25 mg, according to the particular application. In another embodiment, a typical recommended daily dosage regimen for oral administration can range from about 1 mg/day to about 500 mg/day, preferably 1 mg/day to 200 mg/day, in two to four divided doses.

When used in combination with one or more additional therapeutic agents, the compounds of this invention may be administered together or sequentially. When administered sequentially, compounds of the invention may be administered before or after the one or more additional therapeutic agents, as determined by those skilled in the art or patient preference.

If formulated as a fixed dose, such combination products employ the compounds of this invention within the dosage range described herein and the other pharmaceutically active agent or treatment within its dosage range.

Accordingly, another embodiment provides combinations comprising an amount of at least one compound of the invention, or a pharmaceutically acceptable salt, solvate, ester or prodrug thereof, and an effective amount of one or more additional agents described above.

Another embodiment provides for pharmaceutically acceptable compositions comprising a compound of the invention, either as the neat chemical or optionally further comprising additional ingredients. For preparing pharmaceutical compositions from the compounds of the invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g., magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pennsylvania.

Liquid form preparations include solutions, suspensions and emulsions. Non-limiting examples which may be useful include water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations that are intended to be converted, shortly before use, to liquid form preparations for either oral or parenteral administration. Such liquid forms include solutions, suspensions and emulsions.

Another embodiment which may be useful includes compositions comprising a compound of the invention formulated for transdermal delivery. The transdermal compositions can take the form of creams, lotions, aerosols and/or emulsions and can be included in a transdermal patch of the matrix or reservoir type as are conventional in the art for this purpose.

Other embodiment which may be useful includes compositions comprising a compound of the invention formulated for subcutaneous delivery or for oral delivery. In some embodiments, it may be advantageous for the pharmaceutical preparation comprising one or more compounds of the invention be prepared in a unit dosage form. In such forms, the preparation may be subdivided into suitably sized unit doses containing appropriate quantities of the active component, e.g., an effective amount to achieve the desired purpose. Each of the foregoing alternatives, together with their corresponding methods of use, are considered as included in the various embodiments of the invention.

### GENERAL SCHEMES

Several methods for preparing the compounds of this invention are illustrated in the Schemes and Examples herein. Starting materials are made according to procedures known in the art or as illustrated herein. The following examples are provided so that the invention might be more fully understood. These examples are illustrative only and should not be construed as limiting the invention in any way.

### GENERAL SCHEMES

Compounds listed in this invention can be prepared according to the general Schemes A to E and the detailed experimental below. In general, most of the racemic products can be resolved under typical chiral resolution protocol using either normal phase or SFC mediated methods. And in most cases, the chirality (R) or (S) designation is not confirmed as drawn in Tables A to E.

General Scheme A outlines the methods for preparing compounds which have the 4-([4,4'-bipiperidin]-1-y1)-N,N-dimethylbenzamide cores (1-4) with the appropriately functionalized piperidines such as amides, sulfonamides, amines, ureas and carbamates.

In this method, N-Boc protected 4,4'-bipiperidine (1-2) undergoes palladium catalyzed cross-coupling with suitably substituted 4-bromo-N,N-dimethylbenzamide (4-2) to give substituted N-Boc-4,4'-bipiperidine (1-3). Removal of t-Boc protection from 1-3 gives substituted 4,4'-bipiperidine (1-4). Intermediate 1-4 can undergo acylation, sulfonylation, or alkylation to produce the desired products (1-5, 1-7, 1-8, 1-9 and 1-11). The alpha-keto amide 1-5 can react with TMS-CF3 etc. to give product with alpha-hydroxy Mosher amide (1-6). Intermediate 1-5 can also react with Grignard reagent to generate compounds 1-12.

In general, most of the racemic products can be resolved under typical chiral resolution protocol using either normal phase or SFC mediated methods.

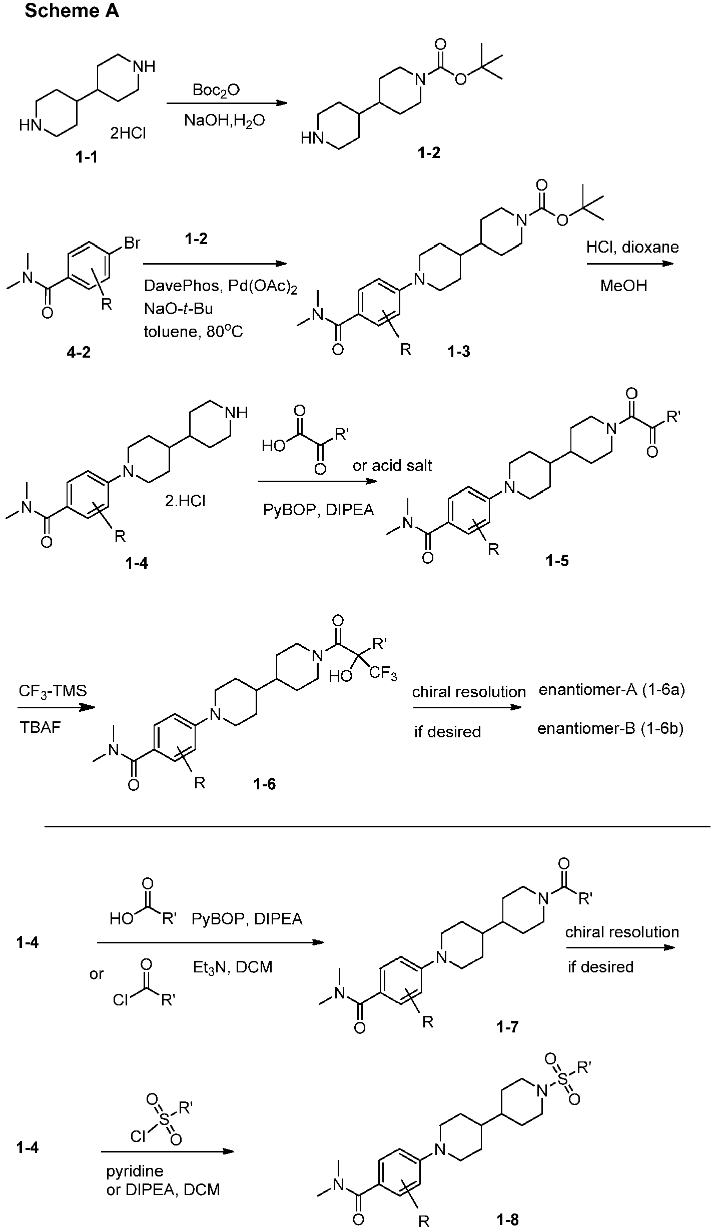

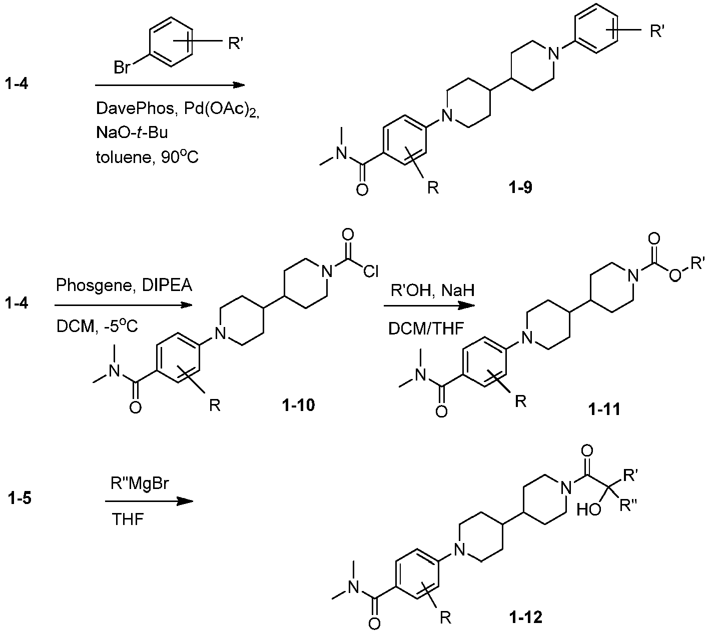

General Scheme B describes the methods for preparing compounds which have the 4-([4,4'-bipiperidin]-1-y1)-N,N-dimethylnicotinamide cores (2-5) with the appropriately functionalized piperidines such as amides, sulfonamides, amines, and carbamates similar to the examples outlined in Scheme A.

In this method, 2-chloro-6-hydroxynicotinic acid (2-1) undergoes amide coupling to give 2-chloro-6-hydroxy-N,N-dimethylnicotinamide (2-2). Nicotinamide 2-2 is deprotonated using NaH and reacts with 1,1,1-trifluoro-N-phenyl-N-((trifluoromethyl)sulfonyl)-methanesulfonamide to generate 6-chloro-5-(dimethylcarbamoyl)pyridin-2-yl trifluoromethanesulfonate (2-3).

Intermediate N-Boc protected 4,4'-bipiperidine (1-2) can undergo SnAr reaction with triflate 2-3 or 2,6-dichloro-N,N-dimethylnicotinamide to produce substituted N-Boc-4,4'-bipiperidine (2-4). Removal of N-Boc protection from 2-4 gives substituted 4,4'-bipiperidine (2-5). Intermediate 2-5 can undergo acylation (2-6 & 2-9), sulfonylation, or alkylation to produce the desired products. A few specific examples are depicted in this scheme. The alpha-keto amide 2-6 can react with Grignard reagent to generate compound 2-7. Intermediate 2-6 can also react with TMS-CF3 etc. to give product with alpha-hydroxy Mosher amide (2-8).

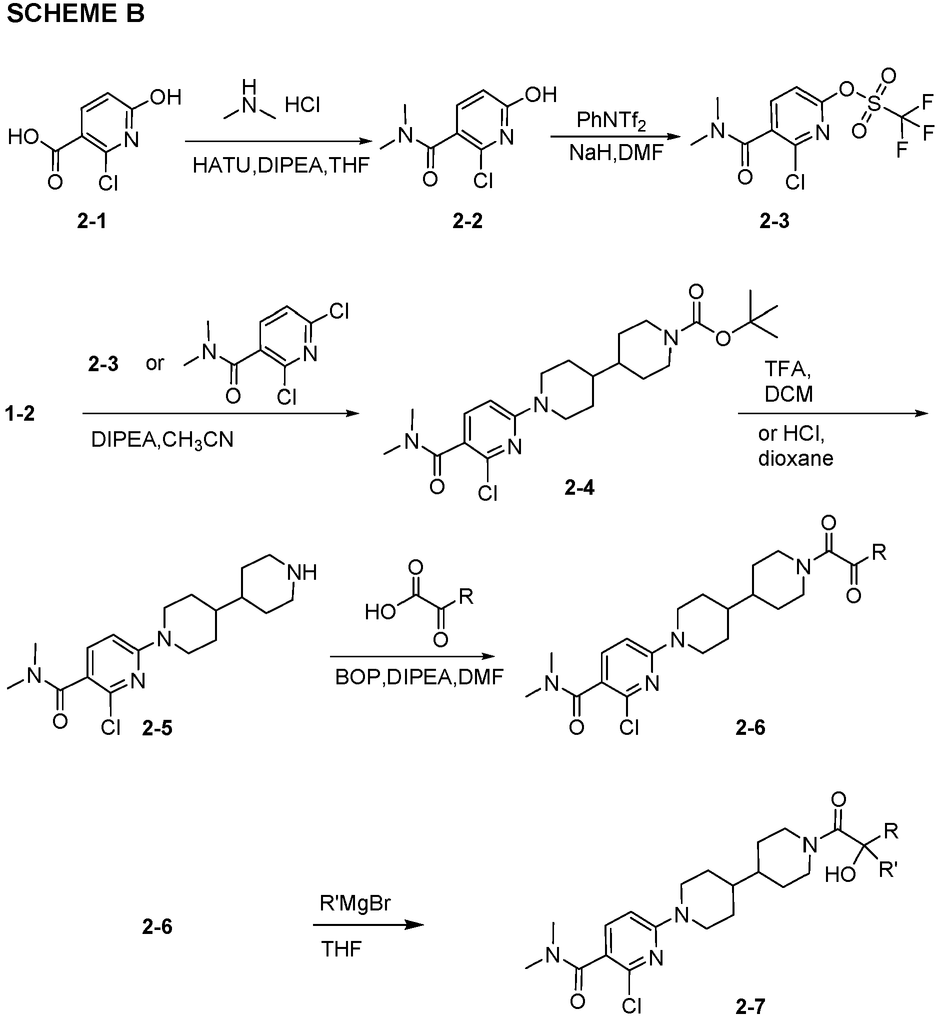

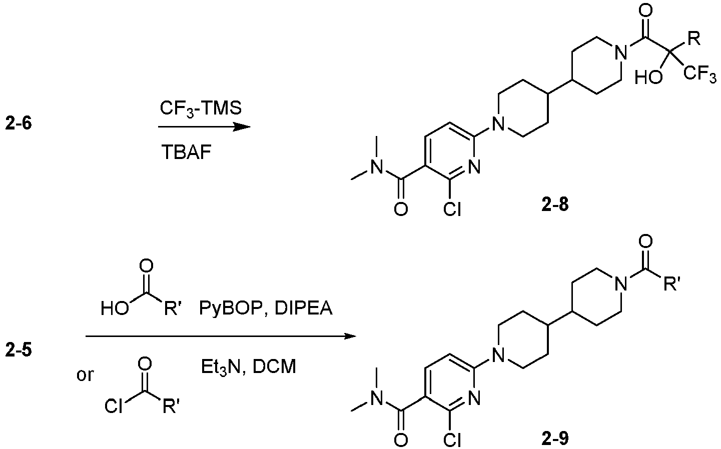

General Scheme C illustrates the methods for preparing compounds which have the 6-([4,4'-bipiperidin]-1-yl)-N,N,2-trimethylnicotinamide cores (3-4) with the appropriately functionalized piperidines such as amides, sulfonamides, amines, ureas and carbamates similar to the examples outlined in Scheme A.

In this method, 6-chloro-2-methylnicotinic acid (3-1) undergoes amide coupling to give 6-chloro-N,N,2-trimethylnicotinamide (3-2). Nicotinamide 3-2 can undergo SnAr reaction with N-Boc protected 4,4'-bipiperidine (1-2) to produce substituted N-Boc-4,4'-bipiperidine (3-3). Removal of t-Boc protection from 3-3 gives the corresponding substituted 4,4'-bipiperidine (3-4). Intermediate 3-4 can then undergo acylation (3-5 & 3-7), sulfonylation, or alkylation to produce the desired products. A few specific examples are described in this scheme. Furthermore, the alpha-keto amide 3-5 can react with TMS-CF3 to give product with alpha-hydroxy Mosher amide (3-6).

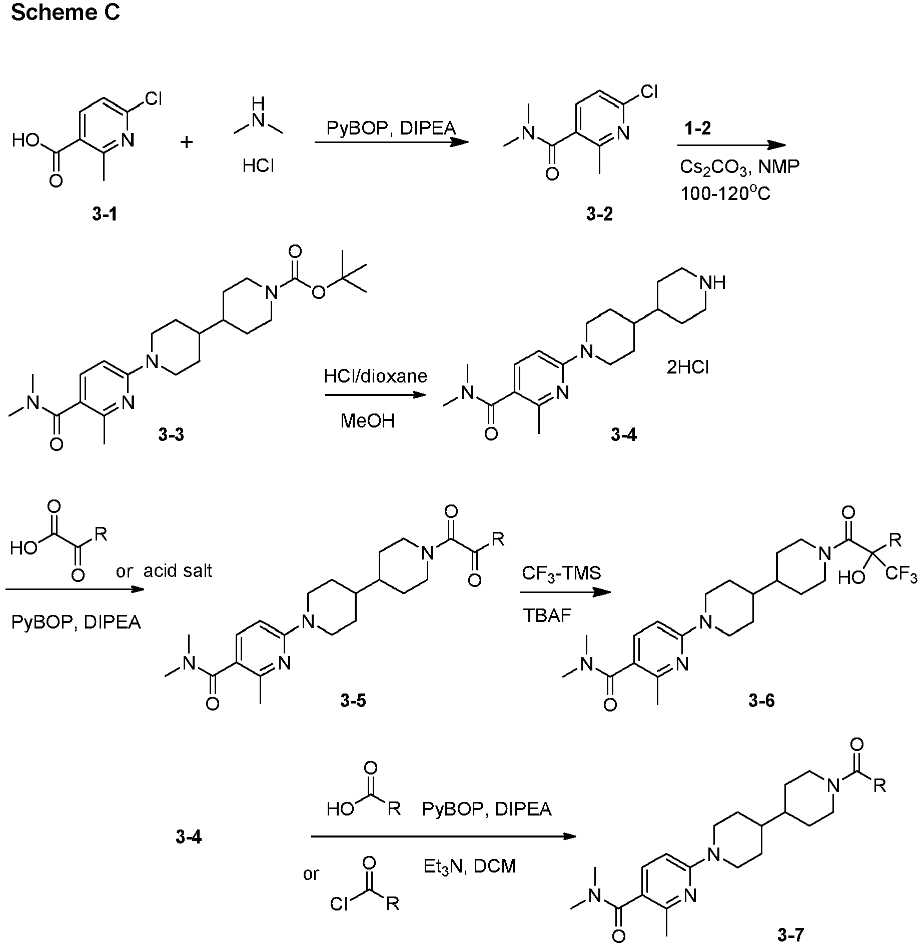

General Scheme D outlines the alternative preparative methods in contrast to Scheme A. Both Scheme A and D can lead to similar compounds by employing different sequence in carrying out the same set of reactions.

In this method the substituted 4-bromo-N,N-dimethylbenzamide (4-2) is either commercially available or synthesized from the corresponding benzoic acid (4-1) by amide coupling. The N-Boc protected 4,4'-bipiperidine (1-2) is acylated first to give the corresponding amide 4-3 by amide coupling. Removal of N-Boc protection from amide 4-3 gives the acylated 4,4'-bipiperidine (4-4). Intermediate 4-4 can undergo palladium catalyzed cross-coupling with suitably substituted 4-bromo-N,N-dimethylbenzamide (4-2) to give final product 4-5.

In addition, intermediate 4-4 can also undergo palladium catalyzed cross-coupling with suitably substituted aryl bromides or iodides to give final product 4-6.

An alternate route to product 4-6 is also listed. The N-Boc protected 4,4'-bipiperidine (1-2) can first undergo palladium catalyzed cross-coupling with suitably substituted 4-bromo-N,N-dimethylbenzamide (4-2) or aryl bromides or iodides to give functionalized N-Boc protected 4,4'-bipiperidine (4-7). Removal of N-Boc protection and followed by acylation provides final product 4-6.

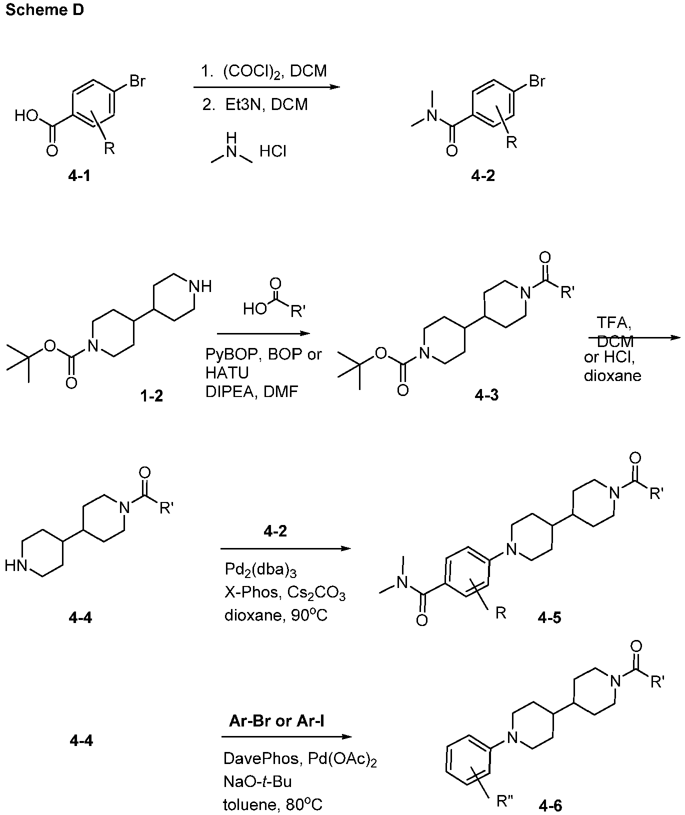

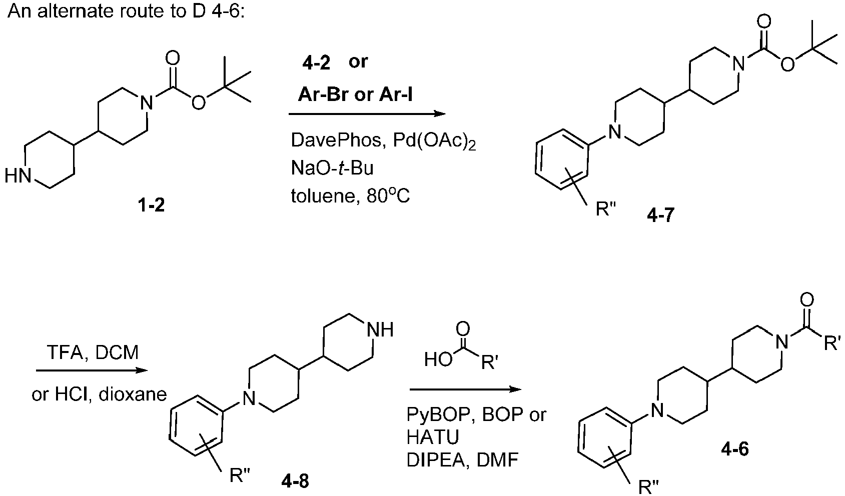

General Scheme E describes the functionalization of the benzamide or the nicotinamide portion of the molecule. Either the commercially available methyl 2-chloro-4-fluorobenzoate (5-1a) or methyl 2,6-dichloronicotinate (5-1b) can undergo SnAr reaction with intermediate N-Boc protected 4,4'-bipiperidine (1-2) to give functionalized N-Boc 4,4'-bipiperidine (5-2). Similarly, methyl 4-bromo-2-chlorobenzoate (5-1c) can also be used to generate intermediate 5-2 via palladium catalyzed cross-coupling. Removal of N-Boc protection from intermediate 5-2 and followed by amide coupling gives the acylated 4,4'-bipiperidine benzoate or nicotinate (5-4). Saponification of the methyl benzoate or nicotinate (5-4) yields the corresponding benzoic or nicotinic acid (5-5). Lastly, acid 5-5 undergoes amide coupling with ammonia, 1^{st} amine or 2^{nd} amine to give the final product 5-6.

An alternate route to product 5-6 is also listed by employing different sequence in carrying out the same set of reactions. The functionalized N-Boc 4,4'-bipiperidine (5-2) is first saponified to the corresponding benzoic or nicotinic acid (5-7). Then acid 5-7 undergoes amide coupling with ammonia, 1^{st} amine or 2^{nd} amine to give intermediate 5-8. Removal of N-Boc protection from intermediate 5-8 and followed by acylation yields final product 5-6.

In addition, the functionalized N-Boc 4,4'-bipiperidine (5-2) can react with methyl Grignard to give compound 5-10.

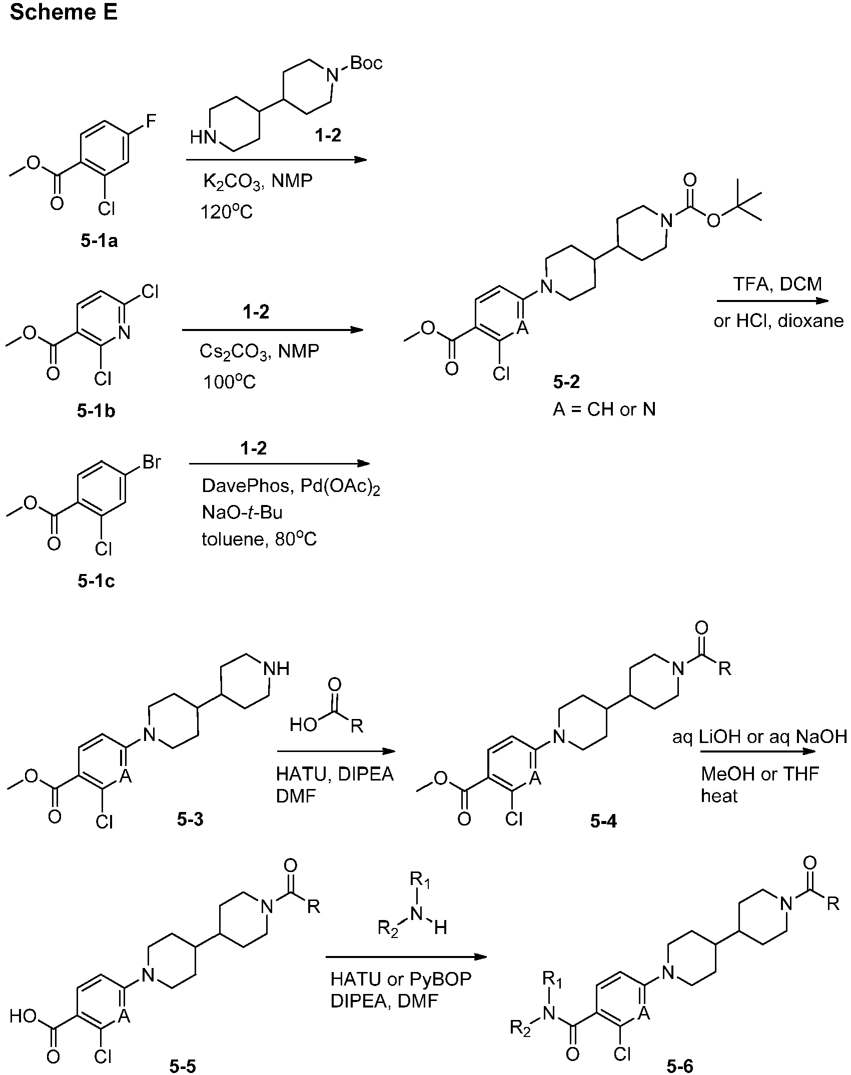

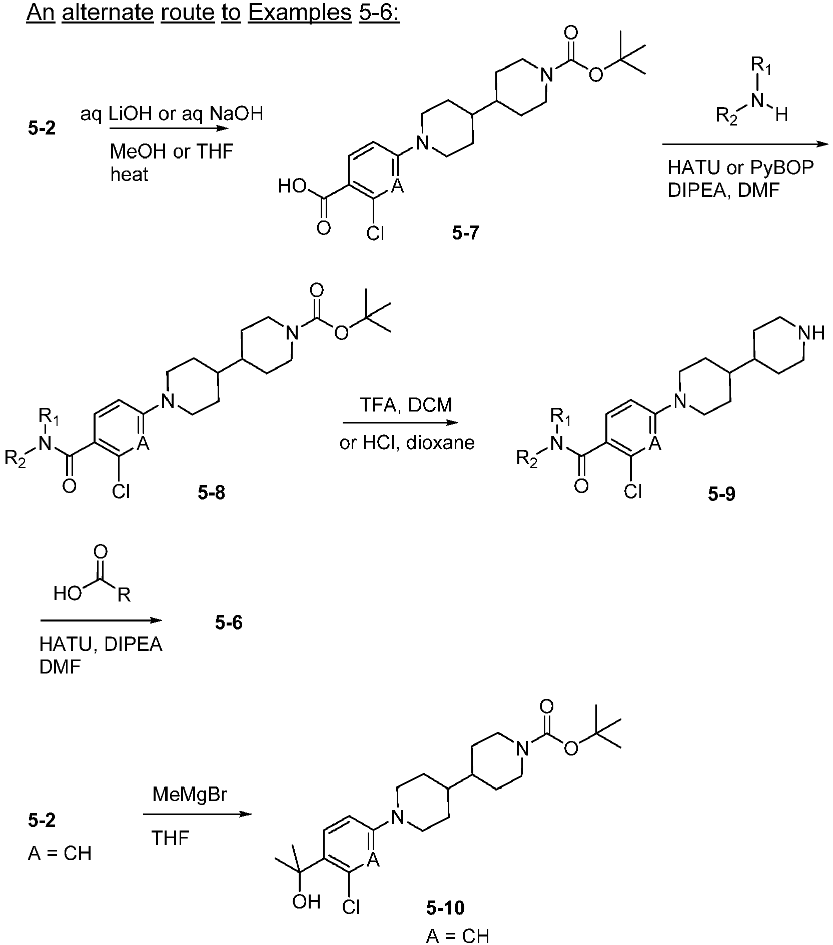

### Abbreviations

- BOP: (benzotriazol-1 -yloxy)tris(dimethylamino)phosphonium hexafluorophosphate
- Br₂BH-SMe₂: dibromoborane-methylsulfide complex
- CDI: N,N'-carbonyldiimidazole
- CS₂CO₃: cesium carbonate
- DCC: *N,N'*-dicyclohexylcarbodiimide
- DCE: dichloroethane
- DCM: dichloromethane
- DIAD: diisopropyl azodicarboxylate
- DIBAL-H: diisobutylaluminum hydride
- DIEA: diisopropylethylamine
- DMF: dimethylformamide
- DMSO: dimethyl sulfoxide
- Dppf: diphenylphosphinoferrocene
- EDC: *N*-(3-dimethylaminopropyl)-*N*'-ethylcarbodiimide
- Et₂O: diethyl ether
- Et₃N: triethylamine
- EtOAc: ethyl acetate
- EtOH: ethanol
- HATU: *O*-(7-azabenzotriazol-1-yl)-*N,N,N',N'*-tetramethyluronium hexafluorophosphate
- HBTU: N,N,N',N'-Tetramethyl-O-(1H-benzotriazol-1-yl)uronium hexafluorophosphate, O-(Benzotriazol- 1 -yl)-N,N,N',N'-tetramethyluronium hexafluorophosphate
- HCl: hydrochloric acid
- (HF)₃-Et₃N: triethylamine trihydrofluoride
- IPA: isopropanol
- K₂CO₃: potassium carbonate
- KHSO₄: potassium bisulfate
- KOAc: potassium acetate
- LiOH: lithium hydroxide
- MeCN: acetonitrile
- MeOH: methanol
- MgSO₄: magnesium sulfate
- MIDA: N-methyliminodiacetic acid
- Na₂SO₄: sodium sulfate
- NaHCO₃: sodium bicarbonate
- NaH: sodium hydride
- NaOH: sodium hydroxide
- n-BuLi: n-butyl lithium
- NIS: N-iodosuccinimide
- Pd(Ph₃P)₄: tetrakis(triphenylphosphine) palladium (0)
- Pd/C: palladium on carbon
- PdCl₂(dppf)-CH₂Cl₂: adduct dichloro[1, 1'-bis(diphenylphosphino)ferrocene] palladium (II) dichloromethane adduct
- PdCl₂(PPh₃)₂: palladium dichloride bis-triphenylphosphine
- PE: Petroleum ether
- POCl₃: phosphorous oxychloride
- PPh₃: triphenylphosphine
- RT, r.t., rt: room temperature
- TBTU: *O*-benzotriazol-1-yl*-N,N,N',N'*-tetramethyluronium tetrafluoroborate
- Tf₂O: triflic anhydride
- TFA: trifluoroacetatic acid
- TfOH: trifluoromethanesulfonic acid
- THF: tetrahydofuran
- Xphos: 2-Dicyclohexylphosphino-2',4',6'-triisopropylbiphenyl

The following examples were prepared according to Scheme A: Examples wherein Q corresponds with -C(O)O- are not comprised in the appendant claims. They are reference examples and do not form part of the invention.

### Examples A-1, A-4, A-6 & A-7

### Preparation of 2-Chloro-4-(1'-(2-hydroxy-3,3-dimethyl-2-(trifluoromethyl)butanoyl)-[4,4'-bipiperidin]-1-yl)-N,N dimethylbenzamide (1-6) Example A-4

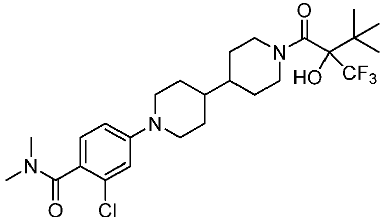

### Step 1: tert-butyl 4,4'-bipiperidine-1-carboxylate (1-2)

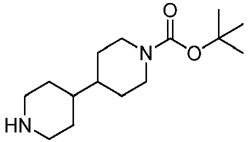

To a solution of 4,4'-bipiperidine dihydrochloride (**1-1**) (10 g, 41.5 mmol, 1.0 eq) in water (20 mL) was added ethanol (400 ml) and a solution of 5N sodium hydroxide to adjust pH to 8∼9, followed by addition of di-tert-butyl dicarbonate (9.06 g, 42.3 mmol, 1.0 eq) in ethanol (20 mL). The mixture was stirred at room temperature overnight and the solvent was removed in vacuo. The white solid (di-substituted product) was filtered off and washed with water (20mLx3). The filtrate was basified (pH=10) and extracted with ethyl acetate (200 mL x3). The combined organic phase was dried over sodium sulfate and concentrated in vacuo to give tert-butyl 4,4'-bipiperidine-1-carboxylate (**1-2**). LRMS *m*/*z* (M+H) 269.2 found, 269.2 required.

### Step 2: tert-Butyl 1'-(3-chloro-4-(dimethylcarbamoyl)phenyl)-[4,4'-bipiperidine]-1-carboxylate (1-3) Example A-1

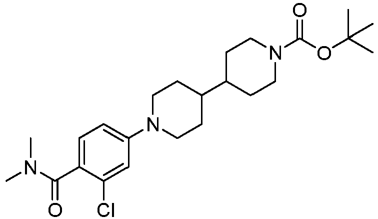

To a mixture of *tert*-butyl [4,4'-bipiperidine]-1-carboxylate (**1-2**) (6 g, 22.4 mmol), DavePhos (2.64 g, 6.71 mmol), Pd(OAc)₂ (502 mg, 2.24 mmol) and sodium-*t*-butoxide (2.58 g, 26.8 mmol), under nitrogen, is added toluene (70 mL) followed by a solution of 4-bromo-2-chloro-*N,N*-dimethylbenzamide (5.87 g, 22.4 mmol) in toluene (30 mL). Heat the reaction mixture at 80°C for 5 h. Cool to rt and partition between water and EtOAc. Layers are separated. Extract aqueous layer with EtOAc (3x). Dry combined organics over Na₂SO₄ and concentrate. Purify by silica gel flash chromatography using a linear gradient of 5 to 100% EtOAc in hexanes to give *tert*-butyl 1'-(3-chloro-4-(dimethylcarbamoyl)phenyl)-[4,4'-bipiperidine]-1-carboxylate (**1-3**). MS [M+H]⁺450.5 found, 450.3 required.

### Step 3: 1-(3-Chloro-4-(dimethylcarbamoyl)phenyl)-[4,4'-bipiperidine]-1,1'-diium chloride (1-4)

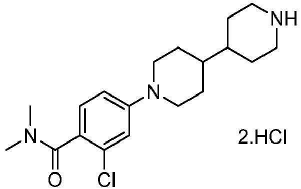

To a solution of *tert*-butyl 1'-(3-chloro-4-(dimethylcarbamoyl)phenyl)-[4,4'-bipiperidine]-1-carboxylate (**1-3**) (6.1 g, 13.56 mmol) in MeOH (50 mL) is added a solution of HCI in dioxane (4 N, 30 mL) and stir at rt for 3 h. Concentrate to give 1-(3-chloro-4-(dimethylcarbamoyl) phenyl)-[4,4'-bipiperidine]-1,1'-diium chloride (**1-4**). MS [M+H]⁺350.5 found, 350.2 required.

### Step 4: 2-Chloro-4-(1'-(3,3-dimethyl-2-oxobutanoyl)-[4,4'-bipiperidin]-1-yl)-N,N-dimethyl benzamide (1-5)

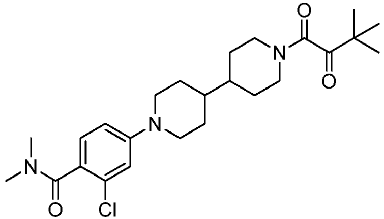

To a solution of 1-(3-chloro-4-(dimethylcarbamoyl)phenyl)-[4,4'-bipiperidine]-1,1'-diium chloride (**1-4**) (40 mg, 0.095 mmol) and PyBOP (59.1 mg, 0.114 mmol) in DMF (0.5 mL) are added trimethylpyruvic acid (24.6 mg, 0.189 mmol) and DIEA (98 mg, 0.76 mmol). Stir the reaction mixture at rt for 10 min and then separate the desired product by reverse phase HPLC (Sunfire C18 30 x 150 mm column) using 20 to 95% MeCN in water (0.1% TFA modifier for the solvents) over 15 min at 35 mL flow rate. Desired fractions are combined and made basic by adding 1 N aqueous NaOH. Extract this basic aqueous solution with EtOAc (3x). Dry combined organics over Na₂SO₄ and concentrate to give 2-chloro-4-(1'-(3,3-dimethyl-2-oxobutanoyl)-[4,4'-bipiperidin]-1-y1)-*N,N*-dimethylbenzamide (**1-5**). LRMS *m*/*z* (M+H) 462.6 found, 462.3 required.

### Step 5: 2-Chloro-4-(1'-(2-hydroxy-3,3-dimethyl-2-(trifluoromethyl)butanoyl)-[4,4'-bipipendin]-1-yl)-N,N-dimethylbenzamide (1-6, 1-6a & 1-6b) (Examples A-4, A-6 & A-7)

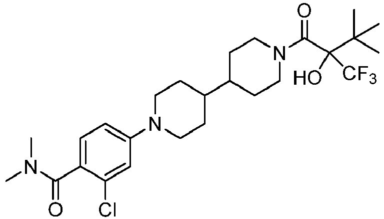

To a solution of 2-chloro-4-(1'-(3,3-dimethyl-2-oxobutanoyl)-[4,4'-bipiperidin]-1-yl)-*N,N-*dimethylbenzamide (**1-5**) (60 mg, 0.13 mmol) in THF (2 mL) is added CF₃TMS (36.9 mg, 0.26 mmol) followed by TBAF solution in THF (1 M, 0.13 mL). After stirring the reaction mixture at rt for 1 h, 0.026 mL more TBAF solution is added and then continue stirring for 15 h. Another aliquot (0.15 mL) of TBAF solution is added. After stirring for 45 min, the reaction mixture is concentrated. Purify by silica gel flash chromatography using a linear gradient of 5 to 100% EtOAc in hexanes to give racemic 2-chloro-4-(1'-(2-hydroxy-3,3-dimethyl-2-(trifluoromethyl) butanoyl)-[4,4'-bipiperidin]-1-y1)-*N,N*-dimethylbenzamide (**1-6**). HRMS [M+H]⁺ 532.2552 found, 532.2554 required. ¹H NMR (400 MHz, CDCl₃) δ 7.15 (d, *J* = 8.4 Hz, 1H), 6.86-6.79 (m, 2H), 5.0-4.85 (m, 2H), 3.74 (d, *J* = 12.5 Hz, 2H), 3.11 (s, 3H), 2.98-2.6 (m, 7H), 1.86-1.78 (m, 4H), 1.40-1.08 (m, 16H).

Racemic 2-chloro-4-(1'-(2-hydroxy-3,3-dimethyl-2-(trifluoromethyl)butanoyl)-[4,4'-bipiperidin]-1-yl)-*N,N*-dimethylbenzamide (**1-6**) is resolved by chiral chromatography (IC column, 3 x 25 cm) using 3:2 (EtOH:heptane with 0.1% TFA). Enantiomer **1-6a** (elution time: 9.35 min); HRMS [M+H]⁺ 532.2540, 532.2554 required. Enantiomer **1-6b** (elution time: 10.47 min); HRMS [M+H]⁺ 532.2543, 532.2554 required.

### Example A-99

### Preparation of 2-chloro-N, N-dimethyl-4-[1'-phenylsulfonyl)-4,4'-bipiperidin-1-yl] benzamide (1-8)

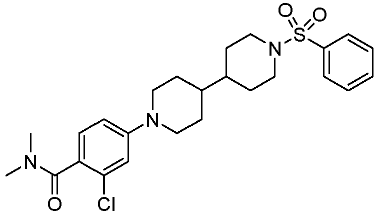

Intermediate (**1-4**) (54 mg, 0.13 mmol, 1 eq) was dissolved in CH₂Cl₂ (5 mL). Triethylamine (0.07 mL, 0.51 mmol, 4 eq) was added followed by phenyl sulfonyl chloride (0.027 g, 0.15 mmol, 1.2 eq) and the reaction was stirred at room temperature for 30 minutes. The reaction mixture concentrated and purified on by RP HPLC to give 2-chloro-N, N-dimethyl-4-[1'-phenylsulfonyl)-4,4'-bipiperidin-1-yl]benzamide (**1-8**). ¹H NMR (400 MHz, CDCl₃): δ 7.74 (d, J = 6.9 Hz, 2H), 7.56 (m, 1H), 7.53-7.51 (m, 2H), 7.11 (d, J = 8.61 Hz, 1H), 6.81 (s, 1H), 6.77 (m, 1H), 4.82 (m, 2H), 4.69 (m, 2H), 3.08 (s, 3H), 2.85 (s, 3H), 2.71-2.6 (m, 2H), 2.21-2.17 (m, 2H), 1.78-1.67 (m, 4H), 1.41-0.93 (m, 6H); LRMS *m*/*z* (M+H) 489.96 found, 489.19 required.

### Example A-107

### Preparation of 2-chloro-4-(1'-(2-chlorophenyl)-[4,4'-bipiperidin]-1-yl)-NN-dimethyl benzamide (1-9)

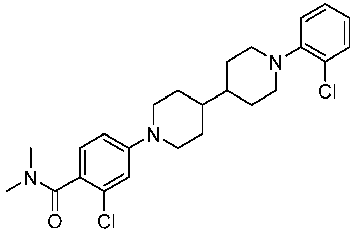

A mixture of 4-([4,4'-bipiperidin]-1-yl)-2-chloro-N,N-dimethylbenzamide dihydrochloride (**1-4**) (40 mg, 0.095 mmol), 1-bromo-2-chlorobenzene (18 mg, 0.095 mmol, 1.0 eq), 2-dicyclohexyl phosphino-2'-(N,N-dimethylamino)biphenyl (Dave Phos) (11 mg, 0.028 mmol, 0.3 eq), and sodium tert-butoxyide (32 mg, 0.33 mmol, 3.5 eq) in toluene (3 mL) was degassed with nitrogen for 15 minutes prior to the addition of palladium aceate (4.2 mg, 0.019 mmol, 0.2 eq). The resulting reaction mixture was heated at 90 °C under nitrogen for 16 hours. The reaction solvent was evaporated and the residue was dissolved in DMSO and filtered. The filtrate was purified by reverse phase liquid chromatography (Gemini-NX C18, 5 µm, 30 x 100 mm column; 0-100% CH₃CN/H₂O gradient w/ 0.10% TFA present) to yield 2-chloro-4-(1'-(2-chlorophenyl)-[4,4'-bipiperidin]-1-yl)-N,N-dimethylbenzamide (**1-9**). 1H NMR (400 MHz, CD₃OD): 7.72 (t, J = 7.4 Hz, 1 H); 7.46 (d, J = 8.0 Hz, 1 H), 7.36 (m, 2 H); 7.20 (m, 1H); 7.10 (m, 2 H); 3.84 (d, J = 12.1 Hz, 2 H); 3.20 (br s, 1 H); 3.10 (s, 3 H); 2.96 (m, 3 H); 2.91 (s, 3 H); 2.83 (m, 2 H); 1.96 (m, 2 H); 1.84 (m, 2 H); 1.50-1.26 (m, 6 H). LRMS m/z (M+H) 460.3 found, 460.2 required.

### Example A-108:

### Preparation of 2,6-dichlorobenzyl 1'-(3-chloro-4-(dimethylcarbamoyl)phenyl)-[4,4'-bipiperidine]-1-carboxylate (1-11)

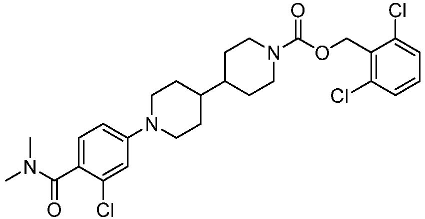

To a solution of 4-([4,4'-bipiperidin]-1-yl)-2-chloro-N,N-dimethylbenzamide dihydrochloride (**1-4**) (0.50 g, 1.2 mmol) and N,N-diisopropylethylamine (0.68 mL, 2.9 mmol, 3.3 eq) in dichloromethane (15 mL) at -5°C was added Phosgene (20% in toluene) (0.76 g, 1.5 mmol, 1.3 eq) and the reaction was stirred to 23°C. After 2 hours the reaction was diluted with dichloromethane (15 mL) and saturated aqueous sodium bicarbonate solution (15 mL) and the organic layer was separated, dried over sodium sulfate and concentrated to yield 1'-(3-chloro-4-(dimethylcarbamoyl)phenyl)-[4,4'-bipiperidine]-1-carbonyl chloride (**1-10**).

To a solution of 2,6-dichlorobenzyl alcohol (26 mg, 0.15 mmol) in tetrahydrofuran (1.5 mL) was added sodium hydride (95%, 25 mg, 1.0 mmol, 24 eq) and the reaction was stirred at 23 °C for 15 minutes before adding a solution of 1'-(3-chloro-4-(dimethylcarbamoyl)phenyl)-[4,4'-bipiperidine]-1-carbonyl chloride (**1-10**) (60 mg, 0.15 mmol, 1.0 eq) in dichloromethane (1.5 mL). The resulting reaction was stirred at 23°C for 16 hours. The reaction mixture was then quenched with water (0.25 mL) and concentrated and the residue was dissolved in DMSO and purified by reverse phase liquid chromatography (Gemini-NX C18, 5 µm, 30 x 100 mm column; 0-100% CH₃CN/H₂O gradient w/ 0.10% TFA present) to yield 2,6-dichlorobenzyl 1'-(3-chloro-4-(dimethylcarbamoyl)phenyl)-[4,4'-bipiperidine]-1-carboxylate (**1-11**). 1H NMR (400 MHz, CD3OD): δ 7.44 (s, 1 H); 7.42 (s, 1 H); 7.34 (dd, J = 9.0, 7.1 Hz, 1 H); 7.25 (d, J = 8.5 Hz, 1 H); 7.20 (d, J = 2.3 Hz, 1 H); 7.13 (dd, J = 8.6, 2.3 Hz, 1 H); 5.36 (s, 2 H); 4.21-3.98 (br m, 2 H); 3.78 (d, J = 12.4 Hz, 2 H); 3.09 (s, 3 H); 2.95 (t, J = 12.0 Hz, 2 H); 2.89 (s, 3 H); 2.77 (t, J = 12.8 Hz, 2 H); 1.89 (d, J = 12.1 Hz, 2 H); 1.74 (br s, 2 H); 1.40-1.33 (m, 4 H); 1.17 (br s, 2 H). LRMS m/z (M+H) 552.3 found, 552.2 required.

Examples reported in Table A can be prepared by the procedures described in Scheme A and the above examples from readily available starting materials and intermediates.

**TABLE A:**

| Example # | **Structure** | IUPAC Name | Exact Mass [M+H] + | Scheme |
|---|---|---|---|---|
| A-1 | 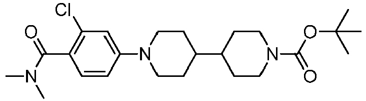 | tert-butyl 1'-[3-chloro-4-(dimethylcarbamoyl) phenyl]-4,4'-bipiperidine-1-carboxylate | Calc'd 450.3, found 450.4 | A 1-3 |
| A-2 | 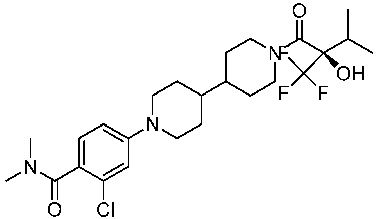 | 2-chloro-4-{1'-[(2R)-2-hydroxy-3-methyl-2-(trifluoromethyl)buta noyl]-4,4'-bipiperidin-1-y1}-N,N-dimethy lbenzamide | Calc'd 518.2, found 518.2 | A 1-6 |
| A-3 | 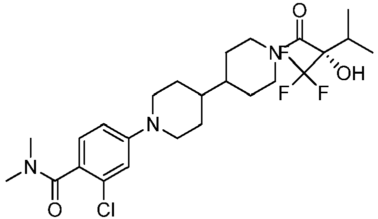 | 2-chloro-4-{1'-[(2S)-2-hydroxy-3-methyl-2-(trifluoromethyl)buta noyl]-4,4'-bipiperidin-1-yl}-N,N-dimethylbenzamide | Calc'd 518.2, found 518.2 | A 1-6 |
| A-4 | 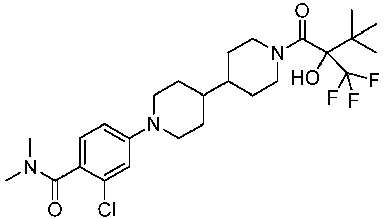 | 2-chloro-4-{1'-[2-hydroxy-3,3-dimethyl-2-(trifluoromethyl)buta noyl]-4,4'-bipiperidin-1-yl}-N,N-dimethylbenzamide | Calc'd 532.3, found 532.3 | A 1-6 |
| A-5 | 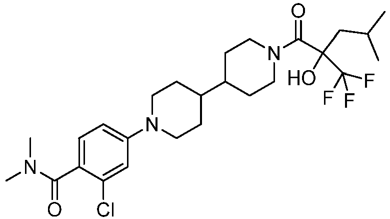 | 2-chloro-4-{1'-[2-hydroxy-4-methyl-2-(trifluoromethyl)pent anoyl]-4,4'-bipiperidin-1-y1}-N,N-dimethylbenzamide | Calc'd 532.3, found 532.3 | A 1-6 |
| A-6 | 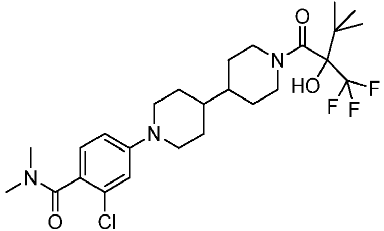 | 2-chloro-4-{1'-[2-hydroxy-3,3-dimethyl-2-(trifluoromethyl)buta noyl]-4,4'-bipiperidin-1-yl}-N,N-dimethylbenzamide | Calc'd 532.3, found 532.3 | A 1-6 |
| A-7 | 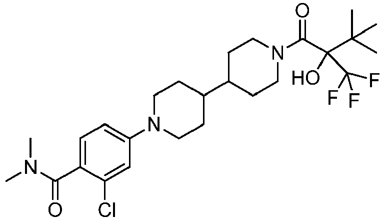 | 2-chloro-4-{1'-[2-hydroxy-3,3-dimethyl-2-(trifluoromethyl)buta noyl]-4,4'-bipiperidin-1-yl}-N,N-dimethy lbenzamide | Calc'd 532.3, found 532.3 | A 1-6 |
| A-8 | 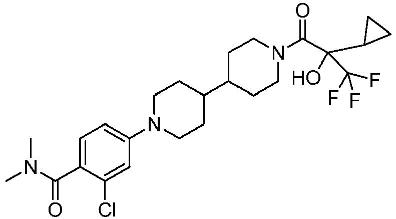 | 2-chloro-4-[1'-(2-cyclopropyl-3,3,3-trifluoro-2-hydroxypropanoyl)-4,4'-bipiperidin-1-y1]-N,N-dimethylbenzamide | Calc'd 516.2, found 516.2 | A 1-6 |
| A-9 | 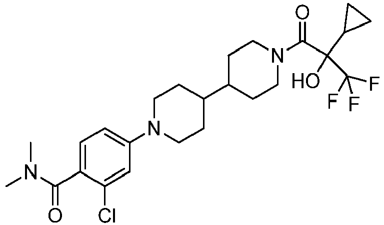 | 2-chloro-4-[1'-(2-cyclopropyl-3,3,3-trifluoro-2-hydroxypropanoyl)-4,4'-bipiperidin-1-yl]-N,N-dimethylbenzamide | Calc'd 516.2 , found 5162 | A 1-6 |
| A-10 | 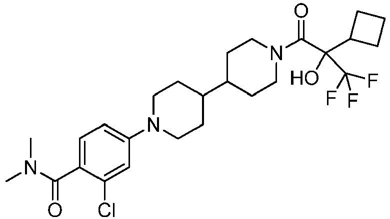 | 2-chloro-4-[1'-(2-cyclobutyl-3,3,3-trifluoro-2-hydroxypropanoyl)-4,4'-bipiperidin-1-yl]-N,N-dimethylbenzamide | Calc'd 530.2 , found 530.2 | A 1-6 |
| A-11 | 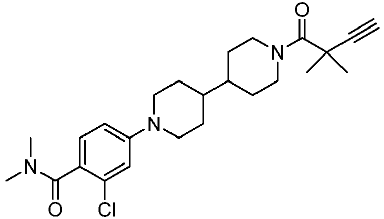 | 2-chloro-4-[1'-(2,2-dimethylbut-3-ynoyl)-4,4'-bipiperidin-1-y1]-N,N-dimethylbenzamide | Calc'd 444.2, found 444.0 | A 1-7 |
| A-12 | 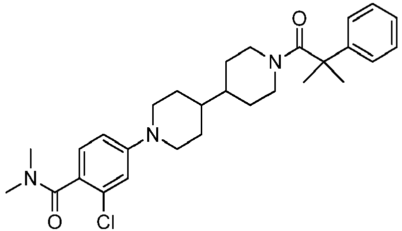 | 2-chloro-N,N-dimethyl-4-[1'-(2-methyl-2-phenylpropanoyl)-4,4'-bipiperidin-1-yl]benzamide | Calc'd 496.3, found 496.4 | A 1-7 |
| A-13 | 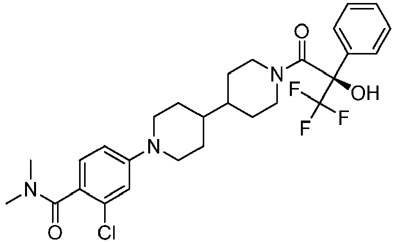 | 2-chloro-N,N-dimethyl-4-{1'-[(2R)-3,3,3-trifluoro-2-hydroxy-2-phenylpropanoyl]-4,4'-bipiperidin-1-yl}benzamide | Calc'd 552.2, found 552.2 | A 1-7 |
| A-14 | 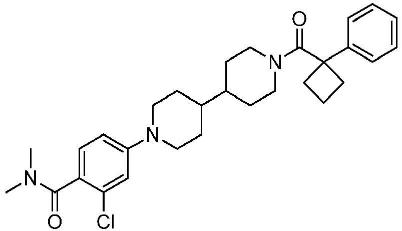 | 2-chloro-N,N-dimethyl-4-{1'-[(1-phenylcyclobutyl)car bonyl]-4,4'-bipiperidin-1-yl}benzamide | Calc'd 508.3, found: 508.3 | A 1-7 |
| A-15 | 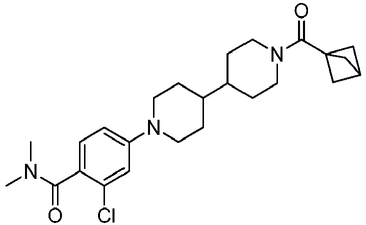 | 4-[1'-(bicyclo[1.1.1]pent-1-ylcarbonyl)-4,4'-bipipendin-1-yl]-2-chloro-N,N-dimethylbenzamide | Calc'd 444.2, found 444.0 | A 1-7 |
| A-16 | 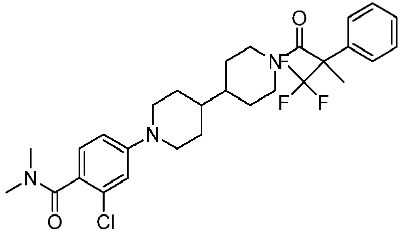 | 2-chloro-N,N-dimethyl-4-[1'-(3,3,3-trifluoro-2-methyl-2-phenylpropanoyl)-4,4'-bipiperidin-1-yl]benzamide | Calc'd 550.2, found 550.0 | A 1-7 |
| A-17 | 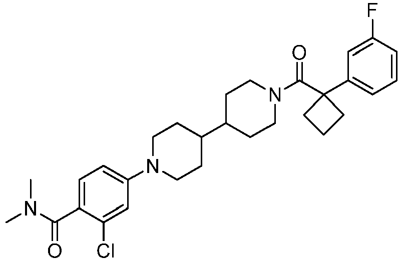 | 2-chloro-4-(1'-{[1-(3-fluorophenyl)cyclobu tyl]carbonyl}-4,4'-bipiperidin-1-y1)-N,N-dimethylbenzamide | Calc'd 526.3, found 526.4 | A 1-7 |
| A-18 | 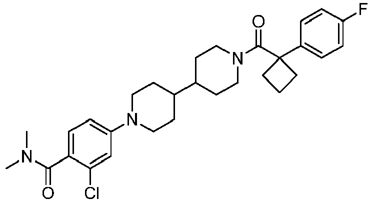 | 2-chloro-4-(1'-{[1-(4-fluorophenyl)cyclobu tyl]carbonyl}-4,4'-bipiperidin-1-yl)-N,N-dimethylbenzamide | Calc'd 526.3, found 526.4 | A 1-7 |
| A-19 | 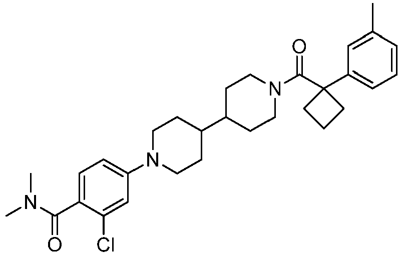 | 2-chloro-N,N-dimethyl-4-(1'-{[1-methylphenyl)cyclob utyl]carbonyl}-4,4'- bipiperidin-1-yl)benzamide | Calc'd 522.3, found 522.5 | A 1-7 |
| A-20 | 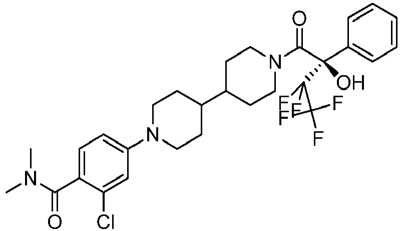 | 2-chloro-N,N-dimethyl-4-{1'-[(2R)-3,3,4,4,4-pentafluoro-2-hydroxy-2-phenylbutanoyl]-4,4' -bipiperidin-1-yl}benzamide | Calc'd 602.2, found 602.2 | A 1-7 |
| A-21 | 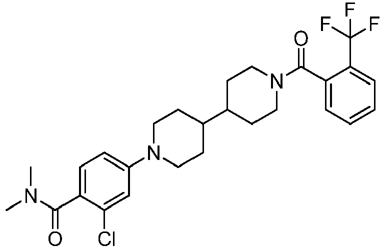 | 2-chloro-N,N-dimethyl-4-(1'-{[2-(trifluoromethyl)phen yl]carbonyl}-4,4'-bipiperidin-1-yl)benzamide | Calc'd 522.2, found 522.4 | A 1-7 |
| A-22 | 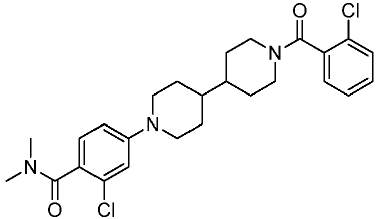 | 2-chloro-4-{1'-[(2-chlorophenyl)carbon yl]-4,4'-bipiperidin-1-y1}-N,N-dimethylbenzamide | Calc'd 488.2, found 488.4 | A 1-7 |
| A-23 | 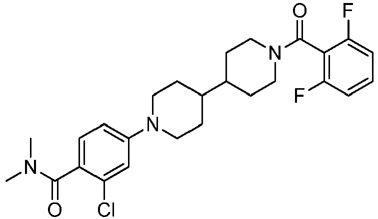 | 2-chloro-4-{1'-[(2,6-difluorophenyl)carbo nyl]-4,4'-bipiperidin-1-yl}-N,N-dimethylbenzamide | Calc'd 490.2, found 490.3 | A 1-7 |
| A-24 | 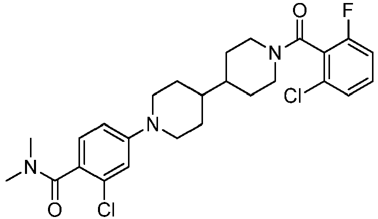 | 2-chloro-4-{1'-[(2-chloro-6-fluorophenyl)carbony l]-4,4'-bipiperidin-1-yl}-N,N-dimethylbenzamide | Calc'd 506.2, found 506.3 | A 1-7 |
| A-25 | 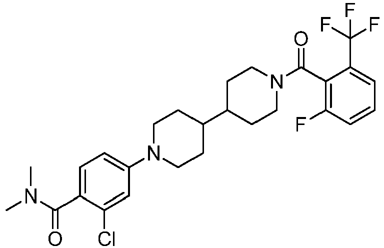 | 2-chloro-4-(1'-{[2-fluoro-6-(trifluoromethyl)phen bipiperidin-1-yl)-N,N-dimethylbenzamide | Calc'd 540.2, found,. 540.1 | A 1-7 |
| A-26 | 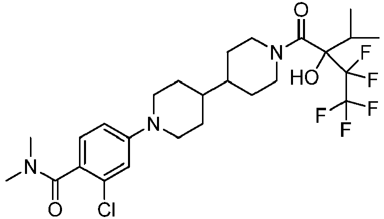 | 2-chloro-N,N-dimethyl-4-{ 1'-[3,3,4,4,4-pentafluoro-2-hydroxy-2-(1-methylethyl)butanoyl ]-4,4'-bipiperidin-1-yl}benzamide | Calc'd 568.2, found 568.3 | A 1-7 |
| A-27 | 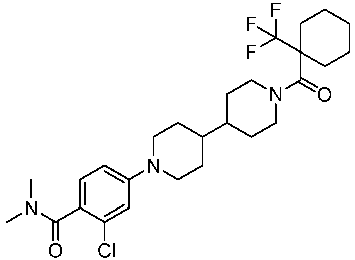 | 2-chloro-N,N-dimethyl-4-(1'-{[1-(trifluoromethyl)cycl ohexyl]carbonyl}-4,4'-bipiperidin-1-yl)benzamide | Calc'd 528.3, found 528.3 | A 1-7 |
| A-28 | 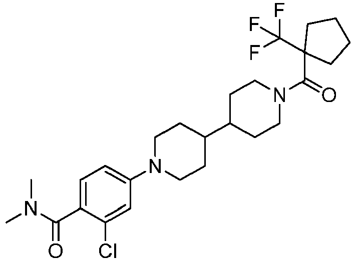 | 2-chloro-N,N-dimethyl-4-(1'-{[1-(trifluoromethyl) cyclopentyl]carbonyl }-4,4'-bipiperidin-1-yl)benzamide | Calc'd 514.2, found: 514.2 | A 1-7 |
| A-29 | 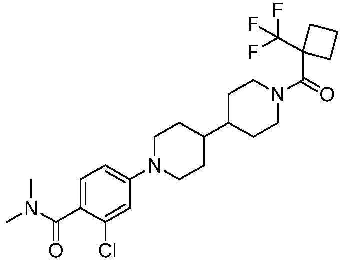 | 2-chloro-N,N-dimethyl-4-(1'-{[1-(trifluoromethyl) cyclobutyl]carbonyl} -4,4'-bipiperidin-1-yl)benzamide | Calc'd 500.2, found 500.2 | A 1-7 |
| A-30 | 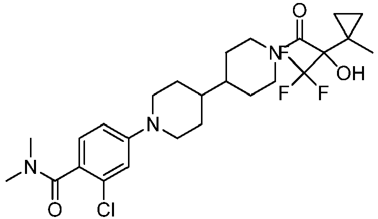 | 2-chloro-N,N-dimethyl-4-{ 1'-[3,3,3-trifluoro-2-hydroxy-2-(1-methylcyclopropyl)pr opanoyl]-4,4'-bipiperidin-1-yl}benzamide | Calc'd 530.2, found 530.1 | A 1-7 |
| A-31 | 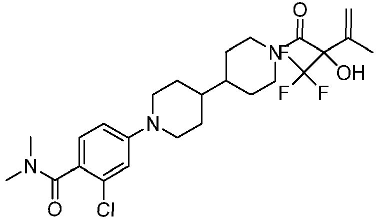 | 2-chloro-4-{1'-[2-hydroxy-3-methyl-2-(trifluoromethyl)but-3-enoyl]-4,4'-bipiperidin-1-yl}-N,N-dimethylbenzamide | Calc'd 516.2, found 516.0 | A 1-7 |
| A-32 | 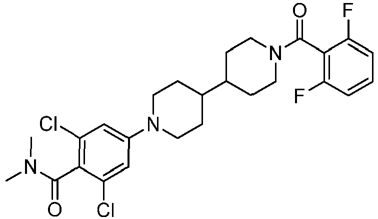 | 2,6-dichloro-4-{1'-[(2,6-difluorophenyl)carbo nyl]-4,4'-bipiperidin-1-yl}-N,N-dimethylbenzamide | Calc'd 524.2, found 524.3 | A 1-7 |
| A-33 | 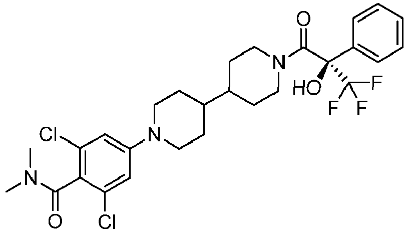 | 2,6-dicliloro-N,N-dimethyl-4-{1'-[(2R)-3,3,3-trifluoro-2-hydroxy-2-phenylpropanoyl]-4,4'-bipiperidin-1-yl}benzamide | Calc'd 586.2, found 586.3 | A 1-7 |
| A-34 | 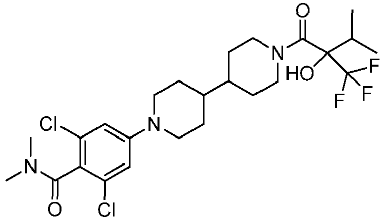 | 2,6-dichloro-4-{1'-[2-hydroxy-3-methyl-2-(trifluoromethyl)buta noyl]-4,4'-bipiperidin-1-yl}-N,N-dimethy lbenzamide | Calc'd 552.2, found 552.3 | A 1-7 |
| A-35 | 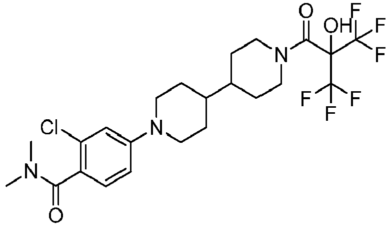 | 2-chloro-N,N-dimethyl-4-{1'-[3,3,3-trifluoro-2-hydroxy-2-(trifluoromethyl)prop anoyl]-4,4'-bipiperidin-1-yl}benzamide | Calc'd 544.2, found 544.0 | A 1-7 |
| A-36 | 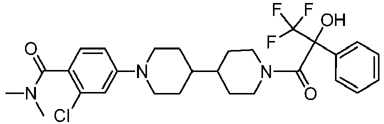 | 2-chloro-N,N-dimethyl-4-[1'-(3,3,3-trifluoro-2-hydroxy-2-phenylpropanoyl)-4,4'-bipiperidin-1-yl]benzamide | Calc'd 552.2, found 552.1 | A 1-7 |
| A-37 | 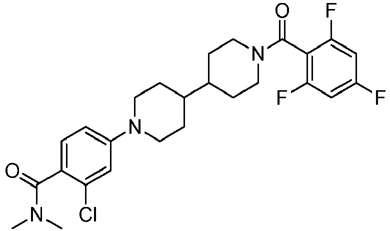 | 2-chloro-N,N-dimethyl-4-{1'-[(2,4,6-trifluorophenyl)carbo nyl]-4,4'-bipiperidin-1-yl}benzamide | Calc'd 508.2, found 508.2 | A 1-7 |
| A-38 | 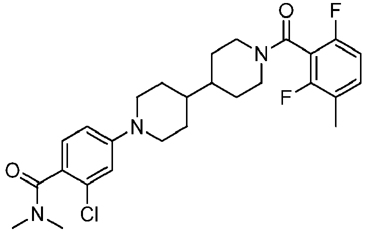 | 2-chloro-4-{1'-[(2,6-difluoro-3-methylphenyl)carbon yl]-4,4'-bipiperidin-1-yl}-N,N-dimethylbenzamide | Calc'd 504.2, found: 504.2 | A 1-7 |
| A-39 | 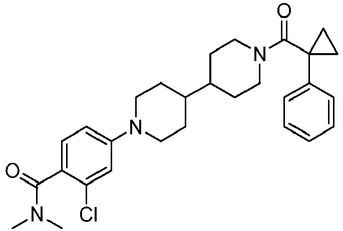 | 2-chloro-N,N-dimethyl-4-{1'-[(1-phenylcyclopropyl)ca rbonyl]-4,4'-bipiperidin-1-yl}benzamide | Calc'd 494.3, found: 494.3 | A 1-7 |
| A-40 | 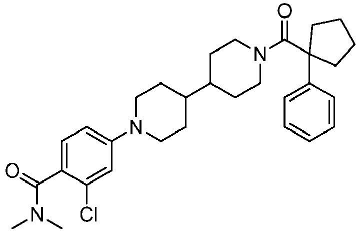 | 2-chloro-N,N-dimethyl-4-{1'-[(1-phenylcyclopentyl)ca rbonyl]-4,4'-bipiperidin-1-yl}benzamide | Calc'd 522.3, found: 522.3 | A 1-7 |
| A-41 | 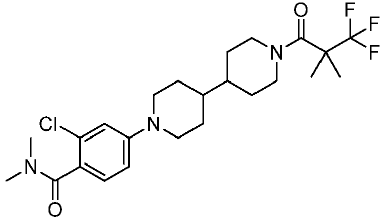 | 2-chloro-N,N-dimethyl-4-[1'-(3,3,3-trifluoro-2,2-dimethylpropanoyl)-4,4'-bipiperidin-1-yl]benzamide | Calc'd 488.2, found 488.2 | A 1-7 |
| A-42 | 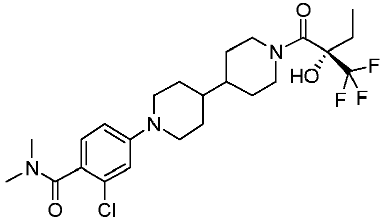 | 2-chloro-4-{1'-[(2S)-2-hydroxy-2-(trifluoromethyl)buta noyl]-4,4'-bipiperidin-1-yl}-N,N-dimethylbenzamide | Calc'd 504.2, found 504.2 | A 1-7 |
| A-43 | 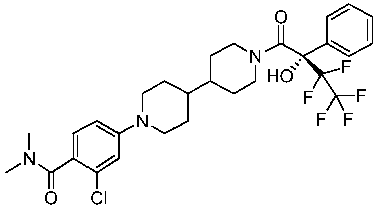 | 2-chloro-N,N-dimethyl-4-{ 1'-[(2S)-3,3,4,4,4-pentafluoro-2-hydroxy-2-phenylbutanoyl]-4,4'-bipiperidin-1-yl}benzamide | Calc'd 602.2, found 602.2 | A 1-7 |
| A-44 | 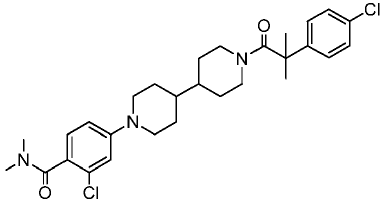 | 2-chloro-4-{1'-[2-(4-chlorophenyl)-2-methylpropanoyl]-4,4'-bipiperidin-1-yl}-N,N-dimethy lbenzamide | Calc'd 530.2, found 530.4 | A 1-7 |
| A-45 | 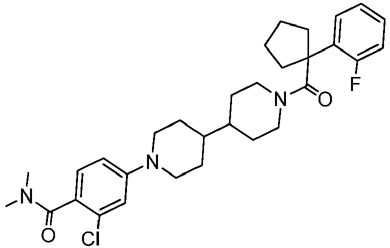 | 2-chloro-4-(1'-{[1-(2-fluorophenyl)cyclope ntyl]carbonyl}-4,4'-bipiperidin-1-yl)-N,N-dimethylbenzamide | Calc'd 540.3, found 540.4 | A 1-7 |
| A-46 | 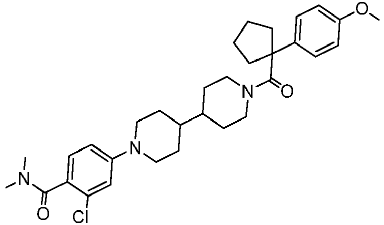 | 2-chloro-4-(1'-{[1-(4-methoxyphenyl)cyclo pentyl]carbonyl}-4,4'-bipiperidin-1-yl)-N,N-dimethylbenzamide | Calc'd 552.3, found 552.5 | A 1-7 |
| A-47 | 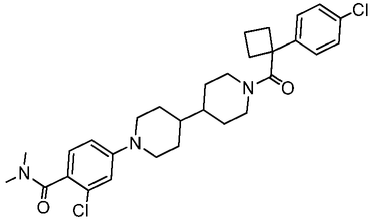 | 2-chloro-4-(1'-{[1-(4-chlorophenyl)cyclob utyl]carbonyl}-4,4'-bipiperidin-1-yl)-N,N-dimethylbenzamide | Calc'd 542.2, found 542.4 | A 1-7 |
| A-48 | 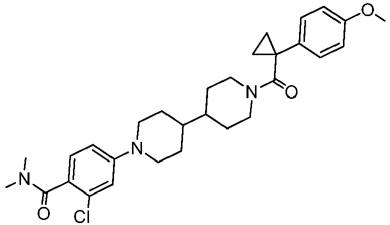 | 2-chloro-4-(1'-{[1-(4-methoxyphenyl)cyclo propyl]carbonyl}-4,4'-bipiperidin-1-yl)-N,N-dimethylbenzamide | Calc'd 524.3, found 524.4 | A 1-7 |
| A-49 | 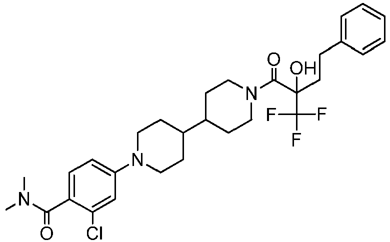 | 2-chloro-4-{1'-[2-hydroxy-4-phenyl-2-(trifluoromethyl)buta noyl]-4,4'-bipiperidin-1-yl}-N,N-dimethylbenzamide | Calc'd 580.3, found 580.4 | A 1-7 |
| A-50 | 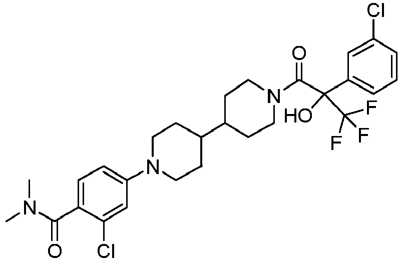 | 2-chloro-4-{1'-[2-(3-chlorophenyl)-3,3,3-trifluoro-2-hydroxypropanoyl]-4,4'-bipiperidin-1-yl}-N,N-dimethylbenzamide | Calc'd 586.2, found 586.4 | A 1-7 |
| A-51 | 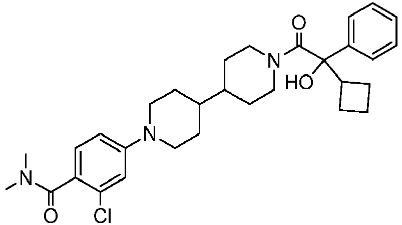 | 2-chloro-4-{1'-[cyclobutyl(hydroxy) phenylacetyl]-4,4'-bipiperidin-1-yl}-N,N-dimethylbenzamide | Calc'd 538.3, found 538.4 | A 1-7 |
| A-52 | 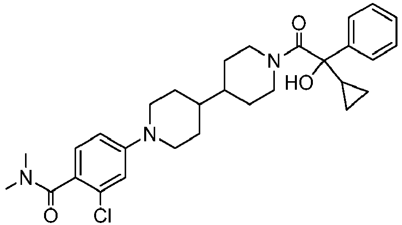 | 2-chloro-4-{1'-[cyclopropyl(hydrox y)phenylacetyl]-4,4'-bipiperidin-1-yl}-N,N-dimethylbenzamide | Calc'd 524.3, found 524.4 | A 1-7 |
| A-53 | 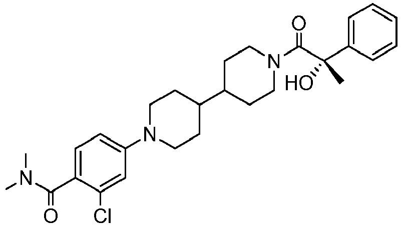 | 2-chloro-4-{1'-[(2S)-2-hydroxy-2-phenylpropanoyl]-4,4'-bipiperidin-1-yl}-N,N-dimethylbenzamide | Calc'd 498.3, found 498.4 | A 1-7 |
| A-54 | 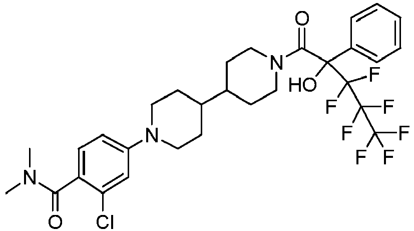 | 2-chloro-4-[1'-(3,3,4,4,5,5,5-heptafluoro-2-hydroxy-2-phenylpentanoyl)-4,4'-bipiperidin-1-yl]-N,N-dimethylbenzamide | Calc'd 652.2, found 652.4 | A 1-7 |
| A-55 | 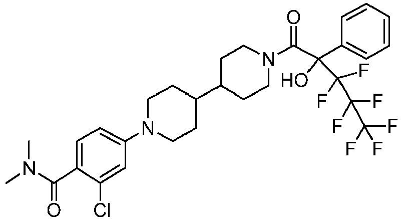 | 2-chloro-4-[1'-(3,3,4,4,5,5,5-heptafluoro-2-hydroxy-2-phenylpentanoyl)-4,4'-bipiperidin-1-yl]-N,N-dimethylbenzamide | Calc'd 652.2, found 652.4 | A 1-7 |
| A-56 | 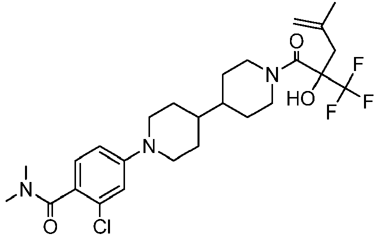 | 2-chloro-4-{1'-[2-hydroxy-4-methyl-2-(trifluoromethyl)pent -4-enoyl]-4,4'-bipiperidin-1-yl}-N,N-dimethylbenzamide | Calc'd 530.2, found 530.4 | A 1-7 |
| A-57 | 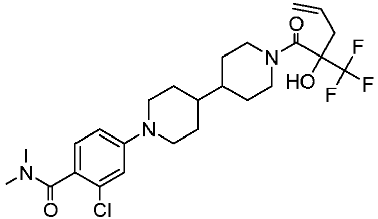 | 2-chloro-4-{1'-[2-hydroxy-2-(trifluoromethyl)pent -4-enoyl]-4,4'-bipiperidin-1-yl}-N,N-dimethylbenzamide | Calc'd 516.2, found 516.4 | A 1-7 |
| A-58 | 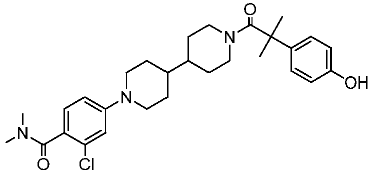 | 2-chloro-4-{1'-[2-(4-hydroxyphenyl)-2-methylpropanoyl]-4,4'-bipiperidin-1-yl}-N,N-dimethylbenzamide | Calc'd 512.3, found 512.4 | A 1-7 |
| A-59 | 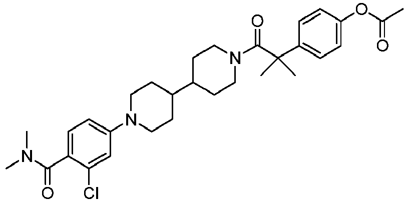 | 4-(2-{1'-[3-chloro-4-(dimethylcarbamoyl) phenyl]-4,4'-bipiperidin-1-yl}-1,1-dimethyl-2-oxoethyl)phenyl acetate | Calc'd 554.3, found 554.5 | A 1-7 |
| A-60 | 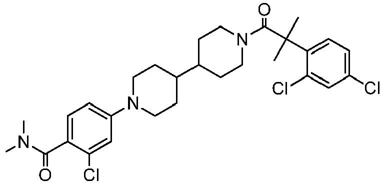 | 2-chloro-4-{1'-[2-(2,4-dichlorophenyl)-2-methylpropanoyl]-4,4'-bipiperidin-1-yl}-N,N-dimethy lbenzamide | Calc'd 564.2, found 564.4 | A 1-7 |
| A-61 | 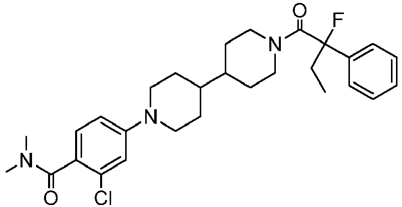 | 2-chloro-4-[1'-(2-fluoro-2-phenylbutanoyl)-4,4'-bipiperidin-1-yl]-N,N-dimethylbenzamide | Calc'd 514.3, found 514.4 | A 1-7 |
| A-62 | 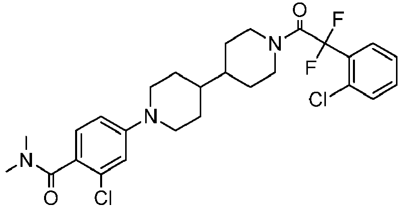 | 2-chloro-4-{1'-[(2-chlorophenyl)(difluor o)acetyl]-4,4'-bipiperidin-1-yl}-N,N-dimethylbenzamide | Calc'd 538.2, found 538.4 | A 1-7 |
| A-63 | 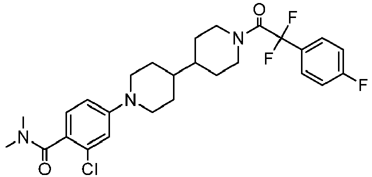 | 2-chloro-4-{1'-[difluoro(4-fluorophenyl)acetyl]-4,4'-bipiperidin-1-yl}-N,N-dimethylbenzamide | Calc'd 522.2, found 522.4 | A 1-7 |
| A-64 | 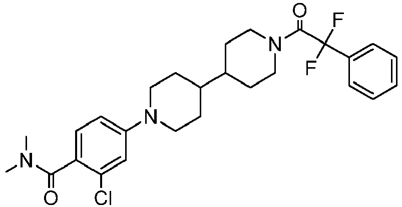 | 2-chloro-4-{1'-[difluoro(phenyl)acet yl]-4,4'-bipiperidin-1-yl}-N,N-dimethylbenzamide | Calc'd 504.2, found 504.4 | A 1-7 |
| A-65 | 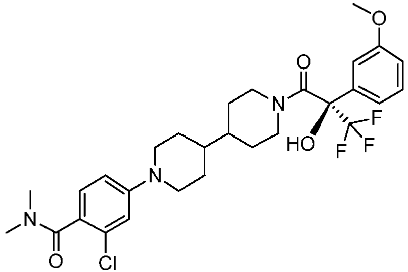 | 2-chloro-N,N-dimethyl-4-{1'-[(2R)-3,3,3-trifluoro-2-hydroxy-2-(3-methoxyphenyl)prop anoyl]-4,4'-bipiperidin-1-yl}benzamide | Calc'd 582.2, found 582.4 | A 1-7 |
| A-66 | 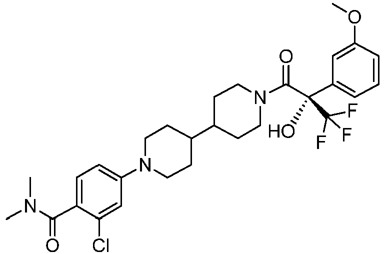 | 2-chloro-N,N-dimethyl-4-{1'-[(2S)-3,3,3-trifluoro-2-hydroxy-2-(3-methoxyphenyl)prop anoyl]-4,4'-bipiperidin-1-yl}benzamide | Calc'd 582.2, found 582.4 | A 1-7 |
| A-67 | 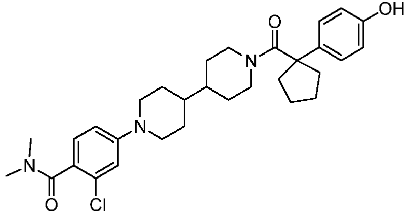 | 2-chloro-4-(1'-{[1-(4-hydroxyphenyl)cyclo pentyl]carbonyl}-4,4'-bipiperidin-1-yl)-N,N-dimethylbenzamide | Calc'd 538.3, found 538.4 | A 1-7 |
| A-68 | 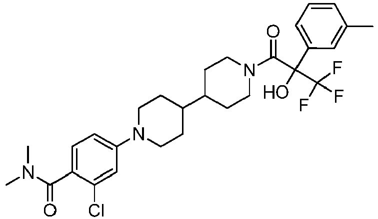 | 2-chloro-N,N-dimethyl-4-{1'-[3,3,3-trifluoro-2-hydroxy-2-(3-methylphenyl)propan oyl]-4,4'-bipiperidin-1-yl}benzamide | Calc'd 566.2, found 566.4 | A 1-7 |
| A-69 | 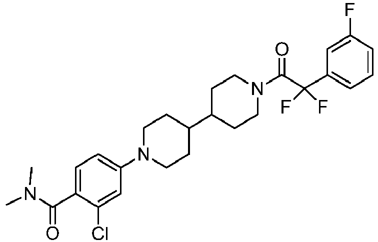 | 2-chloro-4-{1'-[difluoro(3-fluorophenyl)acetyl]-4,4'-bipiperidin-1-yl}-N,N-dimethy lbenzamide | Calc'd 522.2, found 522.4 | A 1-7 |
| A-70 | 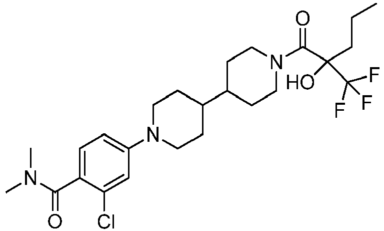 | 2-chloro-4-{1'-[2-hydroxy-2-(trifluoromethyl)pent anoyl]-4,4'-bipiperidin-1-yl}-N,N-dimethylbenzamide | Calc'd 518.2, found 518.4 | A 1-7 |
| A-71 | 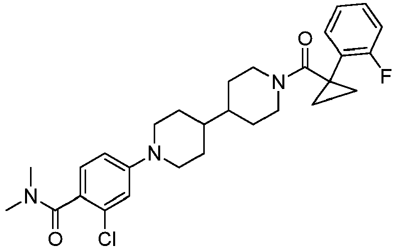 | 2-chloro-4-(1'-{[1-(2-fluorophenyl)cyclopr opyl]carbonyl}-4,4'-bipiperidin-1-yl)-N,N-dimethylbenzamide | Calc'd 512.2, found 512.4 | A 1-7 |
| A-72 | 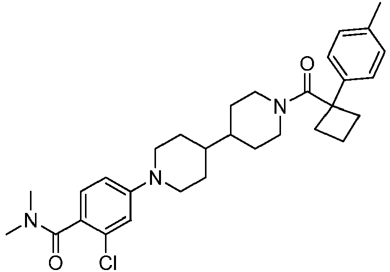 | 2-chloro-N,N-dimethyl-4-(1'-{[1-(4-methylphenyl)cyclob utyl]carbonyl}-4,4'-bipiperidin-1-yl)benzamide | Calc'd 522.3, found 522.4 | A 1-7 |
| A-73 | 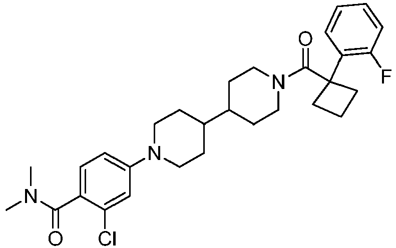 | 2-chloro-4-(1'-{[1-(2-fluorophenyl)cyclobu tyl]carbonyl}-4,4'-bipiperidin-1-yl)-N,N-dimethylbenzamide | Calc'd 526.3, found 526.4 | A 1-7 |
| A-74 | 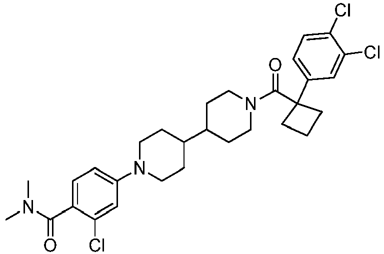 | 2-chloro-4-(1'-{[1-(3,4-dichlorophenyl)cyclo butyl]carbonyl}-4,4'-bipiperidin-1-yl)-N,N-dimethylbenzamide | Calc'd 576.2, found 576.4 | A 1-7 |
| A-75 | 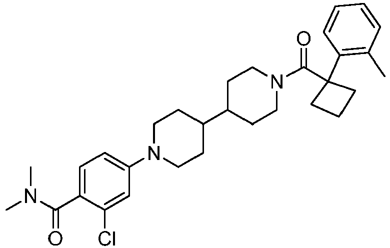 | 2-chloro-N,N-dimethyl-4-(1'-{[1-methylphenyl)cyclob utyl]carbonyl}-4,4'-bipiperidin-1-yl)benzamide | Calc'd 522.3, found 522.5 | A 1-7 |
| A-76 | 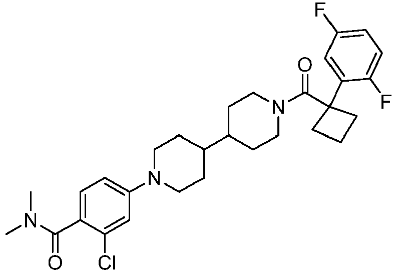 | 2-chloro-4-(1'-{[1-(2,5-difluorophenyl)cyclo butyl]carbonyl}-4,4'-bipiperidin-1-yl)-N,N-dimethylbenzamide | Calc'd 544.3, found 544.4 | A 1-7 |
| A-77 | 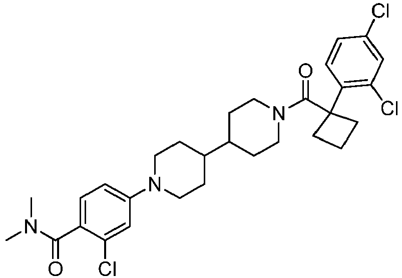 | 2-chloro-4-(1'-{[1-(2,4-dichlorophenyl)cyclo butyl]carbonyl}-4,4'-bipiperidin-1-yl)-N,N-dimethylbenzamide | Calc'd 576.2, found 576.4 | A 1-7 |
| A-78 | 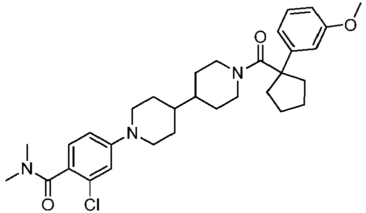 | 2-chloro-4-(1'-{[1-(3-methoxyphenyl)cyclo pentyl]carbonyl}-4,4'-bipiperidin-1-yl)-N,N-dimethylbenzamide | Calc'd 552.3, found 552.5 | A 1-7 |
| A-79 | 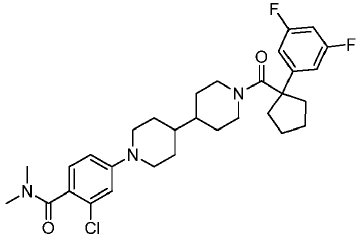 | 2-chloro-4-(1'-{[1-(3,5-difluorophenyl)cyclo pentyl]carbonyl}-4,4'-bipiperidin-1-yl)-N,N-dimethylbenzamide | Calc'd 558.3, found 558.4 | A 1-7 |
| A-80 | 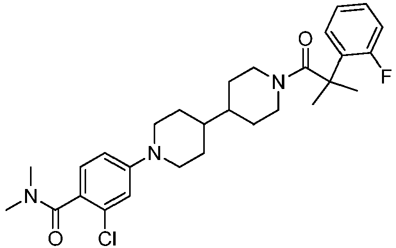 | 2-chloro-4-{1'-[2-(2-fluorophenyl)-2-methylpropanoyl]-4,4'-bipiperidin-1-yl}-N,N-dimethylbenzamide | Calc'd 514.3, found 514.4 | A 1-7 |
| A-81 | 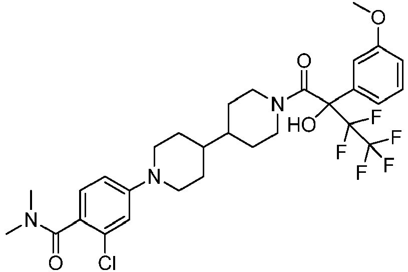 | 2-chloro-N,N-dimethyl-4-{1'-[3,3,4,4,4-pentafluoro-2-hydroxy-2-(3-methoxyphenyl)buta noyl]-4,4'-bipiperidin-1-yl}benzamide | Calc'd 632.2, found 632.4 | A 1-7 |
| A-82 | 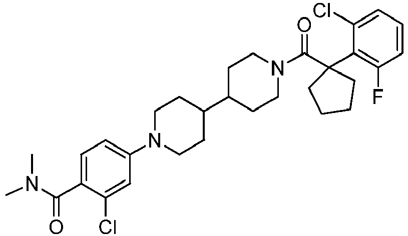 | 2-chloro-4-(1'-{[1-(2-chloro-6-fluorophenyl)cyclope ntyl]carbonyl}-4,4'-bipiperidin-1-yl)-N,N-dimethylbenzamide | Calc'd 574.2, found 574.4 | A 1-7 |
| A-83 | 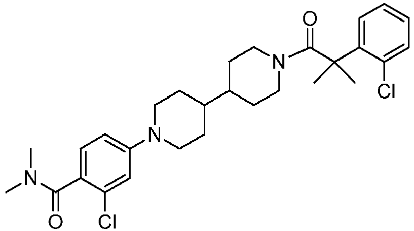 | 2-chloro-4-{1'-[2-(2-chlorophenyl)-2-methylpropanoyl]-4,4'-bipiperidin-1-yl}-N,N-dimethylbenzamide | Calc'd 530.2, found 530.4 | A 1-7 |
| A-84 | 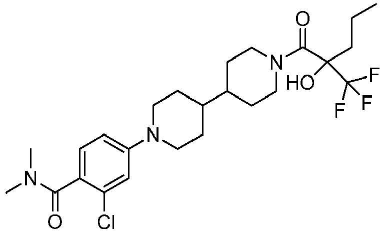 | 2-chloro-4-{1'-[2-hydroxy-2-(trifluoromethyl)pent anoyl]-4,4'-bipiperidin-1-yl}-N,N-dimethylbenzamide | Calc'd 518.2, found 518.4 | A 1-7 |
| A-85 | 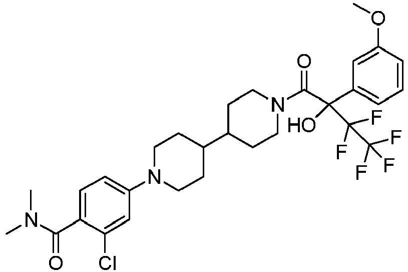 | 2-chloro-N,N-dimethyl-4-{1'-[3,3,4,4,4-pentafluoro-2-hydroxy-2-(3-methoxyphenyl)buta noyl]-4,4'-bipiperidin-1-yl}benzamide | Calc'd 632.2, found 632.4 | A 1-7 |
| A-86 | 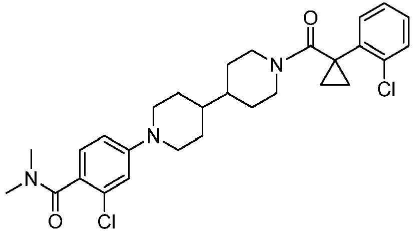 | 2-chloro-4-(1'-{[1-(2-chlorophenyl)cyclopr opyl]carbonyl}-4,4'-bipiperidin-1-yl)-N,N-dimethylbenzamide | Calc'd 528.2, found 528.4 | A 1-7 |
| A-87 | 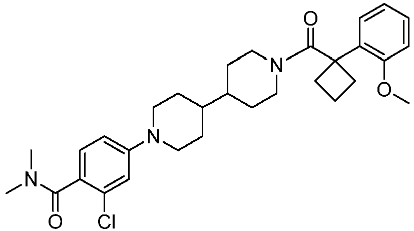 | 2-chloro-4-(1'-{[1-(2-methoxyphenyl)cyclo butyl]carbonyl}-4,4'-bipiperidin-1-yl)-N,N-dimethylbenzamide | Calc'd 538.3, found 538.4 | A 1-7 |
| A-88 | 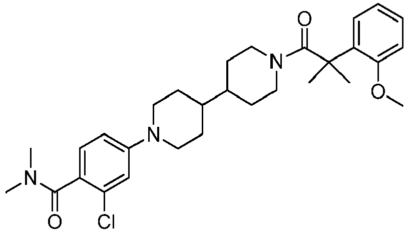 | 2-chloro-4-{1'-[2-(2-methoxyphenyl)-2-methylpropanoyl]-4,4'-bipiperidin-1-yl}-N,N-dimethylbenzamide | Calc'd 526.3, found 526.4 | A 1-7 |
| A-89 | 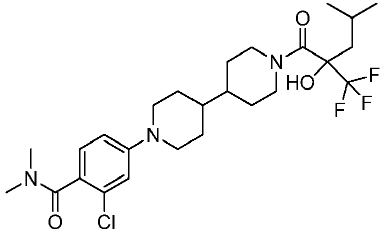 | 2-chloro-4-{1'-[2-hydroxy-4-methyl-2-(trifluoromethyl)pent anoyl]-4,4'-bipiperidin-1-yl}-N,N-dimethylbenzamide | Calc'd 532.3, found 532.4 | A 1-7 |
| A-90 | 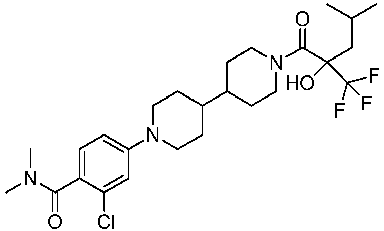 | 2-chloro-4-{1'-[2-hydroxy-4-methyl-2-(trifluoromethyl)pent anoyl]-4,4'-bipiperidin-1-yl}-N,N-dimethylbenzamide | Calc'd 532.3, found 532.4 | A 1-7 |
| A-91 | 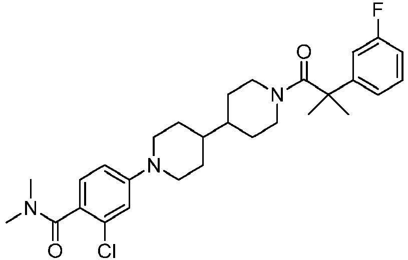 | 2-chloro-4-{1'-[2-(3-fluorophenyl)-2-methylpropanoyl]-4,4'-bipiperidin-1-yl}-N,N-dimethy lbenzamide | Calc'd 514.3, found 514.4 | A 1-7 |
| A-92 | 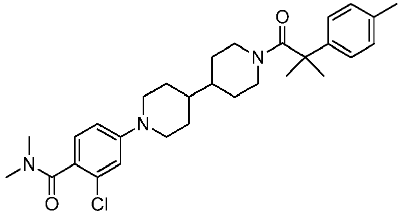 | 2-chloro-N,N-dimethyl-4-{1'-[2-methyl-2-(4-methylphenyl)propan oyl]-4,4'-bipiperidin-1-yl}benzamide | Calc'd 510.3, found 510.5 | A 1-7 |
| A-93 | 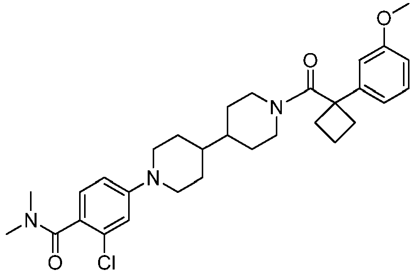 | 2-chloro-4-(1'-{[1-(3-methoxyphenyl)cyclo butyl]carbonyl}-4,4'-bipiperidin-1-yl)-N,N-dimethylbenzamide | Calc'd 538.3, found 538.5 | A 1-7 |
| A-94 | 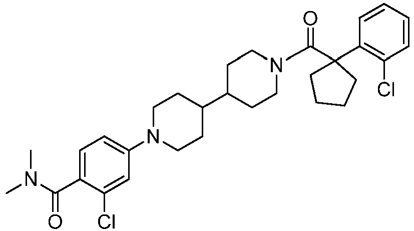 | 2-chloro-4-(1'-{[1-(2-chlorophenyl)cyclope ntyl]carbonyl}-4,4'-bipiperidin-1-yl)-N,N-dimethylbenzamide | Calc'd 556.2, found 556.4 | A 1-7 |
| A-95 | 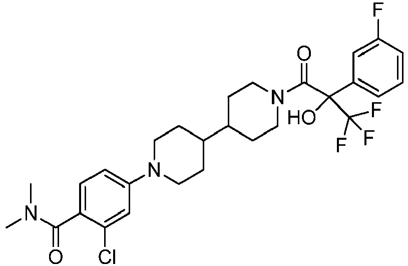 | 2-chloro-N,N-dimethyl-4-{1'-[3,3,3-trifluoro-2-(3-fluorophenyl)-2-hydroxypropanoyl]-4,4'-bipiperidin-1-yl}benzamide | Calc'd 570.2, found 570.4 | A 1-7 |
| A-96 | 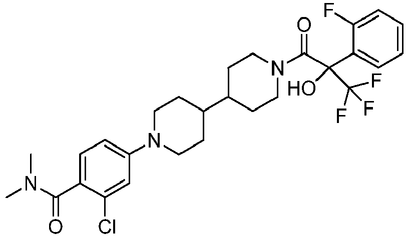 | 2-chloro-N,N-dimethyl-4-{1'-[3,3,3-trifluoro-2-(2-fluorophenyl)-2-hydroxypropanoyl]-4,4'-bipiperidin-1-yl}benzamide | Calc'd 570.2, found 570.4 | A 1-7 |
| A-97 | 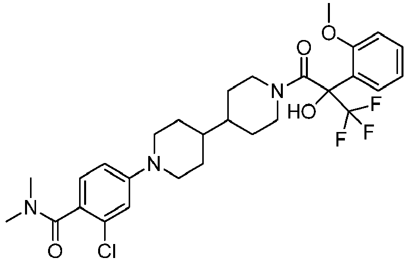 | 2-chloro-N,N-dimethyl-4-{1'-[3,3,3-trifluoro-2-hydroxy-2-(2-methoxyphenyl)prop anoyl]-4,4'-bipiperidin-1-yl}benzamide | Calc'd 582.2, found 582.4 | A 1-7 |
| A-98 | 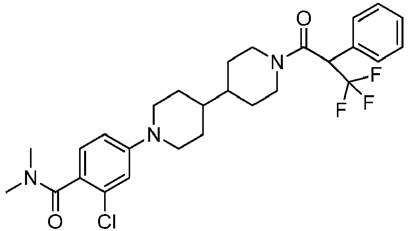 | 2-chloro-N,N-dimethyl-4-[1'-(3,3,3-trifluoro-2-phenylpropanoyl)-4,4'-bipiperidin-1-yl]benzamide | Calc'd 536.2, found 536.4 | A 1-7 |
| A-99 | 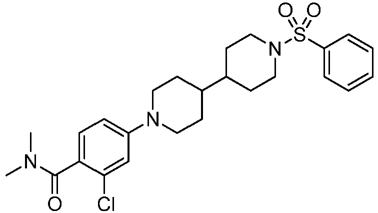 | 2-chloro-N,N-dimethyl-4-[1'-(phenylsulfonyl)-4,4'-bipiperidin-1-yl]benzamide | Calc'd 490.2, found 490.0 | A 1-8 |
| A-100 | 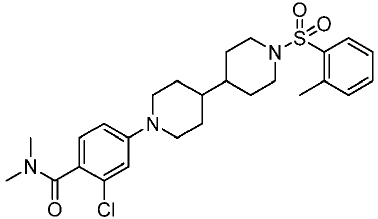 | 2-chloro-N,N-dimethyl-4-{1'-[(2-methylphenyl)sulfon yl]-4,4'-bipiperidin-1-yl}benzamide | Calc'd 504.2, found 504.4 | A 1-8 |
| A-101 | 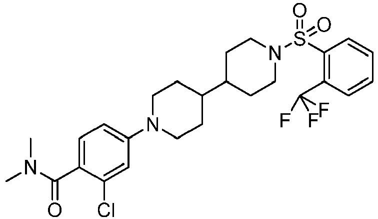 | 2-chloro-N,N-dimethyl-4-(1'-{[2-(trifluoromethyl)phen yl]sulfonyl}-4,4'-bipiperidin-1-yl)benzamide | Calc'd 558.2, found 558.3 | A 1-8 |
| A-102 | 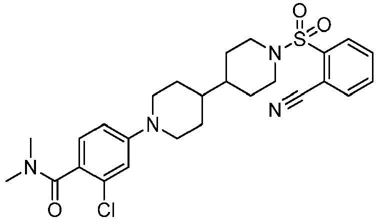 | 2-chloro-4-{1'-[(2-cyanophenyl)sulfonyl ]-4,4'-bipiperidin-1-yl}-N,N-dimethylbenzamide | Calc'd 515.2, found 515.3 | A 1-8 |
| A-103 | 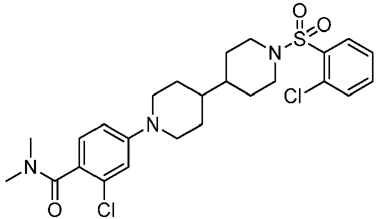 | 2-chloro-4-{1'-[(2-chlorophenyl)sulfony l]-4,4'-bipiperidin-1-yl}-N,N-dimethylbenzamide | Calc'd 524.2, found 524.3 | A 1-8 |
| A-104 | 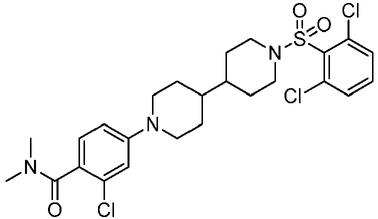 | 2-chloro-4-{1'-[(2,6-dichlorophenyl)sulfo nyl]-4,4'-bipiperidin-1-yl}-N,N-dimethylbenzamide | Calc'd 558.1, found 558.3 | A 1-8 |
| A-105 | 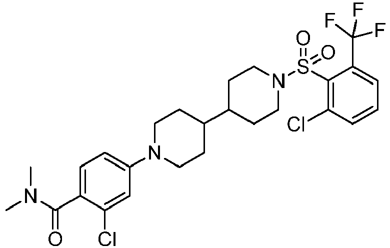 | 2-chloro-4-(1'-{[2-chloro-6-(trifluoromethyl)phen yl]sulfonyl}-4,4'-bipiperidin-1-yl)-N,N-dimethy lbenzamide | Calc'd 592.1, found 592.3 | A 1-8 |
| A106 | 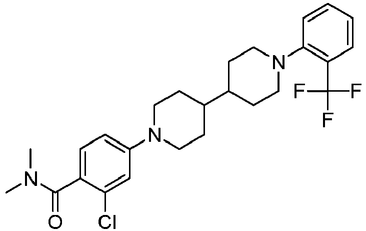 | 2-chloro-N,N-dimethyl-4-{1'-[2-(trifluoromethyl)phen yl]-4,4'-bipipendin-1-yl}benzamide | Calc'd 494.2, found 494.0 | A 1-9 |
| A-107 | 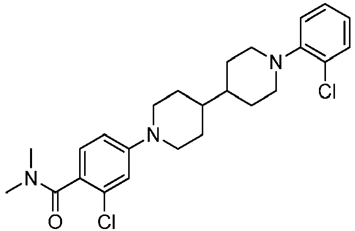 | 2-chloro-4-[1'-(2-, chlorophenyl)-4,4'-bipiperidin-1-yl]-N,N-dimethylbenzamide | Calc'd 460.2, found 460.3 | A 1-9 |
| A-108 | 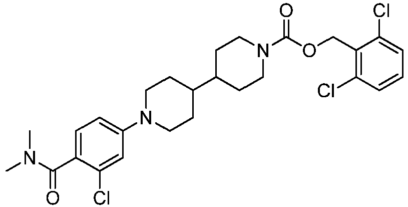 | 2,6-dichlorobenzyl 1'-[3-chloro-4-(dimethylcarbamoyl) phenyl]-4,4'-bipiperidine-1-carboxylate | Calc'd 552.2, found 552.3 | A 1-11 |
| A-109 | 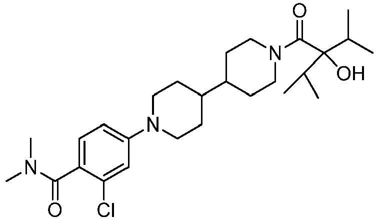 | 2-chloro-4-{1'-[2-hydroxy-3-methyl-2-(1-methylethyl)butanoyl ]-4,4'-bipiperidin-1-yl}-N,N-dimethylbenzamide | Calc'd 492.3, found 492.1 | A 1-12 |
| A-110 | 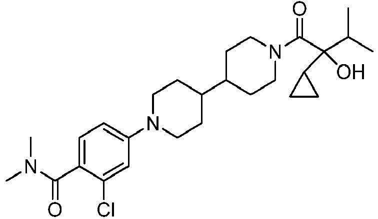 | 2-chloro-4-[1'-(2-cyclopropyl-2-hydroxy-3-methylbutanoyl)-4,4'-bipiperidin-1-yl]-N,N-dimethylbenzamide | Calc'd 490.3, found 490.1 | A 1-12 |

The following examples were prepared according to Scheme B: Examples wherein Q corresponds with -C(O)O- are not comprised in the appendant claims. They are reference examples and do not form part of the invention.

### Example B-2

### Preparation of 2-chloro-6-(1'-(2-hydroxy-2-isopropyl-3-methylbutanoyl)-4,4'-bipiperidin-1-yl)-N,N-dimethylnicotinamide (2-7)

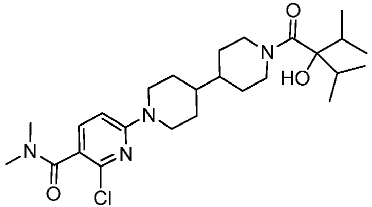

### Step 1: 2-chloro-6-hydroxy-N, N-dimethylnicotinamide (2-2)

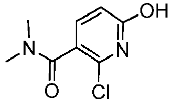

To a solution of 2-chloro-6-hydroxynicotinic acid (**2-1**) (1.0 g, 5.8 mmol,1.0 eq) in THF (20 mL) was added dimethylamine hydrochloride (1.42 g, 17.4 mmol,3 eq), HATU (2.64 g, 6.96 mmol,1.2 eq) and diisopropylethylamine (2.25 g, 17.4 mmol, 3 eq). After stirred at room temperature overnight, the reaction mixture was concentrated and purified by column chromatography (PE:EA=3:1 to 1:1) to give 2-chloro-6-hydroxy-N, N-dimethylnicotinamide (**2-2**). LRMS *m*/*z* (M+H) 201.01 found, 201.1 required.

### Step 2: 6-chloro-5-(dimethylcarbamoyl)pyridin-2-yl trifluoromethanesulfonate (2-3)

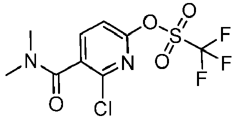

To a 250 ml three-necked round bottom flask equipped with mechanical stirrer and thermometer were charged sodium hydride (0.4g (60% in oil), 10 mmol, 2eq) under N₂. The solution of 2-chloro-6-hydroxy-N, N-dimethylnicotinamide (**2-2**) (1g, 5mmol, 1eq) in DMF (25ml) was added dropwise via syringe to the above sodium hydride at 0°C under N₂. After stirred at room temperature for 1 hour, a solution of 1,1,1-trifluoro-N-phenyl-N-(trifluoromethylsulfonyl) methanesulfonamide (2.7g, 7.5 mmol, 1.5eq) in DMF (25ml) was added via syringe over 5 min at 0° C. Then it was stirred at rt for 1 hour. The reaction was quenched by the addition of saturated ammonium chloride (50ml) at 0°C, The mixture was extracted with ethyl acetate (100 ml×3). The organic layer was combined and concentrated and the residue was purified by column chromatography (PE:EA=3:1) to give 6-chloro-5-(dimethylcarbamoyl)pyridin-2-yl trifluoromethanesulfonate (**2-3**). LRMS *m*/*z* (M+H) 332.9 found, 333.0 required.

### Step 3: tert-butyl 1'-(6-chloro-5-(dimethylcarbamoyl)pyridin-2-yl)-4,4'-bipiperidine-1-carboxylate (2-4) (Example B-1)

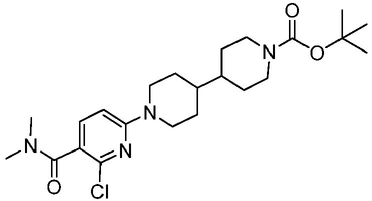

A mixture of tert-butyl 4,4'-bipiperidine-1-carboxylate(**1-2**) (268 mg, 1 mmol, 1.0 eq), 6-chloro-5-(dimethylcarbamoyl)pyridin-2-yl trifluoromethanesulfonate (**2-3**) (365 mg, 1.1 mmol, 1.1 eq)and DIPEA (387 mg, 3 mmol, 3 eq) in CH₃CN(5 ml) was stirred at 50°C for 2 hours. The reaction mixture was concentrated and the residue was diluted with ethyl acetate (80 mL) and washed with water (20 mL×3), brine (10 mL), dried over sodium sulfate and purified by column chromatography (PE:EA =5:1∼3:1) to give tert-butyl 1'-(6-chloro-5-(dimethylcarbamoyl)-pyridin-2-yl)-4,4'-bipiperidine-1-carboxylate (**2-4**). LRMS *m*/*z* (M+H) 451.2 found, 451.2 required.

### Step 4: 6-(4,4'-bipiperidin-1-yl)-2-chloro-N,N-dimethylnicotinamide (2-5)

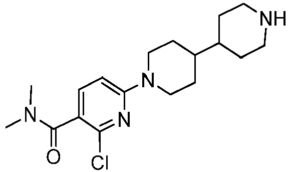

To a solution of tert-butyl 1'-(-(6-chloro-5-(dimethylcarbamoyl)pyridin-2-yl)-4,4'-bipiperidine-1-carboxylate (**2-4**) (350 mg, 0.78mmol, 1eq) in DCM (2.5 ml) was added TFA (2.5 ml) at 0°C. The mixture was stirred at room temperature for 2 hours. The solvent was evaporated and 30 ml of sat. NaHCO₃ was added to the residue. The desired product was extracted with DCM (20 ml x 3), washed with brine (20 mL) and dried over sodium sulfate to give 6-(4,4'-bipiperidin-1-yl)-2-chloro-N,N-dimethylnicotinamide (**2-5**). LRMS *m*/*z* (M+H) 351.2 found, 351.2 required.

### Step 5: 2-chloro-N,N-dimethyl-6-(1'-(3-methyl-2-oxobutanoyl)-4,4'-bipiperudin-1-yl)nicotinamide (2-6)

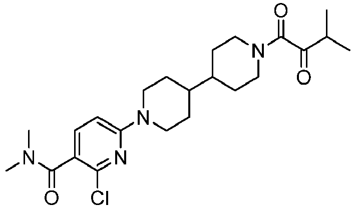

A mixture of calcium 3-methyl-2-oxobutanoate (30 mg, 0.113 mmol,1.2 eq), BOP (50 mg, 0.113 mmol,1.2 eq), DIPEA (36 mg, 0.282mmol,3 eq) and 6-(4,4'-bipiperidin-1-yl)-2-chloro-N,N-dimethylnicotinamide(**2-5**) (33 mg, 0.094 mmol, 1 eq) in dimethylformamide (2 ml) was stirred at room temperature for 3 hours. The resulting mixture was diluted with ethyl acetate (60 ml), washed with water (10ml×3), brine (10ml), dried over anhydrous sodium sulfate, concentrated and purified by prep-TLC (PE:EtOAc = 1:1) to give 2-chloro-N,N-dimethyl-6-(1'-(3-methyl-2-oxobutanoyl)-4,4'-bipiperidin-1-yl)nicotinamide(**2-6**). LRMS *m*/*z* (M+H) 449.2 found, 449.2 required.

### Step 6: 2-chloro-6-(1'-(2-hydroxy-2-isopropyl-3-methylbutanoyl)-4,4'-bipiperidin-1-yl)-N,N-dimethylnicotinamide (2-7)

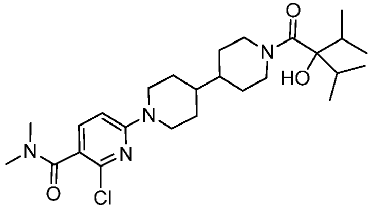

To a solution of 2-chloro-N,N-dimethyl-6-(1'-(3-methyl-2-oxobutanoyl)-4,4'-bipiperidin-1-yl)nicotinamide (**2-6**) (30 mg, 0.067 mmol, 1 eq) in THF (2 ml) at 0°C under N₂, isopropylmagnesium bromide (0.1 mL, 0.2 mmol, 3 eq) was added. After stirred at room temperature for 2 hours, the reaction was quenched by adding saturated ammonium chloride (2 ml). The resulting mixture was diluted with ethyl acetate (40 ml), washed with water (10 ml×3), brine (10 ml), dried over anhydrous sodium sulfate, concentrated and purified by Prep-TLC (PE:EtOAc=2:3) to give 2-chloro-6-(1'-(2-hydroxy-2-isopropyl-3-methylbutanoyl)-4,4'-bipiperidin-1-yl)-N,N-dimethylnicotinamide (**2-7**). LRMS *m*/*z* (M+H) 493.3 found, 493.3 required.

Examples reported in Table B can be prepared by the procedures described in Scheme B and the above examples from readily available starting materials and intermediates.

**TABLE B:**

| Example # | **Structure** | IUPAC Name | Exact Mass [M+H]+ | Scheme |
|---|---|---|---|---|
| B-1 | 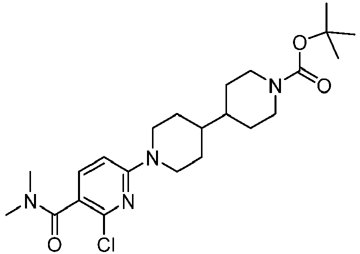 | tert-butyl 1'-[6-chloro-5-(dimethylcarbamoyl)pyri din-2-yl]-4,4'-bipiperidine-1-carboxylate | Calc'd 451.2, found 451.4 | B 2-4 |
| B-2 | 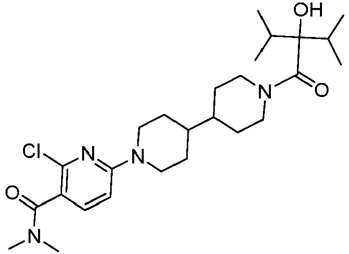 | 2-chloro-6-{1'-[2-hydroxy-3-methyl-2-(1-methylethyl)butanoyl]-4,4'-bipiperidin-1-yl}-N,N-dimethylpyridine-3-carboxamide | Calc'd 493.3, found 493.3 | B 2-7 |
| B-3 | 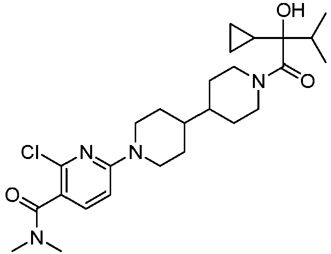 | 2-chloro-6-[1'-(2-cyclopropyl-2-hydroxy-3-methylbutanoyl)-4,4'-bipiperidin-1-yl]-N,N-dimethylpyridine-3-carboxamide | Calc'd 491.3, found 491.3 | B 2-7 |
| B-4 | 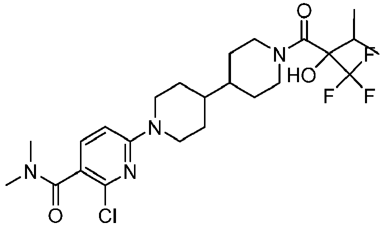 | 2-chloro-6-{1'-[2-hydroxy-3-methyl-2-(trifluoromethyl)butanoy l]-4,4'-bipiperidin-1-yl}-N,N-dimethylpyridine-3-carboxamide | Calc'd 519.2, found 519.1 | B 2-8 |
| B-5 | 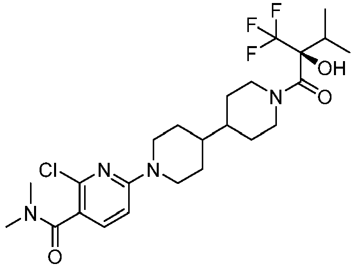 | 2-chloro-6-{1'-[(2R)-2-hydroxy-3-methyl-2-(trifluoromethyl)butanoy 1]-4,4'-bipiperidin-1-yl}-N,N-dimethylpyridine-3-carboxamide | Calc'd 519.2, found 519.5 | B 2-8 |
| B-6 | 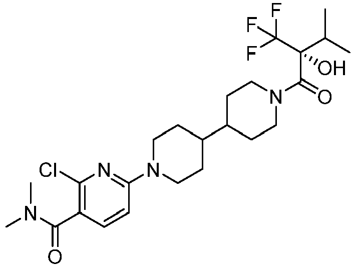 | 2-chloro-6-{1'-[(2S)-2-hydroxy-3-methyl-2-(trifluoromethyl)butanoy l]-4,4'-bipiperidin-1-yl}-N,N-dimethylpyridine-3-carboxamide | Calc'd 519.2, found 519.4 | B 2-8 |
| B-7 | 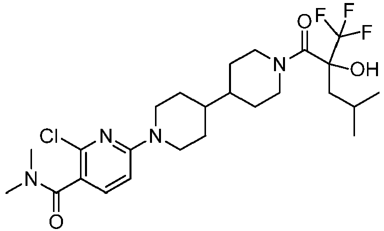 | 2-chloro-6-{1'-[2-hydroxy-4-methyl-2-(trifluoromethyl)pentano yl]-4,4'-bipiperidin-1-yl}-N,N-dimethylpyridine-3-carboxamide | Calc'd 533.3, found 533.3 | B 2-8 |
| B-8 | 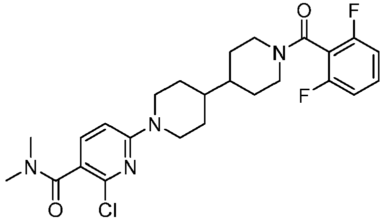 | 2-chloro-6-{1'-[(2,6-difluorophenyl)carbonyl] -4,4'-bipiperidin-1-yl}-N,N-dimethylpyridine-3-carboxamide | Calc'd 491.2, found 491.4 | B 2-9 |
| B-9 | 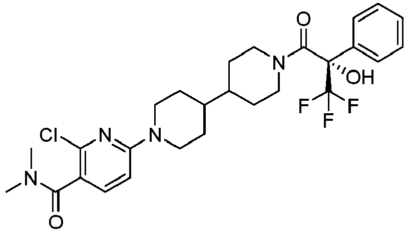 | 2-chloro-N,N-dimethyl-6-{1'-[(2R)-3,3,3-trifluoro-2-hydroxy-2-phenylpropanoyl]-4,4'-bipiperidin-1-yl}pyridine-3-carboxamide | Calc'd 553.2, found 553.1 | B 2-9 |
| B-10 | 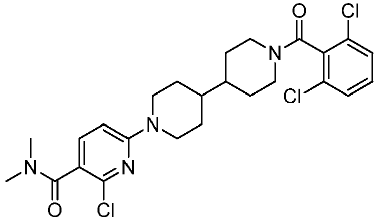 | 2-chloro-6-{1'-[(2,6-dichlorophenyl)carbonyl ]-4,4'-bipiperidin-1-yl}-N,N-dimethylpyridine-3-carboxamide | Calc'd 523.1, found 523.2 | B 2-9 |
| B-11 | 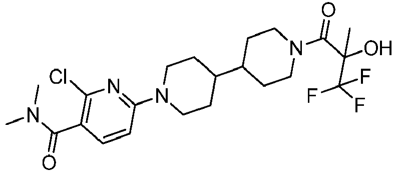 | 2-chloro-N,N-dimethyl-6-[1'-(3,3,3-trifuoro -2-hydroxy-2-methylpropanoyl)-4,4'-bipiperidin-1-yl]pyridine-3-carboxamide | Calc'd 491.2, found 491.3 | B 2-9 |
| B-12 | 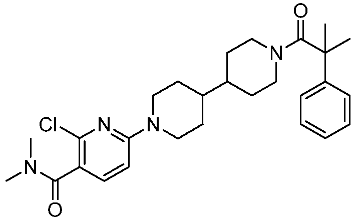 | 2-chloro-N,N-dimethyl-6-[1'-(2-methyl-2-phenylpropanoyl)-4,4'-bipiperidin-1-yl]pyridine-3-carboxamide | Calc'd 497.3, found 497.3 | B 2-9 |
| B-13 | 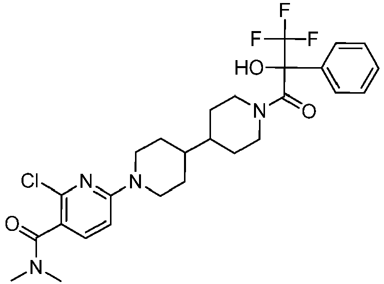 | 2-chloro-N,N-dimethyl-6-[1'-(3,3,3-trifluoro-2-hydroxy-2-phenylpropanoyl)-4,4'-bipiperidin-1-yl]pyridine-3-carboxamide | Calc'd 553.2, found 553.3 | B 2-9 |
| B-14 | 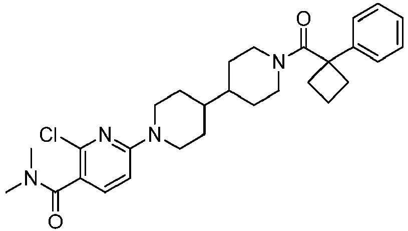 | 2-chloro-N,N-dimethyl-6-{1'-[(1-phenylcyclobutyl)carbon yl]-4,4'-bipiperidin-1-yl}pyridine-3-carboxamide | Calc'd 509.3, found 509.3 | B 2-9 |
| B-15 | 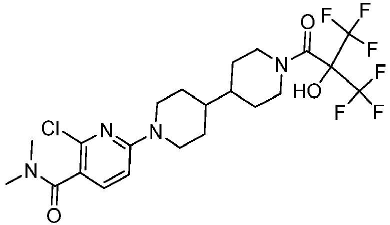 | 2-chloro-N,N-dimethyl-6-{1'-[3,3,3-trifluoro-2-hydroxy-2-(trifluoromethyl)propano yl]-4,4'-bipiperidin-1-yl}pyridine-3-carboxamide | Calc'd 545.2, found 545.2 | B 2-9 |
| B-16 | 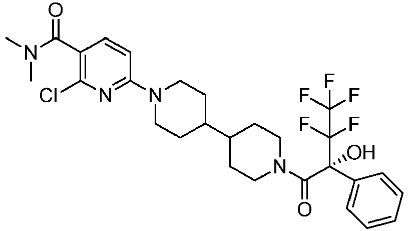 | 2-chloro-N,N-dimethyl-6-{1'-[(2R)-3,3,4,4,4-pentafluoro-2-hydroxy-2-phenylbutanoyl]-4,4'-bipiperidin-1-yl}pyridine-3-carboxamide | Calc'd 603.2, found 603.3 | B 2-9 |
| B-17 | 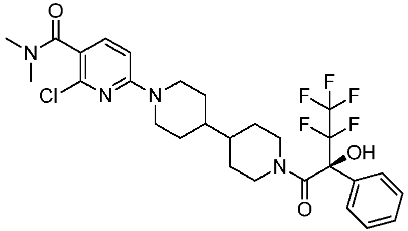 | 2-chloro-N,N-dimethyl-6-{1'-[(2S)-3,3,4,4,4-pentafluoro-2-hydroxy-2-phenylbutanoyl]-4,4'-bipiperidin-1-yl}pyridine-3-carboxamide | Calc'd 603.2, found 603.3 | B 2-9 |

The following example was prepared according to Scheme C:

### Example C-1

### Preparation of 6-(1'-(2-Hydroxy-3-methyl-2-(trifluoromethyl)butanoyl)-[4,4'-bipiperidin]-1-yl)-N,N,2-trimethylnicotinamide (3-6)

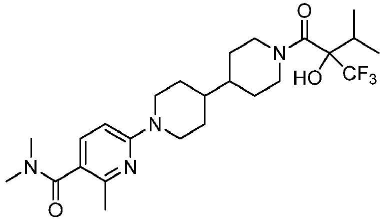

### Step 1: 6-Chloro-N,N,2-trimethylnicotinamide (3-2)

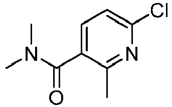

To a mixture of 6-chloro-2-methylnicotinic acid (**3-1**) (806 mg, 4.7 mmol), dimethylammonium chloride (498 mg, 6.11 mmol) and PyBOP (2.56 g, 4.93 mmol) in DCM (15 mL) is added DIEA (3.64 g, 28.2 mmol). Stir the reaction mixture at rt for 15 h and then partition between DCM and water. Layers are separated. Extract aqueous layer with DCM (1x). Dry combined organics over Na₂SO₄ and concentrate. Purify by silica gel flash chromatography using a linear gradient of 5 to 100% EtOAc in hexanes to give 6-chloro-*N*,*N,*2-trimethylnicotinamide (*3-2*). MS [M+H]⁺ 199.0 found, 199.1 required.

### Step 2: tert-Butyl 1'-(5-(dimethylcarbamoyl)-6-methylpyridin-2-yl)-[4,4'-bipiperidine]-1-carboxylate (3-3)

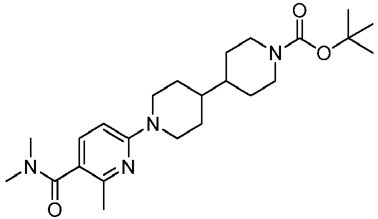

A mixture of 6-chloro-*N*,*N*,2-trimethylnicotinamide (**3-2**) (300 mg, 1.51 mmol), *tert*-butyl [4,4'-bipiperidine]-1-carboxylate (**1-2**) (397 mg, 1.48 mmol) and CS₂CO₃ (590 mg, 1.81 mmol) in *N-*methyl-2-pyrrolidone (2 mL) is heated at 120°C for 15 h, and then partition between EtOAc and water. Layers are separated. Extract aqueous layer with EtOAc (3x). Dry combined organics over Na₂SO₄ and concentrate. Purify by silica gel flash chromatography using a linear gradient of 5 to 100% EtOAc in hexanes to give tert-butyl 1'-(5-(dimethylcarbamoyl)-6-methylpyridin-2-yl)-[4,4'-bipiperidine]-1-carboxylate (**3-3**). MS [M+H]⁺431.1 found, 431.3 required.

### Step 3: 3-(Dimethylcarbamoyl)-2-methyl-6-(4-(piperidin-1-ium-4-yl)piperidin-1-yl)pyridin-1-ium chloride (3-4)

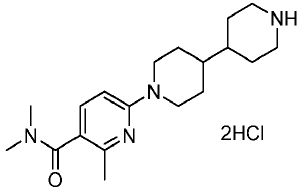

To a solution of tert-butyl 1'-(5-(dimethylcarbamoyl)-6-methylpyridin-2-yl)-[4,4'-bipiperidine]-1-carboxylate (**3-3**) (650 mg, 1.51 mmol) in MeOH (3 mL) is added a solution of HCl in dioxane (4 *N*, 3 mL) and stir at rt for 3 h. Concentrate to give 3-(dimethylcarbamoyl)-2-methyl-6-(4-(piperidin-1-ium-4-yl)piperidin-1-yl)pyridin-1-ium chloride (**3-4**). MS [M+H]⁺ 331.4 found, 331.3 required.

### Step 4: N,N,2-Trimethyl-6-(1'-(3-methyl-2-oxobutanoyl)-[4,4'-bipiperidin]-1-yl)nicotinamide (3-5)

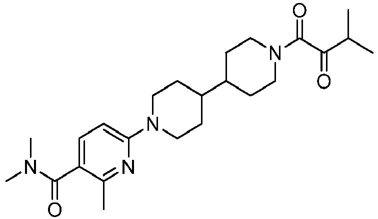

To a mixture of 3-(dimethylcarbamoyl)-2-methyl-6-(4-(piperidin-1-ium-4-yl)piperidin-1-yl)pyridin-1-ium chloride (**3-4**) (170 mg, 0.42 mmol) and DIEA (381 mg, 2.95 mmol) in DCM (6 mL) is added ketovaine sodium salt (87 mg, 0.63 mmol). Stir the reaction mixture at rt for 4 h and then partition between DCM and water. Extract aqueous layer with DCM (2x). Dry combined organics over Na₂SO₄ and concentrate. Purify by silica gel flash chromatography using a liner gradient of 80 to 100% EtOAc in hexanes to give *N*,*N*,2-trimethyl-6-(1'-(3-methyl-2-oxobutanoyl)-[4,4'-bipiperidin]-1-yl)nicotinamide (**3-5**). MS [M+H]⁺ 429.5 found, 429.3 required.

### Step 5: 6-(1'-(2-Hydroxy-3-methyl-2-(trifluoromethyl)butanoyl)-[4,4'-bipiperidin]-1-yl)-N,N,2-trimethylnicotinamide (3-6)

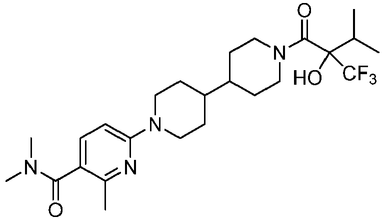

To a solution of *N*,*N*,2-trimethyl-6-(1'-(3-methyl-2-oxobutanoyl)-[4,4'-bipiperidin]-1-yl)nicotinamide (**3-5**) (200 mg, 0.467 mmol) in THF is added a solution of CF₃TMS in THF (0.5 M, 1.4 mL) followed by TBAF-solution in THF (1 M, 0.093 mL). After stirring at rt for 15 min neat CF₃TMS (0.3 mL, 2.03 mmol) is added followed by TBAF-solution in THF (1 M, 0.5 mL). Stir the reaction mixture for 10 min and then partition between water and EtOAc. Layers are separated. Extract aqueous layer with EtOAc (2x). Dry combined organics over Na₂SO₄ and concentrate. Purify by silica gel flash chromatography using a liner gradient of 0 to 5% MeOH in DCM to give a slightly impure product which is then purified by reverse phase HPLC (Sunfire C18 30 x 150 mm column) using 5 to 75% MeCN in water (0.1% TFA modifier for the solvents) over 15 min at 35 mL flow rate. Desired fractions are combined and made basic by adding saturated aqueous NaHCO₃ solution. Extract this basic aqueous solution with EtOAc (3x). Dry combined organics over Na₂SO₄ and concentrate to give 6-(1'-(2-Hydroxy-3-methyl-2-(trifluoromethyl)butanoyl)-[4,4'-bipiperidin]-1-yl)-*N*,*N*,2-trimethylnicotinamide (**3-6**). HRMS [M+H]⁺499.2895 found, 499.2896 required. ¹H NMR (400 MHz, DMSO-d₆) δ 7.3 (d, *J* = 8.6 Hz, 1H), 6.6 (d, *J* = 8.6 Hz, 1H), 6.46 (br, 1H), 5.1-4.4 (m, 2H), 4.43 (d, *J* = 13 Hz, 2H), 2.96-2.67 (m, 9 H), 2.46-2.41 (m, 1H), 2.21 (s, 3H), 1.82-1.66 (m, 4H), 1.41-0.81 (m, 13 H). Example C-1 was prepared by the procedures described in Scheme C above.

**TABLE C:**

| Example# | Structure | IUPAC Name | Exact Mass [M+H]+ | Scheme |
|---|---|---|---|---|
| C-1 | 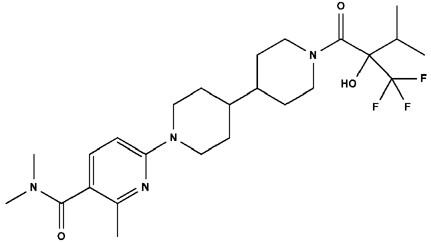 | 6-{1'-[2-hydroxy-3-methyl-2-(trifluoromethyl)buta noyl]-4,4'-bipiperidin-1-yl}-N,N,2-trimethylpyridine-3-carboxamide | Calc'd 499.3, found 499.3 | C 3-6 |

The following examples were prepared according to Scheme D: Examples wherein Q corresponds with -C(O)O- are not comprised in the appendant claims. They are reference examples and do not form part of the invention.

### Example D-3

### Preparation of 3-chloro-4-(1'-(2-hydroxy-3-methyl-2-(trifluoromethyl)butanoyl)-4,4'-bipiperidin-1-yl)-N,N-dimethylbenzamide (4-5)

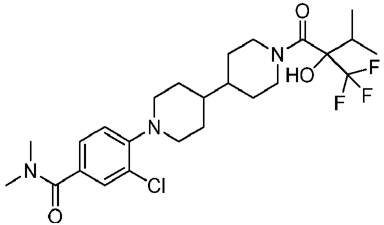

### Step 1: 4-bromo-3-chloro-N,N-dimethylbenzamide (4-2)

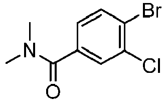

A mixture of 4-bromo-3-chlorobenzoic acid (**4-1**) (2.35 g, 10 mmol, 1.0 eq), oxalyl chloride (5 mL, 50 mmol, 5 eq) in dichloromethane (20 mL) was stirred for 2 hours at room temperature. The mixture was concentrated under reduced pressure to give white solid, which was redissolved in dichloromethane (10 mL). To the solution was added a solution of dimethylamine hydrochloride (1.63 g, 20 mmol, 2 eq) and triethylamine (3.1 g, 30 mmol, 3 eq) in dichloromethane (20 mL) at 0°C. The mixture was stirred at room temperature for 2 hours and followed by adding 50 mL DCM/MeOH (10:1), washed with saturated sodium bicarbonate (50 mL), ammonium chloride (50 mL), dried by sodium sulfate and concentrated to give 4-bromo-3-chloro-N,N-dimethylbenzamide (**4-2**). LRMS *m*/*z* (M+H) 262.0 found, 261.9 required.

### Step 2: tert-butyl 1'-(2-hydroxy-3-methyl-2-(trifluoromethyl)butanoyl)-4,4'-bipiperidine-1-carboxylate (4-3)

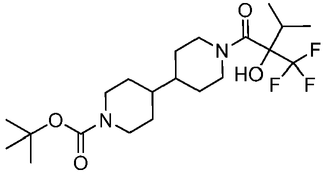

A mixture of tert-butyl 4,4'-bipiperidine-1-carboxylate (**1-2**) (107 mg, 0.4 mmol, 1 eq), 2-hydroxy-3-methyl-2-(trifluoromethyl)butanoic acid (159 mg, 0.8 mmol, 2 eq), BOP (212 mg, 0.48 mmol, 1.2 eq), DIPEA (155 mg, 1.2 mmol, 3 eq) in dimethylformamide (5 mL) was stirred at room temperature overnight. The resulting mixture was diluted with water (50 mL) and extracted with ethyl acetate (50 mL×3). The organic layer was combined, concentrated and purified on prep-HPLC to give tert-butyl 1'-(2-hydroxy-3-methyl-2-(trifluoromethyl)butanoyl)-4,4'-bipiperidine-1-carboxylate (**4-3**). LRMS *m*/*z* (M+Na) 459.4 found, 459.2 required.

### Step 3: 1-(4,4'-bipiperidin-1-yl)-2-hydroxy-3-methyl-2-(trifluoromethyl)butan-1-one (4-4)

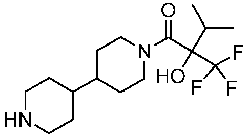

A mixture of tert-butyl 1'-(2-hydroxy-3-methyl-2-(trifluoromethyl)butanoyl)-4,4'-bipiperidine-1-carboxylate (**4-3**) (80 mg, 0.18 mmol, 1 eq) and trifluoroacetic acid (2 mL) in dichloromethane (5 mL) was stirred at room temperature for 2 hours. The solvent was evaporated under reduced pressure to give an oil, which was dissolved in dichloromethane (30 mL) and washed with water (30 mL). PH of the aqueous phase was adjusted to 8 with saturated sodium bicarbonate and desired product was extracted with chloroform/isopropanol (3:1) (20 mL×3). The combined organic layer was concentrated under reduced pressure to give 1-(4,4'-bipiperidin-1-yl)-2-hydroxy-3-methyl-2-(trifluoromethyl)butan-1-one (**4-4**). LRMS *m*/*z* (M+H) 337.2 found, 337.2 required.

### Step 4: 3-chloro-4-(1'-(2-hydroxy-3-methyl-2-(trifluoromethyl)butanoyl)-4,4'-bipiperidin-1-yl)-N,N-dimethylbenzamide (4-5)

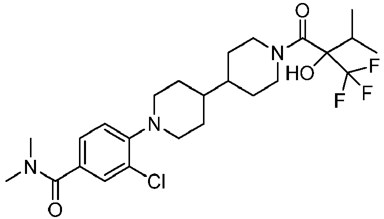

A mixture of 1-(4,4'-bipiperidin-1-yl)-2-hydroxy-3-methyl-2-(trifluoromethyl)butan-1-one (**4-4**) (55 mg, 0.26 mmol, 1 eq), 4-bromo-3-chloro-N,N-dimethylbenzamide (**4-2**) (66 mg, 0.25 mmol, 1.0 eq), Pd₂(dba)₃ (23 mg, 0.025 mmol, 0.1 eq), X-Phos (36 mg, 0.075 mmol, 0.3 eq), cesium carbonate (245 mg, 0.75 mmol, 3 eq) in 1,4-dioxane (3 mL) was stirred at 90°C under N₂ protection for 1.5 hours. The reaction mixture was filtered, concentrated and further purified on Prep-TLC (PE:EA=1:1) to give 3-chloro-4-(1'-(2-hydroxy-3-methyl-2-(trifluoromethyl)butanoyl)-4,4'-bipiperidin-1-yl)-N,N-dimethylbenzamide (**4-5**). ¹H NMR (400 MHz, CDCl₃): δ 7.45 (s, 1H) ; 7.29 (d, J = 8.4 Hz, 1 H); 7.01 (d, J=8.4Hz, 1 H); 4.72(m, 1H); 4.32(m, 1H); 3.46 (d, J=12Hz, 2H); 3.08(s, 3H); 3.02(s, 3H); 2.73 (m, 1H); 2.63 (t, J= 11.2Hz, 2H); 2.44(m, 1H); 1.91-1.79(m, 5H); 1.61-1.47(m, 3H); 1.25-1.11(m, 6H); 0.88-0.84(m, 3H). LRMS *m*/*z* (M+H) 518.3 found, 518.2 required.

### Examples D-7 & D-9

### Preparation of 2(R)-3-{1'[3-chloro-4-(methylsulfonyl)phenyl]-4-4'bipiperidin-1-yl}-1,1,1-trifluoro-3-oxo-2-phenylproropan-2-ol (4-6)

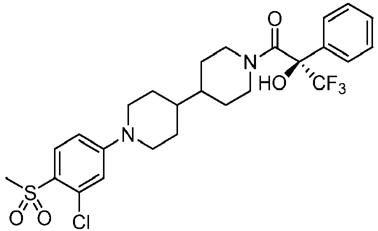

### Step 1: tert-butyl-1'-[3-chloro-4-(methylsulfonyl)phenyl]-4-4'-bipiperidine-1-carboxylate (4-7) Example D-9

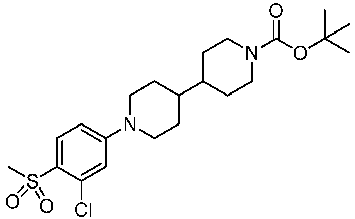

A mixture tert-butyl-[4,4'-bipiperidine]-1-carboxylate (**1-2**) (0.20 g, 0.76 mmol, 1.0 eq), 4-bromo-2-chloro-1-(methylsulfonyl) benzene (0.20 g, 0.76 mmol, 1 eq), sodium tert-butoxide (0.087 g, 0.91 mmol, 1.2 eq), palladium(II) acetate (0.017 g, 0.076 mmol, 0.1 eq) and 2-dicyclohexylphosphino-2'-(*N*,*N*-dimethylamino)biphenyl (0.089 g, 0.227 mmol, 0.3 eq) were suspended in toluene (3 mL) in sealed tube and heated to 80°C overnight. The mixture was quenched with water (10 mL) and extracted with ethyl acetate (2 x 15 mL). The organic phase was dried over MgSO4, filtered and concentrated under reduced pressure. The residue was purified on silica gel (Ethyl acetate/hexanes) and concentrated to give tert-butyl-l'-[3-chloro-4-(methylsulfonyl)phenyl]-4-4'-bipiperidine-1-carboxylate (**4-7**). LRMS *m*/*z* (M+H) 457.0 found, 457.0 required.

### Step 2: 2(R)-3-{1'[3-chloro-4-(methylsulfonyl)phenyl]-4-4'bipiperidin-1-yl}-1,1,1-trifluoro-3-oxo-2-phenylproropan-2-ol (4-6) Example D-7

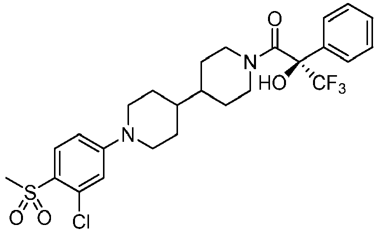

Tert-Butyl-1'-[3-chloro-4-(methylsulfonyl)phenyl]-4-4'-bipiperidine-1-carboxylate (**4-7**) (0.11 g, 0.24 mmol, 1 eq) was dissolved in 1:1 MeOH: EtOAc (15 mL) and cooled to 0°C. HCl gas was bubbled through the solution for 10 min. The reaction was warmed to RT and stirred for 30 min. The reaction was concentrated to give 1-(3-chloro-4-(methylsulfonyl)phenyl)-4,4'-bipiperidine bis hydrochloride (**4-8**) which was used without further purification.

Intermediate **4-8** (0.03 g, 0.07 mmol, 1 eq) was suspended in CH₂Cl₂. (R)-3,3,3-trifluoro-2-hydroxy-2-phenylpropanoic acid (0.018 g, 0.084 mmol, 1.2 eq), HBTU (0.032 g, 0.084 mmol, 1.2 eq), and DIPEA (0.061 mL, 0.35 mmol, 5 eq) were added and the reaction was stirred at room temperature overnight. The reaction was concentrated and purified by reverse phase HPLC. The fraction containing product were adjusted to pH 8 and extracted with EtOAc (3 x 10 mL) and concentrated to provide the title compound **4-6**. ¹H NMR (400 MHz, CDCl₃): δ 7.89 (d, J = 9.0 Hz, 1H), 7.42-7.26 (m, 5H), 6.86 (s, 1H), 6.75 (d, J = 9.0 Hz, 1H), 3.85 (d, J = 12.8 Hz, 2H), 3.20 (s, 3H), 2.81 (t, J = 11.9 Hz, 2H), 2.67-2.61 (m, 1H), 1.72-1.15 (m, 13H); LRMS *m*/*z* (M+H) 559.0 found, 559.2 required.

Examples reported in Table D can be prepared by the procedures described in Scheme D and the above examples from readily available starting materials and intermediate.

**TABLE D:**

| Example # | **Structure** | IUPAC Name | Exact Mass [M+H]+ | Scheme |
|---|---|---|---|---|
| D-1 | 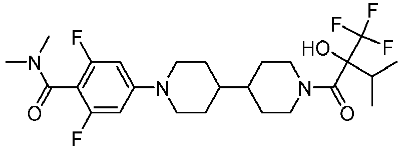 | 2,6-difluoro-4-{1'-[2-hydroxy-3-methyl-2-(trifluoromethyl)butano yl]-4,4'-bipiperidin-1-yl}-N,N-dimethylbenzamide | Calc'd 520.3, found 520.3 | D 4-5 |
| D-2 | 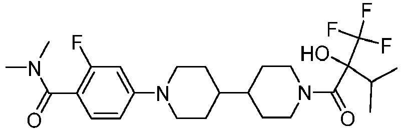 | 2-fluoro-4-{1'-[2-hydroxy-3-methyl-2-(trifluoromethyl)butano yl]-4,4'-bipiperidin-1-yl}-N,N-dimethylbenzamide | Calc'd 502.3, found 502.3 | D 4-5 |
| D-3 | 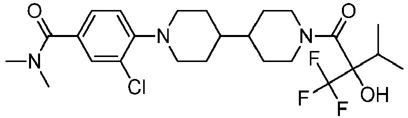 | 3-chloro-4-{1'-[2-hydroxy-3-methyl-2-(trifluoromethyl)butano yl]-4,4'-bipiperidin-1-yl}-N,N-dimethylbenzamide | Calc'd 518.2, found 518.3 | D 4-5 |
| D-4 | 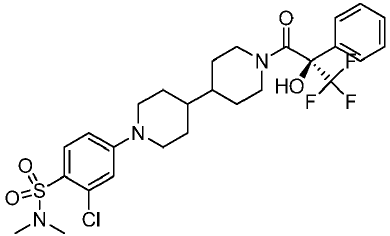 | 2-chloro-N,N-dimethyl-4-{1'-[(2R)-3,3,3-trifluoro-2-hydroxy-2-phenylpropanoyl]-4,4'-bipiperidin-1-yl}benzenesulfonamide | Calc'd 588.2, found 588.2 | D 4-6 |
| D-5 | 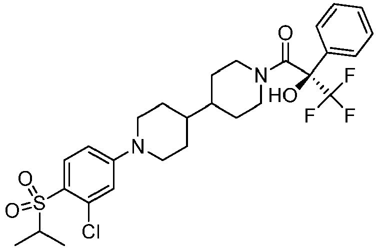 | (2R)-3-(1'-{3-chloro-4-[(1-methylethyl)sulfonyl]p henyl}-4,4'-bipiperidin-1-yl)-1,1,1-trifluoro-3-oxo-2-phenylpropan-2-ol | Calc'd 587.2, found 587.2 | D 4-6 |
| D-6 | 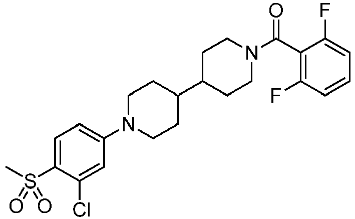 | 1-[3-chloro-4-(methylsulfonyl)phenyl ]-1'-[(2,6-difluorophenyl)carbony 1]-4,4'-bipiperidine | Calc'd 497.1, found 497.0 | D 4-6 |
| D-7 | 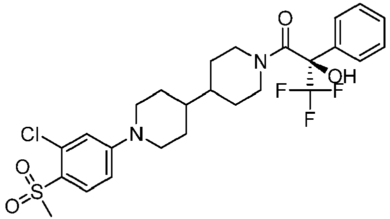 | (2R)-3-{1'-[3-chloro-4-(methylsulfonyl)phenyl ]-4,4'-bipiperidin-1-yl}-1,1,1-trifluoro-3-oxo-2-phenylpropan-2-ol | Calc'd 559.2, found 559.0 | D 4-6 |
| D-8 | 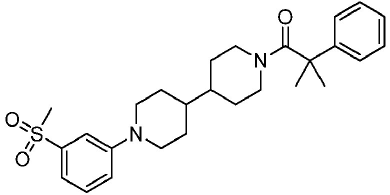 | 1-(2-methyl-2-phenylpropanoyl)-1'-[3-(methylsulfonyl)phenyl ]-4,4'-bipiperidine | Calc'd 469.3, found: 469.4 | D 4-6 |
| D-9 | 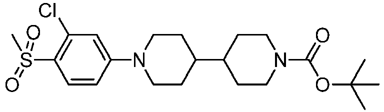 | tert-butyl 1'-[3-chloro-(methylsulfonyl)phenyl ]-4,4'-bipiperidine-1-carboxylate | Calc'd 457.2, found 457.0 | D 4-7 |

The following examples were prepared according to Scheme E: Examples wherein Q corresponds with -C(O)O- are not comprised in the appendant claims. They are reference examples and do not form part of the invention.

### Examples E-2 & E-16

### 2-chloro-4-(1'-(2,6-difluorobenzoyl)-4,4'-bipiperidin-1-yl)-N-(isoxazol-3-ylmethyl)-N-methylbenzamide (5-6) Example E-16

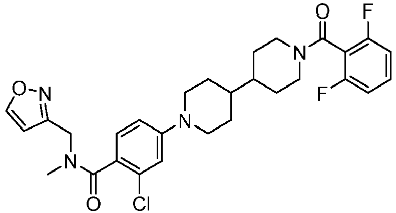

### Step 1: tert-butyl 1'-(3-chloro-4-(methoxycarbonyl)phenyl)-4,4'-bipiperidine-1-carboxylate (5-2)

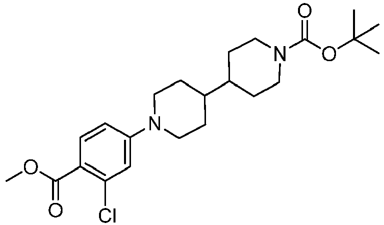

A mixture of tert-butyl 4,4'-bipiperidine-1-carboxylate (**1-2**) (268 mg, 1 mmol, 1.0 eq), methyl 2-chloro-4-fluorobenzoate (**5-1**) (226 mg, 1.2 mmol, 1.2 eq) and K₂CO₃ (414 mg, 3 mmol, 3 eq) in NMP (5 ml) was stirred at 120 °C for 3 h. After cooling to room temperature, the mixture was diluted with EtOAc (80 mL), washed with water (20 mL ×3), brine (10 mL), dried over sodium sulfate and purified by column chromatography (PE:EA =5:1∼3:1) to give tert-butyl 1'-(3-chloro-4-(methoxycarbonyl)phenyl)-4,4'-bipiperidine-1-carboxylate (**5-2**). LRMS *m*/*z* (M+Na) 459.2 found, 459.2 required.

### Step 2: methyl 4-(4,4'-bipiperidin-1-yl)-2-chlorobenzoate (5-3)

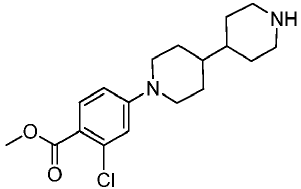

To a solution of tert-butyl 1'-(3-chloro-4-(methoxycarbonyl)phenyl)-4,4'-bipiperidine-1-carboxylate (**5-2**) (361 mg, 0.83 mmol) in DCM (3 ml) was added TFA (2 ml) at 0°C. The solution was stirred at room temperature for 2h. The solvent was evaporated and saturated sodium bicarbonate (30 ml) was added. The desired product was extracted with dicholormethane (20 ml x3) and the combined organic phase was washed with brine (20 mL), dried over sodium sulfate, concentrated to give methyl 4-(4,4'-bipiperidin-1-yl)-2-chlorobenzoate (**5-3**). LRMS *m*/*z* (M+H) 336.1 found, 336.2 required.

### Step 3: methyl 2-chloro-4-(1'-(2,6-difluorobenzoyl)-4,4'-bipiperidin-1-yl)benzoate (5-4) (Example E-2)

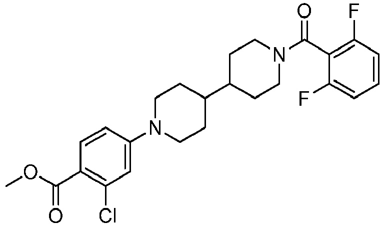

A mixture of 2,6-difluorobenzoic acid (190 mg, 1.2 mmol, 1.2 eq), HATU (460 mg, 1.2 mmol, 1.2 eq), DIPEA (260 mg, 2 mmol, 2 eq) and methyl 4-(4,4'-bipiperidin-1-yl)-2-chlorobenzoate (**5-3**) (340 mg, 1 mmol, 1 eq) in DMF (5 ml) was stirred at room temperature for 3 h. Then the mixture was diluted with ethyl acetate (60 ml), washed with water (10ml ×3), brine (10ml), dried over sodium sulfate, concentrated and purified by column chromatography (PE/EtOAc = 1/1) to give methyl 2-chloro-4-(1'-(2,6-difluorobenzoyl)-4,4'-bipiperidin-1-yl)benzoate (**5-4**). LRMS *m*/*z* (M+H) 477.1 found, 477.2 required.

### Step 4: 2-chloro-4-(1'-(2,6-difluorobenzoyl)-4,4'-bipiperidin-1-yl)benzoic acid (5-5)

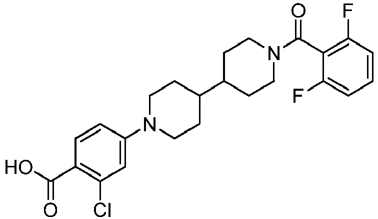

To a solution of methyl 2-chloro-4-(1'-(2,6-difluorobenzoyl)-4,4'-bipiperidin-1-yl)benzoate (**5-4**) (350 mg, 0.74 mmol, 1 eq) in THF (3.0 ml) and water (3.0 ml) was added lithium hydroxide monohydrate (154 mg, 3.7 mmol, 5 eq). The mixture was stirred at room temperature for 24 hours and monitored by LC-MS. The organic solvent was evaporated and the pH was adjusted to 2 with 2N HCl. The desired product was then extracted with ethyl acetate (30 ml x 3),washed with water (10 ml ×3) and brine (10 ml), dried over anhydrous sodium sulfate, concentrated to give 2-chloro-4-(1'-(2,6-difluorobenzoyl)-4,4'-bipiperidin-1-yl)benzoic acid (**5-5**). LRMS *m*/*z* (M+H) 463.1 found, 463.2 required.

### Step 5: 2-chloro-4-(1'-(2,6-difluorobenzoyl)-4,4'-bipiperidin-1-yl)-N-(isoxazol-3-ylmethyl)-N-methylbenzamide (5-6) Example E-16

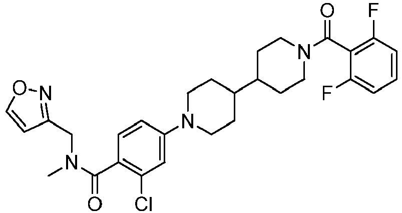

A mixture of 2-chloro-4-(1'-(2,6-difluorobenzoyl)-4,4'-bipiperidin-1-yl)benzoic acid (**5-5**) (23 mg, 0.05 mmol, 1 eq), HATU(23 mg, 0.06 mmol, 1.2 eq), DIPEA (13 mg, 0.1 mmol, 2 eq) and 1-(isoxazol-3-yl)-N-methylmethanamine (7 mg, 0.06 mmol, 1.2 eq) in DMF (1 ml) was stirred at room temperature for 3 h. The resulting mixture was diluted with ethyl acetate (40 ml), washed with water (8 ml ×3) and brine (10 ml), dried over anhydrous sodium sulfate, concentrated and purified by Prep-TLC (PE:EtOAc = 1:1) to give 2-chloro-4-(1'-(2,6-difluorobenzoyl)-4,4'-bipiperidin-1-yl)-N-(isoxazol-3-ylmethyl)-N-methylbenzamide (**5-6**). ¹H NMR (400 MHz, CDCl₃): δ 8.39(s, 1H); 7.34(m, 1H); 7.17(t, J=8 Hz, 1H); 6.95(t, J=8Hz, 2H); 6.88-6.81 (m, 2H); 6.52(s, 1H); 4.85(d, J=13.2Hz, 2H); 3.75(d, J=12Hz, 2H); 3.56(d, J=13.2Hz, 1H); 3.11-2.98(m, 2H); 2.85(s, 2H); 2.8-2.62 (m, 3H); 1.93-1.57(m, 5H); 1.47-1.15(m, 6H). LRMS *m*/*z* (M+H) 557.2 found, 557.2 required

### Example E-69

### Preparation of tert-butyl 1'-(3-chloro-4-(2-hydroxypropan-2-yl)phenyl)-[4,4'-bipiperidine]-1-carboxylate (5-10)

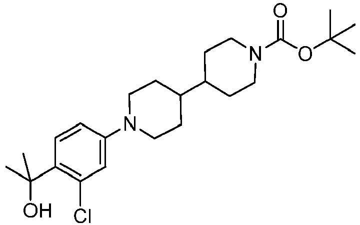

To a solution of *tert*-butyl 1'-(3-chloro-4-(methoxycarbonyl)phenyl)-[4,4'-bipiperidine]-1-carboxylate (147 mg, 0.336 mmol) in THF (2.5 mL) was added a solution of methylmagnesium bromide in THF (3 M, 0.336 mL) at rt. After stirring at rt for 1 h, 0.4 mL more of methylmagnesium bromide solution was added to the reaction mixture and allowed to stir for 30 min. The reaction was then quenched with water and partitioned between EtOAc and water. Layers were separated. Organic layer was dried over Na₂SO₄, concentrated and purified by flash chromatography (24 g SiO₂) using a linear gradient of 3 to 50% EtOAc in hexanes to give the title compound **5-10**. ¹H NMR (400 MHz, CDCl₃) δ 7.45 (d, *J* = 8.8 Hz, 1H), 6.89 (d, *J* = 2.6 1H), 6.79-6.76 (m, 1H), 4.13 (br, 2H), 3.71-3.68 (m, 2H), 2.68-2.56 (m, 4H), 1.8-1.78 (m, 2H), 1.71-1.68 (m, 8H), 1.46 (s, 9H), 1.42-1.1 (m, 7H). HRMS [M+H]⁺ 437.2584 found, 437.2565 required.

Examples reported in Table E can be prepared by the procedures described in Scheme E and the above examples from readily available starting materials and intermediates.

**TABLE E:**

| Example # | **Structure** | IUPAC Name | Exact Mass [M+H]+ | Scheme |
|---|---|---|---|---|
| E-1 | 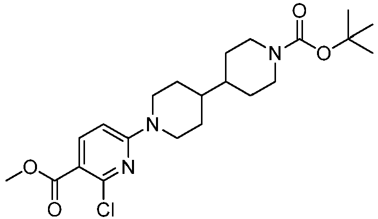 | tert-butyl 1'-[6-chloro-5-(methoxycarbonyl) pyridin-2-yl]-4,4'-bipiperidine-1-carboxylate | Calc'd 438.2, found 438.4 | E 5-2 |
| E-2 | 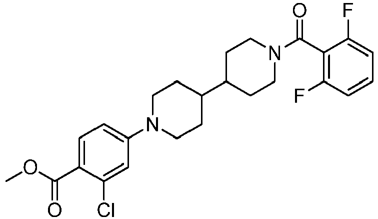 | methyl 2-chloro-4-{1'-[(2,6-difluorophenyl)carbonyl]-4,4'-bipiperidin-1-yl}benzoate | Calc'd 477.2, found 477.2 | E 5-4 |
| E-3 | 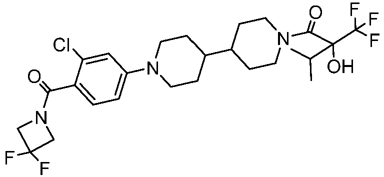 | 2-[(1'-{3-chloro-4-[(3,3-difluoroazetidin-1-yl)carbonyl]phenyl} -4,4'-bipiperidin-1-yl)carbonyl]-1,1,1-trifluoro-3-methylbutan-2-ol | Calc'd 566.2, found 566.2 | E 5-6 |
| E-4 | 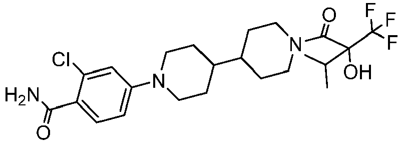 | 2-chloro-4-{1'-[2-hydroxy-3-methyl-2-(trifluoromethyl)but anoyl]-4,4'-bipiperidin-1-yl}benzamide | Calc'd 490.2, found 490.2 | E 5-6 |
| E-5 | 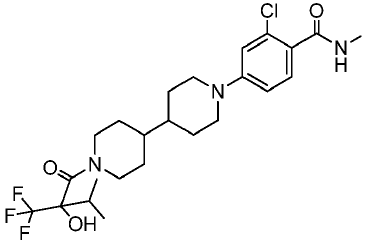 | 2-chloro-4-{1'-[2-hydroxy-3-methyl-(trifluoromethyl)but anoyl]-4,4'-bipiperidin-1-yl}-N-methylbenzamide | Calc'd 504.2, found 504.2 | E 5-6 |
| E-6 | 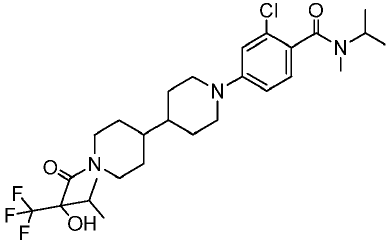 | 2-chloro-4-{1'-[2-hydroxy-3-methyl-2-(trifluoromethyl)but anoyl]-4,4'-bipiperidin-1-yl}-N-methyl-N-(1-methylethyl)benzam ide | Calc'd 546.3, found 546.3 | E 5-6 |
| E-7 | 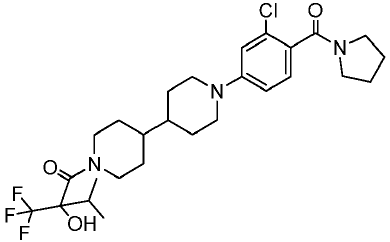 | 2-({1'-[3-chloro-4-(pyrrolidin-1-ylcarbonyl)phenyl]-4,4'-bipiperidin-1-yl}carbonyl)-1,1,1-trifluoro-3-methylbutan-2-ol | Calc'd 544.3, found 544.3 | E 5-6 |
| E-8 | 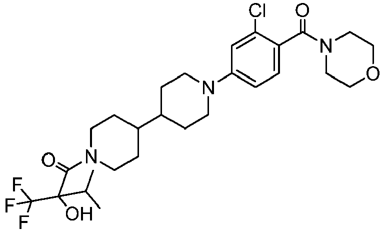 | 2-({1'-[3-chloro-4-(morpholin-4-ylcarbonyl)phenyl]-4,4'-bipiperidin-1-yl}carbonyl)-1,1,1-trifluoro-3-methylbutan-2-ol | Calc'd 560.2 , found 560.3 | E 5-6 |
| E-9 | 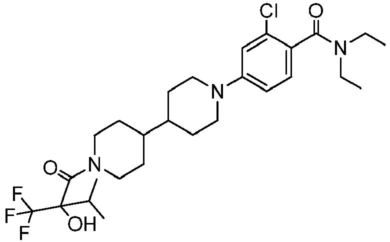 | 2-chloro-N,N-diethyl-4-{1'-[2-hydroxy-3-methyl-2-(trifluoromethyl)but anoyl]-4,4'-bipiperidin-1-yl}benzamide | Calc'd 546.3, found 546.3 | E 5-6 |
| E-10 | 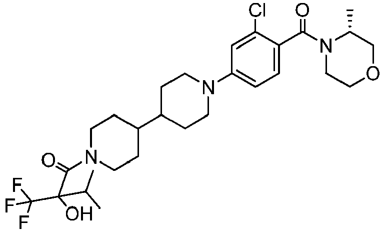 | 2-{[1'-(3-chloro-4-{[(3R)-3-methylmorpholin-4-yl]carbonyl}phenyl) -4,4'-bipiperidin-1-yl]carbonyl}-1,1,1-trifluoro-3-methylbutan-2-ol | Calc'd 574.3, found 574.3 | E 5-6 |
| E-11 | 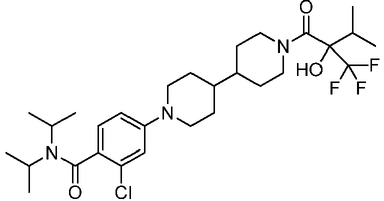 | 2-chloro-4-{1'-[2-hydroxy-3-methyl-2-(trifluoromethyl)but anoyl]-4,4'-bipiperidin-1-yl}-N,N-bis(1-methylethyl)benzam ide | Calc'd 574.3, found 574.3 | E 5-6 |
| E-12 | 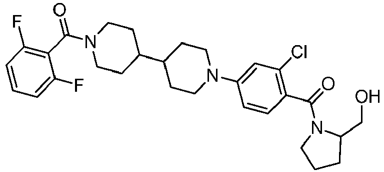 | {1-[(2-chloro-4-{1'-[(2,6-difluorophenyl)carb onyl]-4,4'-bipiperidin-1-yl}phenyl)carbonyl] pyrrolidin-2-yl}methanol | Calc'd 546.2, found 546.0 | E 5-6 |
| E-13 | 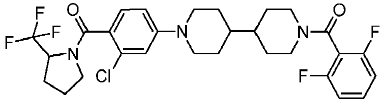 | 1-(3-chloro-4-{[2-(trifluoromethyl)pyr rolidin-1-yl]carbonyl}phenyl) -1'-[(2,6-difluorophenyl)carb onyl]-4,4'-bipiperidine | Calc'd 584.2, found 584.3 | E 5-6 |
| E-14 | 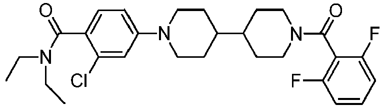 | 2-chloro-4-{1'-[(2,6-difluorophenyl)carb onyl]-4,4'-bipiperidin-1-yl}-N,N-diethylbenzamide | Calc'd 518.2, found 518.3 | E 5-6 |
| E-15 | 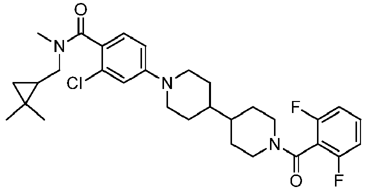 | 2-chloro-4-{1'-[(2,6-difluorophenyl)carb onyl]-4,4'-bipiperidin-1-yl}-N-[(2,2-dimethylcyclopropyl )methyl]-N-methylbenzamide | Calc'd 558.3, found 558.3 | E 5-6 |
| E-16 | 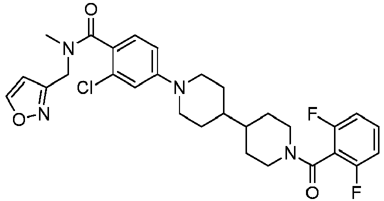 | 2-chloro-4-{1'-[(2,6-difluorophenyl)carb onyl]-4,4'-bipiperidin-1-yl}-N-(isoxazol-3-ylmethyl)-N-methylbenzamide | Calc'd 557.2 , found 557.3 | E 5-6 |
| E-17 | 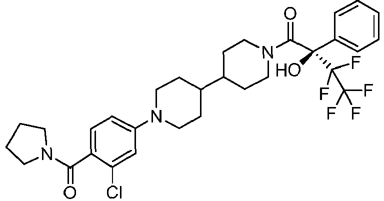 | (2R)-1-{1'-[3-chloro-4-(pyrrolidin-1-ylcarbonyl)phenyl]-4,4'-bipiperidin-1-yl}-3,3,4,4,4-pentafluoro-1-oxo-2-phenylbutan-2-ol | Calc'd 628.2, found 628.24 | E 5-6 (chirali ty not confir med) |
| E-18 | 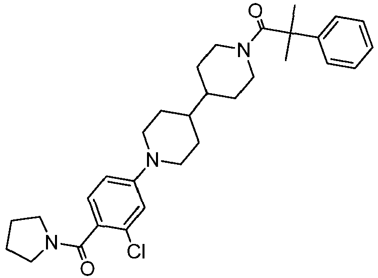 | 1-[3-chloro-4-(pyrrolidin-1-ylcarbonyl)phenyl]-1'-(2-methyl-2-phenylpropanoyl)-4,4'-bipiperidine | Calc'd 522.3, found 522.2 | E 5-6 |
| E-19 | 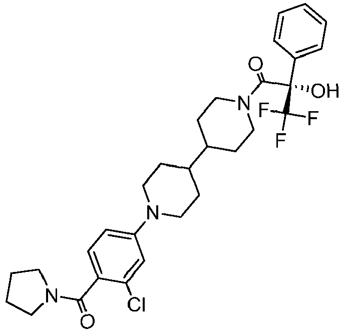 | (2S)-3-{1'-[3-chloro-4-(pyrrolidin-1-ylcarbonyl)phenyl]-4,4'-bipiperidin-1-yl}-1,1,1-trifluoro-3-oxo-2-phenylpropan-2-ol | Calc'd 578.2, found 578.2 | E 5-6 |
| E-20 | 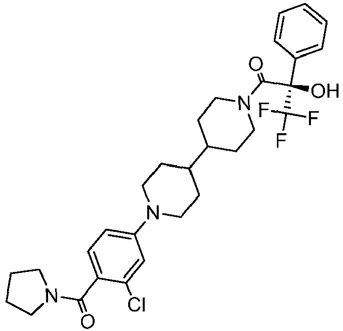 | (2R)-3-{1'-[3-chloro-4-(pyrrolidin-1-ylcarbonyl)phenyl]-4,4'-bipiperidin-1-yl}-1,1,1-trifluoro-3-oxo-2-phenylpropan-2-ol | Calc'd 578.2, found 578.2 | E 5-6 |
| E-21 | 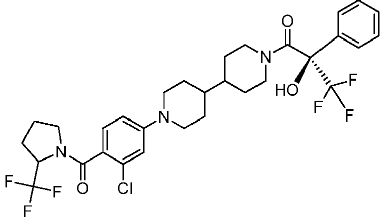 | (2R)-3-[1'-(3-chloro-4-{[2-(trifluoromethyl)pyr rolidin-1-yl]carbonyl}phenyl) -4,4'-bipiperidin-1-yl]-1,1,1-trifluoro-3-oxo-2-phenylpropan-2-ol | Calc'd 646.2, found 646.4 | E 5-6 |
| E-22 | 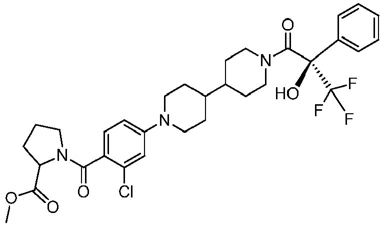 | methyl 1-[(2-chloro-4-{1'-[(2R)-3,3,3-trifluoro-2-hydroxy-2-phenylpropanoyl]-4,4'-bipiperidin-1-yl}phenyl)carbonyl] prolinate | Calc'd 636.2, found 636.4 | E 5-6 |
| E-23 | 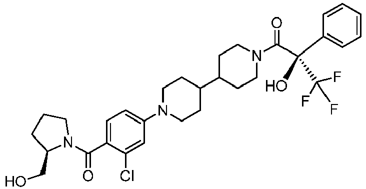 | (2R)-3-[1'-(3-chloro-4-{[(2R)-2-(hydroxymethyl)pyr rolidin-1-yl]carbonyl}phenyl) -4,4'-bipiperidin-1-yl]-1,1,1-trifluoro-3-oxo-2-phenylpropan-2-ol | Calc'd 608.2, found 608.4 | E 5-6 |
| E-24 | 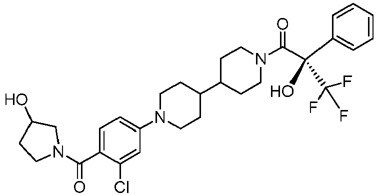 | 1-[(2-cliloro-4-{1'-[(2R)-3,3,3-trifluoro-2-hydroxy-2-phenylpropanoyl]-4,4'-bipiperidin-1-yl}phenyl)carbonyl] pyrrolidin-3-ol | Calc'd 594.2, found 594.4 | E 5-6 |
| E-25 | 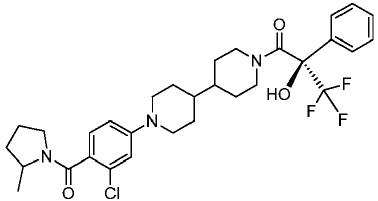 | (2R)-3-(1'-{3-chloro-4-[(2-methylpyrrolidin-1-yl)carbonyl]phenyl} -4,4'-bipiperidin-1-yl)-1,1,1-trifluoro-3-oxo-2-phenylpropan-2-ol | Calc'd 592.3, found 592.4 | E 5-6 |
| E-26 | 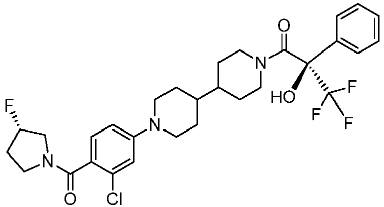 | (2R)-3-[1'-(3-chloro-4-{[(3S)-3-fluoropyrrolidin-1-yl]carbonyl}phenyl) -4,4'-bipiperidin-1-yl]-1,1,1-trifluoro-3-oxo-2-phenylpropan-2-ol | Calc'd 596.2, found 596.4 | E 5-6 |
| E-27 | 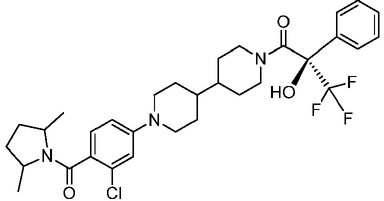 | (2R)-3-(1'-{3-chloro-4-[(2,5-dimethylpyrrolidin-1-yl)carbonyl]phenyl} -4,4'-bipiperidin-1-yl)-1,1,1-trifluoro-3-oxo-2-phenylpropan-2-ol | Calc'd 606.3, found | E 5-6 |
| E-28 | 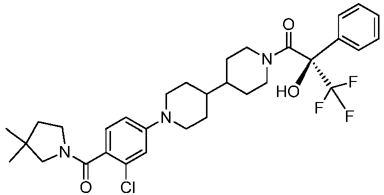 | (2R)-3-(1'-{3-chloro-4-[(3,3-dimethylpyrrolidin-yl)carbonyl]phenyl} -4,4'-bipiperidin-1-yl)-1,1,1-trifluoro-3-oxo-2-phenylpropan-2-ol | Calc'd 606.3, found 606.4 | E 5-6 |
| E-29 | 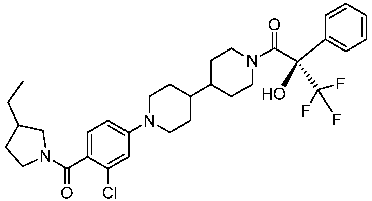 | (2R)-3-(1'-{3-chloro-4-[(3-ethylpyrrolidin-1-yl)carbonyl]phenyl} -4,4'-bipiperidin-1-yl)-1,1,1-trifluoro-3-oxo-2-phenylpropan-2-ol | Calc'd 606.3, found 606.4 | E 5-6 |
| E-30 | 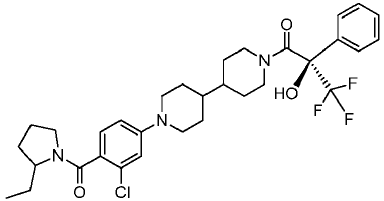 | (2R)-3-(1'-{3-chloro-4-[(2-ethylpyrrolidin-1-yl)carbonyl]phenyl} -4,4'-bipiperidin-1-yl)-1,1,1-trifluoro-3-oxo-2-phenylpropan-2-ol | Calc'd 606.3, found 606.4 | E 5-6 |
| E-31 | 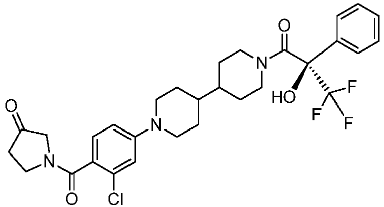 | 1-[(2-cliloro-4-{1'-[(2R)-3,3,3-trifluoro-2-hydroxy-2-phenylpropanoyl]-4,4'-bipiperidin-1-yl}phenyl)carbonyl] pyrrolidin-3-one | Calc'd 592.2, found 592.4 | E 5-6 |
| E-32 | 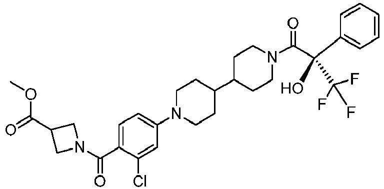 | methyl 1-[(2-chloro-4-{1'-[(2R)-3,3,3-trifluoro-2-hydroxy-2-phenylpropanoyl]-4,4'-bipiperidin-1-yl}phenyl)carbonyl] azetidine-3-carboxylate | Calc'd 622.2, found 622.4 | E 5-6 |
| E-33 | 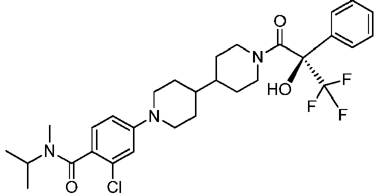 | 2-chloro-N-methyl-N-(1-methylethyl)-4-{1'-[(2R)-3,3,3-trifluoro-2-hydroxy-2-phenylpropanoyl]-4,4'-bipiperidin-1-yl}benzamide | Calc'd 580.3, found 580.4 | E 5-6 |
| E-34 | 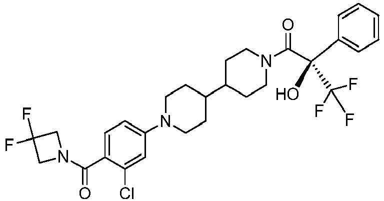 | (2R)-3-(1'-{3-chloro-4-[(3,3-difluoroazetidin-1-yl)carbonyl]phenyl} -4,4'-bipiperidin-1-yl)-1,1,1-trifluoro-3-oxo-2-phenylpropan-2-ol | Calc'd 600.2, found 600.4 | E 5-6 |
| E-35 | 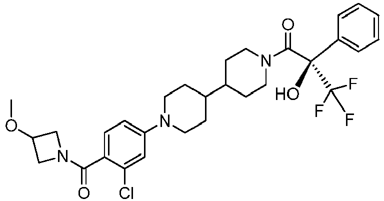 | (2R)-3-(1'-{3-chloro-4-[(3-methoxyazetidin-1-yl)carbonyl]phenyl} -4,4'-bipiperidin-1-yl)-1,1,1-trifluoro-3-oxo-2-phenylpropan-2-ol | Calc'd 594.2, found 594.4 | E 5-6 |
| E-36 | 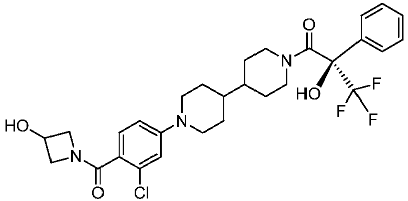 | 1-[(2-cliloro-4-{1'-[(2R)-3,3,3-trifluoro-2-hydroxy-2-phenylpropanoyl]-4,4'-bipiperidin-1-yl}phenyl)carbonyl] azetidin-3-ol | Calc'd 580.2, found 580.4 | E 5-6 |
| E-37 | 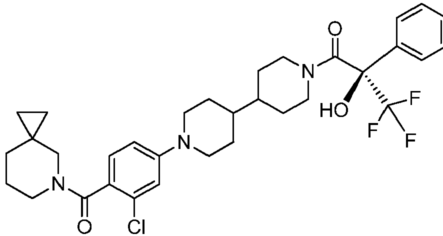 | (2R)-3-{1'-[4-(5-azaspiro[2.5]oct-5-ylcarbonyl)-3-chlorophenyl]-4,4'-bipiperidin-1-yl}-1,1,1-trifluoro-3-oxo-2-phenylpropan-2-ol | Calc'd 618.3, found 618.4 | E 5-6 |
| E-38 | 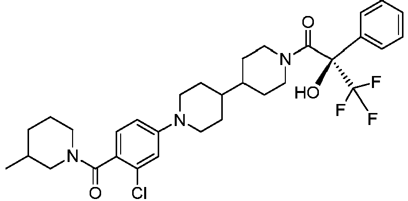 | (2R)-3-(1'-{3-chloro-4-[(3-methylpiperidin-1-yl)carbonyl]phenyl} -4,4'-bipiperidin-1-yl)-1,1,1-trifluoro-3-oxo-2-phenylpropan-2-ol | Calc'd 606.3, found 606.4 | E 5-6 |
| E-39 | 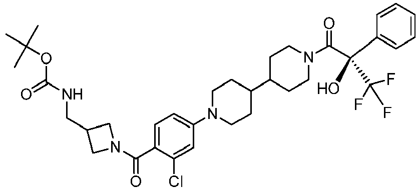 | tert-butyl ({1-[(2-chloro-4-{1'-[(2R)-3,3,3-trifluoro-2-hydroxy-2-phenylpropanoyl]-4,4'-bipiperidin-1-yl}phenyl)carbonyl] azetidin-3-yl}methyl)carbamat e | Calc'd 693.3, found 693.2 | E 5-6 |
| E-40 | 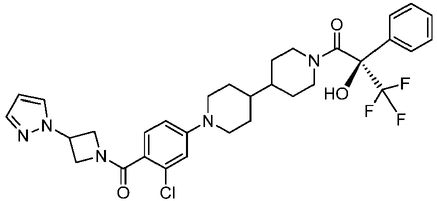 | (2R)-3-[1'-(3-chloro-4-{[3-(1H-pyrazol-1-yl)azetidin-1-yl]carbonyl}phenyl) -4,4'-bipiperidin-1-yl]-1,1,1-trifluoro-3-oxo-2-phenylpropan-2-ol | Calc'd 630.2, found 630.2 | E 5-6 |
| E-41 | 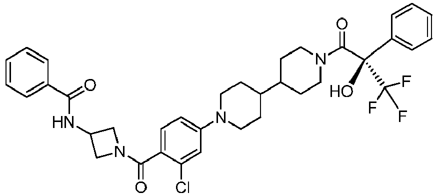 | N-{ 1-[(2-chloro-4-{1'-[(2R)-3,3,3-trifluoro-2-hydroxy-2-phenylpropanoyl]-4,4'-bipiperidin-1-yl}phenyl)carbonyl] azetidin-3-yl}benzamide | Calc'd 683.3, found 683.2 | E 5-6 |
| E-42 | 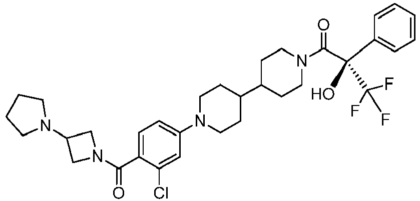 | (2R)-3-(1'-{3-chloro-4-[(3-pyrrolidin-1-ylazetidin-1-yl)carbonyl]phenyl} -4,4'-bipiperidin-1-yl)-1,1,1-trifluoro-3-oxo-2-phenylpropan-2-ol | Calc'd 633.3, found 633.2 | E 5-6 |
| E-43 | 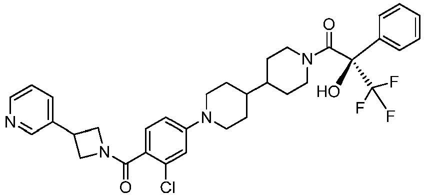 | (2R)-3-(1'-{3-chloro-4-[(3-pyridin-3-ylazetidin-1-yl)carbonyl]phenyl} -4,4'-bipiperidin-1-yl)-1,1,1-trifluoro-3-oxo-2-phenylpropan-2-ol | Calc'd 641.3, found 641.2 | E 5-6 |
| E-44 | 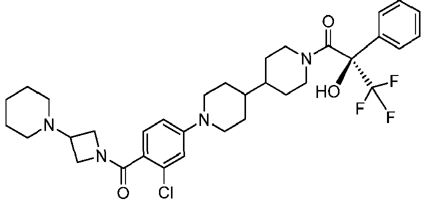 | (2R)-3-(1'-{3-chloro-4-[(3-piperidin-1-ylazetidin-1-yl)carbonyl]phenyl} -4,4'-bipiperidin-1-yl)-1,1,1-trifluoro-3-oxo-2-phenylpropan-2-ol | Calc'd 647.3, found 647.2 | E 5-6 |
| E-45 | 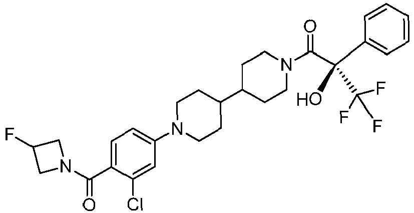 | (2R)-3-(1'-{3-chloro-4-[(3-fluoroazetidin-1-yl)carbonyl]phenyl} -4,4'-bipiperidin-1-yl)-1,1,1-trifluoro-3-oxo-2-phenylpropan-2-ol | Calc'd 582.2, found 582.1 | E 5-6 |
| E-46 | 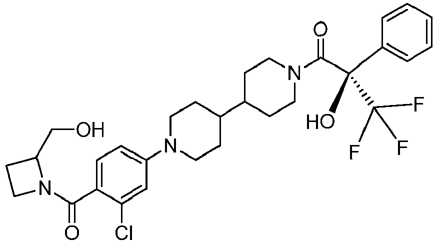 | (2R)-3-[1'-(3-chloro-4-{[2-(hydroxymethyl)aze tidin-1-yl]carbonyl}phenyl) -4,4'-bipiperidin-1-yl]-1,1,1-trifluoro-3-oxo-2-phenylpropan-2-ol | Calc'd 594.2, found 594.4 | E 5-6 |
| E-47 | 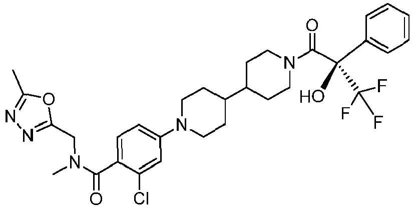 | 2-chloro-N-methyl-N-[(5-methyl-1,3,4-oxadiazol-2-yl)methyl]-4-{ 1'-[(2R)-3,3,3-trifluoro-2-hydroxy-2-phenylpropanoyl]-4,4'-bipiperidin-1-yl}benzamide | Calc'd 634.2, found 634.1 | E 5-6 |
| E-48 | 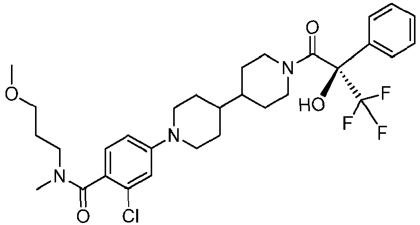 | 2-chloro-N-(3-methoxypropyl)-N-methyl-4-{1'-[(2R)-3,3,3-trifluoro-2-hydroxy-2-phenylpropanoyl]-4,4'-bipiperidin-1-yl}benzamide | Calc'd 610.3, found 610.2 | E 5-6 |
| E-49 | 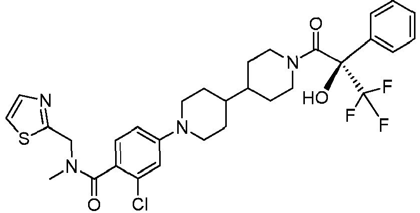 | 2-chloro-N-methyl-N-(1,3-thiazol-2-ylmethyl)-4-{ 1'-[(2R)-3,3,3-trifluoro-2-hydroxy-2-phenylpropanoyl]-4,4'-bipiperidin-1-yl}benzamide | Calc'd 635.2, found 635.1 | E 5-6 |
| E-50 | 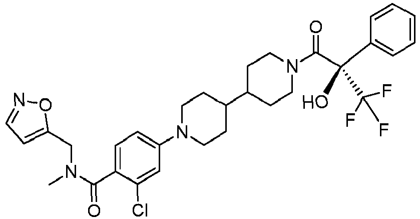 | 2-chloro-N-(isoxazol-5-ylmethyl)-N-methyl-4-{1'-[(2R)-3,3,3-trifluoro-2-hydroxy-2-phenylpropanoyl]-4,4'-bipiperidin-1-yl}benzamide | Calc'd 619.2, found 619.1 | E 5-6 |
| E-51 | 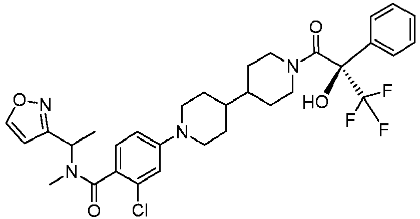 | 2-chloro-N-(1-isoxazol-3-ylethyl)-N-methyl-4-{ 1'-[(2R)-3,3,3-trifluoro-2-hydroxy-2-phenylpropanoyl]-4,4'-bipiperidin-1-yl}benzamide | Calc'd 633.2, found 633.1 | E 5-6 |
| E-52 | 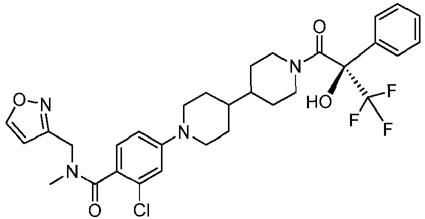 | 2-chloro-N-(isoxazol-3-ylmethyl)-N-methyl-4-{1'-[(2R)-3,3,3-trifluoro-2-hydroxy-2-phenylpropanoyl]-4,4'-bipiperidin-1-yl}benzamide | Calc'd 619.2, found 619.1 | E 5-6 |
| E-53 | 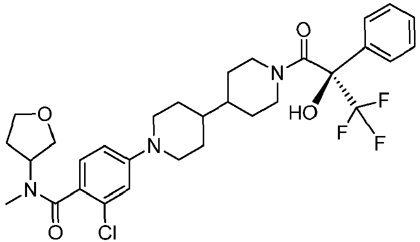 | 2-chloro-N-methyl-N-(tetrahydrofuran-3-yl)-4-{1'-[(2R)-3,3,3-trifluoro-2-hydroxy-2-phenylpropanoyl]-4,4'-bipiperidin-1-yl}benzamide | Calc'd 608.2, found 608.2 | E 5-6 |
| E-54 | 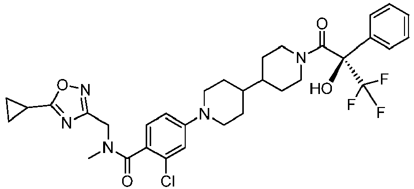 | 2-chloro-N-[(5-cyclopropyl-1,2,4-oxadiazol-3-yl)methyl]-N-methyl-4-{1'-[(2R)-3,3,3-trifluoro-2-hydroxy-2-phenylpropanoyl]-4,4'-bipiperidin-1-yl}benzamide | Calc'd 660.3, found 660.1 | E 5-6 |
| E-55 | 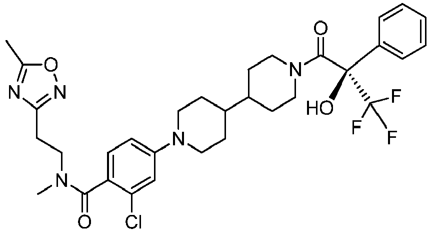 | 2-chloro-N-methyl-N-[2-(5-methyl-1,2,4-oxadiazol-3-yl)ethyl]-4-{ 1'-[(2R)-3,3,3-trifluoro-2-hydroxy-2-phenylpropanoyl]-4,4'-bipiperidin-1-yl}benzamide | Calc'd 648.3, found 648.2 | E 5-6 |
| E-56 | 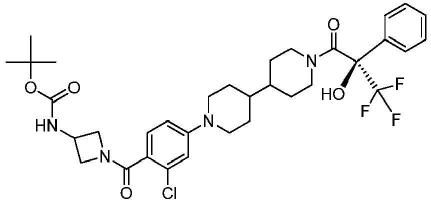 | tert-butyl {1-[(2-chloro-4-{1'-[(2R)-3,3,3-trifluoro-2-hydroxy-2-phenylpropanoyl]-4,4'-bipiperidin-1-yl}phenyl)carbonyl] azetidin-3-yl}carbamate | Calc'd 679.3, found 679.2 | E 5-6 |
| E-57 | 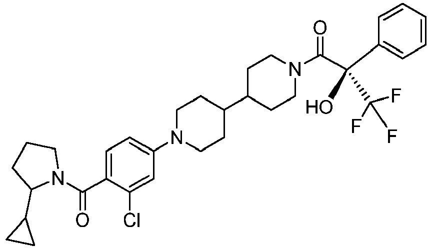 | (2R)-3-(1'-{3-chloro-4-[(2-cyclopropylpyrrolidi n-1-yl)carbonyl]phenyl} -4,4'-bipiperidin-1-yl)-1,1,1-trifluoro-3-oxo-2-phenylpropan-2-ol | Calc'd 618.3, found 618.2 | E 5-6 |
| E-58 | 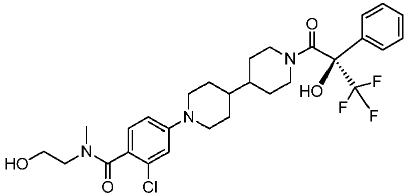 | 2-chloro-N-(2-hydroxyethyl)-N-methyl-4-{ 1'-[(2R)-3,3,3-trifluoro-2-hydroxy-2-phenylpropanoyl]-4,4'-bipiperidin-1-yl}benzamide | Calc'd 582.2, found 582.1 | E 5-6 |
| E-59 | 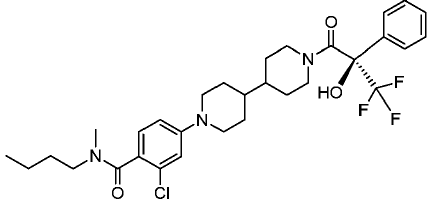 | N-butyl-2-chloro-N-methyl-4-{1'-[(2R)-3,3,3-trifluoro-2-hydroxy-2-phenylpropanoyl]-4,4'-bipiperidin-1-yl}benzamide | Calc'd 594.3, found 594.2 | E 5-6 |
| E-60 | 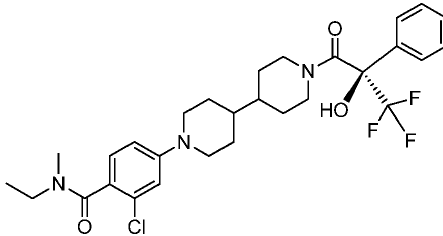 | 2-chloro-N-ethyl-N-methyl-4-{1'-[(2R)-3,3,3-trifluoro-2-hydroxy-2-phenylpropanoyl]-4,4'-bipiperidin-1-yl}benzamide | Calc'd 566.2, found 566.2 | E 5-6 |
| E-61 | 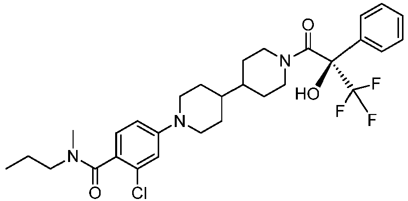 | 2-chloro-N-methyl-N-propyl-4-{1'-[(2R)-3,3,3-trifluoro-2-hydroxy-2-phenylpropanoyl]-4,4'-bipiperidin-1-yl}benzamide | Calc'd 580.3, found 580.1 | E 5-6 |
| E-62 | 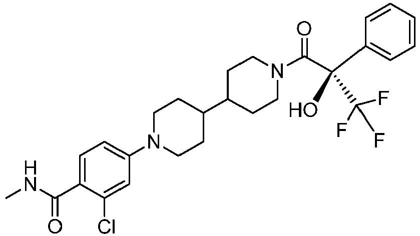 | 2-chloro-N-methyl-4-{1'-[(2R)-3,3,3-trifluoro-2-hydroxy-2-phenylpropanoyl]-4,4'-bipiperidin-1-yl}benzamide | Calc'd 538.2, found 538.1 | E 5-6 |
| E-63 | 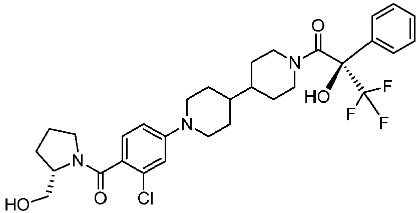 | (2R)-3-[1'-(3-chloro-4-{[(2S)-2-(hydroxymethyl)pyr rolidin-1-yl]carbonyl}phenyl) -4,4'-bipiperidin-1-yl]-1,1,1-trifluoro-3-oxo-2-phenylpropan-2-ol | Calc'd 608.2, found 608.3 | E 5-6 |
| E-64 | 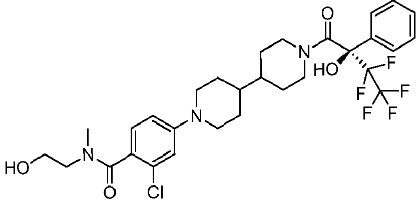 | 2-chloro-N-(2-hydroxyethyl)-N-methyl-4-{ 1'-[(2R)-3,3,4,4,4-pentafluoro-2-hydroxy-2-phenylbutanoyl]-4,4'-bipiperidin-1-yl}benzamide | Calc'd 632.2, found 632.2 | E 5-6 |
| E-65 | 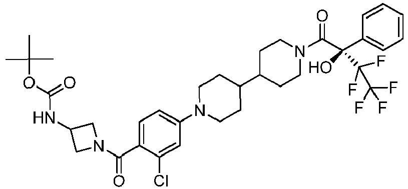 | tert-butyl{1-[(2-chloro-4-{1'-[(2R)-3,3,4,4,4-pentafluoro-2-hydroxy-2-phenylbutanoyl]-4,4'-bipiperidin-1-yl}phenyl)carbonyl] azetidin-3-yl}carbamate | Calc'd 729.3, found 729.2 | E 5-6 |
| E-66 | 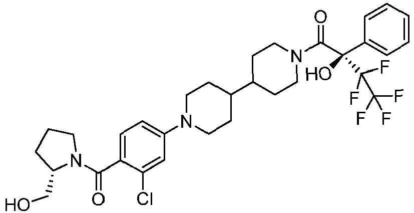 | (2R)-1-[1'-(3-chloro-4-{[(2S)-2-(hydroxymethyl)pyr rolidin-1-yl]carbonyl}phenyl) -4,4'-bipiperidin-1-yl]-3,3,4,4,4-pentafluoro-1-oxo-2-phenylbutan-2-ol | Calc'd 658.2, found 658.2 | E 5-6 (chirali ty not confir med) |
| E-67 | 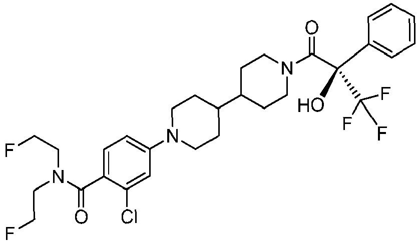 | 2-chloro-N,N-bis(2-fluoroethyl)-4-{1'-[(2R)-3,3,3-trifluoro-2-hydroxy-2-phenylpropanoyl]-4,4'-bipiperidin-1-yl}benzamide | Calc'd 616.2, found 616.5 | E 5-6 |
| E-68 | 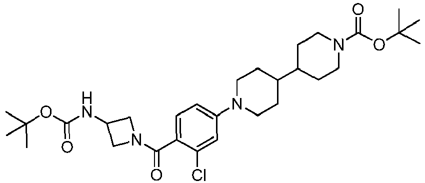 | tert-butyl 1'-[4-({3-[(tert - butoxycarbonyl)ami no]azetidin-1-yl}carbonyl)-3-chlorophenyl]-4,4'-bipiperidine-1-carboxylate | Calc'd 577.3, found 577.5 | E 5-6 |
| E-69 | 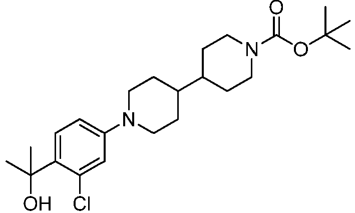 | tert-butyl 1'-[3-chloro-4-(1-hydroxy-1-methylethyl)phenyl] -4,4'-bipiperidine-1-carboxylate | Calc'd 437.3, found 437.2584 | E 5-10 |

### Biological Assays

Potency (Inflection Point, IP) and efficacy (Emax) are evaluated via compound-induced co-activator recruitment to glutathione-S-transferase (GST) tagged LXRbeta and LXRalpha LBD (ligand binding domain) proteins in relation to reference dual agonist compound T0901317 (N-(2,2,2-Trifluoroethyl)-*N*-[4-[2,2,2-trifluoro-1-hydroxy-1-(trifluoromethyl)ethyl]phenyl] benzenesulfonamide) using the LanthaScreen™ TR-FRET Liver X Receptor Coactivator Assays according to manufacturer's instructions (Invitrogen catalog number pv4658.pps and pv4655). While running the LanthaScreen™ TR-FRET Liver X Receptor Coactivator Assay, LXR alpha-LBD or LXR beta-LBD was added to ligand test compounds followed by addition of a mixture of a fluorescein-labelled coactivator peptide and terbium-conjugated anti-GST antibody. After an incubation period at room temperature, TR-FRET (time-resolved fluorescence resonance energy transfer) was measured using a filter-based instrument capable of TR- FRET, e.g. PerkinElmer Envision. When the terbium label on the anti-GST antibody was excited at 340 nm, energy was transferred to the fluorescein label on the coactivator peptide and detected as emission at 520 nm, providing an indication of ligand binding that enables ligand-dependent recruitment of coactivator peptide, and the ratio of 520 nm:495 nm is calculated and is used to determine the ligands potencies and efficacies from appropriate dose response curves of the compound. IP and %Emax values for compounds of the invention were measured in accordance with the above and are provided in the Table F below.

**TABLE F**

| Example | LXR beta IP (nM) | LXR beta act @ max dose (%) | LXR alpha IP (nM) | LXR alpha act @ max dose (%) |
|---|---|---|---|---|
| A-1 | 16 | 106 | 748 | 76 |
| A-2 | 35 | 100 | 1028 | 41 |
| A-3 | 63 | 103 | 3554 | 36 |
| A-4 | 29 | 104 | 735 | 65 |
| A-5 | 51 | 113 | 869 | 55 |
| A-6 | 24 | 107 | 626 | 70 |
| A-7 | 62 | 93 | 1396 | 56 |
| A-8 | 247 | 95 | 1808 | 60 |
| A-9 | 116 | 110 | 1196 | 56 |
| A-10 | 30 | 109 | 756 | 59 |
| A-11 | 512 | 125 | 2099 | 62 |
| A-12 | 14 | 114 | 2460 | 47 |
| A-13 | 52 | 106 | 1507 | 19 |
| A-14 | 36 | 114 | 1532 | 38 |
| A-15 | 314 | 93 | 3127 | 31 |
| A-16 | 14 | 109 | 861 | 33 |
| A-17 | 204 | 100 | 2258 | 14 |
| A-18 | 207 | 81 | 4201 | 15 |
| A-19 | 124 | 94 | 1747 | 17 |
| A-20 | 35 | 146 | 625 | 0 |
| A-21 | 243 | 95 | 2999 | 37 |
| A-22 | 52 | 58 | 1 | 3 |
| A-23 | 79 | 92 | 2981 | 23 |
| A-24 | 89 | 107 | 4849 | 31 |
| A-25 | 113 | 109 | 3537 | 54 |
| A-26 | 112 | 103 | 967 | 45 |
| A-27 | 26 | 119 | 300 | 70 |
| A-28 | 173 | 123 | 2466 | 73 |
| A-29 | 526 | 106 | 3585 | 46 |
| A-30 | 73 | 119 | 2122 | 62 |
| A-31 | 61 | 86 | 362 | 27 |
| A-32 | 101 | 88 | 748 | 24 |
| A-33 | 48 | 86 | 483 | 25 |
| A-34 | 119 | 103 | 1350 | 46 |
| A-35 | 7 | 86 | 217 | 59 |
| A-36 | 108 | 113 | 1799 | 27 |
| A-37 | 274 | 93 | 4097 | 35 |
| A-38 | 164 | 85 | 3451 | 22 |
| A-39 | 140 | 101 | 5505 | 32 |
| A-40 | 37 | 113 | 940 | 41 |
| A-41 | 84 | 112 | 3392 | 71 |
| A-42 | 61 | 117 | 3003 | 67 |
| A-43 | 54 | 128 | 620 | 40 |
| A-44 | 143 | 95 | 1976 | 22 |
| A-45 | 11 | 108 | 577 | 53 |
| A-46 | 187 | 34 | NO IP | 0 |
| A-47 | 178 | 78 | NO IP | 4 |
| A-48 | 289 | 69 | NO IP | 0 |
| A-49 | 429 | 32 | NO IP | 16 |
| A-50 | 93 | 99 | 1007 | 20 |
| A-51 | 128 | 109 | 1586 | 33 |
| A-52 | 171 | 96 | 2065 | 22 |
| A-53 | 256 | 108 | 3226 | 29 |
| A-54 | 410 | 75 | 827 | 15 |
| A-55 | 408 | 93 | 1340 | 25 |
| A-56 | 64 | 120 | 1907 | 68 |
| A-57 | 311 | 112 | 4794 | 58 |
| A-58 | 100 | 112 | 3692 | 59 |
| A-59 | 91 | 106 | 2631 | 54 |
| A-60 | 33 | 109 | 717 | 32 |
| A-61 | 56 | 115 | 888 | 53 |
| A-62 | 22 | 131 | 782 | 74 |
| A-63 | 218 | 114 | 2875 | 52 |
| A-64 | 54 | 144 | 936 | 74 |
| A-65 | 119 | 100 | 2125 | 19 |
| A-66 | 251 | 90 | 2475 | 21 |
| A-67 | 107 | 75 | NO IP | 26 |
| A-68 | 280 | 91 | NO IP | 27 |
| A-69 | 86 | 113 | 1774 | 51 |
| A-70 | 177 | 109 | 3001 | 52 |
| A-71 | 64 | 127 | 1641 | 62 |
| A-72 | 112 | 96 | 1260 | 30 |
| A-73 | 30 | 113 | 1018 | 61 |
| A-74 | 413 | 57 | NO IP | 0 |
| A-75 | 18 | 118 | 446 | 65 |
| A-76 | 374 | 87 | 2004 | 31 |
| A-77 | 52 | 86 | 618 | 31 |
| A-78 | 210 | 81 | NO IP | 15 |
| A-79 | 216 | 125 | 2540 | 35 |
| A-80 | 31 | 116 | 1166 | 67 |
| A-81 | 283 | 104 | 3667 | 25 |
| A-82 | 7 | 107 | 148 | 58 |
| A-83 | 15 | 115 | 594 | 74 |
| A-84 | 173 | 116 | 1373 | 63 |
| A-85 | 180 | 115 | 1120 | 41 |
| A-86 | 16 | 112 | 671 | 73 |
| A-87 | 11 | 110 | 499 | 58 |
| A-88 | 74 | 101 | 1076 | 37 |
| A-89 | 102 | 94 | 1221 | 36 |
| A-90 | 108 | 70 | 686 | 45 |
| A-91 | 147 | 112 | 2089 | 42 |
| A-92 | 193 | 99 | 2842 | 31 |
| A-93 | 171 | 100 | 2267 | 22 |
| A-94 | 13 | 124 | 250 | 67 |
| A-95 | 84 | 116 | 1334 | 38 |
| A-96 | 132 | 107 | 2336 | 29 |
| A-97 | 114 | 100 | 2428 | 37 |
| A-98 | 324 | 92 | 4960 | 33 |
| A-99 | 496 | 54 | NO IP | 0 |
| A-100 | 140 | 121 | 5772 | 48 |
| A-101 | 8 | 138 | 618 | 65 |
| A-102 | 45 | 130 | 57080 | 48 |
| A-103 | 16 | 122 | 2186 | 54 |
| A-104 | 21 | 129 | 2336 | 67 |
| A-105 | 55 | 118 | 1090 | 44 |
| A106 | 526 | 91 | 2108 | 27 |
| A-107 | 825 | 62 | NO IP | 17 |
| A-108 | 8 | 131 | 522 | 74 |
| A-109 | 61 | 98 | 54170 | 22 |
| A-110 | 52 | 127 | 1040 | 69 |
| B-1 | 20 | 119 | 1553 | 74 |
| B-2 | 57 | 123 | 1709 | 47 |
| B-3 | 325 | 143 | 1794 | 71 |
| B-4 | 26 | 123 | 493 | 48 |
| B-5 | 27 | 115 | 1097 | 61 |
| B-6 | 38 | 108 | 1671 | 46 |
| B-7 | 72 | 127 | 1429 | 55 |
| B-8 | 71 | 95 | 885 | 38 |
| B-9 | 48 | 104 | 1969 | 31 |
| B-10 | 161 | 106 | 1372 | 55 |
| B-11 | 177 | 116 | 2202 | 48 |
| B-12 | 65 | 140 | 684 | 69 |
| B-13 | 291 | 95 | NO IP | 28 |
| B-14 | 73 | 114 | 1230 | 43 |
| B-15 | 34 | 121 | 2241 | 87 |
| B-16 | 89 | 91 | 1335 | 17 |
| B-17 | 30 | 130 | 854 | 39 |
| C-1 | 148 | 101 | 21 | 24 |
| D-1 | 157 | 101 | 1446 | 43 |
| D-2 | 400 | 99 | 2770 | 41 |
| D-3 | 273 | 118 | 1573 | 67 |
| D-4 | 371 | 89 | 2046 | 29 |
| D-5 | 418 | 86 | 1516 | 28 |
| D-6 | 380 | 49 | 772 | 18 |
| D-7 | 180 | 82 | 413 | 20 |
| D-8 | 185 | 134 | 2489 | 64 |
| D-9 | 124 | 81 | 822 | 41 |
| E-1 | 237 | 36 | 857 | 29 |
| E-2 | 153 | 25 | 58 | 26 |
| E-3 | 297 | 81 | 127 | 30 |
| E-4 | 51 | 94 | 180 | 67 |
| E-5 | 111 | 123 | 1066 | 56 |
| E-6 | 111 | 101 | 761 | 53 |
| E-7 | 108 | 74 | 1916 | 32 |
| E-8 | 149 | 65 | 1194 | 29 |
| E-9 | 30 | 96 | 114 | 49 |
| E-10 | 219 | 72 | 1260 | 32 |
| E-11 | 156 | 33 | 1071 | 1 |
| E-12 | 128 | 84 | 1223 | 58 |
| E-13 | 473 | 46 | 2146 | 15 |
| E-14 | 289 | 78 | 1893 | 30 |
| E-15 | 283 | 54 | NO IP | 10 |
| E-16 | 137 | 92 | 1697 | 38 |
| E-17 | 105 | 130 | 760 | 33 |
| E-18 | 73 | 99 | 1279 | 52 |
| E-19 | 284 | 88 | 3144 | 33 |
| E-20 | 197 | 78 | NO IP | 25 |
| E-21 | 95 | 62 | NO IP | 13 |
| E-22 | 437 | 81 | 1666 | 18 |
| E-23 | 125 | 72 | 1784 | 25 |
| E-24 | 323 | 76 | 3092 | 27 |
| E-25 | 332 | 88 | 769 | 15 |
| E-26 | 279 | 92 | 1451 | 25 |
| E-27 | 53 | 58 | NO IP | 0 |
| E-28 | 200 | 57 | NO IP | 15 |
| E-29 | 383 | 63 | NO IP | 13 |
| E-30 | 143 | 74 | 1521 | 15 |
| E-31 | 182 | 79 | 2994 | 28 |
| E-32 | 144 | 57 | 2263 | 22 |
| E-33 | 71 | 95 | 893 | 24 |
| E-34 | 450 | 83 | NO IP | 28 |
| E-35 | 364 | 57 | 2460 | 19 |
| E-36 | 453 | 74 | 3819 | 23 |
| E-37 | 216 | 70 | NO IP | 5 |
| E-38 | 276 | 57 | NO IP | 0 |
| E-39 | 141 | 99 | 1426 | 43 |
| E-40 | 297 | 52 | NO IP | 39 |
| E-41 | 118 | 74 | 1681 | 54 |
| E-42 | 98 | 39 | 1460 | 25 |
| E-43 | 355 | 50 | 3006 | 24 |
| E-44 | 90 | 67 | 1687 | 31 |
| E-45 | 414 | 68 | 1022 | 12 |
| E-46 | 395 | 55 | 2246 | 27 |
| E-47 | 334 | 71 | NO IP | 10 |
| E-48 | 175 | 106 | 1716 | 26 |
| E-49 | 184 | 77 | 1382 | 30 |
| E-50 | 349 | 74 | 1397 | 31 |
| E-51 | 145 | 62 | 1716 | 18 |
| E-52 | 206 | 78 | 1419 | 28 |
| E-53 | 219 | 98 | 1598 | 34 |
| E-54 | 35 | 93 | 1461 | 55 |
| E-55 | 66 | 106 | 1113 | 41 |
| E-56 | 21 | 89 | 488 | 45 |
| E-57 | 134 | 65 | 975 | 16 |
| E-58 | 57 | 98 | 1827 | 48 |
| E-59 | 208 | 89 | 2376 | 21 |
| E-60 | 100 | 114 | 1853 | 34 |
| E-61 | 100 | 94 | 1277 | 29 |
| E-62 | 341 | 99 | 2062 | 33 |
| E-63 | 21 | 86 | 359 | 51 |
| E-64 | 26 | 146 | 913 | 54 |
| E-65 | 17 | 137 | 635 | 64 |
| E-66 | 12 | 126 | 211 | 72 |
| E-67 | 171 | 106 | 2098 | 30 |
| E-68 | 29 | 92 | 367 | 64 |
| E-69 | 322 | 69 | 996 | 56 |

## Claims

1. A compound having the structural Formula (I): 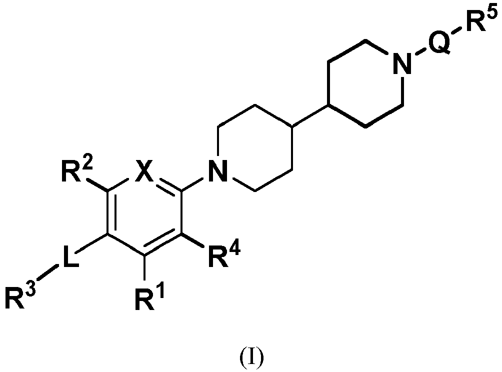 or a pharmaceutically acceptable salt thereof, wherein:
X is selected from the group consisting of -N- and -CH-;
R¹ is selected from the group consisting of H, methyl, and halogen;
R² is selected from the group consisting of H and halogen;
R⁴ is selected from the group consisting of H and methyl;
L is a bond and R³ is selected from the group consisting of -(C₁-C₆)alkyl and -(C₁-C₆)alkyl-OH;
or, alternatively, L is a divalent moiety selected from the group consisting of -C(O)- and - S(O)₂-; and R³ is -N(R^{N1})(R^{N2}), wherein:
R^{N1} is selected from the group consisting of H and -(C₁-C₆)alkyl; and
R^{N2} is selected from the group consisting of: H, -(C₁-C₆)alkyl, -O-(C₁-C₆)alkyl, -OH, halogen, -CN, and -(C₁-C₆)alkyl which is substituted with 1 or 2 groups independently selected from:
-OH, halogen, -CN,
optionally substituted phenyl, (wherein said optional substitutents on said phenyl are 1 to 3 groups independently selected from OH, CN, -(C₁-C₄)alkyl, - (C₁-C₄)alkoxyl),
optionally substituted heteroaryl, (wherein said optional substituents on said heteroaryl are 1 to 3 groups independently selected from -(C₁-C₆)alkyl, -(C₁-C₄)alkoxyl, and cyclopropyl),
optionally substituted cyclopropyl (wherein said optional substituents on said cyclopropyl are 1 to 3 groups independently selected from -(C₁-C₆)alkyl), and
optionally substituted heterocycloalkyl (wherein said optional substitutents on said heterocycloalkyl are 1 to 3 groups independently selected from halogen - OH, oxo, CN, and -(C₁-C₆)alkyl,
or, alternatively, R^{N1} and R^{N2} are taken together with the nitrogen atom to which they are shown attached to form a 4-, 5-, or 6-membered fully saturated heterocyclic ring comprising (including said nitrogen atom) 1, 2, or 3 ring heteroatoms selected from the group consisting of N, N-oxide, O, S, and S-oxide,
wherein said heterocyclic ring is unsubstituted or substituted with 1, 2, or 3 substituents independently selected from the group consisting of halogen -OH, oxo, CN, -(C₁-C₆)alkyl, amino-substituted -(C₁-C₆)alkyl (wherein said amino is 1, 2, or 3 groups independently selected from the group consisting of -NH₂, -N(C₁-C₄alkyl)₂, and -NH(C₁-C₄alkyl)), -O-(C₁-C₆)alkyl, -(C₁-C₆)alkyl-OH, -(C₁-C₆)haloalkyl, -C(O)O-(C₁-C₆)alkyl, cyclopropyl, spirocyclopropyl, -CH₂-NHC(O)O-(C₁-C₆)alkyl, -CH₂-N(CH₃)C(O)O-(C₁-C₆)alkyl, phenyl, benzyl, - NHC(O)-phenyl, heteroaryl, and -(C₁-C₄)alkylheteroaryl, heterocycloalkyl;
Q is a bond or a divalent moiety selected from the group consisting of -C(O)-, and -S(O)₂-; and
R⁵ is selected from the group consisting of:
-C(R^{5A})(R^{5B})(R^{5C}), wherein:
each of R^{5A}, R^{5B} and R^{5C} is independently selected from the group consisting of: H, halogen, OH, -(C₁-C₆)alkyl, -(C₁-C₆)haloalkyl, -(C₁-C₆)alkenyl, -(C₁-C₆)alkynyl, -(C₃-C₆)cycloalkyl, -(C₃-C₆)cycloalkyl substituted with -(C₁-C₆)alkyl, -(C₁-C₆)alkenyl, -(C₁-C₆)alkynyl, phenyl, phenyl substituted with from 1 to 3 groups independently selected from halogen, -(C₁-C₆)alkyl, -O-(C₁-C₆)alkyl, and -C(O)O-(C₁-C₆)alkyl, 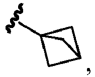 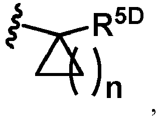 wherein:
n is an integer from 1 to 4; and R^{5D} is selected from the group consisting of H, -(C₁-C₆)alkyl, -(C₁-C₆)haloalkyl, phenyl, and phenyl substituted with from 1 to 3 groups independently selected from the group consisting of OH, halogen, - (C₁-C₆)alkyl, and -O-(C₁-C₆)alkyl, and
phenyl, wherein:
said phenyl is unsubstituted or substituted with 1, 2, or 3 groups independently selected from the group consisting of halogen, CN, -(C₁-C₆)alkyl, and -(C₁-C₆)haloalkyl.

2. The compound of claim 1, or a pharmaceutically acceptable salt thereof, wherein:
Q is a bond or a divalent moiety selected from the group consisting of -C(O)- and - S(O)₂-; and
R⁵ is-C(R^{5A})(R^{5B})(R^{5C}),
wherein each of R^{5A}, R^{5B} and R^{5C} is independently selected from the group consisting of: H, F, Cl, OH, -(C₁-C₆)alkyl, -(C₁-C₆)haloalkyl, -(C₁-C₆)alkenyl, -(C₁-C₆)alkynyl, -(C₃-C₆)cycloalkyl, -(C₃-C₆)cycloalkyl substituted with -(C₁-C₆)alkyl, -(C₁-C₆)alkenyl, -(C₁-C₆)alkynyl, phenyl, phenyl substituted with from 1 to 3 groups independently selected from F, Cl, -(C₁-C₆)alkyl, -O-(C₁-C₆)alkyl, and -C(O)O-(C₁-C₆)alkyl.

3. The compound of claim 1, or a pharmaceutically acceptable salt thereof, wherein:
Q is a bond or a divalent moiety selected from the group consisting of -C(O)- and - S(O)₂-; and
R⁵ is selected from the group consisting of 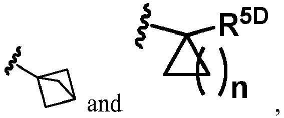 wherein n is an integer from 1 to 4; and R^{5D} is selected from the group consisting of H, -(C₁-C₆)alkyl, -(C₁-C₆)haloalkyl, phenyl, and phenyl substituted with from 1 to 3 groups independently selected from the group consisting of OH, F, Cl, -(C₁-C₆)alkyl, and -O-(C₁-C₆)alkyl.

4. The compound of claim 1, or a pharmaceutically acceptable salt thereof, wherein:
R⁵ is phenyl,
wherein said phenyl is unsubstituted or substituted with 1, 2, or 3 groups independently selected from the group consisting of F, Cl, CN, -(C₁-C₆)alkyl, and -(C₁-C₆)haloalkyl.

5. The compound according to any
previous claim, or a pharmaceutically acceptable salt thereof, wherein:
L is -C(O)-; and R³ is -N(R^{N1})(R^{N2}), wherein:
R^{N1} is selected from the group consisting of H and -(C₁-C₆)alkyl; and
R^{N2} is -(C₁-C₆)alkyl which is optionally substituted with 1 or 2 groups independently selected from:
optionally substituted phenyl, (wherein said optional substitutents on said phenyl are 1 to 3 groups independently selected from OH, CN, -(C₁-C₄)alkyl, - (C₁-C₄)alkoxyl),
optionally substituted heteroaryl, (wherein said optional substituents on said heteroaryl are 1 to 3 groups independently selected from -(C₁-C₆)alkyl, -(C₁-C₄)alkoxyl, and cyclopropyl),
optionally substituted cyclopropyl (wherein said optional substituents on said cyclopropyl are 1 to 3 groups independently selected from -(C₁-C₆)alkyl),
optionally substituted heterocycloalkyl (wherein said optional substitutents on said heterocycloalkyl are 1 to 3 groups independently selected from halogen - OH, oxo, CN, and -(C₁-C₆)alkyl, and
-O-(C₁-C₆)alkyl, -OH, F, Cl, and -CN.

6. The compound according to any one of claims 1 to 4, or a pharmaceutically acceptable salt thereof, wherein:
L is selected from the group consisting of -C(O)- and -S(O)₂-; and
R³ is -N(R^{N1})(R^{N2}), wherein R^{N1} and R^{N2} are each independently selected from the group consisting of H and -(C₁-C₆)alkyl.

7. The compound according to any one of claims 1 to 4, or a pharmaceutically acceptable salt thereof, wherein:
L is -C(O)-; and R³ is -N(R^{N1})(R^{N2}), wherein:
R^{N1} and R^{N2} are taken together with the nitrogen atom to which they are shown attached to form a 4-, 5-, or 6-membered fully saturated heterocyclic ring comprising (including said nitrogen atom) 1, 2, or 3 ring heteroatoms selected from the group consisting of N, N-oxide, O, S, and S-oxide,
wherein said heterocyclic ring is unsubstituted or substituted with 1, 2, or 3 substituents independently selected from the group consisting of halogen -OH, oxo, CN, -(C₁-C₆)alkyl, amino-substituted -(C₁-C₆)alkyl (wherein said amino is 1, 2, or 3 groups independently selected from the group consisting of -NH₂, -N(C₁-C₄alkyl)₂, and -NH(C₁-C₄alkyl)), -O-(C₁-C₆)alkyl, -(C₁-C₆)alkyl-OH, -(C₁-C₆)haloalkyl, -C(O)O-(C₁-C₆)alkyl, cyclopropyl, spirocyclopropyl, -CH₂-NHC(O)O-(C₁-C₆)alkyl, -CH₂-N(CH₃)C(O)O-(C₁-C₆)alkyl, phenyl, benzyl, - NHC(O)-phenyl, heteroaryl, and -(C₁-C₄)alkylheteroaryl, heterocycloalkyl.

8. The compound according to any one of claims 1 to 4, or a pharmaceutically acceptable salt thereof, wherein:
L is a bond and R³ is -(C₁-C₆)alkyl which is optionally substituted with OH.

9. The compound according to claim 1, or a pharmaceutically acceptable salt thereof, said compound selected from the group consisting of:

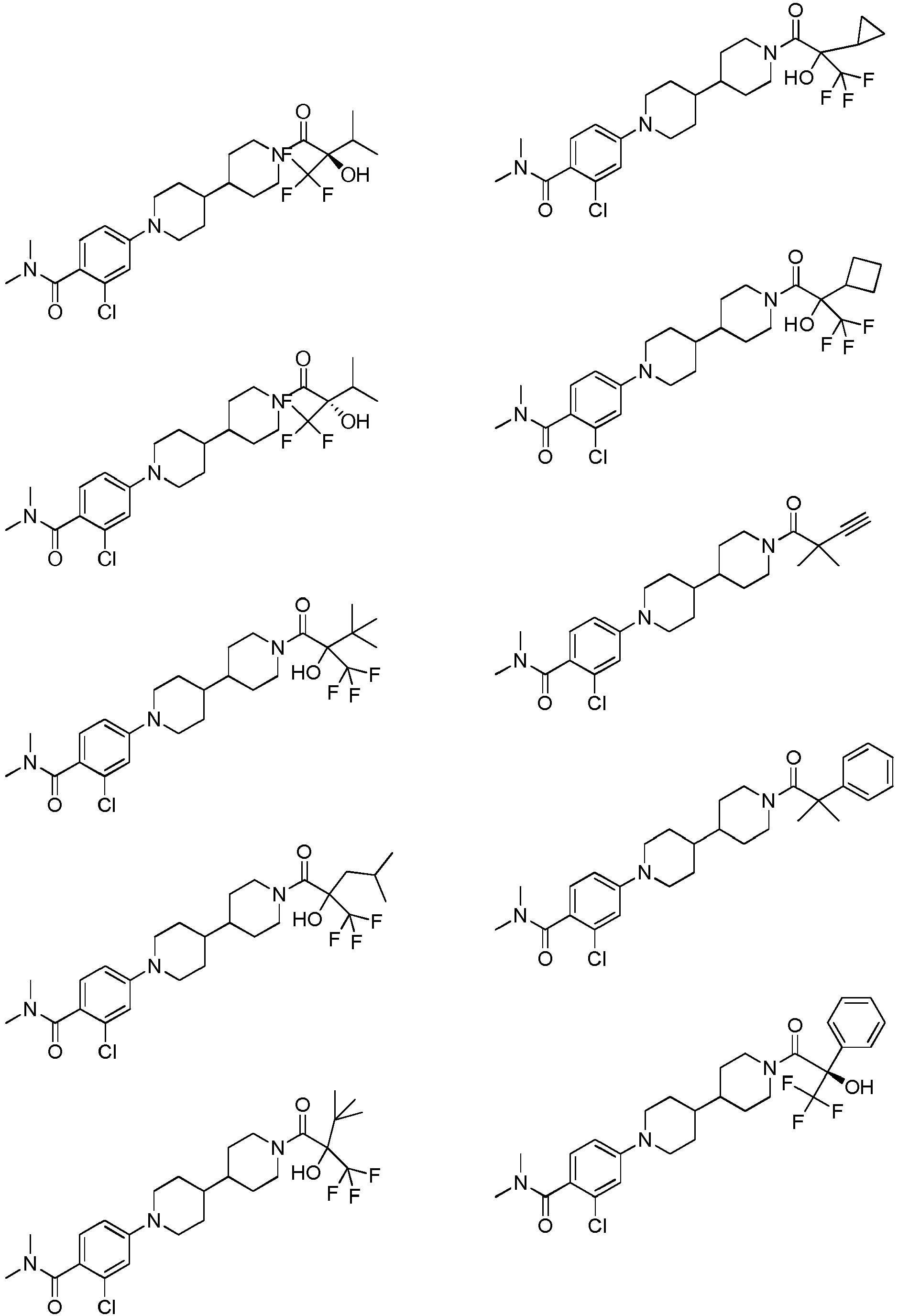

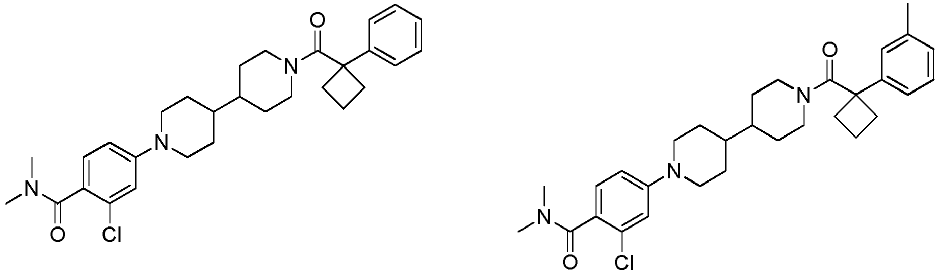

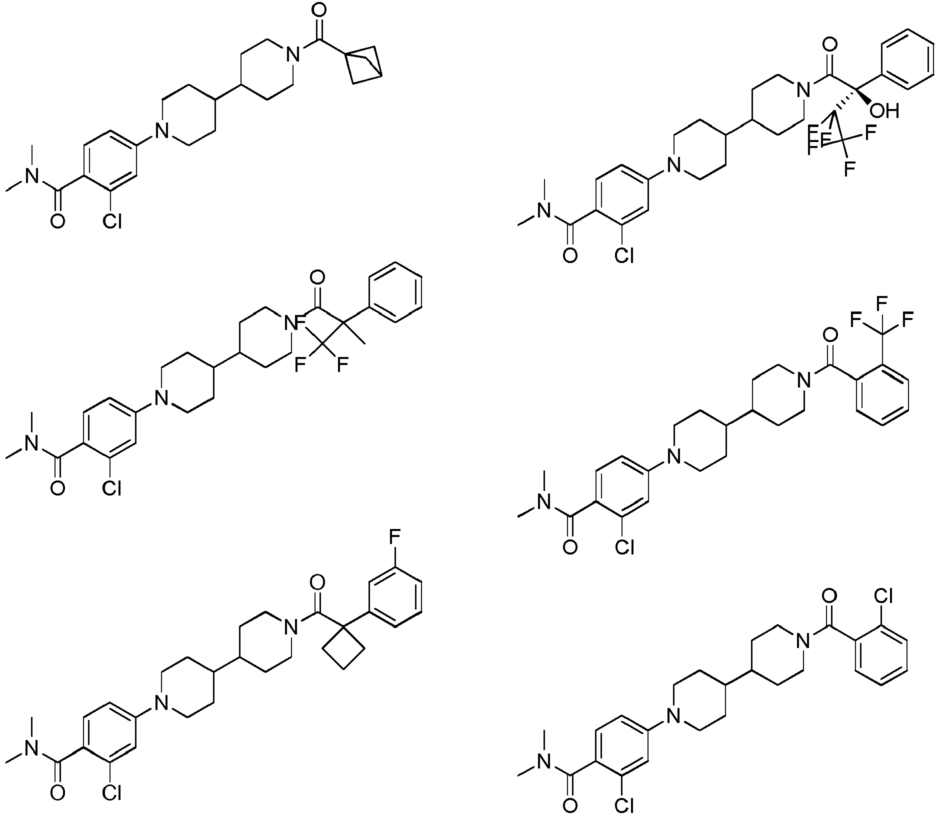

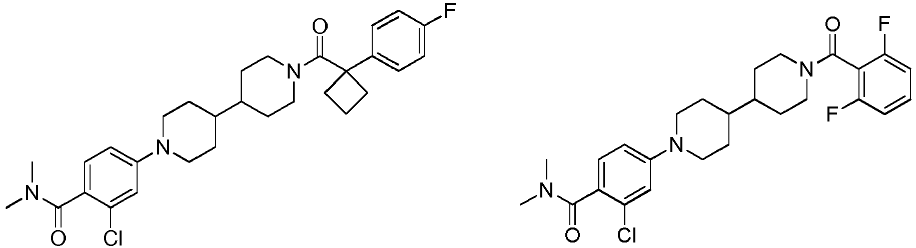

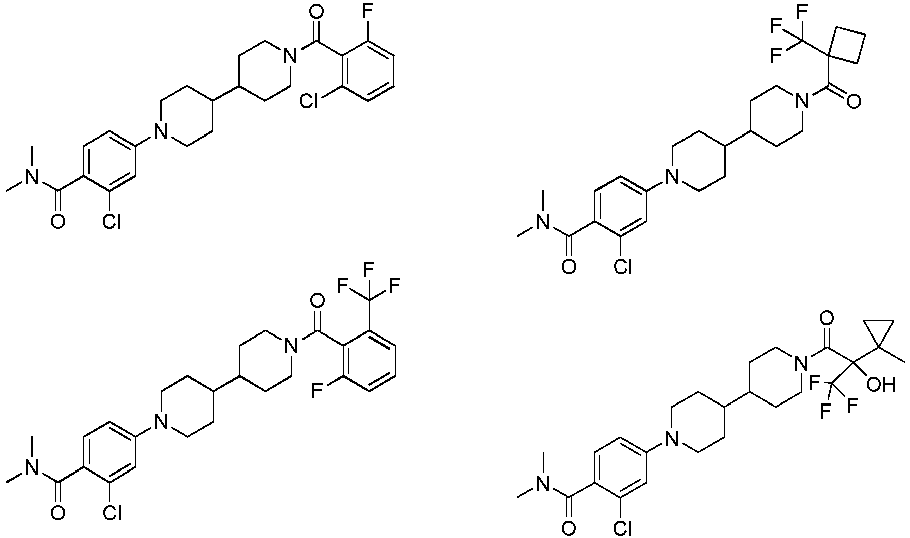

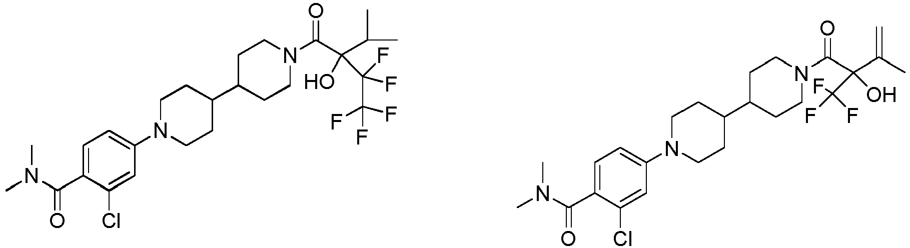

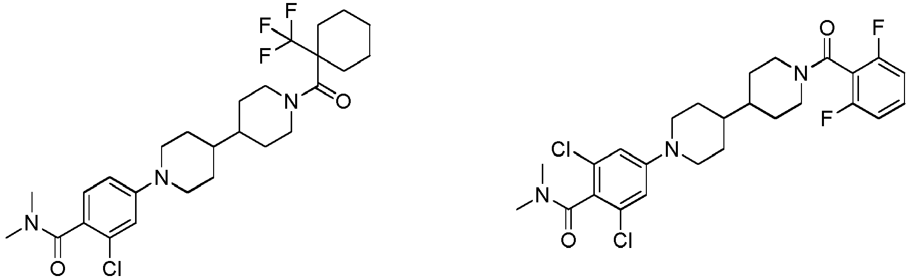

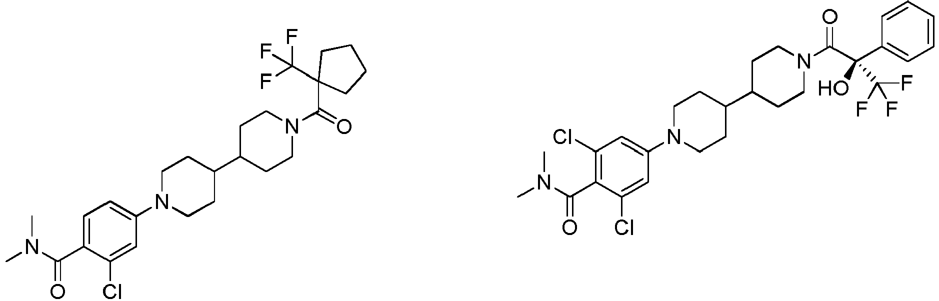

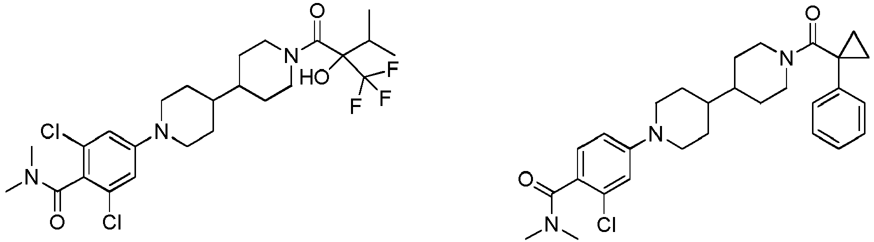

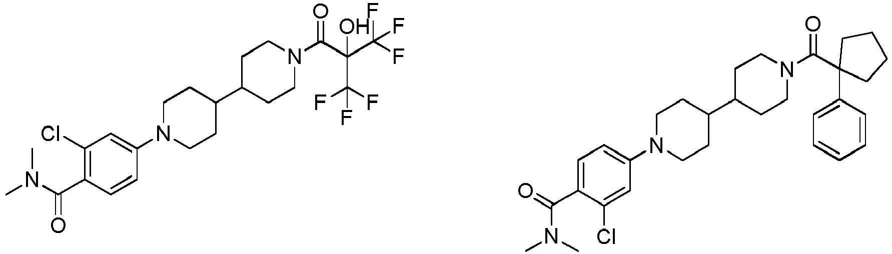

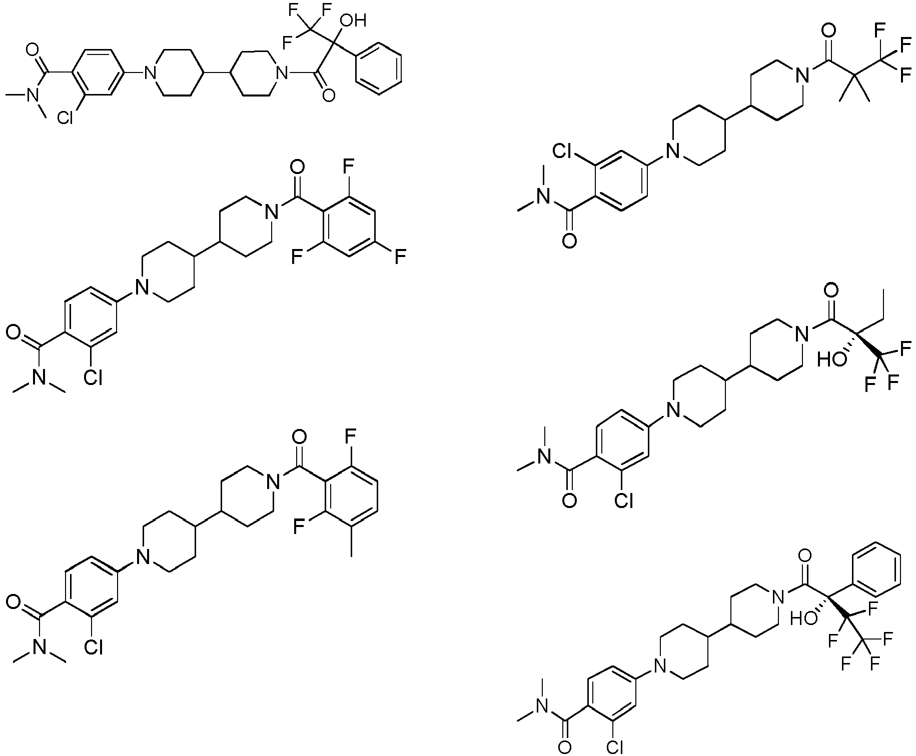

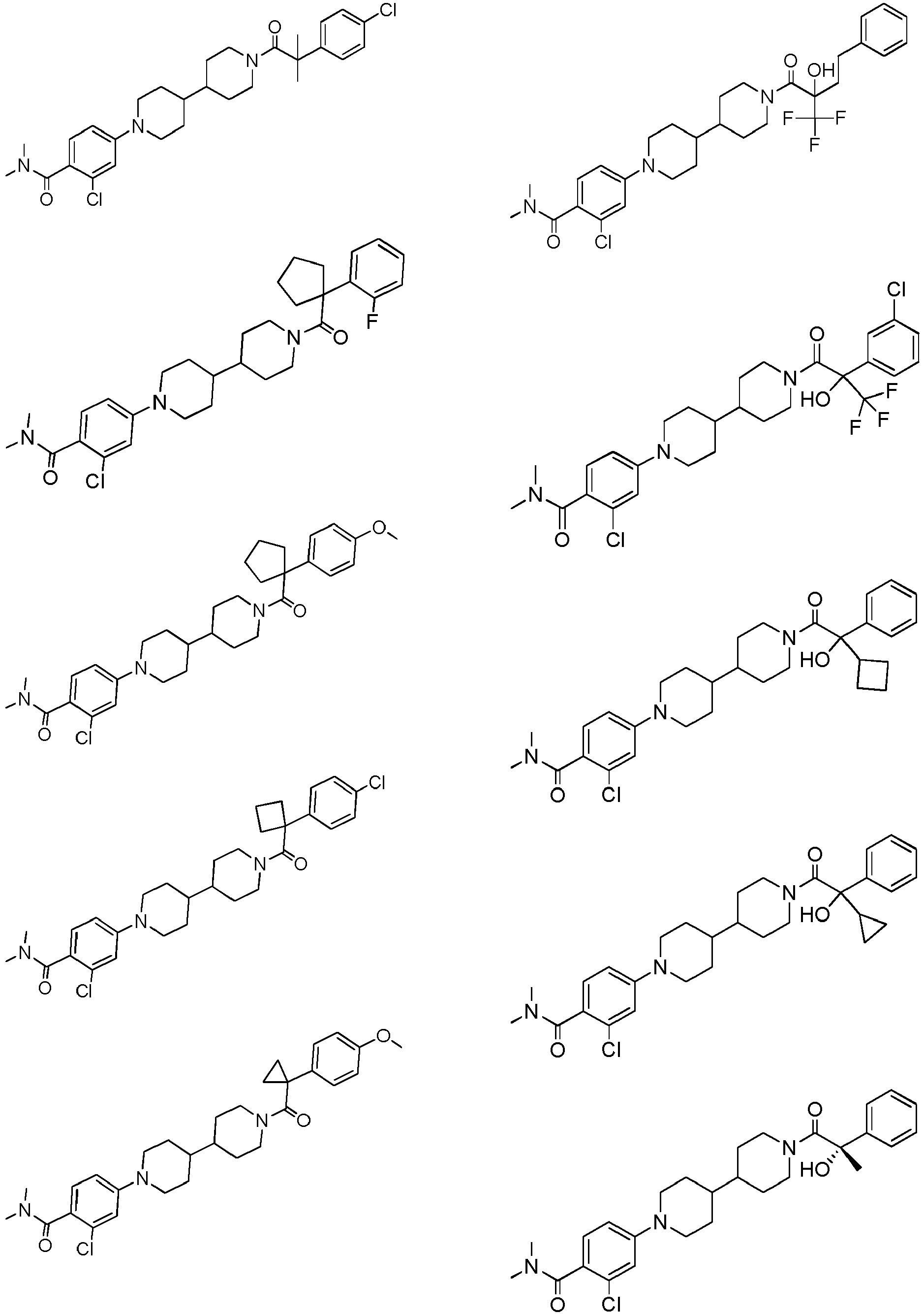

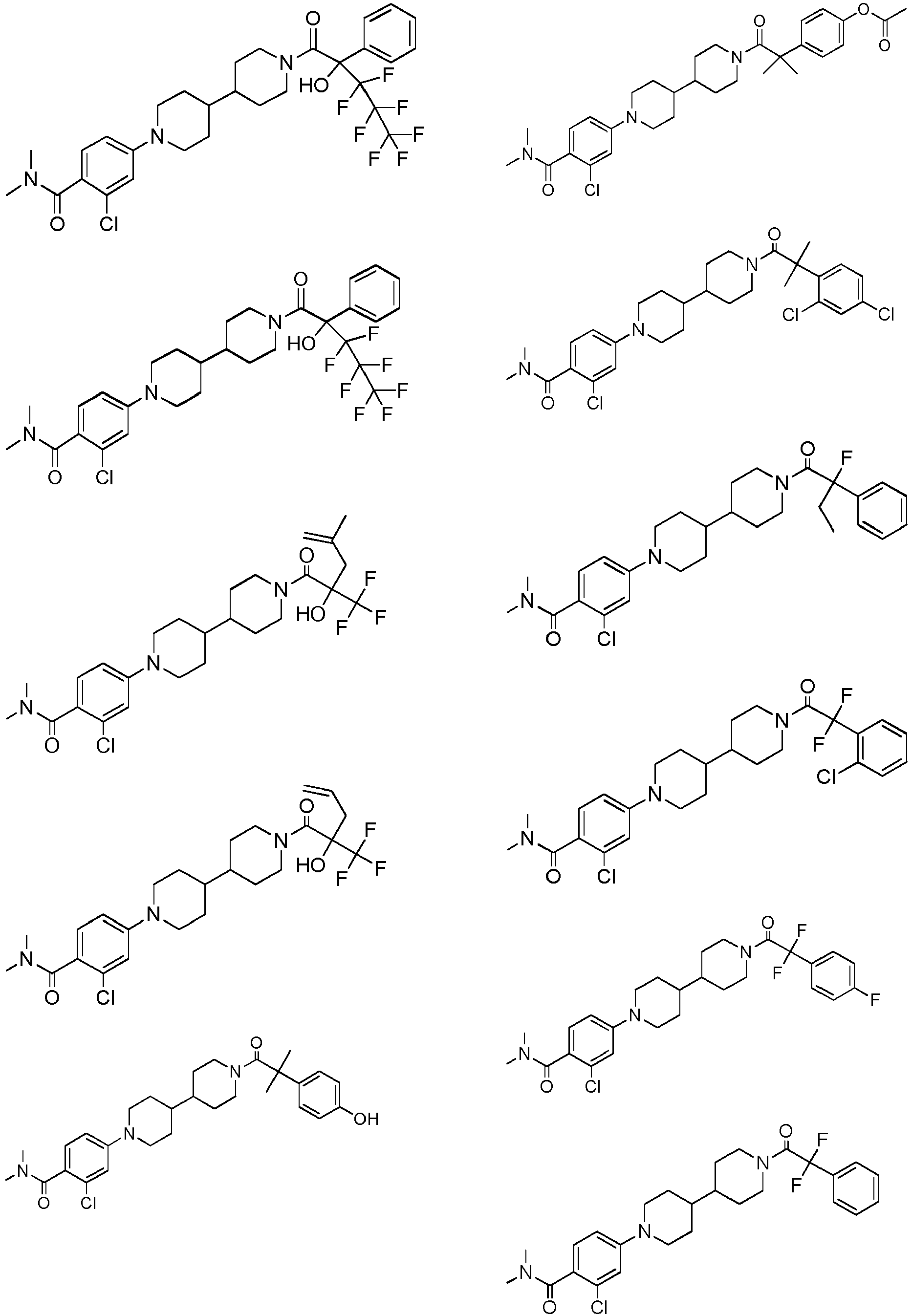

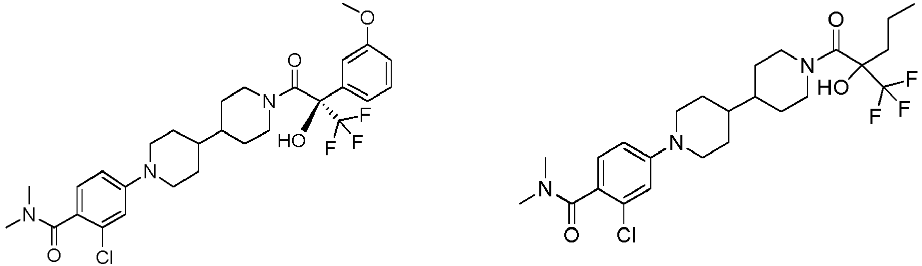

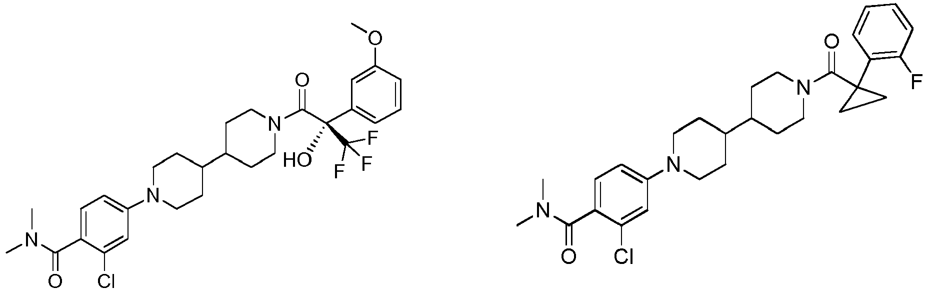

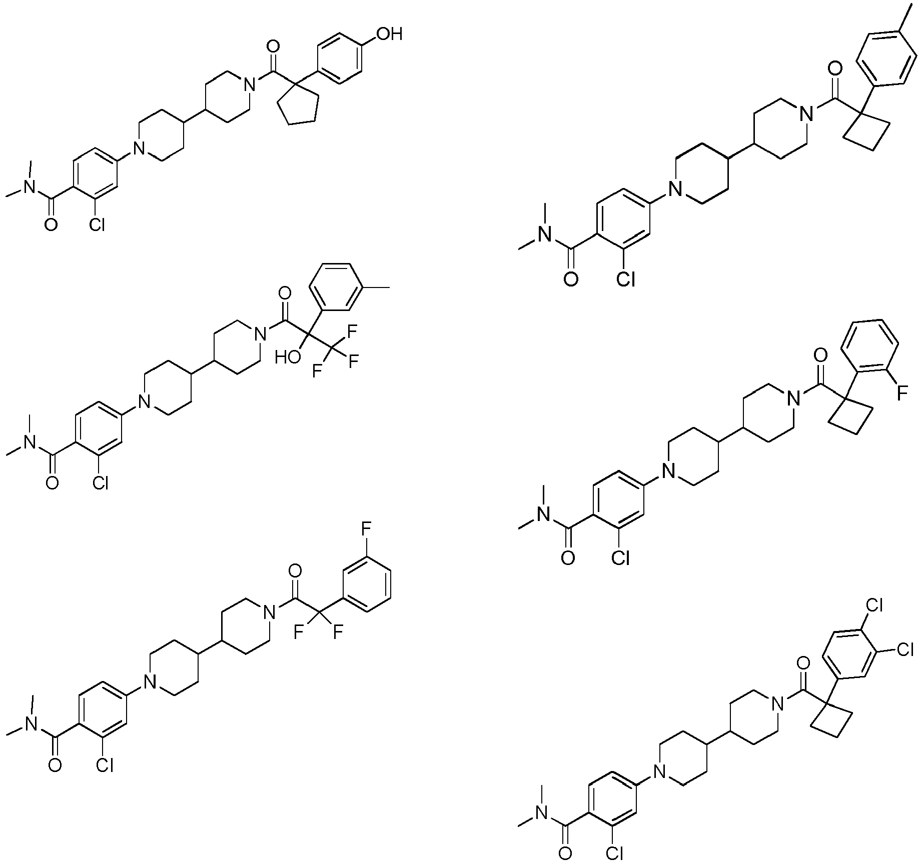

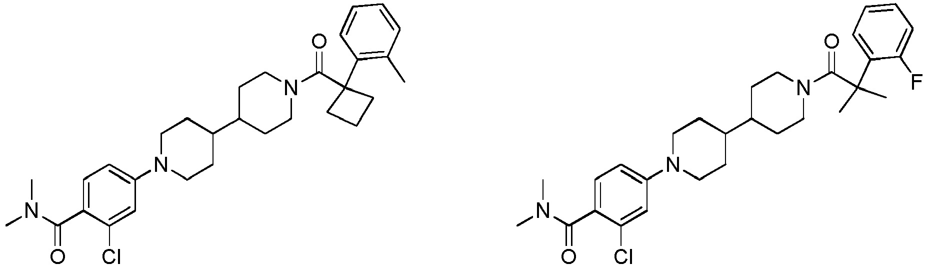

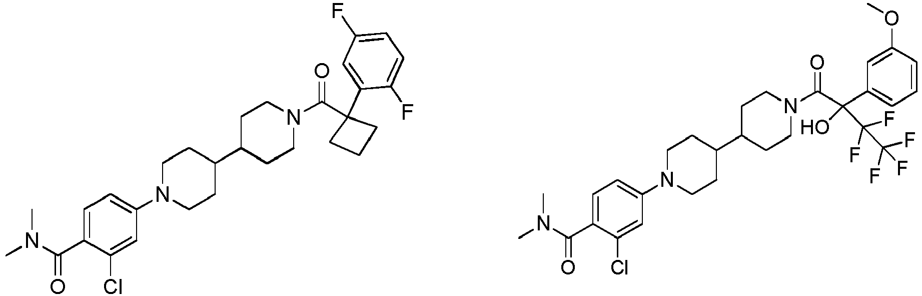

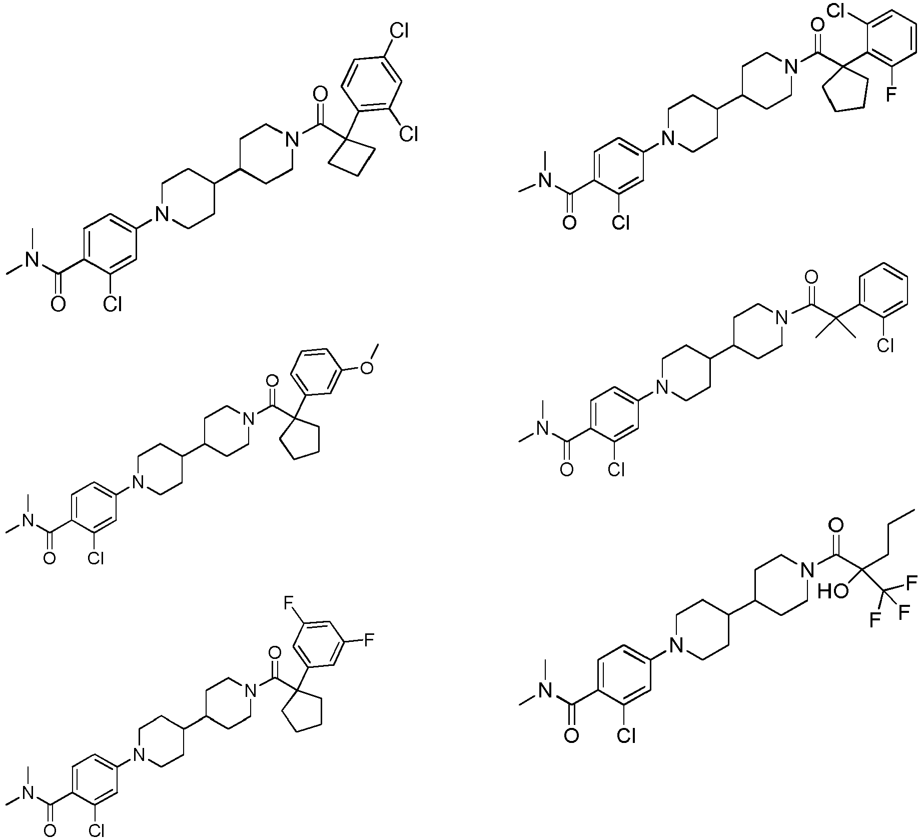

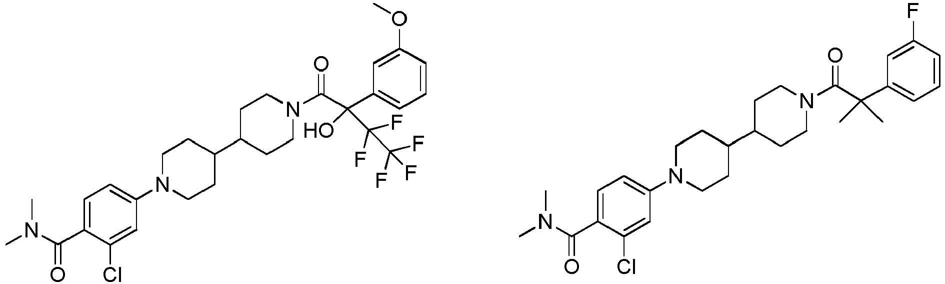

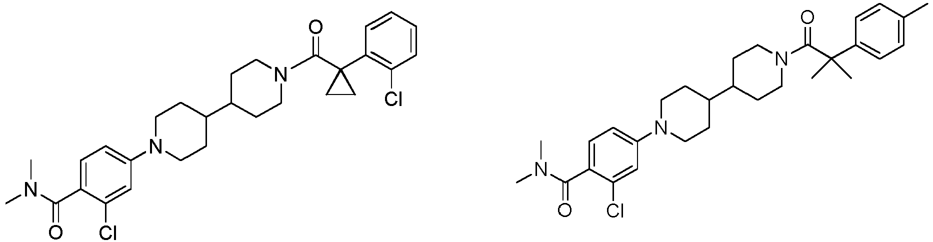

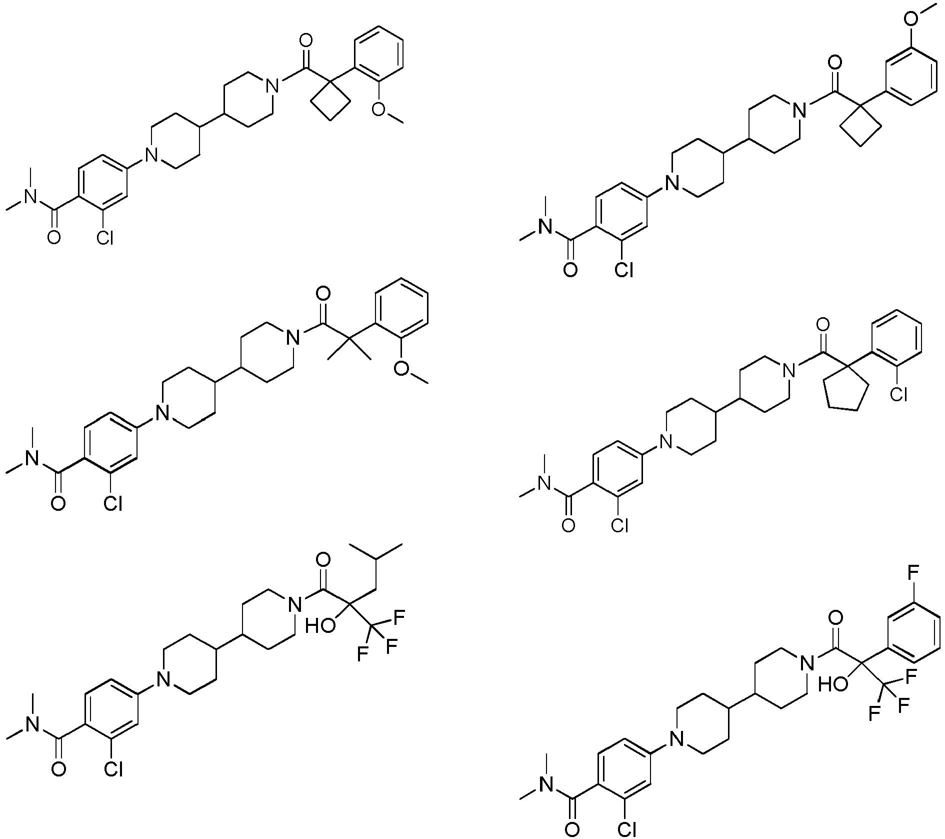

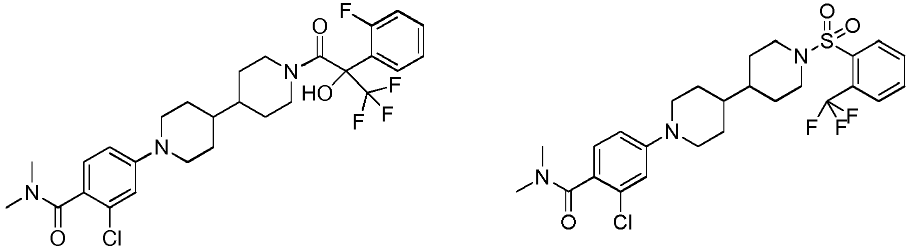

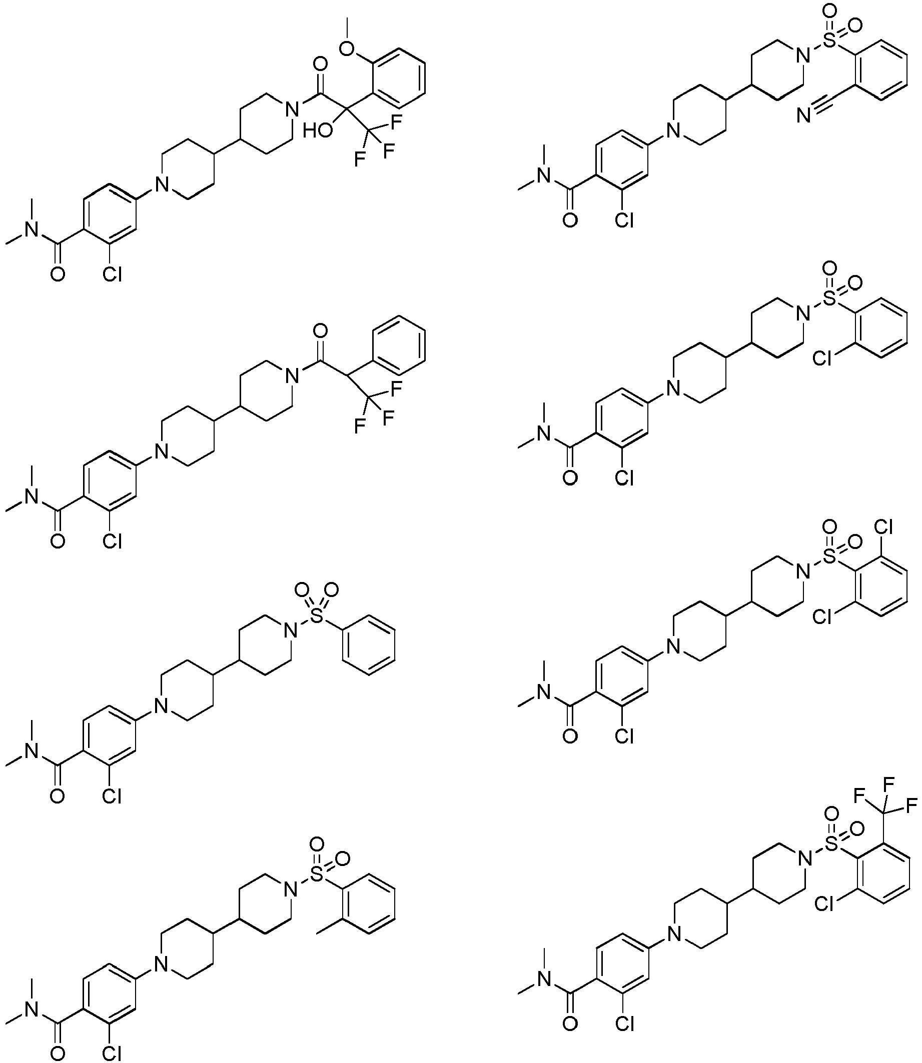

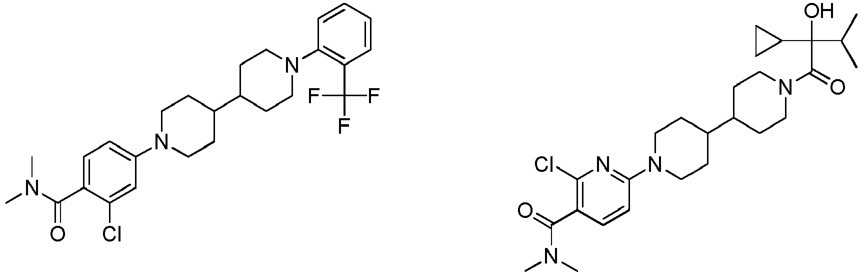

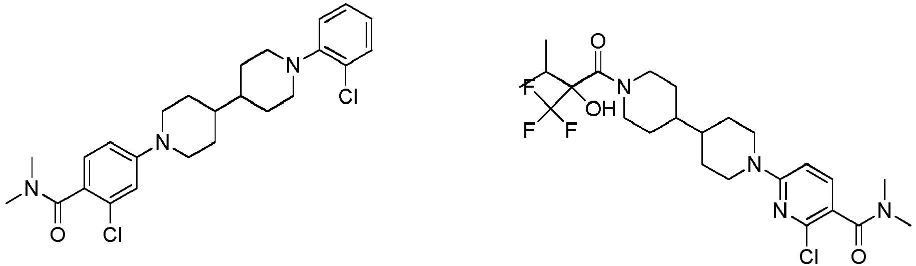

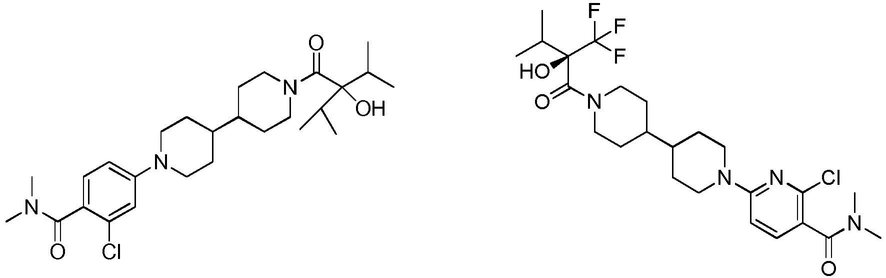

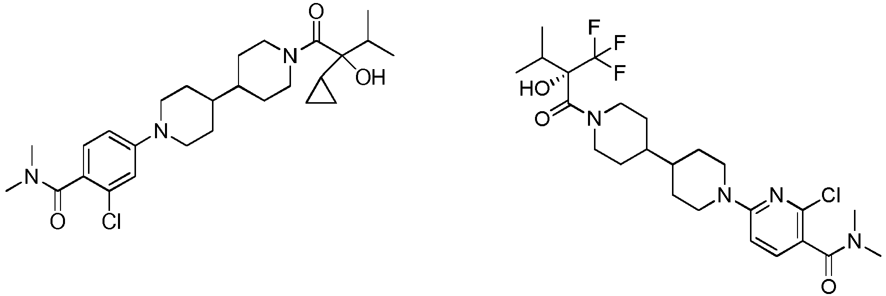

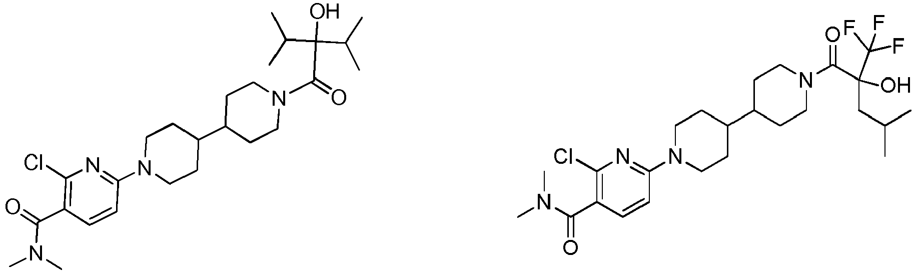

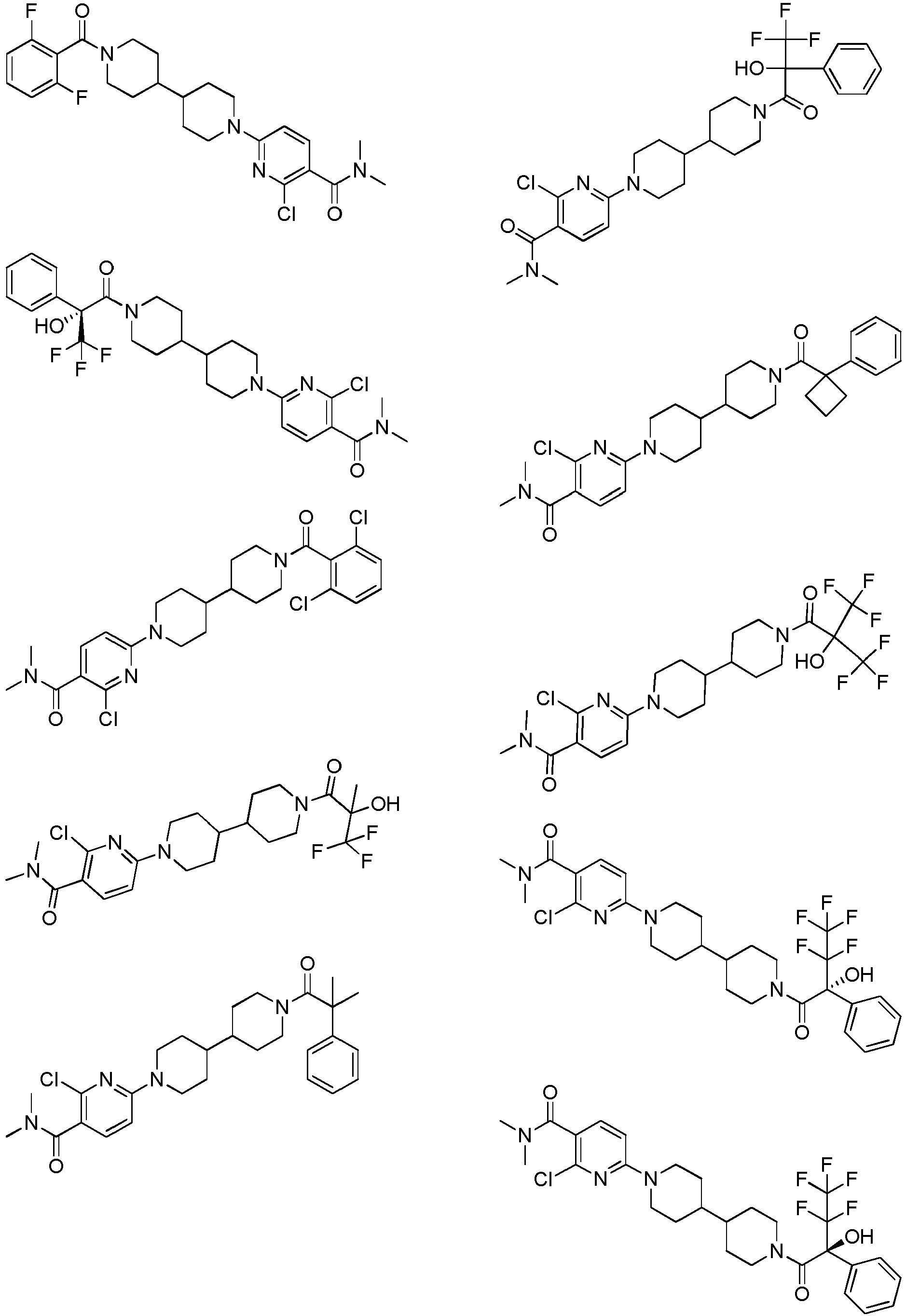

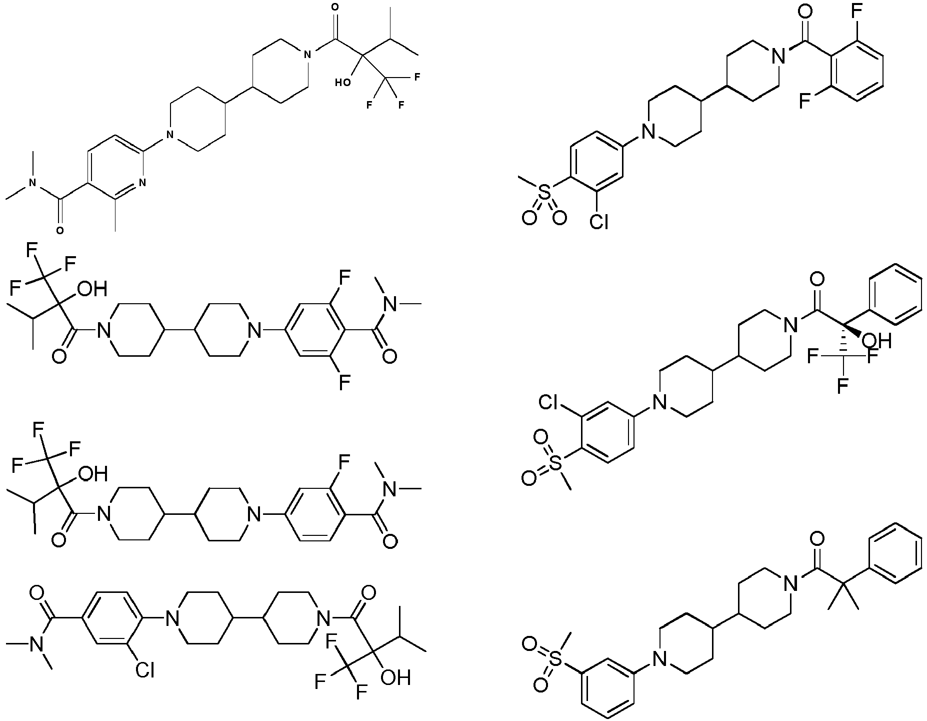

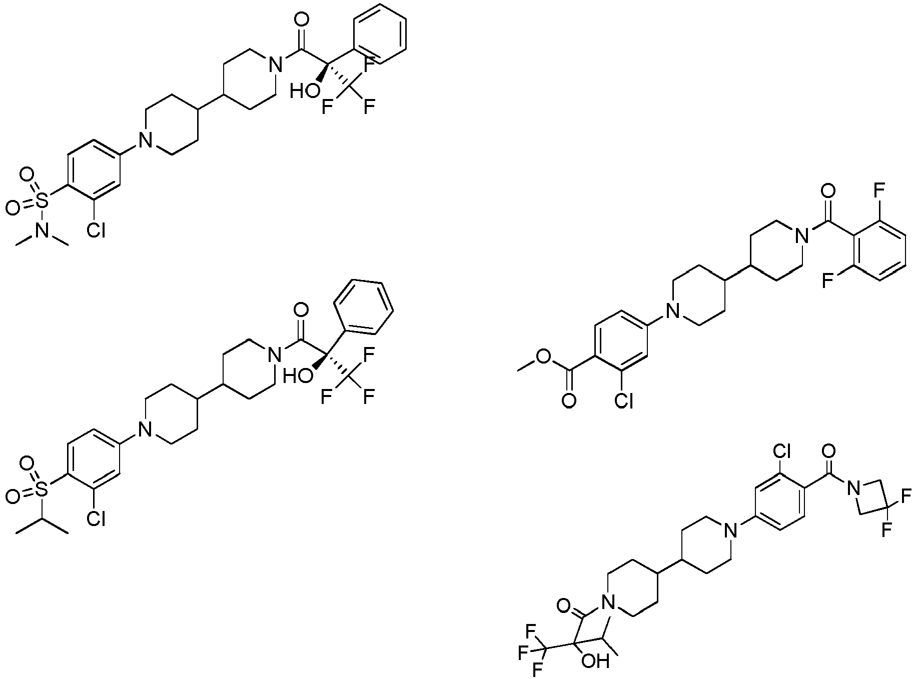

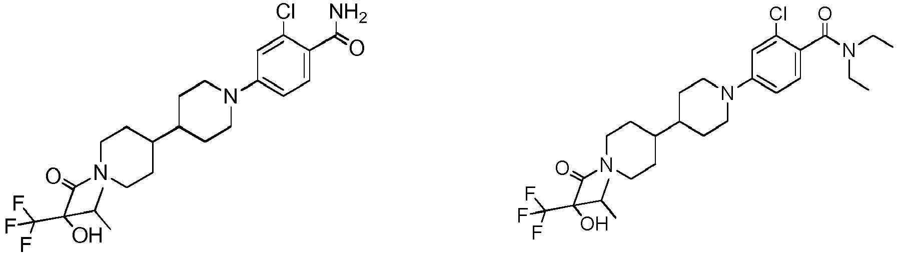

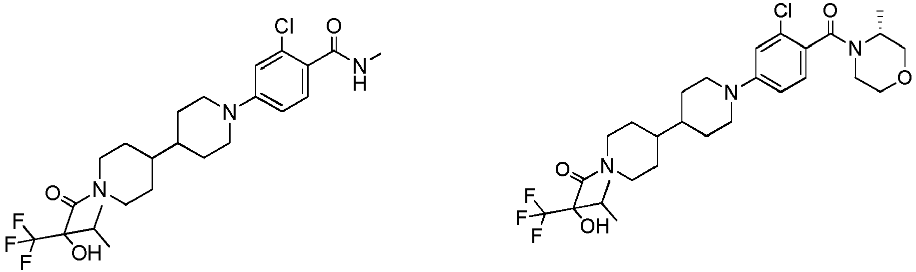

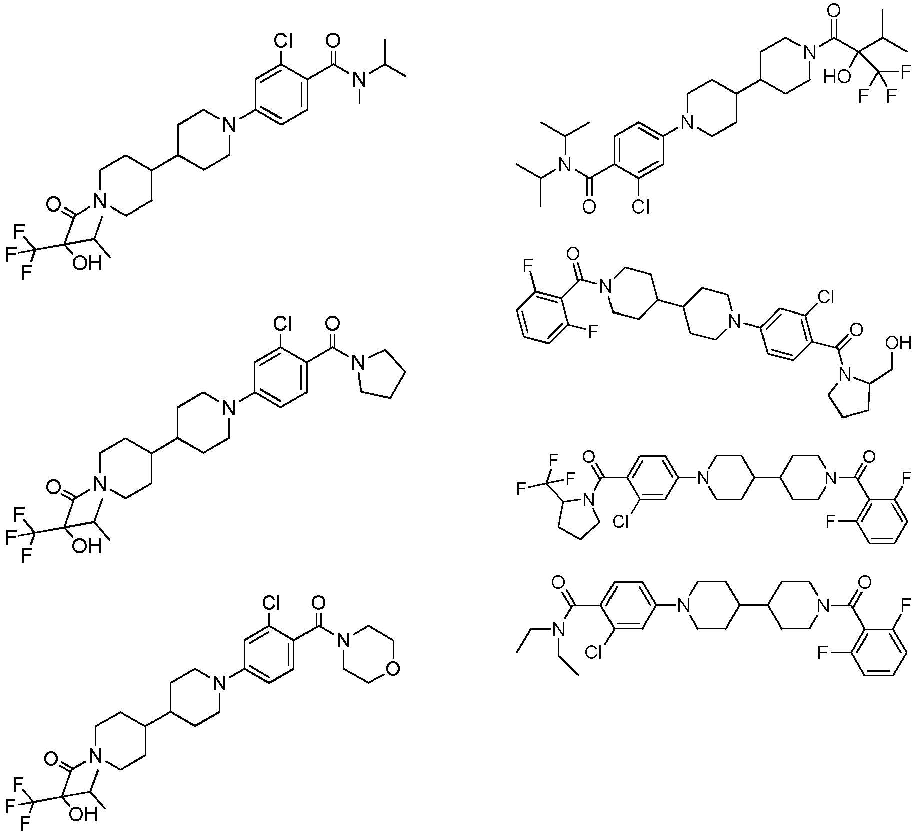

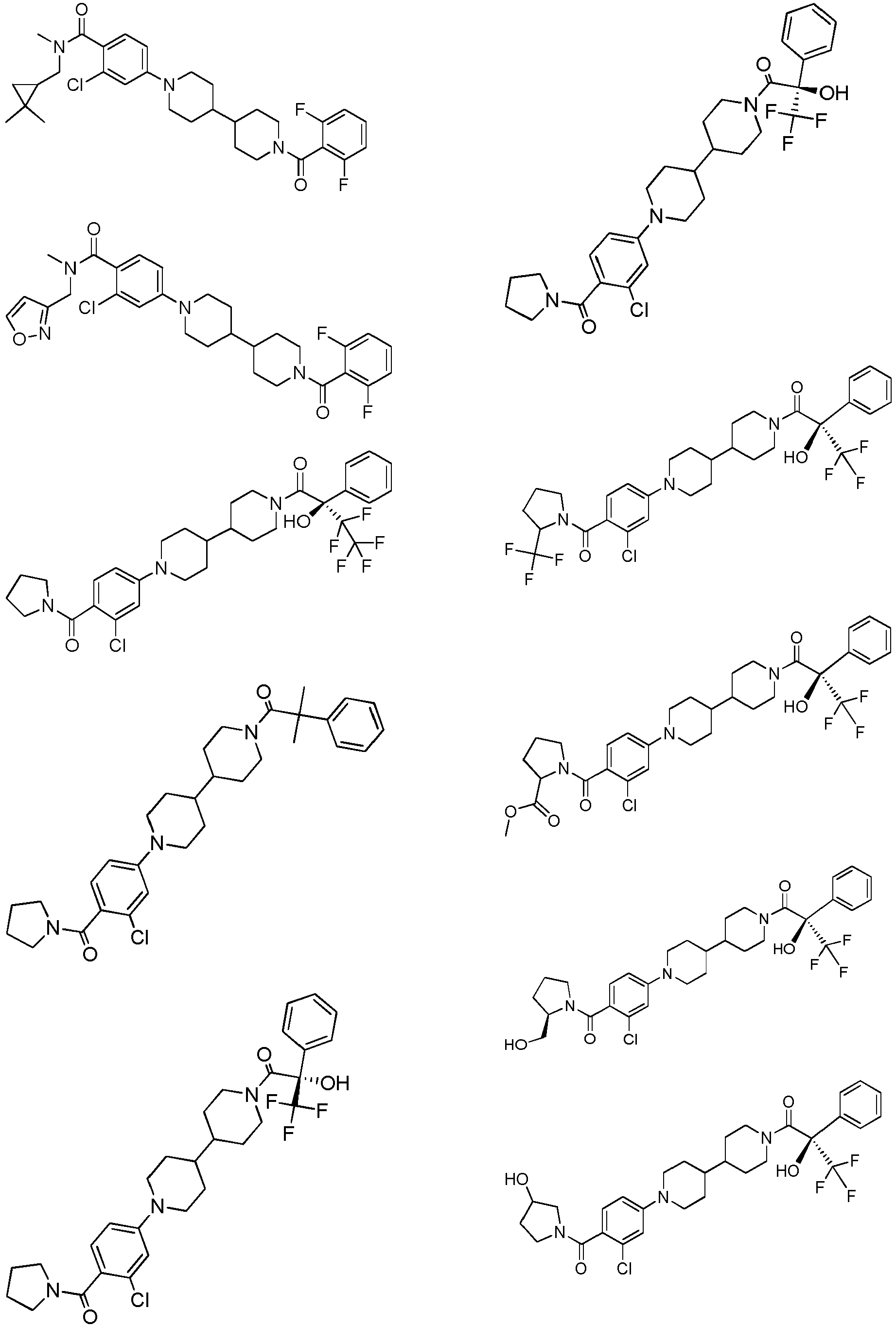

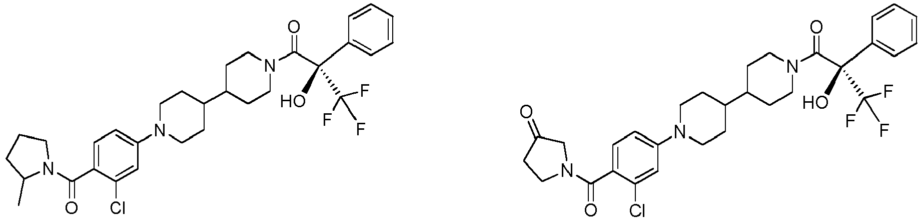

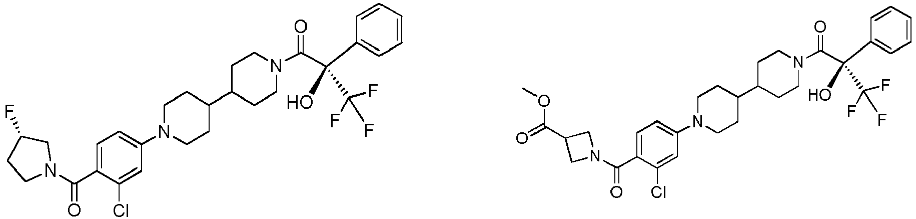

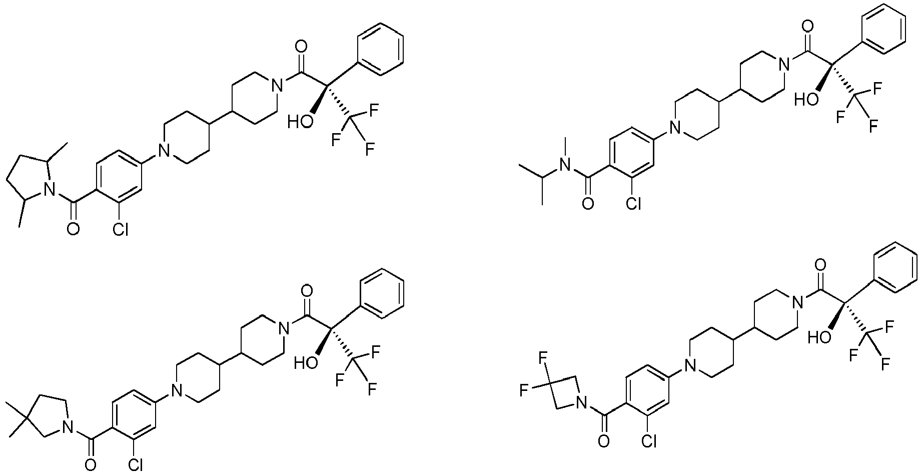

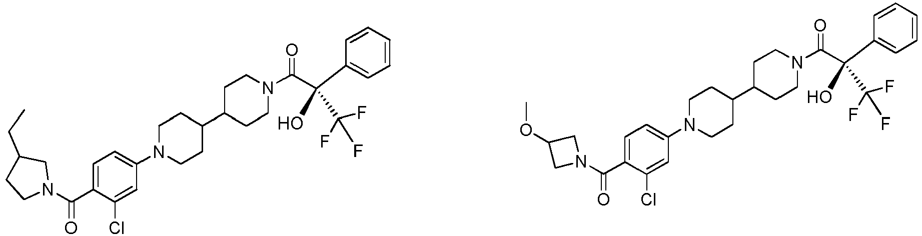

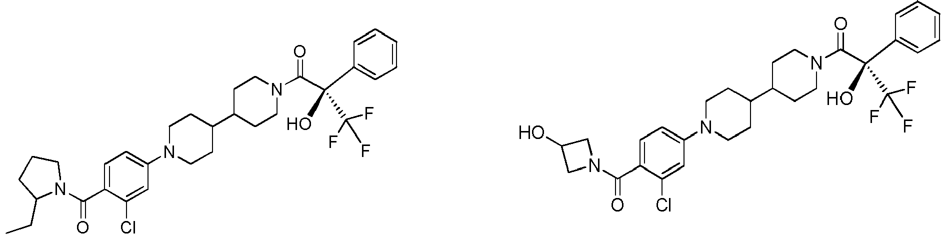

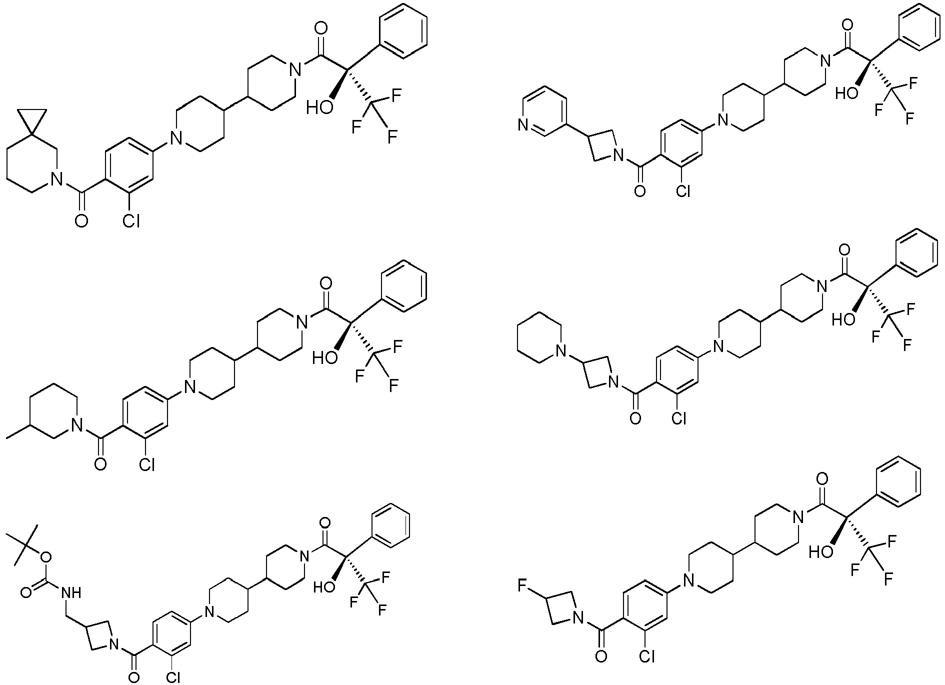

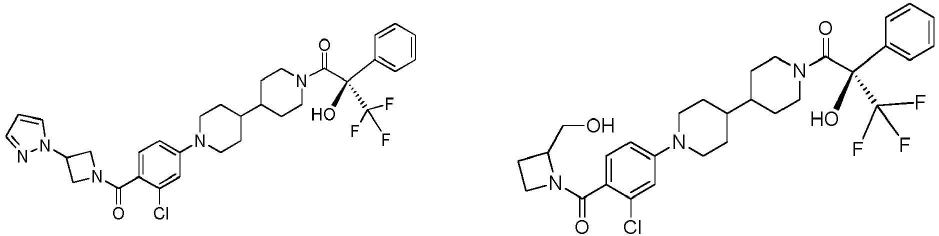

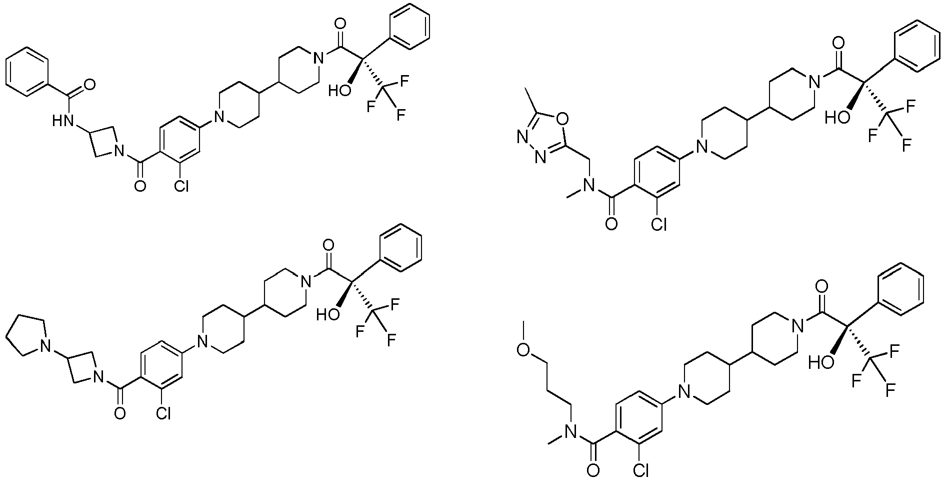

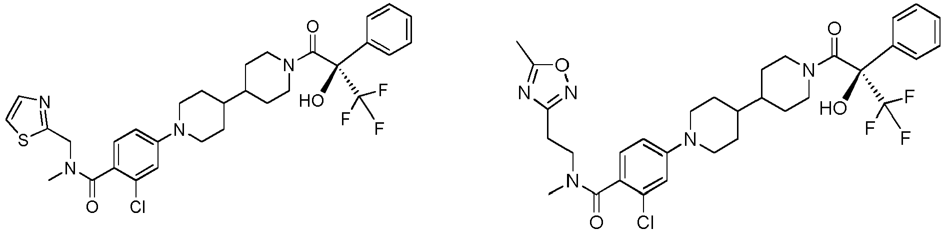

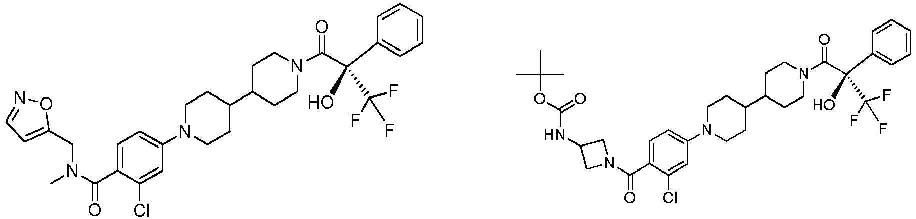

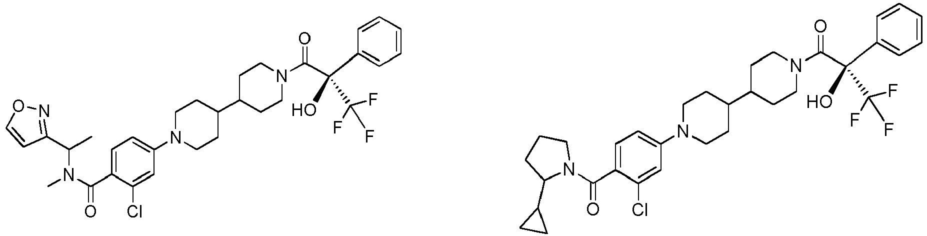

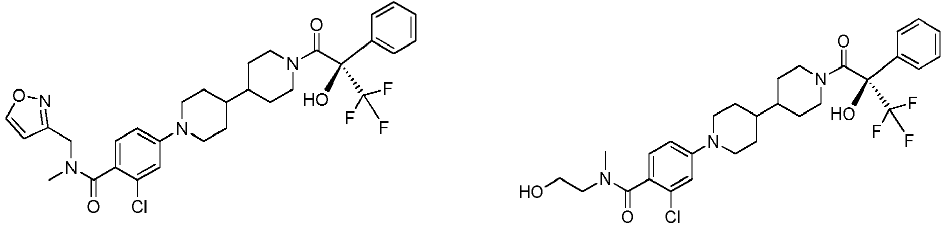

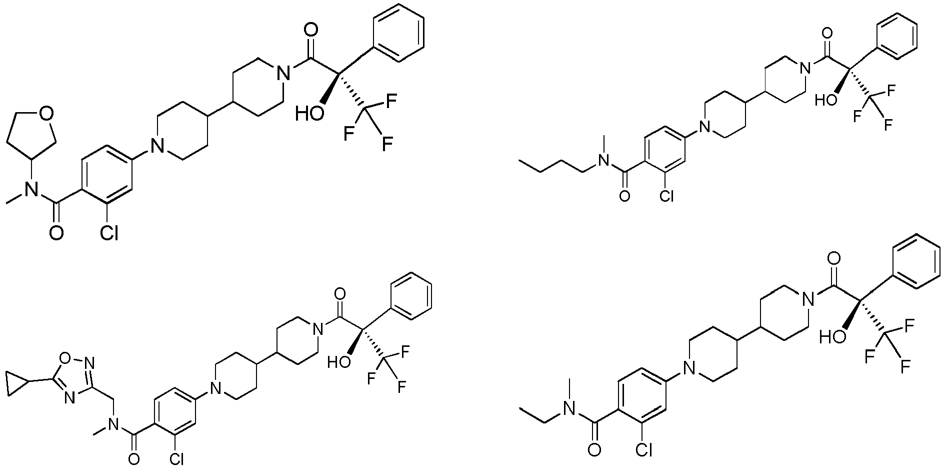

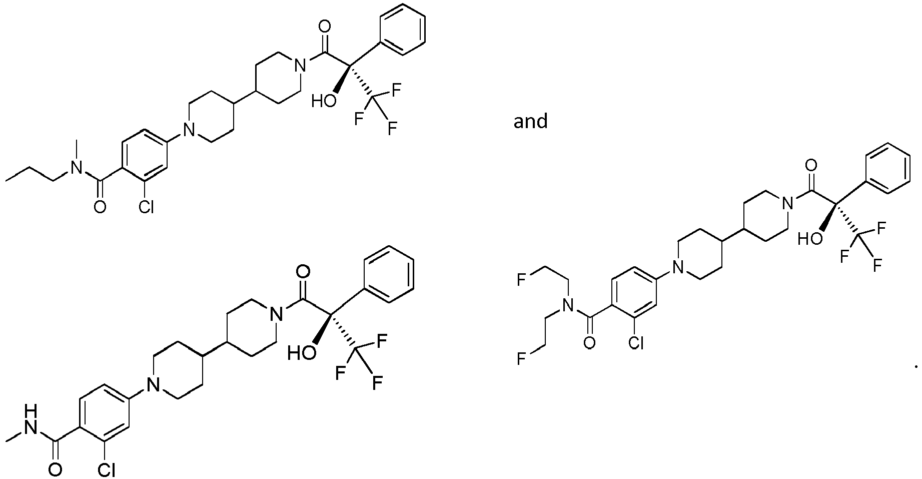

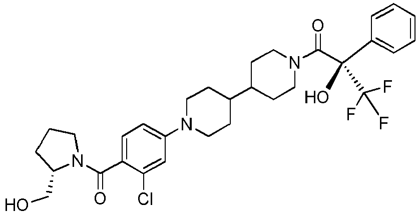

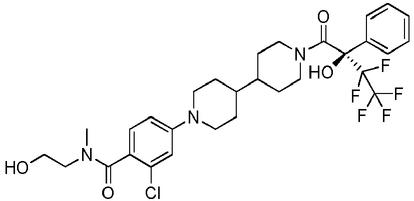

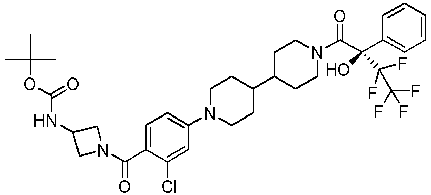

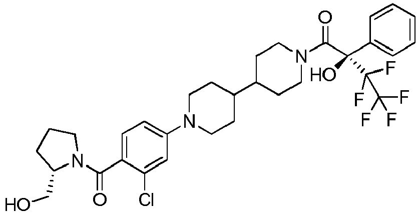

10. A pharmaceutical composition comprising a compound according to any one of claims 1 to 9, a pharmaceutically acceptable salt thereof, and a pharmaceutically acceptable carrier or diluent.

11. A compound according to any one of claims 1 to 9, or pharmaceutically acceptable salt thereof, for use as a medicament.

12. A compound according to any one of claims 1 to 9, or pharmaceutically acceptable salt thereof, for use in the treatment of Alzheimer's Disease, Neimann-Pick disease type C1, Parkinson's Disease, amyotrophic lateral sclerosis, stroke, age-related macular degeneration, schizophrenia, depression, cardiovascular disease, obesity or diabetes.

13. A pharmaceutical composition of claim 10 further comprising one or more other active ingredients.

## Patentansprüche

1. Eine Verbindung mit der Strukturformel (I): 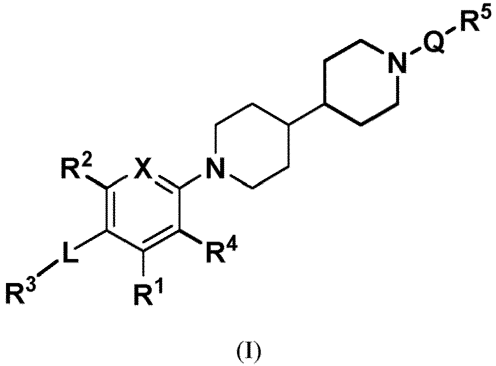 oder ein pharmazeutisch annehmbares Salz davon, wobei:
X ausgewählt ist aus der Gruppe bestehend aus -N- und -CH-,
R¹ ausgewählt ist aus der Gruppe bestehend aus H, Methyl und Halogen,
R² ausgewählt ist aus der Gruppe bestehend aus H und Halogen,
R⁴ ausgewählt ist aus der Gruppe bestehend aus H und Methyl,
L eine Bindung ist und R³ ausgewählt ist aus der Gruppe bestehend aus -(C₁-C₆)Alkyl und -(C₁-C₆)Alkyl-OH,
oder, alternativ, L eine bivalente Gruppe ist, ausgewählt aus der Gruppe bestehend aus -C(O)- und -S(O)₂-, und R³-N(R^{N1})(R^{N2}) ist, wobei:
R^{N1} ausgewählt ist aus der Gruppe bestehend aus H und -(C₁-C₆)Alkyl, und
R^{N2} ausgewählt ist aus der Gruppe bestehend aus: H, -(C₁-C₆)Alkyl,
-O-(C₁-C₆)Alkyl, -OH, Halogen, -CN und -(C₁-C₆)Alkyl, das mit 1 oder 2 Gruppen substituiert ist, unabhängig ausgewählt aus:
-OH, Halogen, -CN,
gegebenenfalls substituiertem Phenyl (wobei die optionalen Substituenten am Phenyl 1 bis 3 Gruppen, unabhängig ausgewählt aus OH, CN, -(C₁-C₄)Alkyl, -(C₁-C₄)Alkoxyl, sind),
gegebenenfalls substituiertem Heteroaryl (wobei die optionalen Substituenten am Heteroaryl 1 bis 3 Gruppen, unabhängig ausgewählt aus -(C₁-C₆)Alkyl, -(C₁-C₄)Alkoxyl und Cyclopropyl, sind),
gegebenenfalls substituiertem Cyclopropyl (wobei die optionalen Substituenten am Cyclopropyl 1 bis 3 Gruppen, unabhängig ausgewählt aus -(C₁-C₆)Alkyl, sind), und
gegebenenfalls substituiertem Heterocycloalkyl (wobei die optionalen Substituenten am Heterocycloalkyl 1 bis 3 Gruppen, unabhängig ausgewählt aus Halogen, -OH, Oxo, CN und -(C₁-C₆)Alkyl, sind),
oder, alternativ, R^{N1} und R^{N2} mit dem Stickstoffatom, an das sie gebunden gezeigt sind, zusammengenommen sind unter Bildung eines 4-, 5- oder 6-gliedrigen vollständig gesättigten heterocyclischen Rings, der (einschließlich dieses Stickstoffatoms) 1, 2 oder 3 Ring-Heteroatome, ausgewählt aus der Gruppe bestehend aus N, N-Oxid, O, S und S-Oxid, umfasst,
wobei der heterocyclische Ring unsubstituiert oder substituiert ist mit 1, 2 oder 3 Substituenten, unabhängig ausgewählt aus der Gruppe bestehend aus Halogen, - OH, Oxo, CN, -(C₁-C₆)Alkyl, aminosubstituiertem
-(C₁-C₆)Alkyl (wobei das Amino 1, 2 oder 3 Gruppen, unabhängig ausgewählt aus der Gruppe bestehend aus -NH₂, -N(C₁-C₄-Alkyl)₂ und -NH(C₁-C₄-Alkyl), ist), -O-(C₁-C₆)Alkyl, -(C₁-C₆)Alkyl-OH,
-(C₁-C₆)Halogenalkyl, -C(O)O-(C₁-C₆)Alkyl, Cyclopropyl, Spirocyclopropyl, -CH₂-NHC(O)O-(C₁-C₆)Alkyl, -CH₂-N(CH₃)C(O)O-(C₁-C₆)Alkyl, Phenyl, Benzyl, -NHC(O)-Phenyl, Heteroaryl und -(C₁-C₄)Alkylheteroaryl, Heterocycloalkyl,
Q eine Bindung oder eine bivalente Gruppe, ausgewählt aus der Gruppe bestehend aus -C(O)- und -S(O)₂-, ist, und
R⁵ ausgewählt ist aus der Gruppe bestehend aus:
-C(R^{5A})(R^{5B})(R^{5C}), wobei:
jedes von R^{5A}, R^{5B} und R^{5C} unabhängig ausgewählt ist aus der Gruppe bestehend aus: H, Halogen, OH, -(C₁-C₆)Alkyl, -(C₁-C₆)Halogenalkyl,
-(C₁-C₆)Alkenyl, -(C₁-C₆)Alkinyl, -(C₃-C₆)Cycloalkyl, -(C₃-C₆)Cycloalkyl, substituiert mit -(C₁-C₆)Alkyl, -(C₁-C₆)Alkenyl, -(C₁-C₆)Alkinyl, Phenyl, Phenyl, substituiert mit 1 bis 3 Gruppen, unabhängig ausgewählt aus Halogen, -(C₁-C₆)Alkyl, -O-(C₁-C₆)Alkyl und -C(O)O-(C₁-C₆)Alkyl, 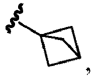 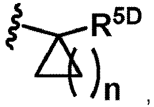 wobei:
n eine ganze Zahl von 1 bis 4 ist, und R^{5D} ausgewählt ist aus der Gruppe bestehend aus H, -(C₁-C₆)Alkyl, -(C₁-C₆)Halogenalkyl, Phenyl und Phenyl, das mit 1 bis 3 Gruppen, unabhängig ausgewählt aus der Gruppe bestehend aus OH, Halogen, - (C₁-C₆)Alkyl und -O-(C₁-C₆)Alkyl, substituiert ist, und
Phenyl, wobei:
das Phenyl unsubstituiert oder substituiert ist mit 1, 2 oder 3 Gruppen, unabhängig ausgewählt aus der Gruppe bestehend aus Halogen, CN, -(C₁-C₆)Alkyl und -(C₁-C₆)Halogenalkyl.

2. Die Verbindung nach Anspruch 1 oder ein pharmazeutisch annehmbares Salz davon, wobei:
Q eine Bindung oder eine bivalente Gruppe, ausgewählt aus der Gruppe bestehend aus - C(O)- und -S(O)₂-, ist, und
R⁵ -C(R^{5A})(R^{5B})(R^{5C}) ist,
wobei jedes von R^{5A}, R^{5B} und R^{5C} unabhängig ausgewählt ist aus der Gruppe bestehend aus: H, F, Cl, OH, -(C₁-C₆)Alkyl, -(C₁-C₆)Halogenalkyl, -(C₁-C₆)Alkenyl, - (C₁-C₆)Alkinyl, -(C₃-C₆)Cycloalkyl, -(C₃-C₆)Cycloalkyl, substituiert mit -(C₁-C₆)Alkyl, - (C₁-C₆)Alkenyl, -(C₁-C₆)Alkinyl, Phenyl, Phenyl, substituiert mit 1 bis 3 Gruppen, unabhängig ausgewählt aus F, Cl, -(C₁-C₆)Alkyl, -O-(C₁-C₆)Alkyl und -C(O)O-(C₁-C₆)Alkyl.

3. Die Verbindung nach Anspruch 1 oder ein pharmazeutisch annehmbares Salz davon, wobei:
Q eine Bindung oder eine bivalente Gruppe, ausgewählt aus der Gruppe bestehend aus - C(O)- und -S(O)₂-, ist, und
R⁵ ausgewählt ist aus der Gruppe bestehend aus 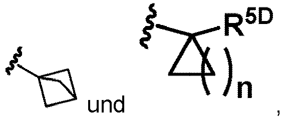 wobei
n eine ganze Zahl von 1 bis 4 ist und R^{5D} ausgewählt ist aus der Gruppe bestehend aus H, -(C₁-C₆)Alkyl,
-(C₁-C₆)Halogenalkyl, Phenyl und Phenyl, substituiert mit 1 bis 3 Gruppen, unabhängig ausgewählt aus der Gruppe bestehend aus OH, F, Cl, -(C₁-C₆)Alkyl und -O-(C₁-C₆)Alkyl.

4. Die Verbindung nach Anspruch 1 oder ein pharmazeutisch annehmbares Salz davon, wobei:
R⁵ Phenyl ist,
wobei das Phenyl unsubstituiert oder substituiert ist mit 1, 2 oder 3 Gruppen, unabhängig ausgewählt aus der Gruppe bestehend aus F, Cl, CN, -(C₁-C₆)Alkyl und -(C₁-C₆) Halogenalkyl.

5. Die Verbindung gemäß einem vorhergehenden Anspruch oder ein pharmazeutisch annehmbares Salz davon, wobei:
L -C(O)- ist und R³ -N(R^{N1})(R^{N2}) ist, wobei:
R^{N1} ausgewählt ist aus der Gruppe bestehend aus H und -(C₁-C₆)Alkyl, und
R^{N2} -(C₁-C₆)Alkyl ist, das gegebenenfalls substituiert ist mit 1 oder 2 Gruppen, unabhängig ausgewählt aus:
gegebenenfalls substituiertem Phenyl (wobei die optionalen Substituenten am Phenyl 1 bis 3 Gruppen, unabhängig ausgewählt aus OH, CN, -(C₁-C₄)Alkyl, -(C₁-C₄)Alkoxyl, sind),
gegebenenfalls substituiertem Heteroaryl (wobei die optionalen Substituenten am Heteroaryl 1 bis 3 Gruppen, unabhängig ausgewählt aus -(C₁-C₆)Alkyl, -(C₁-C₄)Alkoxyl und Cyclopropyl, sind),
gegebenenfalls substituiertem Cyclopropyl (wobei die optionalen Substituenten am Cyclopropyl 1 bis 3 Gruppen, unabhängig ausgewählt aus -(C₁-C₆)Alkyl, sind),
gegebenenfalls substituiertem Heterocycloalkyl (wobei die optionalen Substituenten am Heterocycloalkyl 1 bis 3 Gruppen, unabhängig ausgewählt aus Halogen, -OH, Oxo, CN und -(C₁-C₆)Alkyl, sind), und
-O-(C₁-C₆)Alkyl, -OH, F, CI und -CN.

6. Die Verbindung gemäß einem der Ansprüche 1 bis 4 oder ein pharmazeutisch annehmbares Salz davon, wobei:
L ausgewählt ist aus der Gruppe bestehend aus -C(O)- und -S(O)₂, und
R³ -N(R^{N1})(R^{N2}) ist, wobei R^{N1} und R^{N2} jeweils unabhängig ausgewählt sind aus der Gruppe bestehend aus H und -(C₁-C₆)Alkyl.

7. Die Verbindung gemäß einem der Ansprüche 1 bis 4 oder ein pharmazeutisch annehmbares Salz davon, wobei:
L -C(O)- ist und R³ -N(R^{N1})(R^{N2}) ist, wobei:
R^{N1} und R^{N2} mit dem Stickstoffatom, an das sie gebunden gezeigt sind, zusammengenommen sind unter Bildung eines 4-, 5- oder 6-gliedrigen vollständig gesättigten heterocyclischen Rings, der (einschließlich dieses Stickstoffatoms) 1, 2 oder 3 Ring-Heteroatome, ausgewählt aus der Gruppe bestehend aus N, N-Oxid, O, S und S-Oxid, umfasst,
wobei der heterocyclische Ring unsubstituiert oder substituiert ist mit 1, 2 oder 3 Substituenten, unabhängig ausgewählt aus der Gruppe bestehend aus Halogen, - OH, Oxo, CN, -(C₁-C₆)Alkyl, aminosubstituiertem
-(C₁-C₆)Alkyl (wobei das Amino 1, 2 oder 3 Gruppen, unabhängig ausgewählt aus der Gruppe bestehend aus -NH₂, -N(C₁-C₄-Alkyl)₂ und -NH(C₁-C₄-Alkyl), ist), -O-(C₁-C₆)Alkyl, -(C₁-C₆)Alkyl-OH,
-(C₁-C₆)Halogenalkyl, -C(O)O-(C₁-C₆)Alkyl, Cyclopropyl, Spirocyclopropyl, -CH₂-NHC(O)O-(C₁-C₆)Alkyl, -CH₂-N(CH₃)C(O)O-(C₁-C₆)Alkyl, Phenyl, Benzyl, -NHC(O)-Phenyl, Heteroaryl und -(C₁-C₄)Alkylheteroaryl, Heterocycloalkyl.

8. Die Verbindung gemäß einem der Ansprüche 1 bis 4 oder ein pharmazeutisch annehmbares Salz davon, wobei:
L eine Bindung ist und R³ -(C₁-C₆)Alkyl ist, das gegebenenfalls mit OH substituiert ist.

9. Die Verbindung gemäß Anspruch 1 oder ein pharmazeutisch annehmbares Salz davon, wobei die Verbindung ausgewählt ist aus der Gruppe bestehend aus: 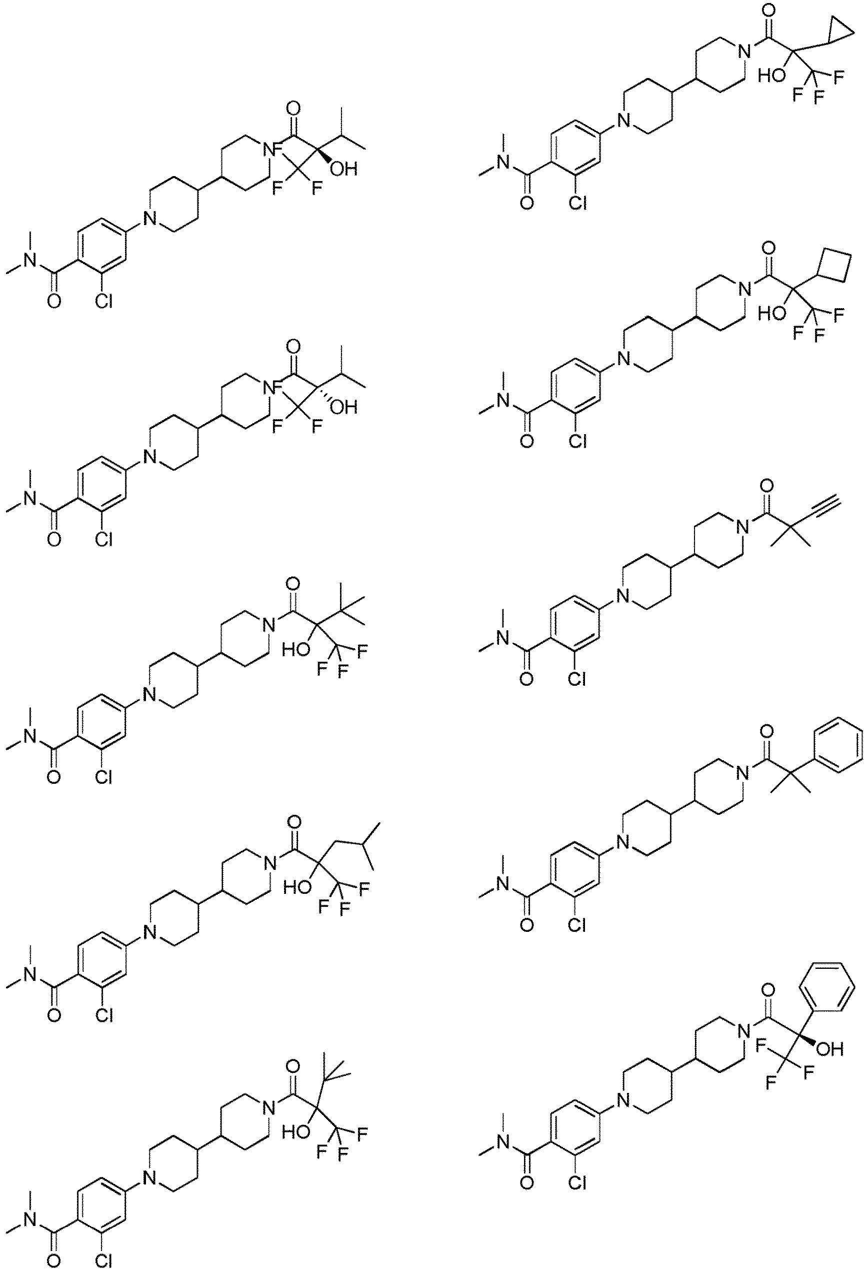 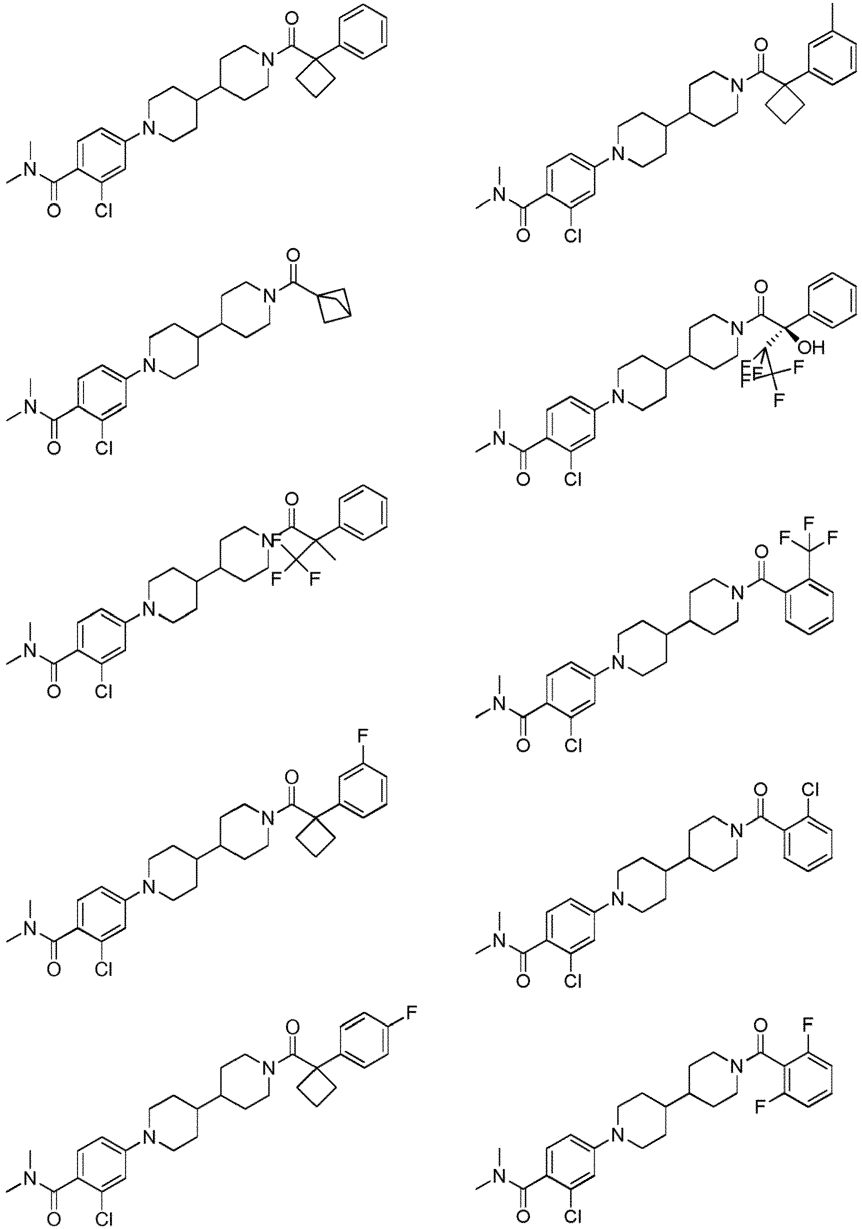 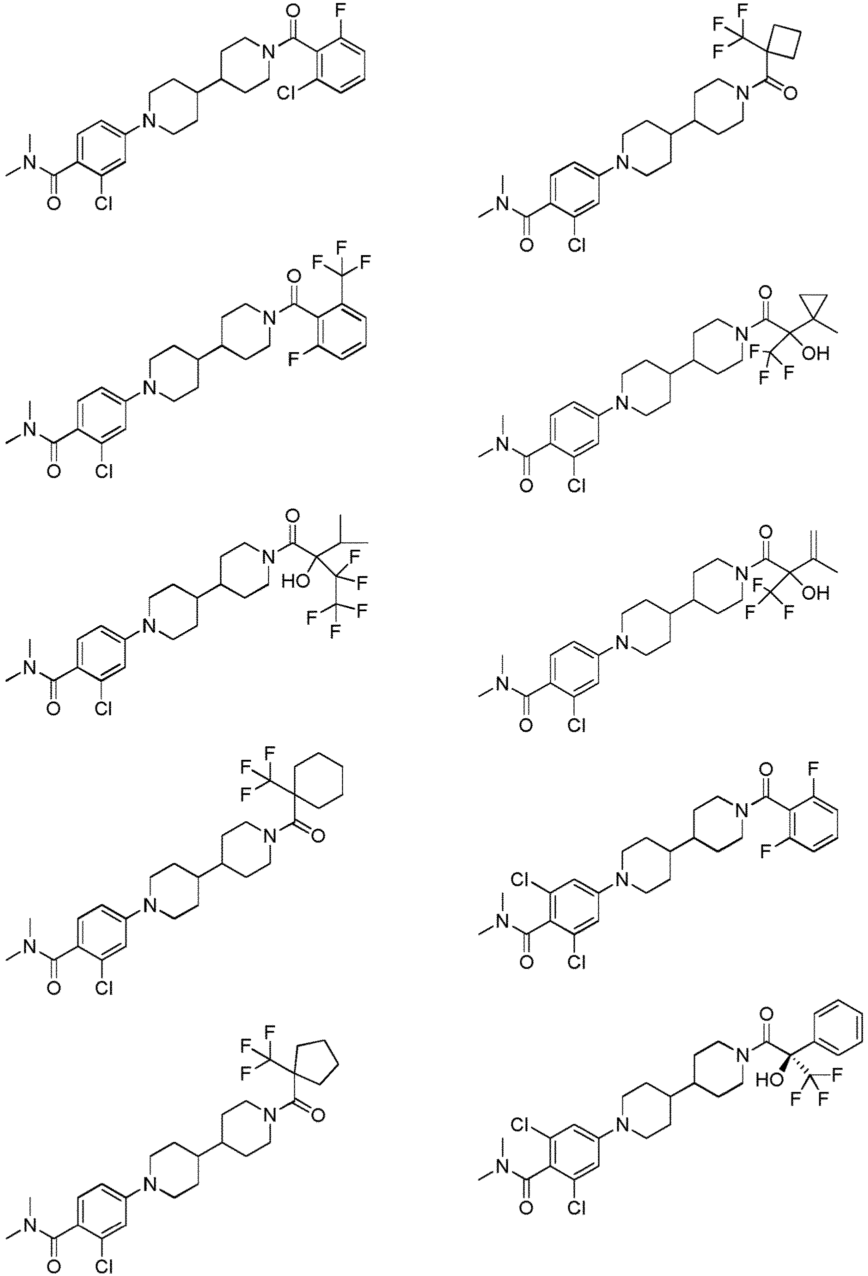 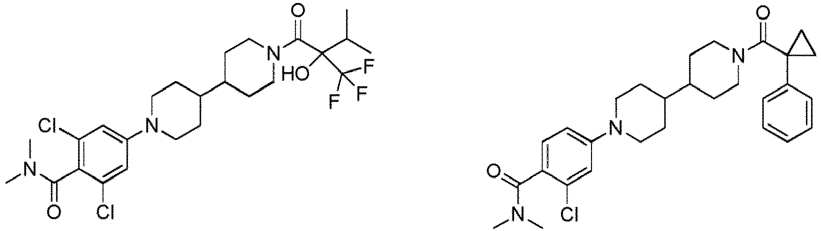 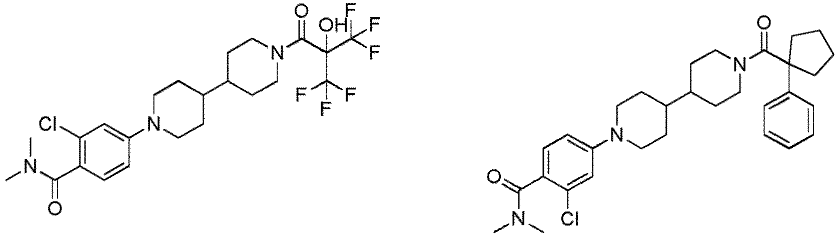 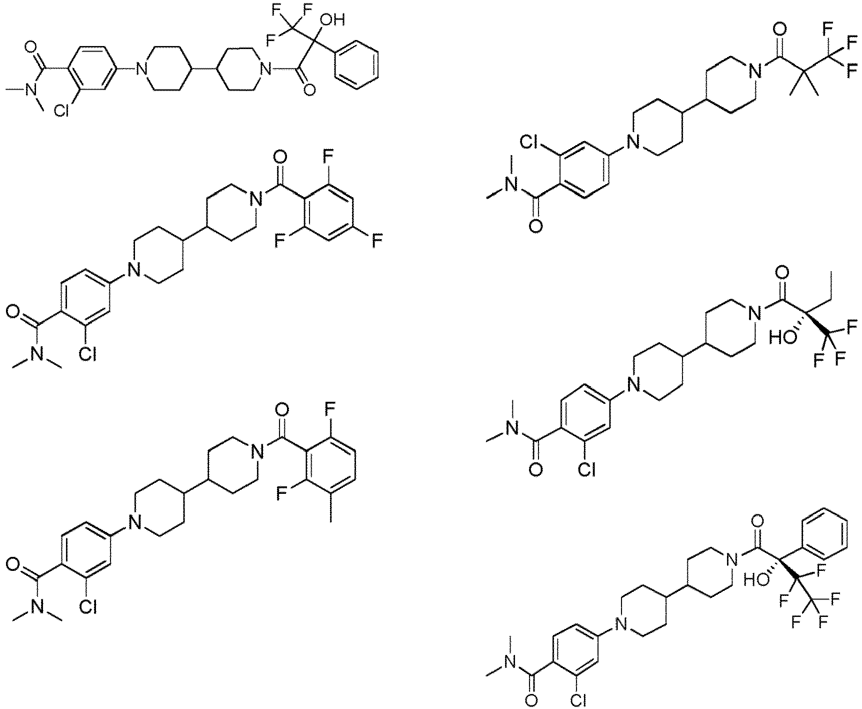 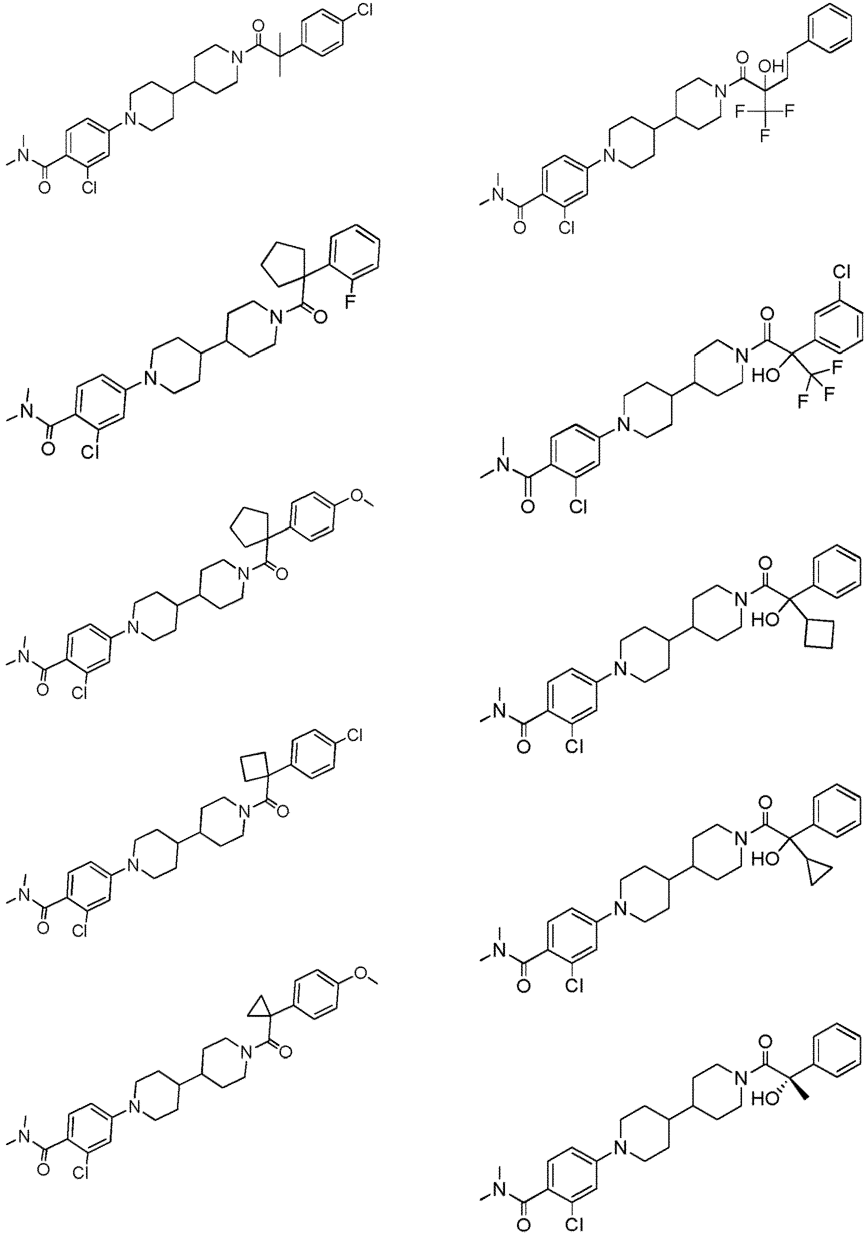 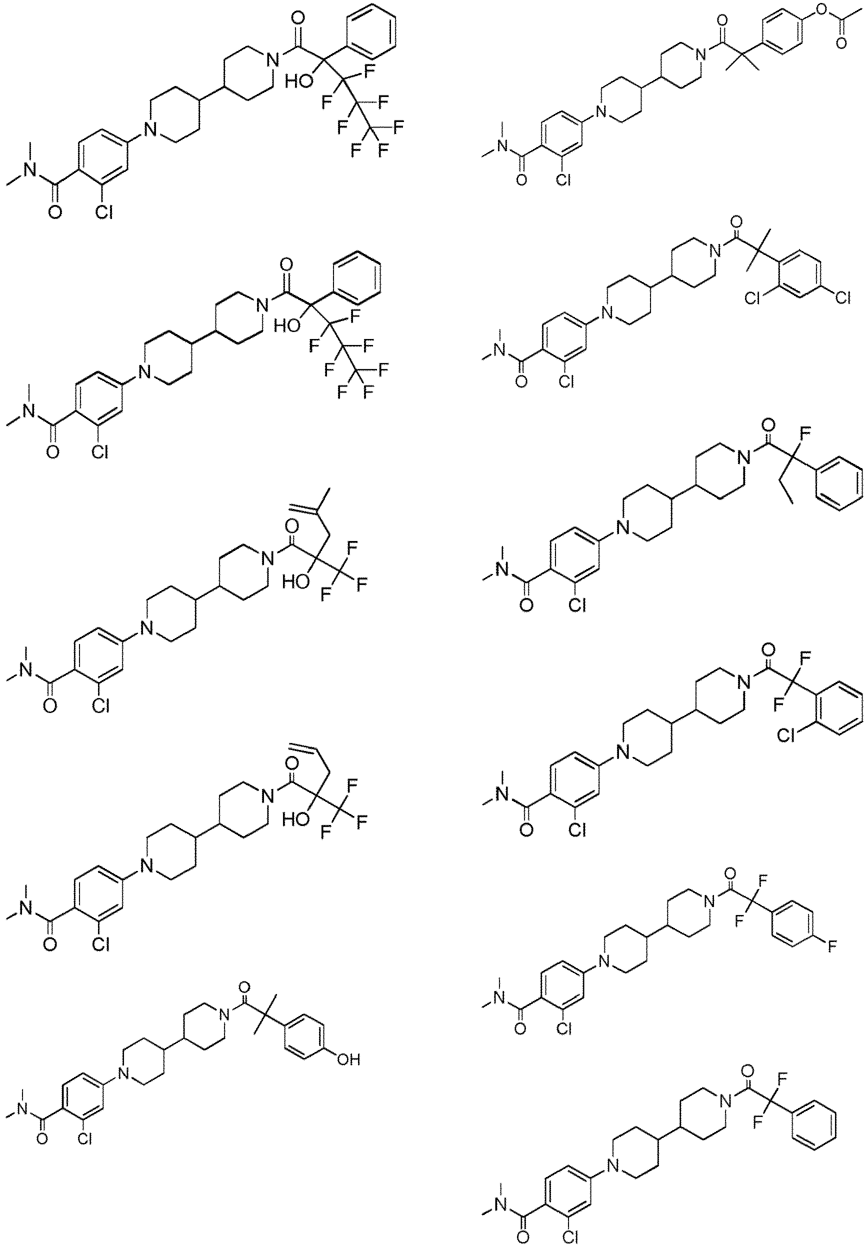 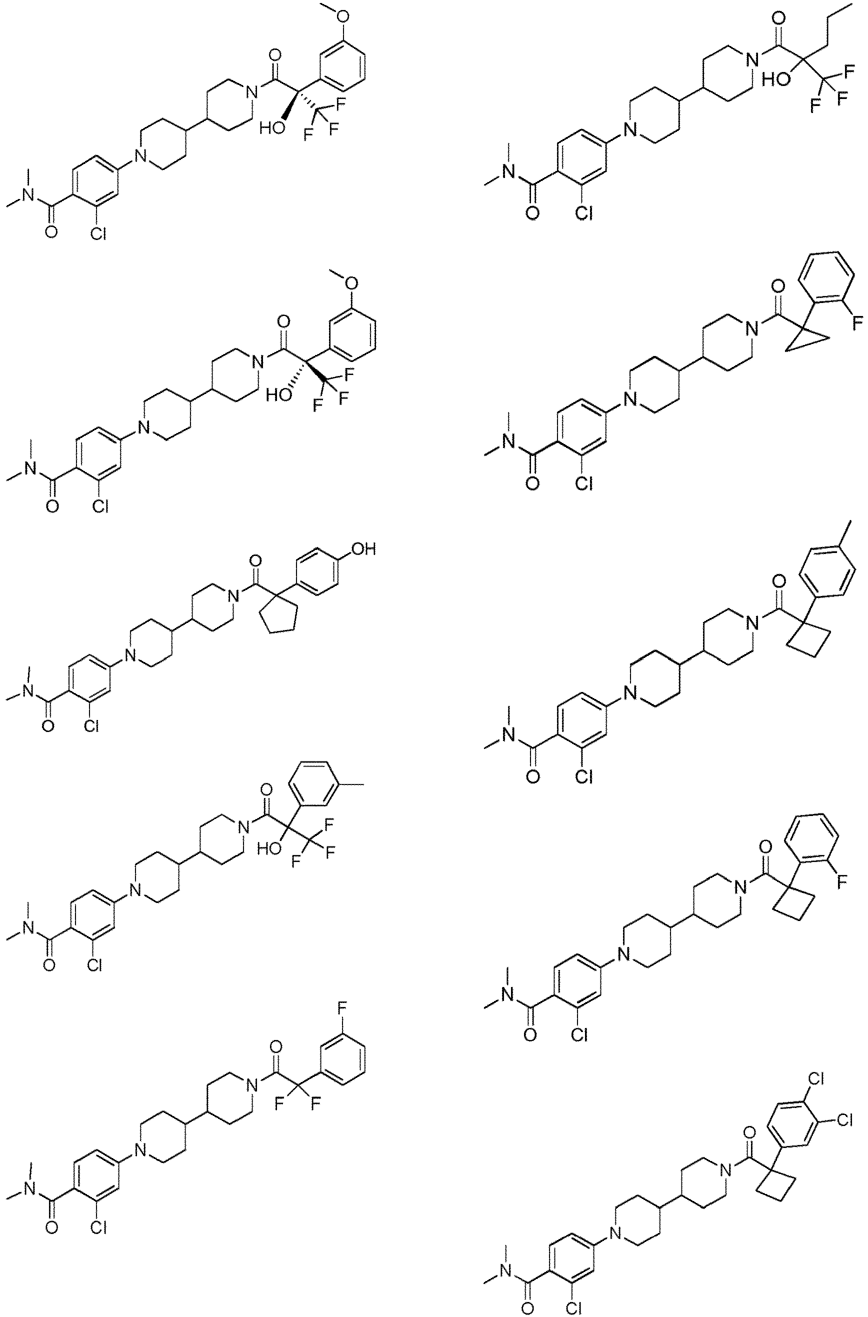 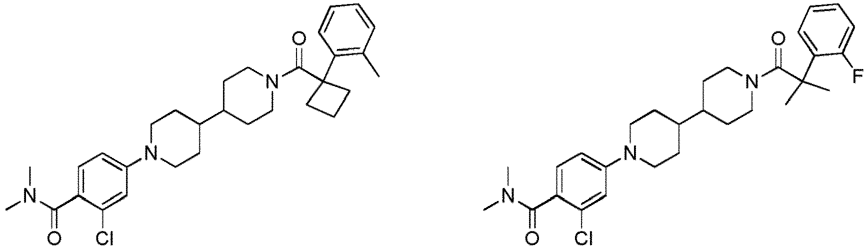 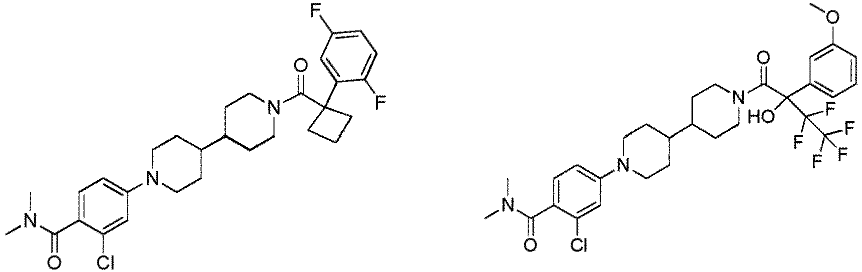 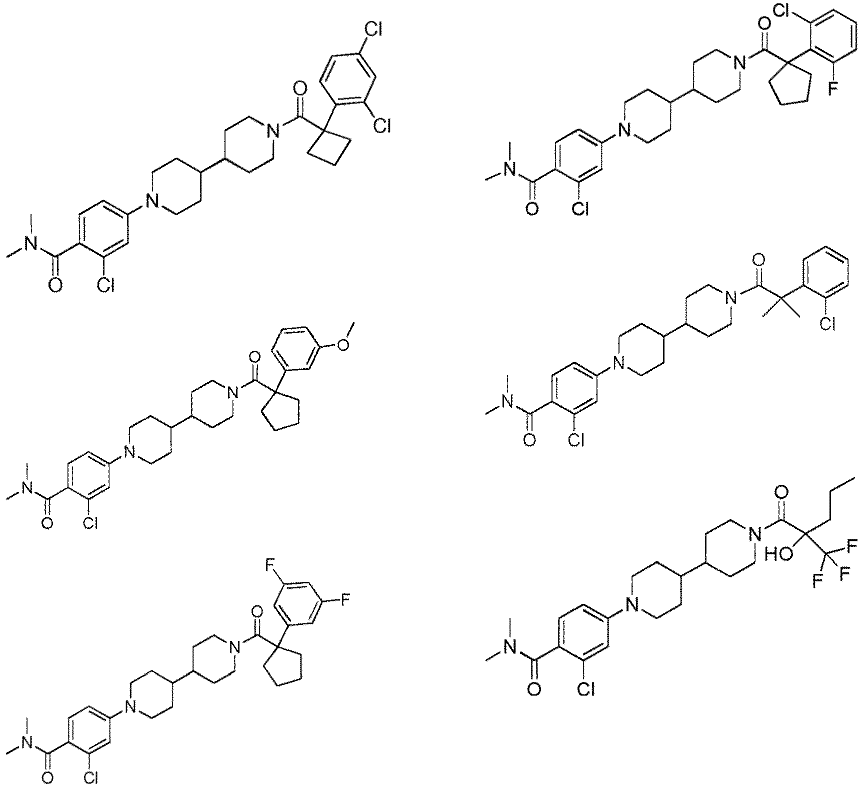 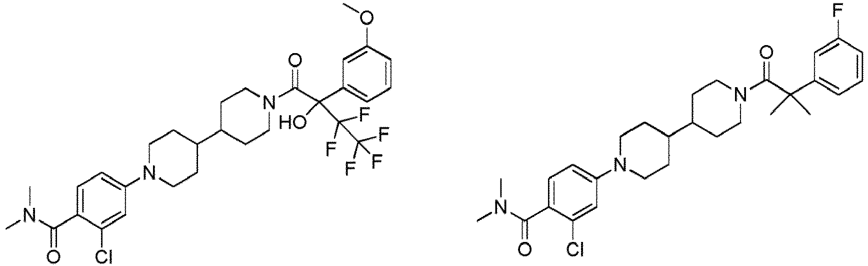 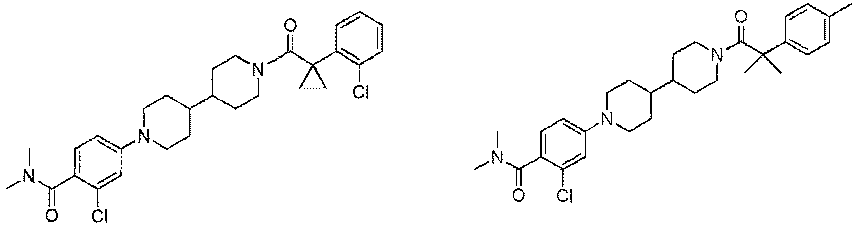 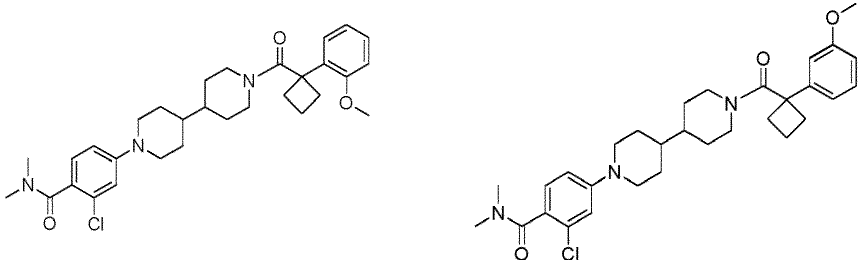 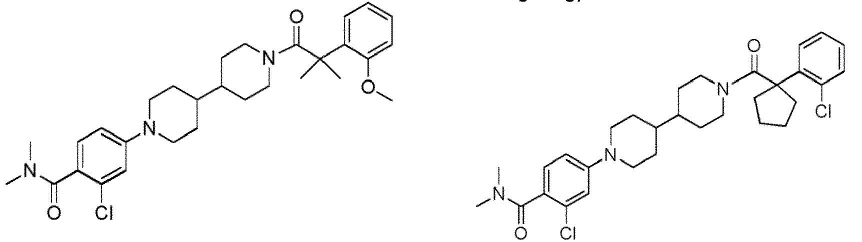 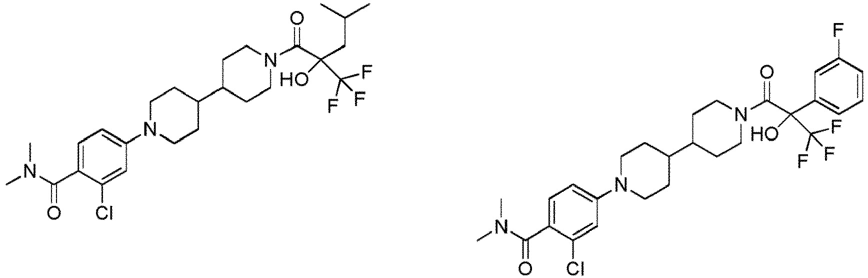 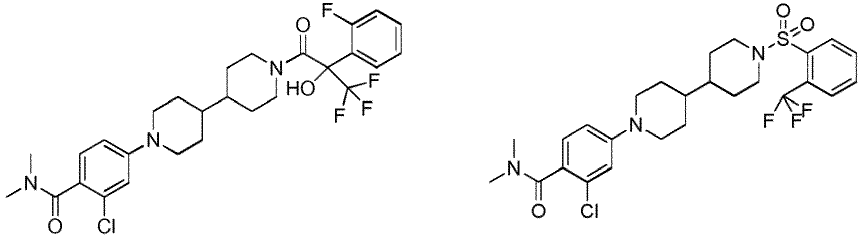 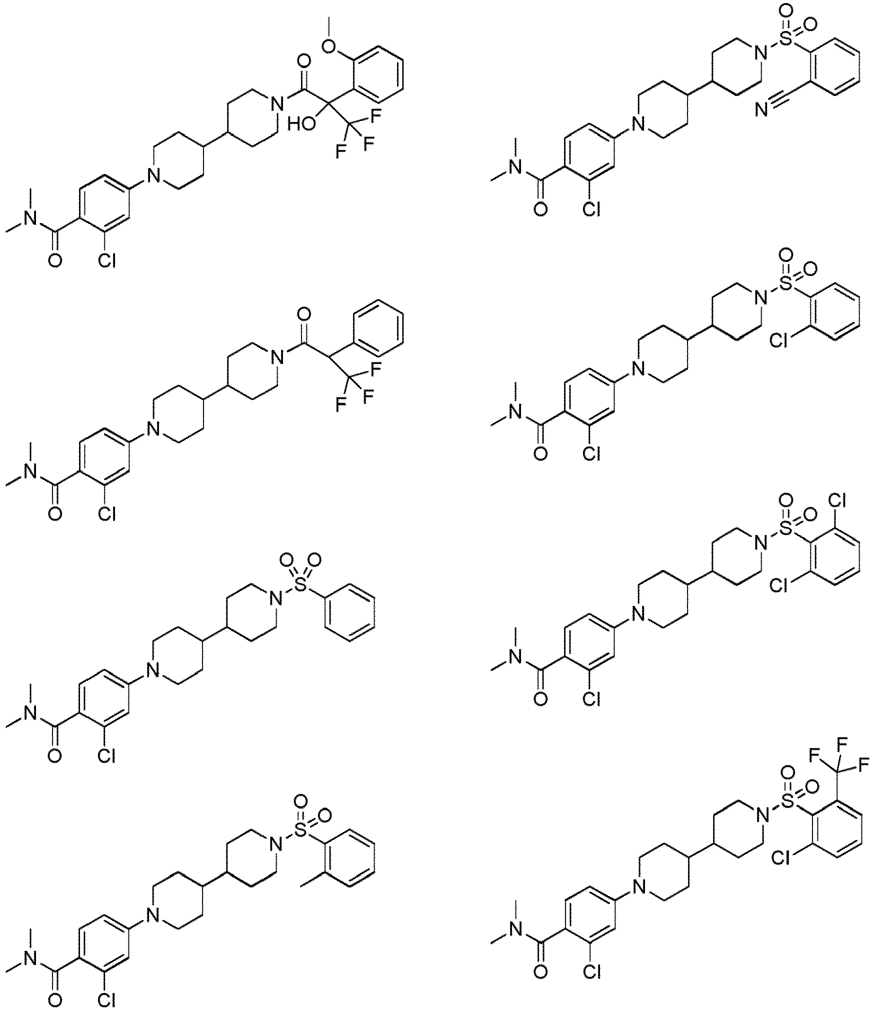 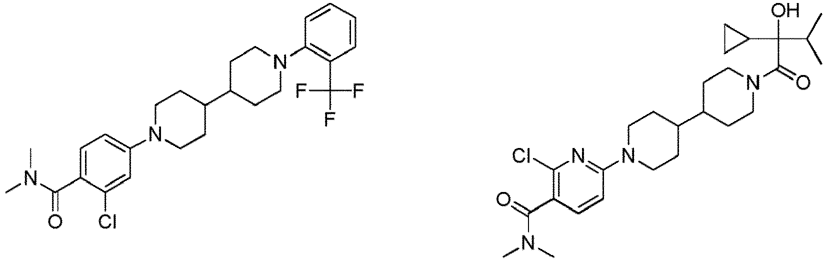 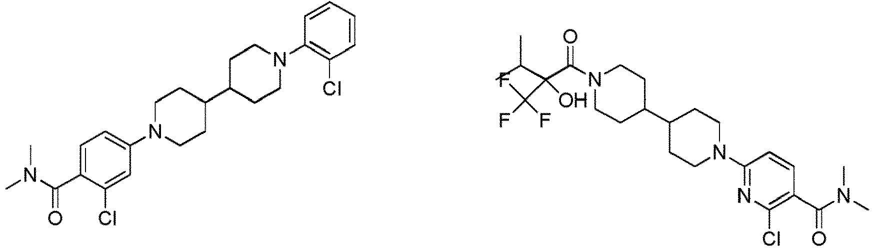 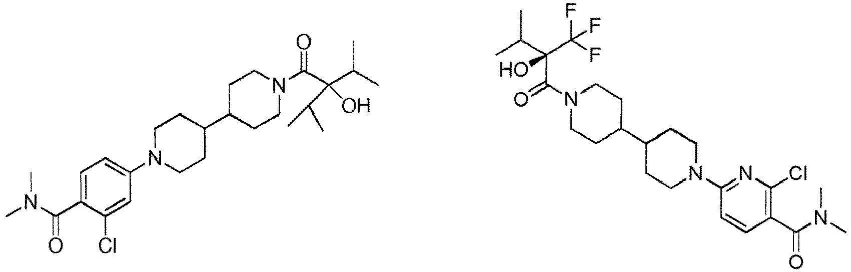 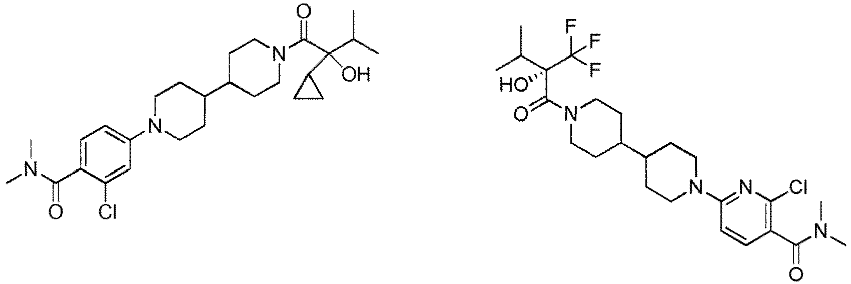 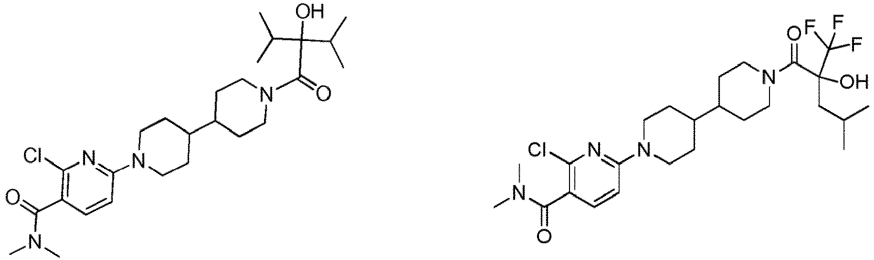 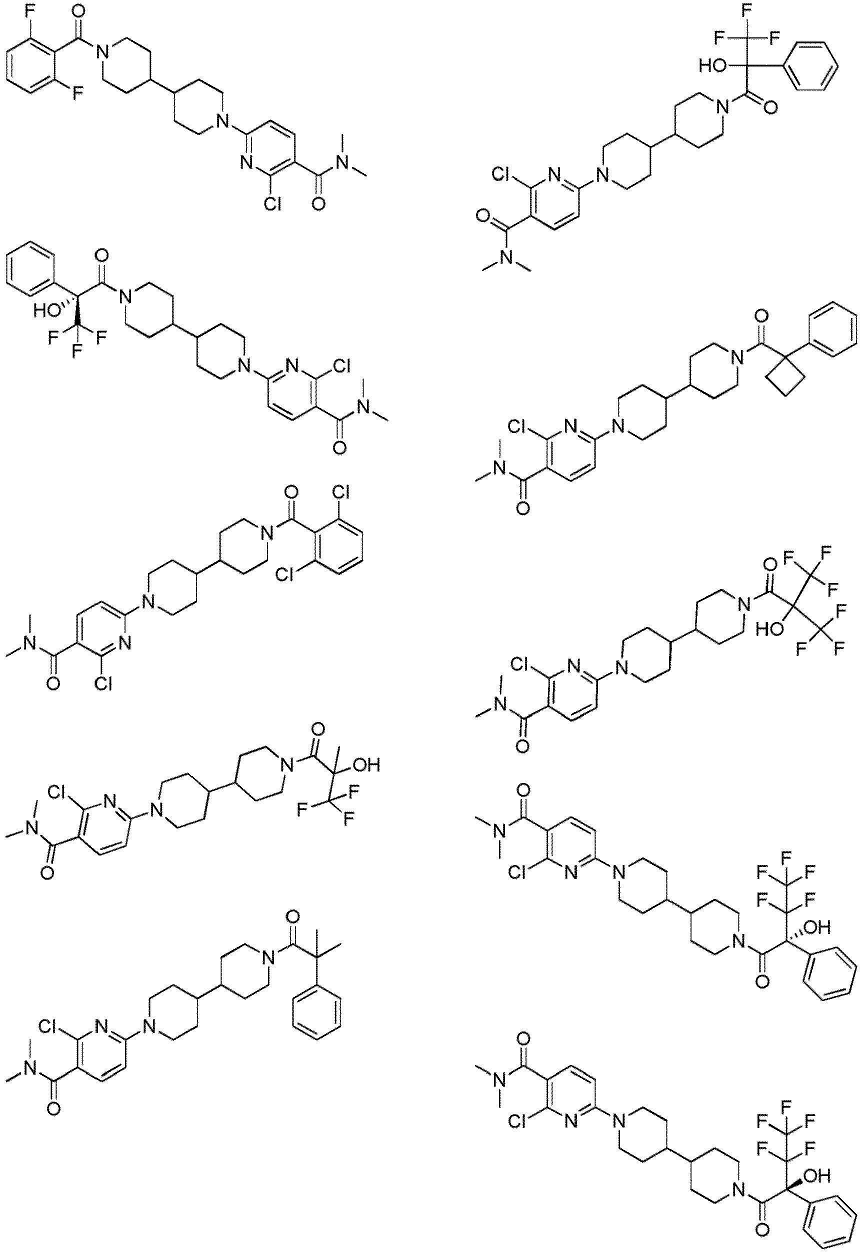 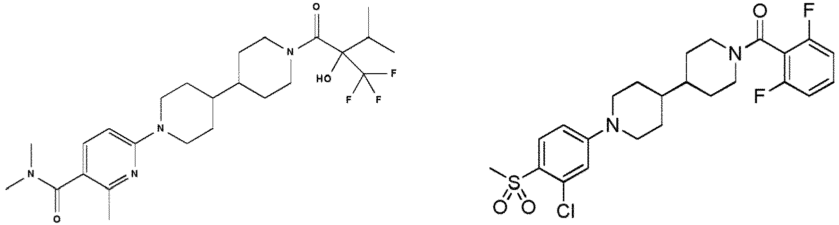 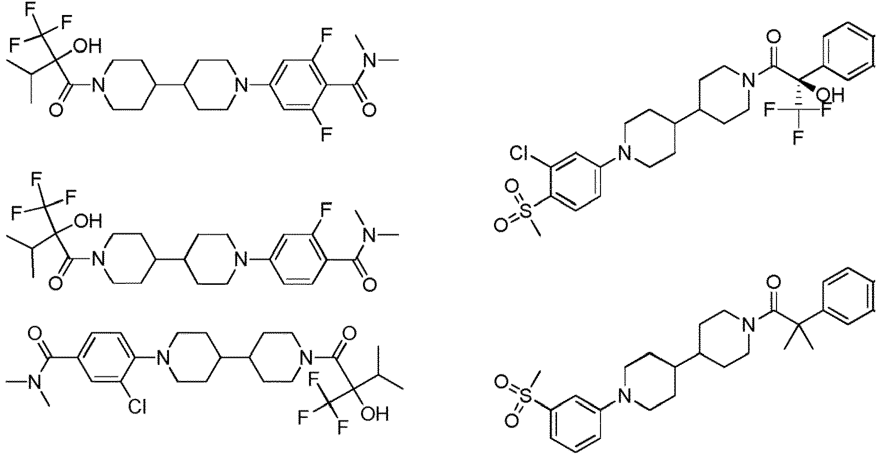 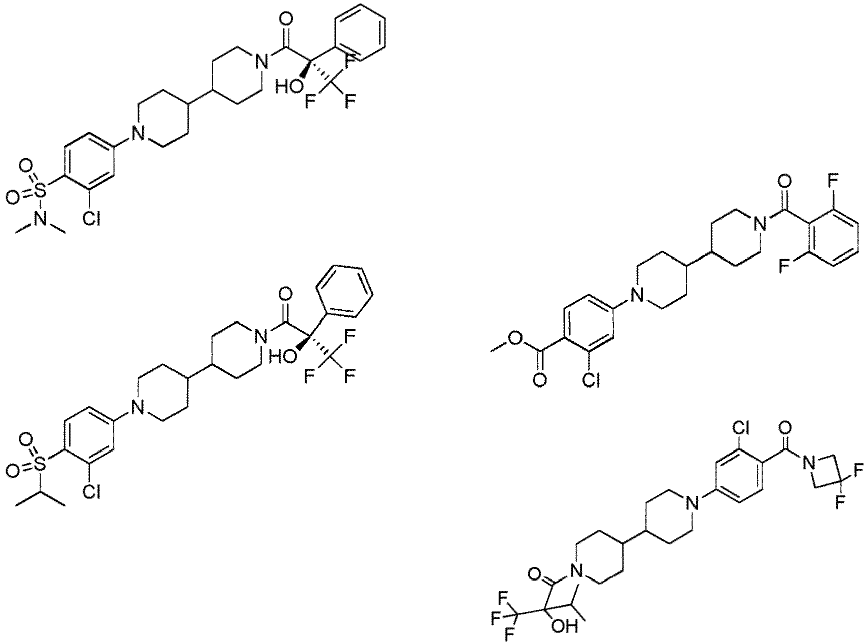 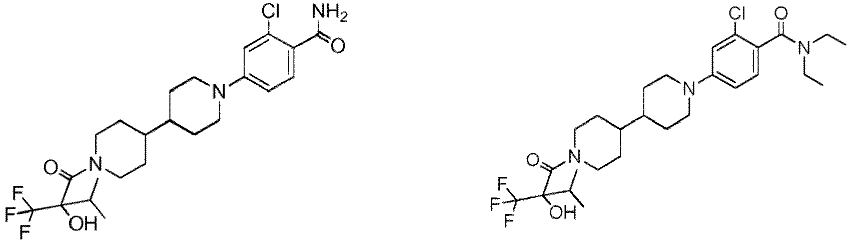 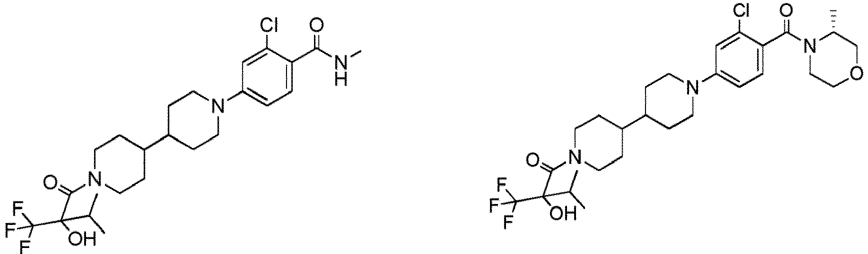 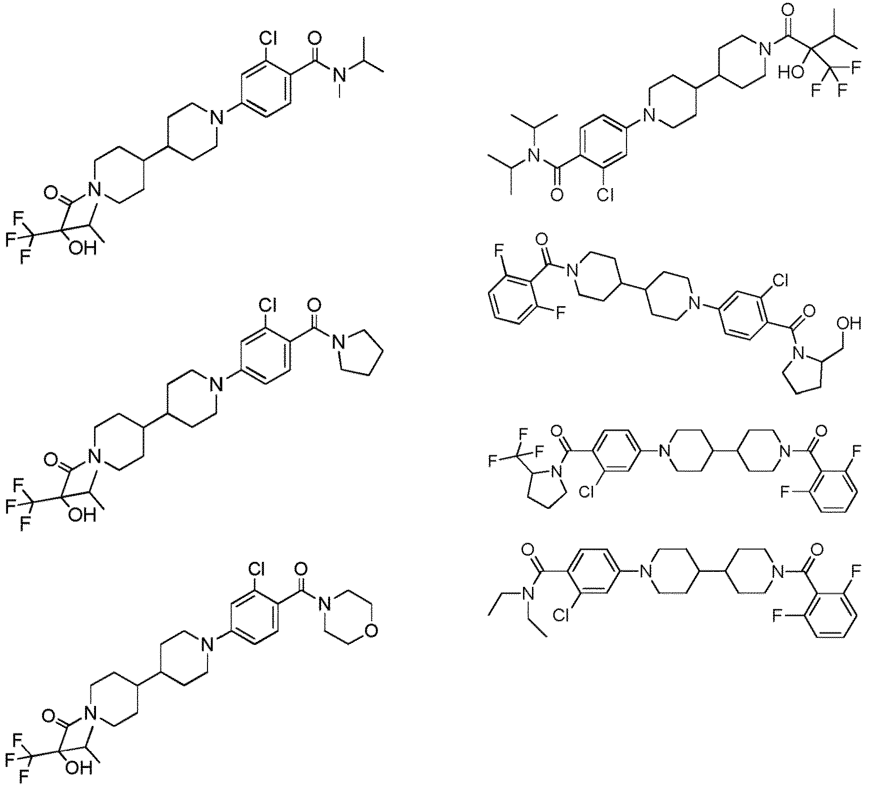 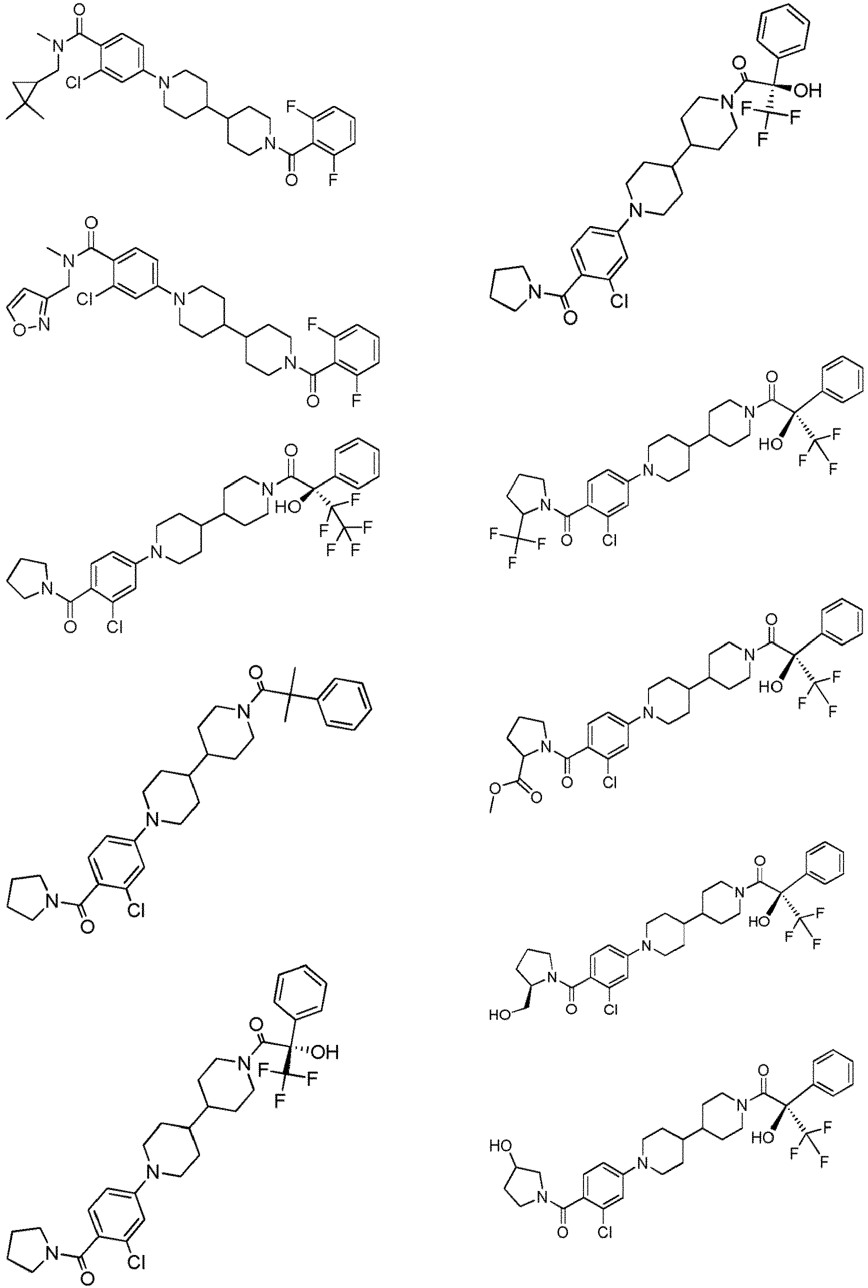 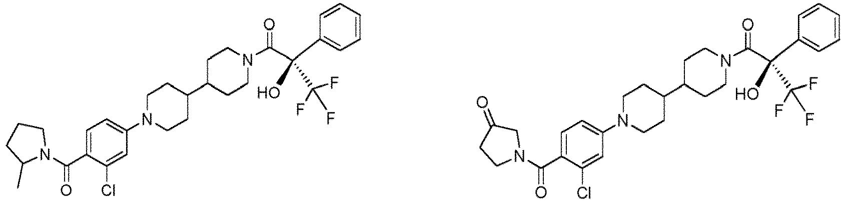 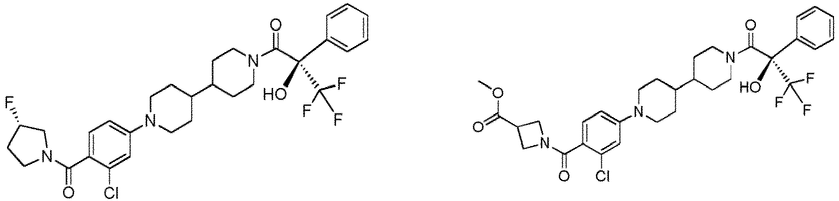 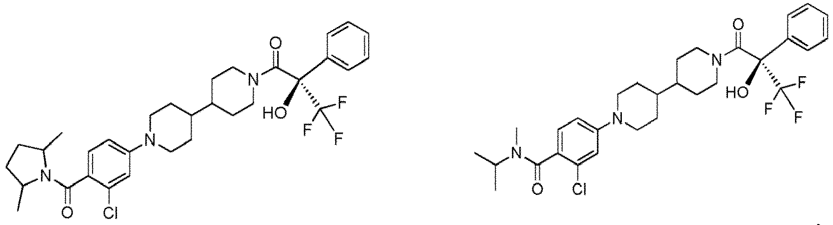 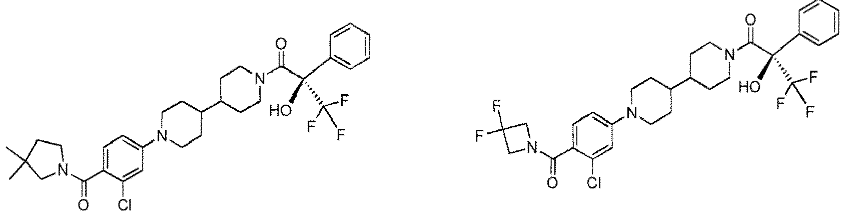 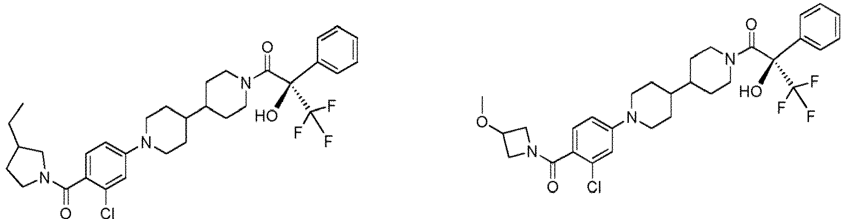 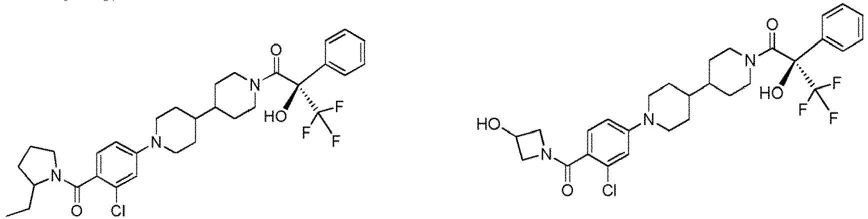 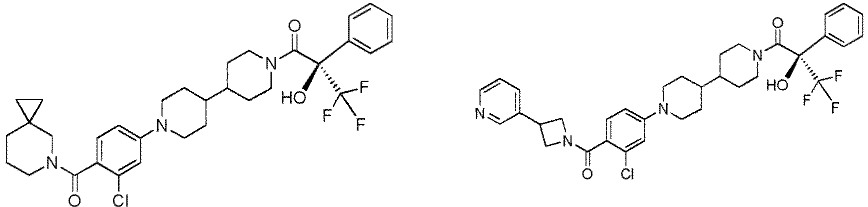 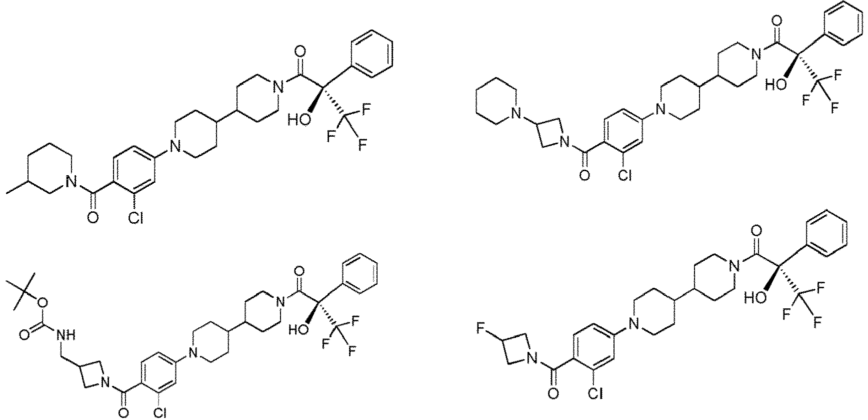 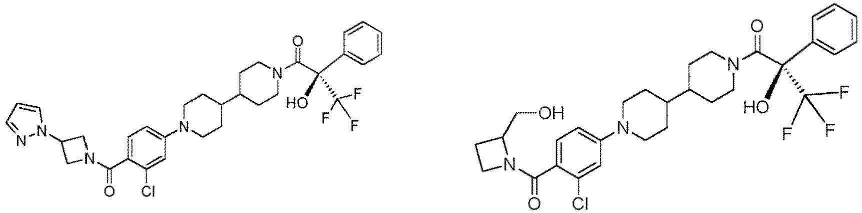 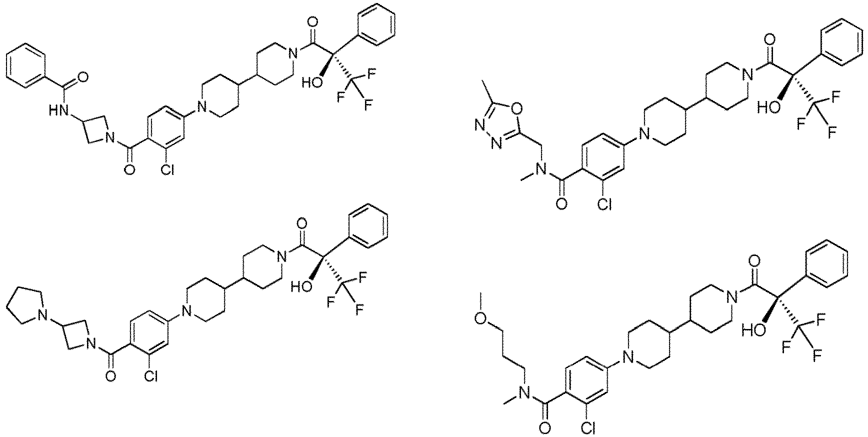 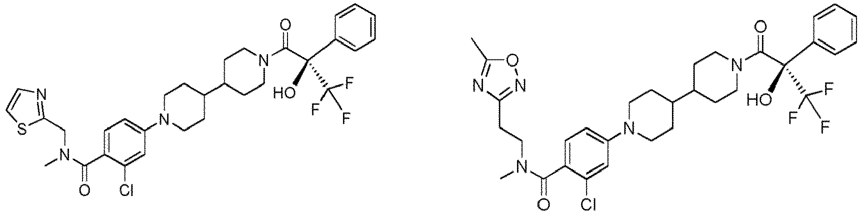 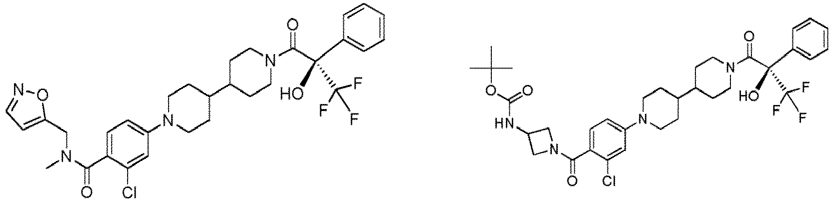 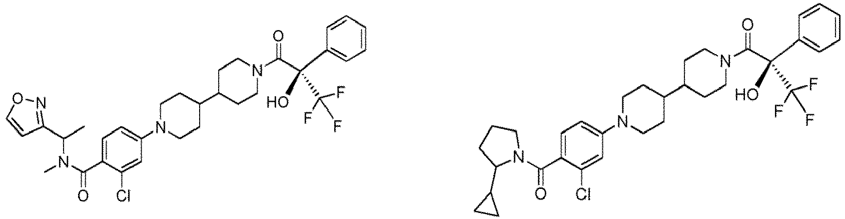 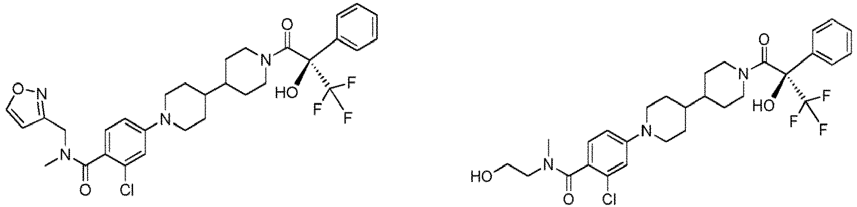 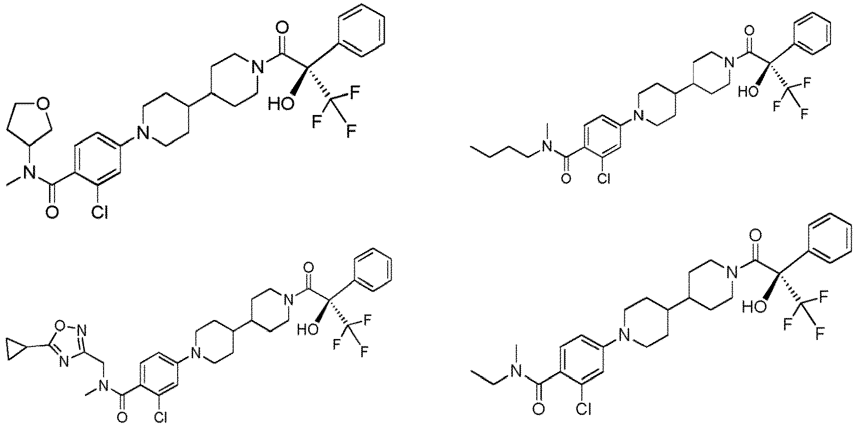 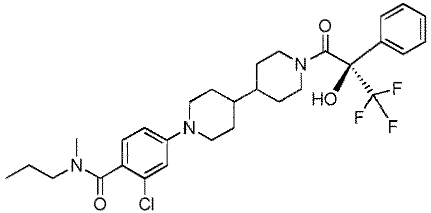 und

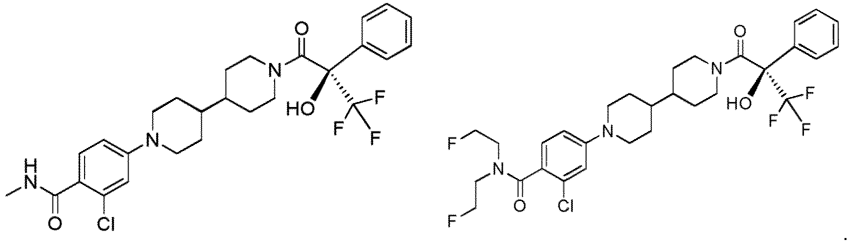

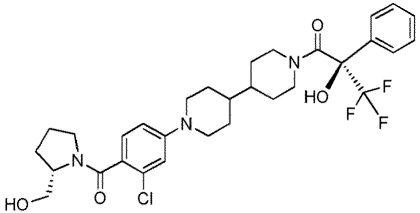

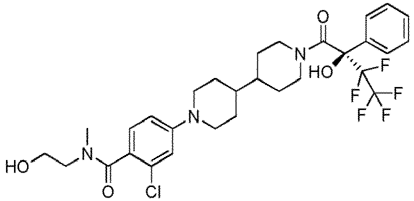

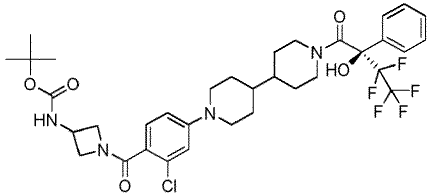

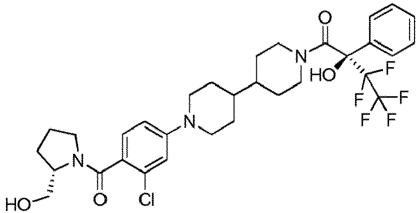

10. Eine pharmazeutische Zusammensetzung, die eine Verbindung gemäß einem der Ansprüche 1 bis 9, ein pharmazeutisch annehmbares Salz davon und einen pharmazeutisch annehmbaren Träger oder ein pharmazeutisch annehmbares Verdünnungsmittel umfasst.

11. Eine Verbindung gemäß einem der Ansprüche 1 bis 9 oder ein pharmazeutisch annehmbares Salz davon zur Verwendung als Medikament.

12. Eine Verbindung gemäß einem der Ansprüche 1 bis 9 oder ein pharmazeutisch annehmbares Salz davon zur Verwendung bei der Behandlung von Alzheimer-Krankheit, Neimann-Pick-Krankheit Typ C1, Parkinson-Krankheit, amyotropher Lateralsklerose, Schlaganfall, altersbedingter Makuladegeneration, Schizophrenie, Depression, kardiovaskulärer Erkrankung, Adipositas oder Diabetes.

13. Eine pharmazeutische Zusammensetzung nach Anspruch 10, die ferner ein oder mehrere andere Wirkstoffe umfasst.

## Revendications

1. Composé présentant la Formule structurale (I): 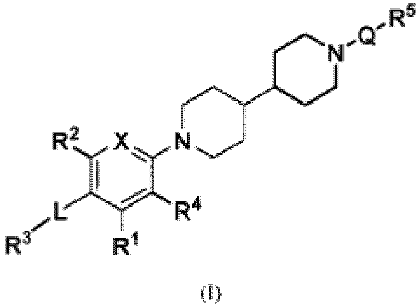 ou un sel pharmaceutiquement acceptable de celui-ci, dans lequel:
X est choisi dans le groupe constitué de -N- et -CH-;
R¹ est choisi dans le groupe constitué de H, méthyle et halogène;
R² est choisi dans le groupe constitué de H et halogène;
R⁴ est choisi dans le groupe constitué de H et méthyle;
L est une liaison et R³ est choisi dans le groupe constitué de -(C₁-C₆)alkyle et -(C₁-C₆)alkyl-OH;
ou, selon une variante, L est une fraction divalente choisie dans le groupe constitué de - C(O)- et -S(O)₂-; et R³ est -N(R^{N1})(R^{N2}), dans lequel:
R^{N1} est choisi dans le groupe constitué de H et -(C₁-C₆)alkyle; et
R^{N2} est choisi dans le groupe constitué de: H, -(C₁-C₆)alkyle, -O-(C₁-C₆)alkyle, -OH, halogène, -CN et -(C₁-C₆)alkyle qui est substitué avec 1 ou 2 groupes indépendamment choisis parmi:
-OH, halogène, -CN,
phényle optionnellement substitué, (dans lequel lesdits substituants optionnels sur ledit phényle sont 1 à 3 groupes indépendamment choisis parmi OH, CN, -(C₁-C₄)alkyle, -(C₁-C₄)alcoxyle),
hétéroaryle optionnellement substitué, (dans lequel lesdits substituants optionnels sur ledit hétéroaryle sont 1 à 3 groupes indépendamment choisis parmi -(C₁-C₆)alkyle, -(C₁-C₄)alcoxyle et cyclopropyle),
cyclopropyle optionnellement substitué, (dans lequel lesdits substituants optionnels sur ledit cyclopropyle sont 1 à 3 groupes indépendamment choisis parmi -(C₁-C₆)alkyle), et
hétérocycloalkyle optionnellement substitué, (dans lequel lesdits substituants optionnels sur ledit hétérocycloalkyle sont 1 à 3 groupes indépendamment choisis parmi halogène, -OH, oxo, CN et -(C₁-C₆)alkyle),
ou, selon une variante, R^{N1} et R^{N2} sont pris ensemble avec l'atome d'azote auquel ils sont montrés attachés pour former un cycle hétérocyclique de 4, 5 ou 6 membres totalement saturé comprenant (y compris ledit atome d'azote) 1, 2 ou 3 hétéroatomes de cycle choisis dans le groupe constitué de N, N-oxyde, O, S et S-oxyde,
dans lequel ledit cycle hétérocyclique est non substitué ou substitué avec 1, 2 ou 3 substituants indépendamment choisis dans le groupe constitué de halogène, -OH, oxo, CN, -(C₁-C₆)alkyle, -(C₁-C₆)alkyle à substitution amino (dans lequel ledit amino est 1, 2 ou 3 groupes indépendamment choisis dans le groupe constitué de -NH₂, -N(C₁-C₄ alkyle)₂ et -NH(C₁-C₄ alkyle)), -O-(C₁-C₆)alkyle, -(C₁-C₆)alkyl-OH, -(C₁-C₆)haloalkyle, -C(O)O-(C₁-C₆)alkyle, cyclopropyle, spirocyclopropyle, -CH₂-NHC(O)O-(C₁-C₆)alkyle, -CH₂-N(CH₃)C(O)O-(C₁-C₆)alkyle, phényle, benzyle, -NHC(O)-phényle, hétéroaryle et -(C₁-C₄)alkylhétéroaryle, hétérocycloalkyle;
Q est une liaison ou une fraction divalente choisie dans le groupe constitué de -C(O)- et - S(O)₂-; et
R⁵ est choisi dans le groupe constitué de:
-C(R^{5A})(R^{5B})(R^{5C}), dans lequel:
chacun de R^{5A}, R^{5B} et R^{5C} est indépendamment choisi dans le groupe constitué de: H, halogène, OH, -(C₁-C₆)alkyle, -(C₁-C₆)haloalkyle, -(C₁-C₆)alcényle, -(C₁-C₆)alcynyle, -(C₃-C₆)cycloalkyle, -(C₃-C₆)cycloalkyle substitué avec -(C₁-C₆)alkyle, -(C₁-C₆)alcényle, -(C₁-C₆)alcynyle, phényle, phényle substitué avec de 1 à 3 groupes indépendamment choisis parmi halogène, -(C₁-C₆)alkyle, -O-(C₁-C₆)alkyle et -C(O)O-(C₁-C₆)alkyle, 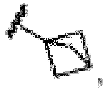 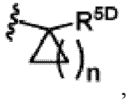 dans lequel:
n est un nombre entier de 1 à 4; et R^{5D} est choisi dans le groupe constitué de H, -(C₁-C₆)alkyle, -(C₁-C₆)haloalkyle, phényle et phényle substitué avec de 1 à 3 groupes indépendamment choisis dans le groupe constitué de OH, halogène, -(C₁-C₆)alkyle et -O-(C₁-C₆)alkyle, et
phényle, dans lequel:
ledit phényle est non substitué ou substitué avec 1, 2 ou 3 groupes indépendamment choisis dans le groupe constitué de halogène CN, -(C₁-C₆)alkyle et -(C₁-C₆)haloalkyle.

2. Composé selon la revendication 1, ou un sel pharmaceutiquement acceptable de celui-ci, dans lequel:
Q est une liaison ou une fraction divalente choisie dans le groupe constitué de -C(O)- et - S(O)₂-; et
R⁵ est -C(R^{5A})(R^{5B})(R^{5C}),
dans lequel chacun de R^{5A}, R^{5B} et R^{5C} est indépendamment choisi dans le groupe constitué de: H, F, Cl, OH, -(C₁-C₆)alkyle, -(C₁-C₆)haloalkyle, -(C₁-C₆)alcényle, -(C₁-C₆)alcynyle, -(C₃-C₆)cycloalkyle, -(C₃-C₆)cycloalkyle substitué avec -(C₁-C₆)alkyle, -(C₁-C₆)alcényle, -(C₁-C₆)alcynyle, phényle, phényle substitué avec de 1 à 3 groupes indépendamment choisis parmi F, Cl, -(C₁-C₆)alkyle, -O-(C₁-C₆)alkyle et -C(O)O-(C₁-C₆)alkyle.

3. Composé selon la revendication 1, ou un sel pharmaceutiquement acceptable de celui-ci, dans lequel:
Q est une liaison ou une fraction divalente choisie dans le groupe constitué de -C(O)- et - S(O)₂-; et
R⁵ est choisi dans le groupe constitué de 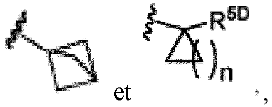 dans lequel n est un nombre entier de 1 à 4; et R^{5D} est choisi dans le groupe constitué de H, -(C₁-C₆)alkyle, -(C₁-C₆)haloalkyle, phényle et phényle substitué avec de 1 à 3 groupes indépendamment choisis dans le groupe constitué de OH, F, Cl, -(C₁-C₆)alkyle et -O-(C₁-C₆)alkyle.

4. Composé selon la revendication 1, ou un sel pharmaceutiquement acceptable de celui-ci, dans lequel:
R⁵ est un phényle,
dans lequel ledit phényle est non substitué ou substitué avec 1, 2 ou 3 groupes indépendamment choisis dans le groupe constitué de F, Cl CN, -(C₁-C₆)alkyle et -(C₁-C₆)haloalkyle.

5. Composé selon l'une quelconque des revendications précédentes, ou un sel pharmaceutiquement acceptable de celui-ci, dans lequel:
L est -C(O)-; et R³ est -N(R^{N1})(R^{N2}), dans lequel:
R^{N1} est choisi dans le groupe constitué de H et -(C₁-C₆)alkyle; et
R^{N2} est un -(C₁-C₆)alkyle qui est substitué avec 1 ou 2 groupes indépendamment choisis parmi:
phényle optionnellement substitué, (dans lequel lesdits substituants optionnels sur ledit phényle sont 1 à 3 groupes indépendamment choisis parmi OH, CN, -(C₁-C₄)alkyle, -(C₁-C₄)alcoxyle),
hétéroaryle optionnellement substitué, (dans lequel lesdits substituants optionnels sur ledit hétéroaryle sont 1 à 3 groupes indépendamment choisis parmi -(C₁-C₆)alkyle, -(C₁-C₄)alcoxyle et cyclopropyle),
cyclopropyle optionnellement substitué, (dans lequel lesdits substituants optionnels sur ledit cyclopropyle sont 1 à 3 groupes indépendamment choisis parmi -(C₁-C₆)alkyle),
hétérocycloalkyle optionnellement substitué, (dans lequel lesdits substituants optionnels sur ledit hétérocycloalkyle sont 1 à 3 groupes indépendamment choisis parmi halogène, OH, oxo, CN et -(C₁-C₆)alkyle), et
-O-(C₁-C₆)alkyle, -OH, F, Cl et -CN.

6. Composé selon l'une quelconque des revendications 1 à 4, ou un sel pharmaceutiquement acceptable de celui-ci, dans lequel:
L est choisi dans le groupe constitué de -C(O)- et -S(O)₂-; et
R³ est -N(R^{N1})(R^{N2}), dans lequel R^{N1} et R^{N2} sont chacun indépendamment choisis dans le groupe constitué de H et -(C₁-C₆)alkyle.

7. Composé selon l'une quelconque des revendications 1 à 4, ou un sel pharmaceutiquement acceptable de celui-ci, dans lequel:
L est -C(O)-; et R³ est -N(R^{N1})(R^{N2}), dans lequel:
R^{N1} et R^{N2} sont pris ensemble avec l'atome d'azote auquel ils sont montrés attachés pour former un cycle hétérocyclique de 4, 5 ou 6 membres totalement saturé comprenant (y compris ledit atome d'azote) 1, 2 ou 3 hétéroatomes de cycle choisis dans le groupe constitué de N, N-oxyde, O, S et S-oxyde,
dans lequel ledit cycle hétérocyclique est non substitué ou substitué avec 1, 2 ou 3 substituants indépendamment choisis dans le groupe constitué de halogène, -OH, oxo, CN, -(C₁-C₆)alkyle, -(C₁-C₆)alkyle à substitution amino (dans lequel ledit amino est 1, 2 ou 3 groupes indépendamment choisis dans le groupe constitué de -NH₂, -N(C₁-C₄ alkyle)₂ et -NH(C₁-C₄ alkyle)), -O-(C₁-C₆)alkyle, -(C₁-C₆)alkyl-OH, -(C₁-C₆)haloalkyle, -C(O)O-(C₁-C₆)alkyle, cyclopropyle, spirocyclopropyle, -CH₂-NHC(O)O-(C₁-C₆)alkyle, -CH₂-N(CH₃)C(O)O-(C₁-C₆)alkyle, phényle, benzyle, -NHC(O)-phényle, hétéroaryle et -(C₁-C₄)alkylhétéroaryle, hétérocycloalkyle.

8. Composé selon l'une quelconque des revendications 1 à 4, ou un sel pharmaceutiquement acceptable de celui-ci, dans lequel:
L est une liaison et R³ est un -(C₁-C₆)alkyle qui est optionnellement substitué avec OH.

9. Composé selon la revendication 1, ou un sel pharmaceutiquement acceptable de celui-ci, ledit composé étant choisi dans le groupe constitué de:

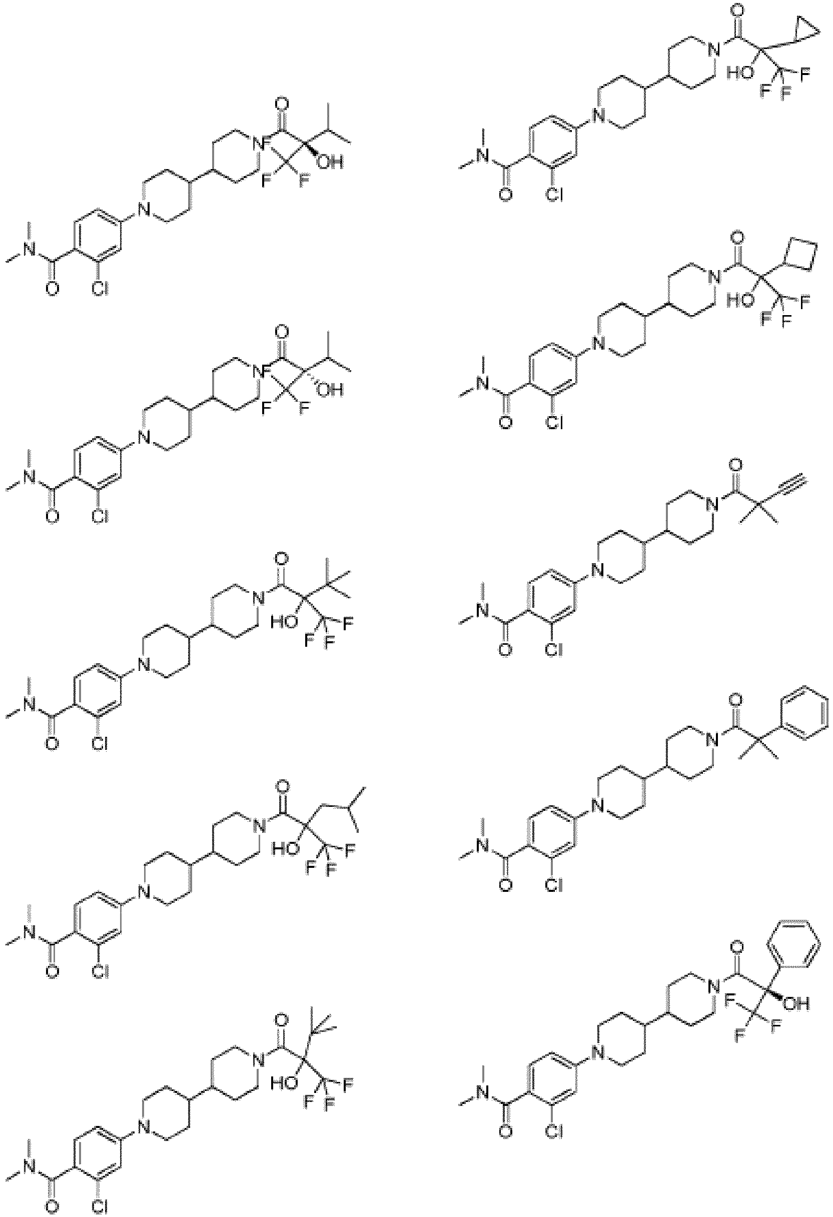

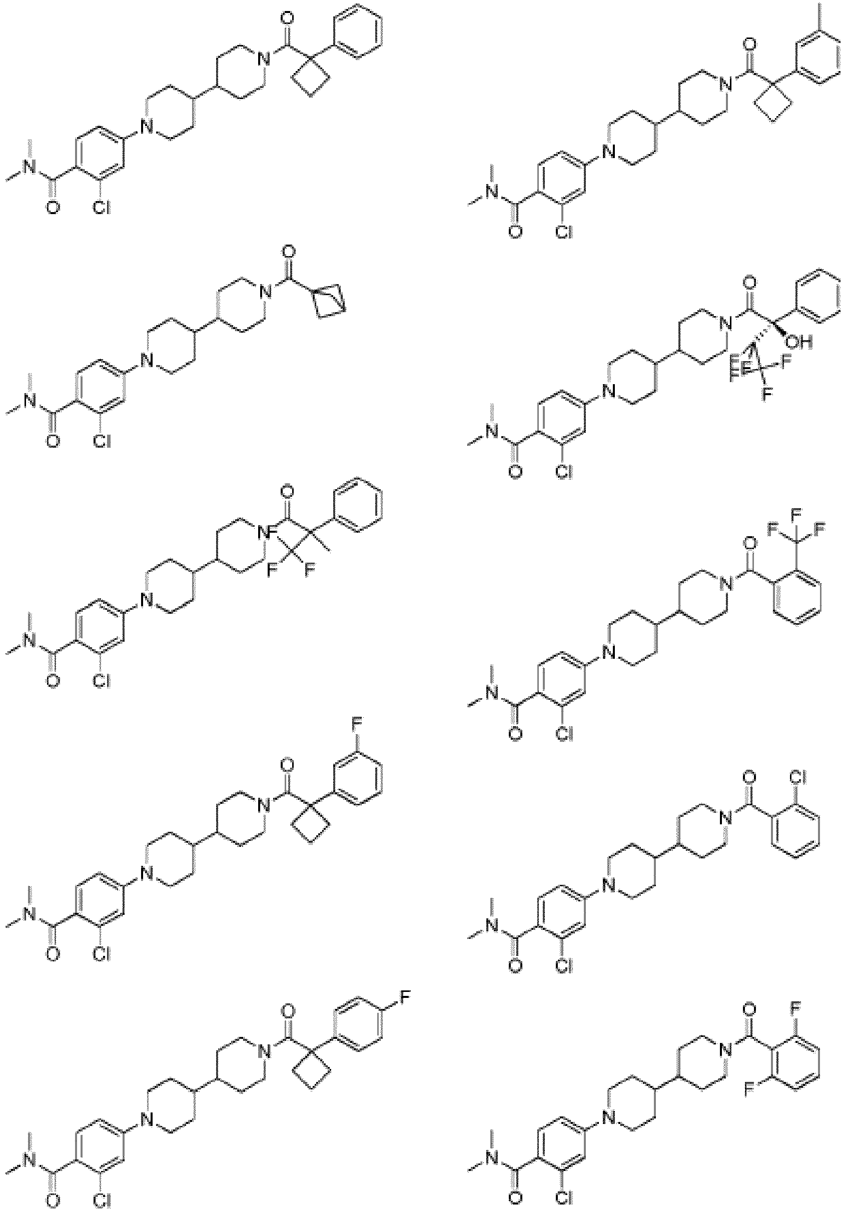

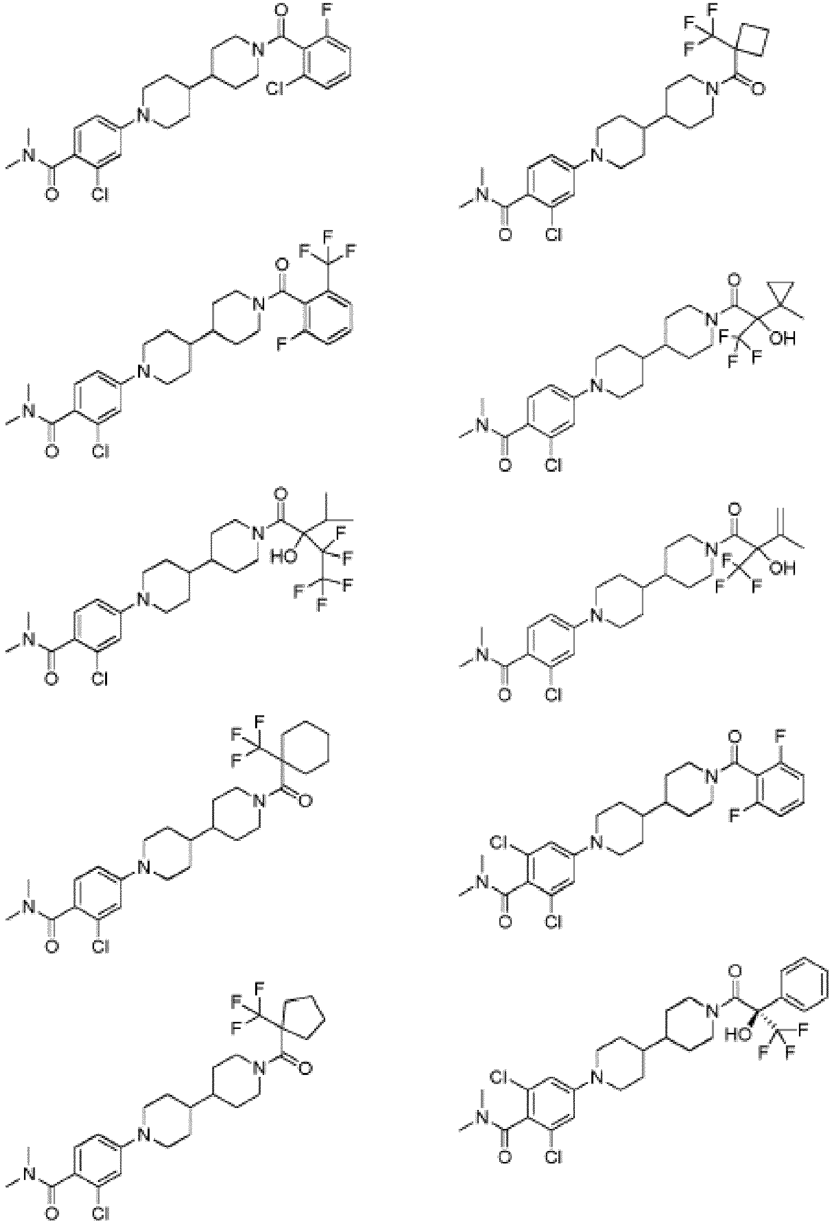

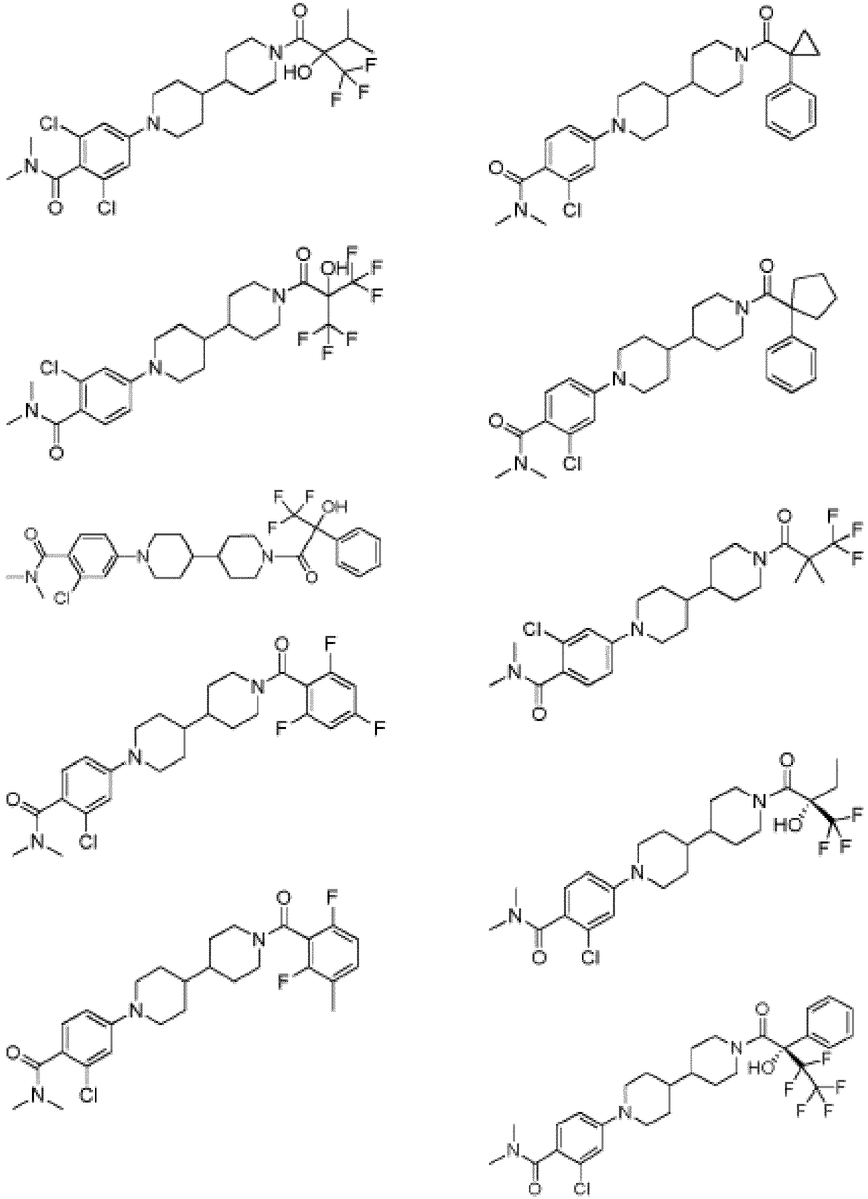

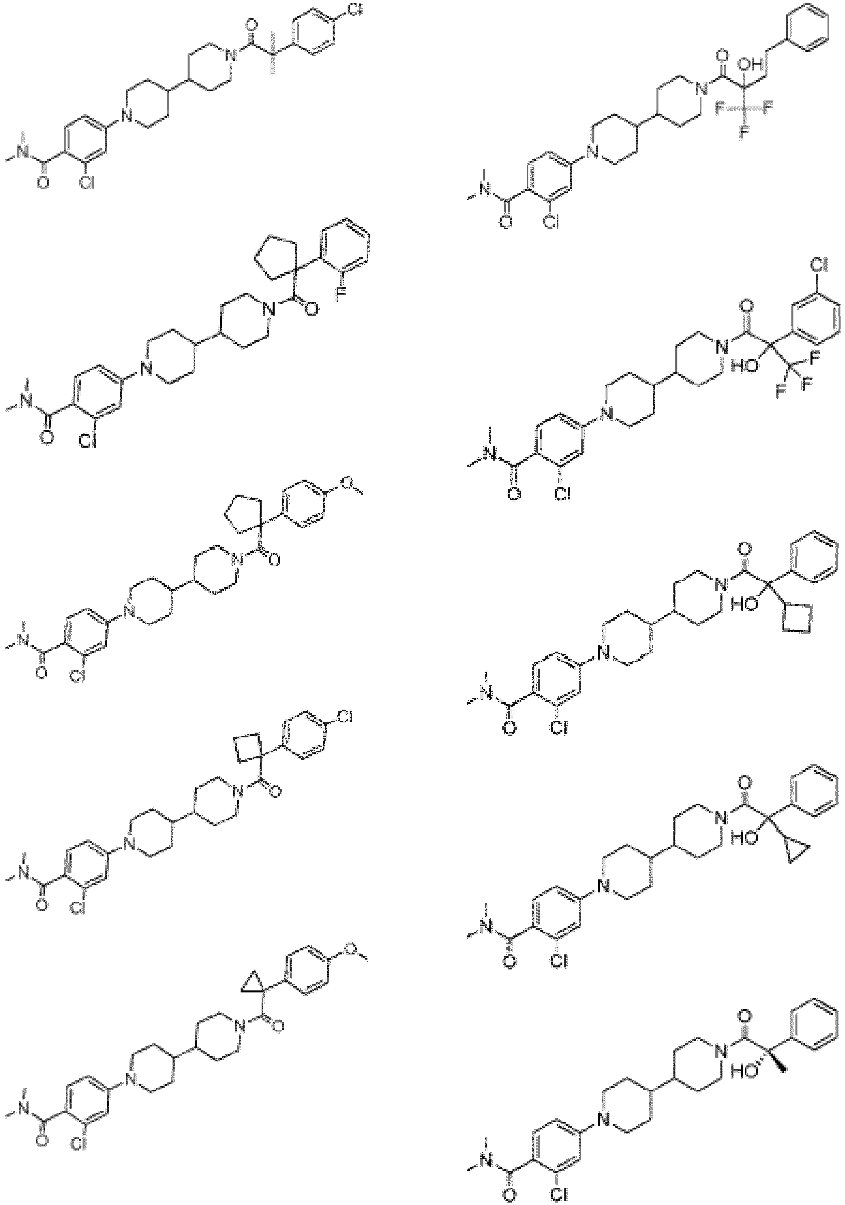

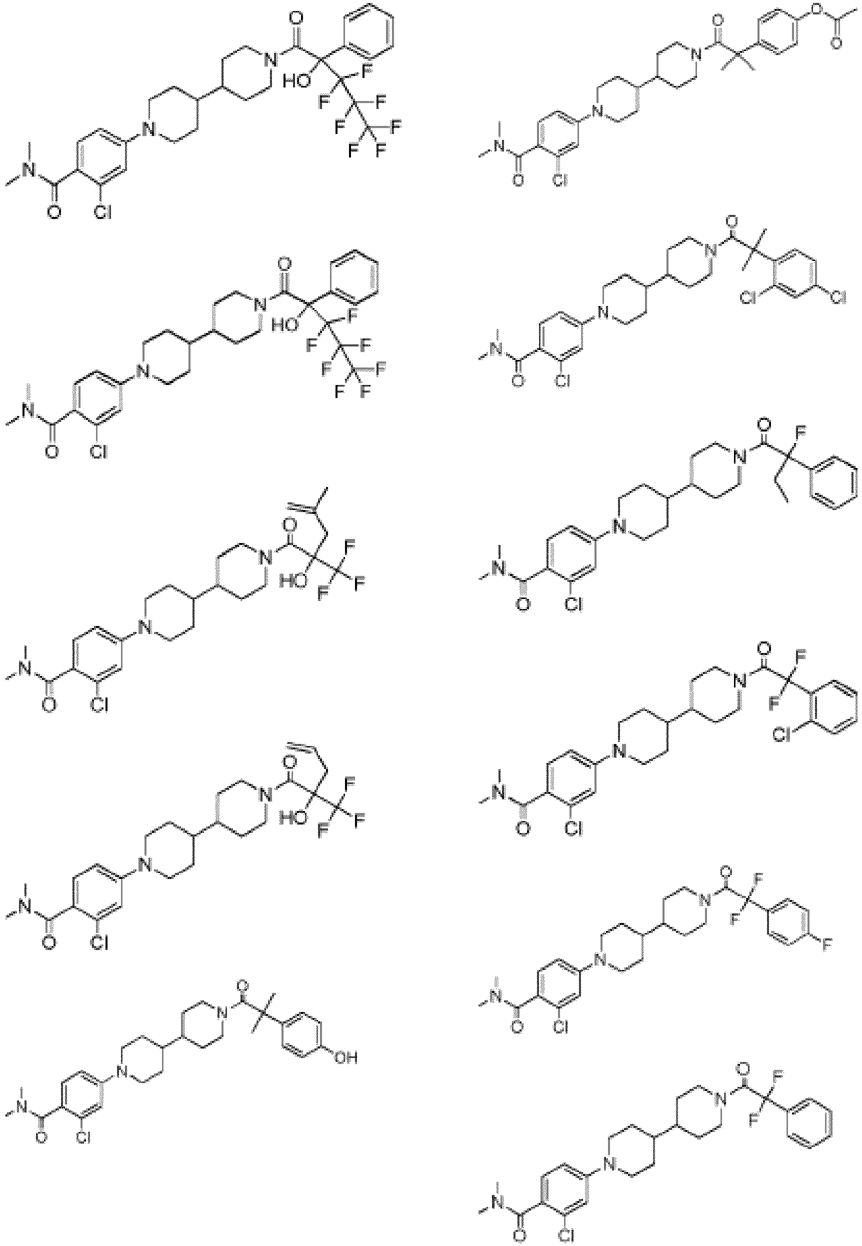

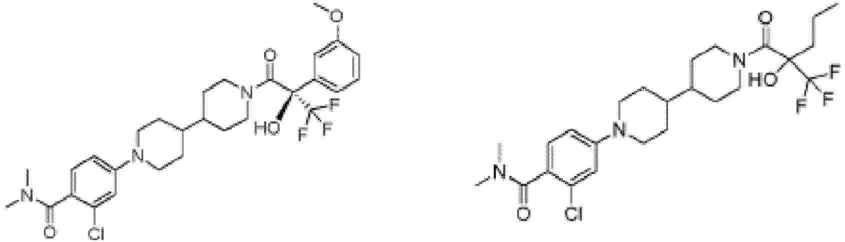

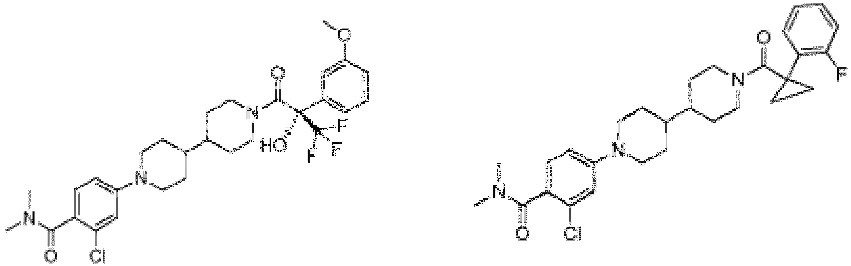

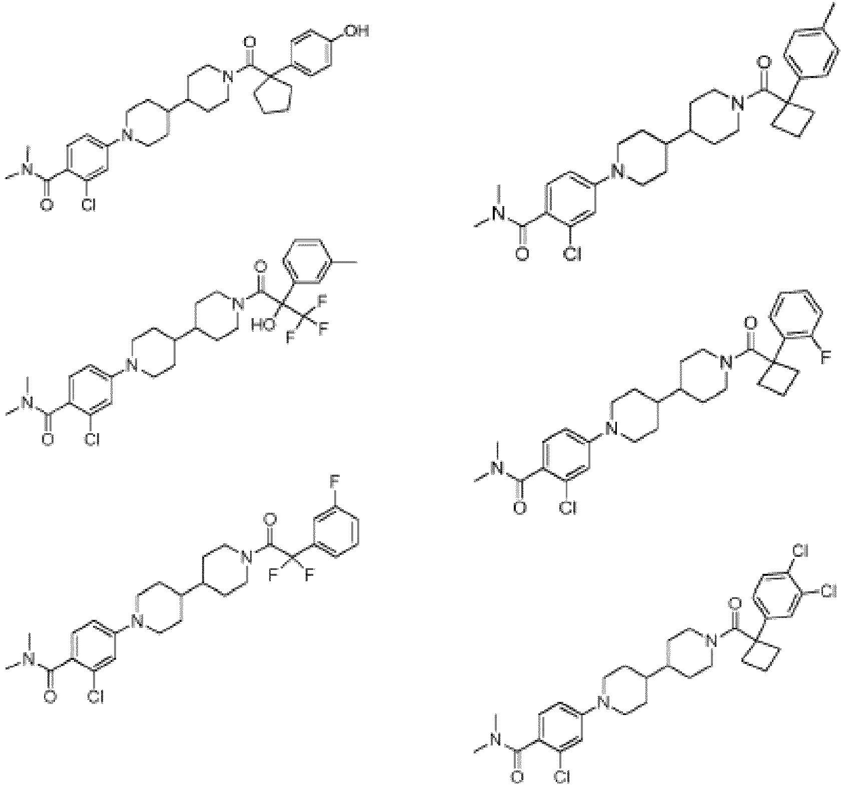

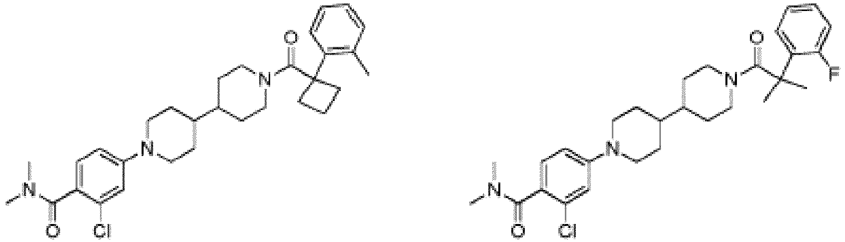

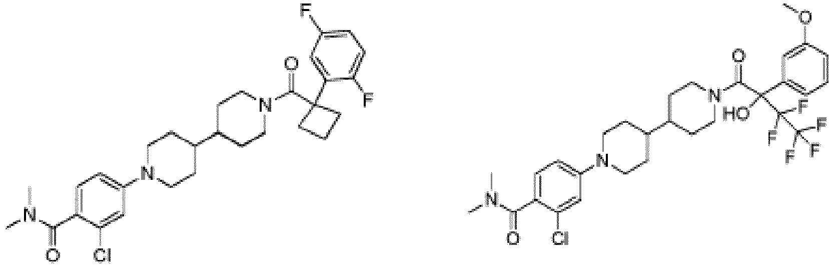

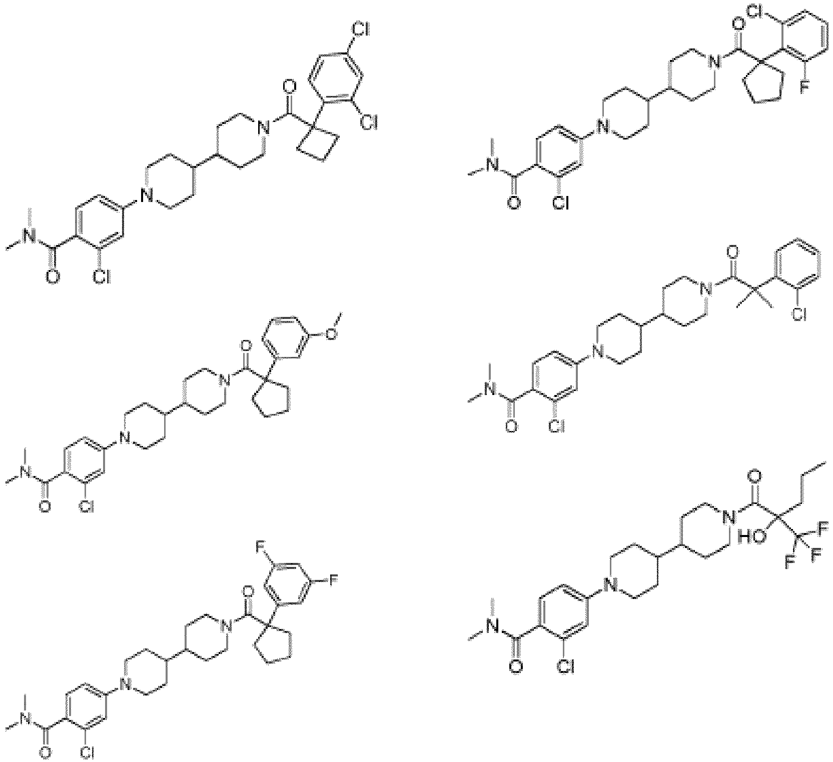

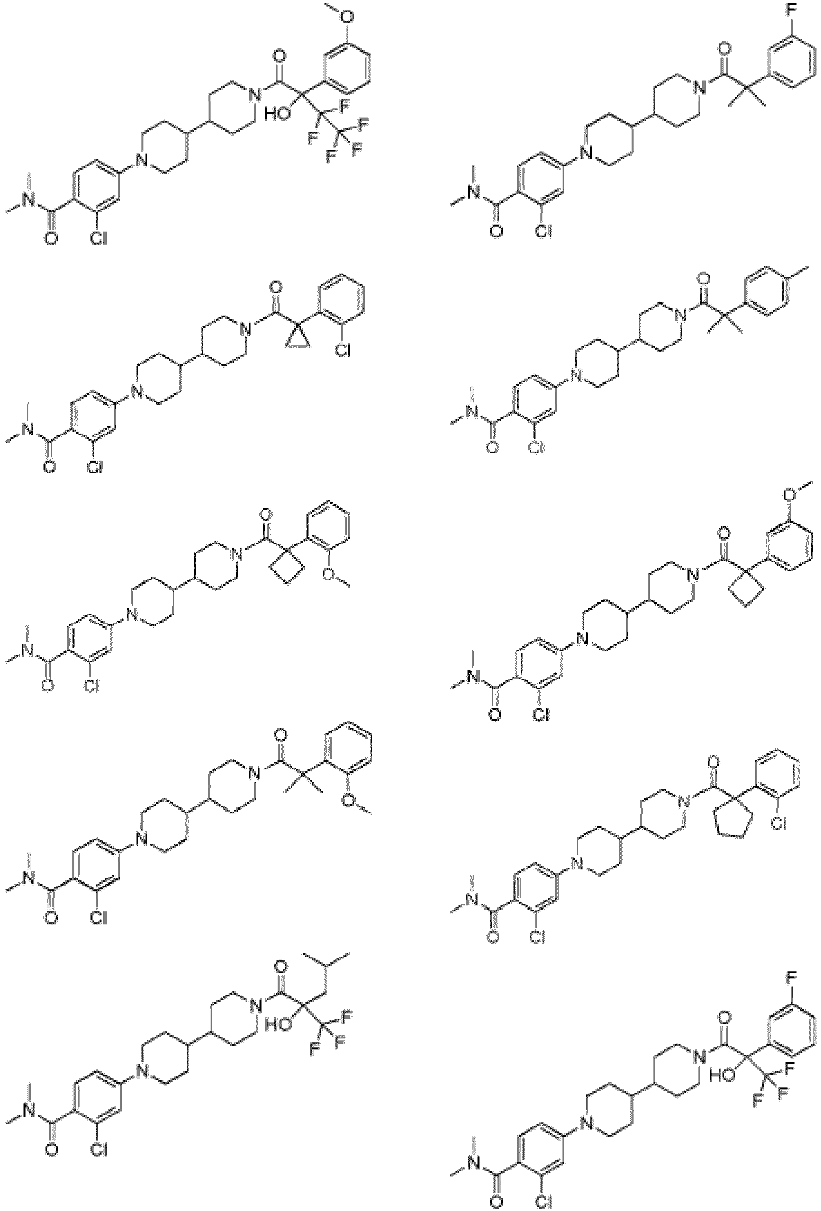

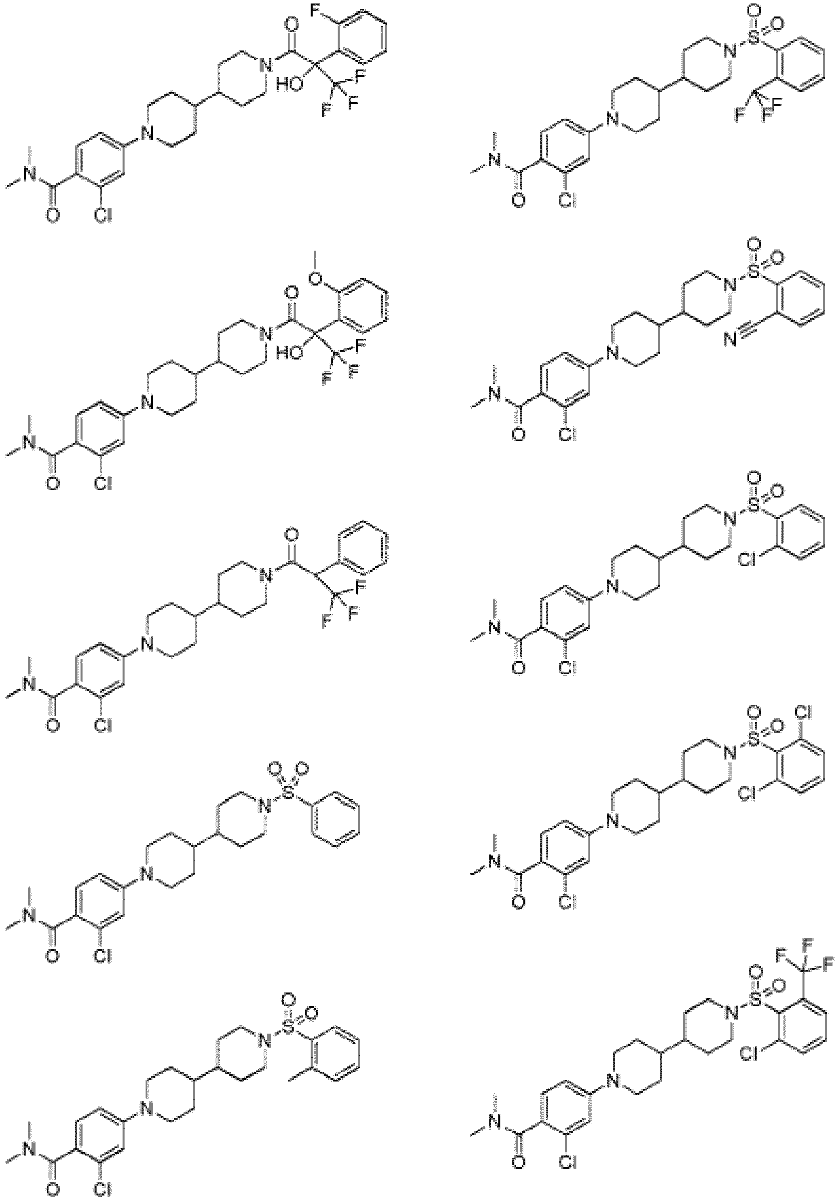

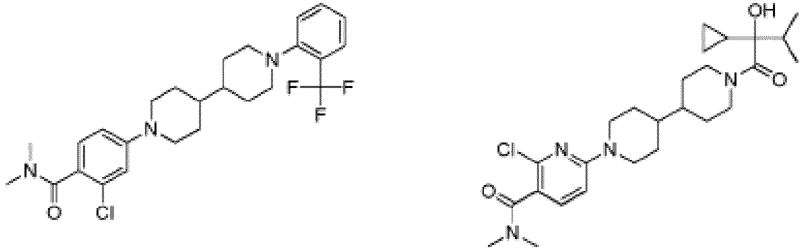

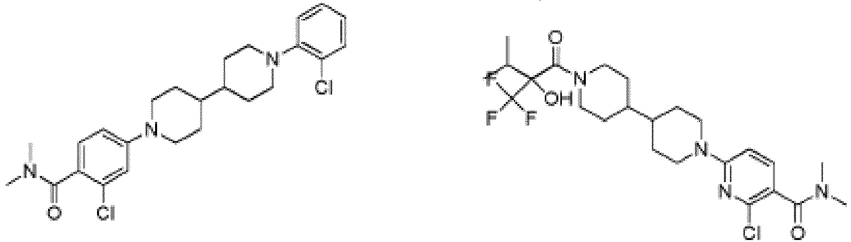

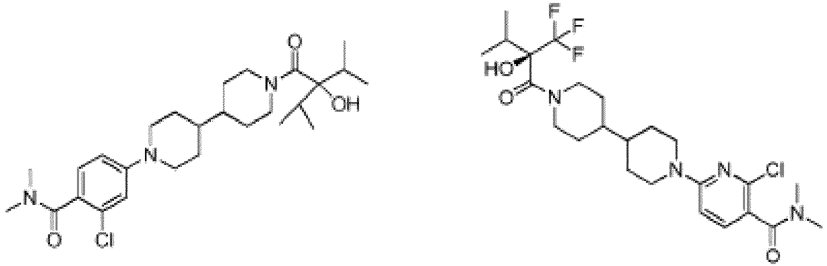

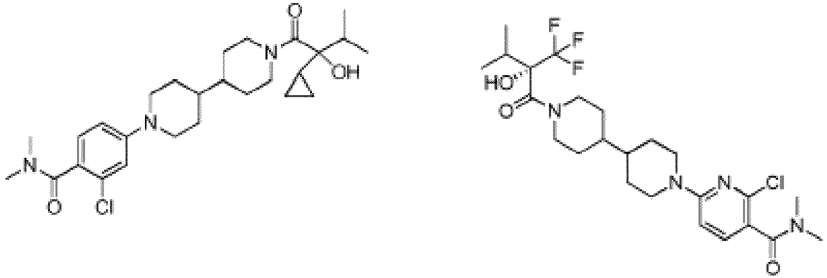

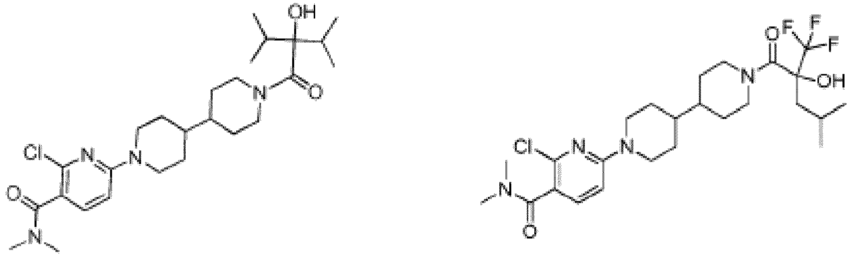

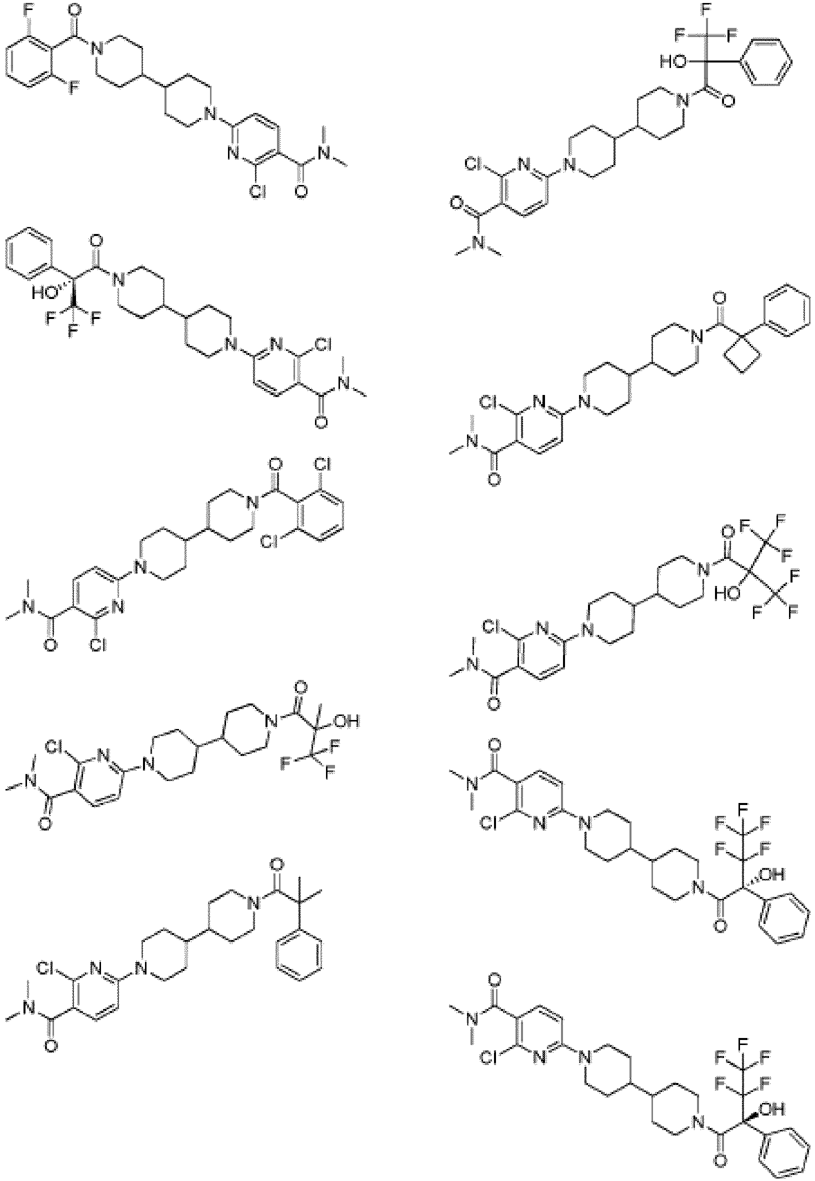

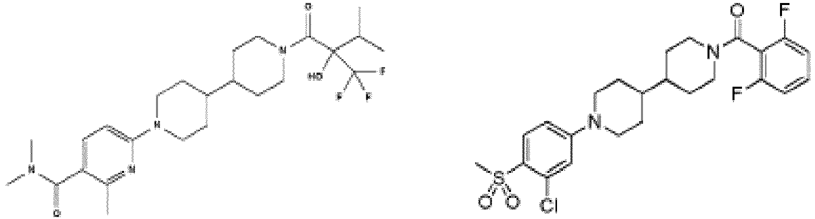

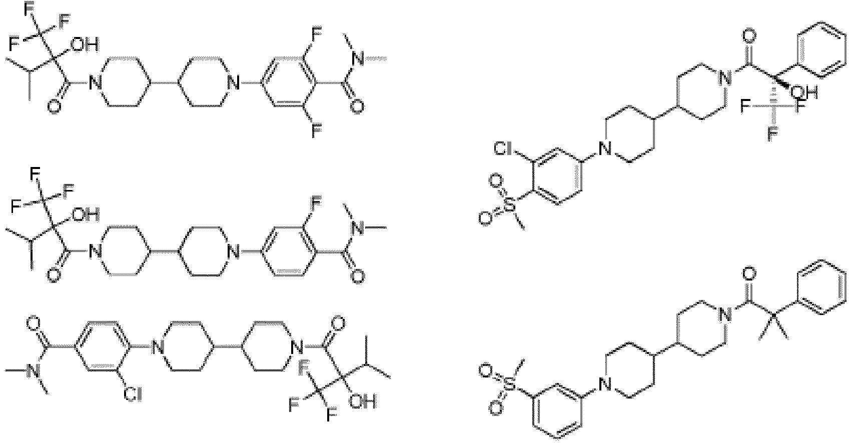

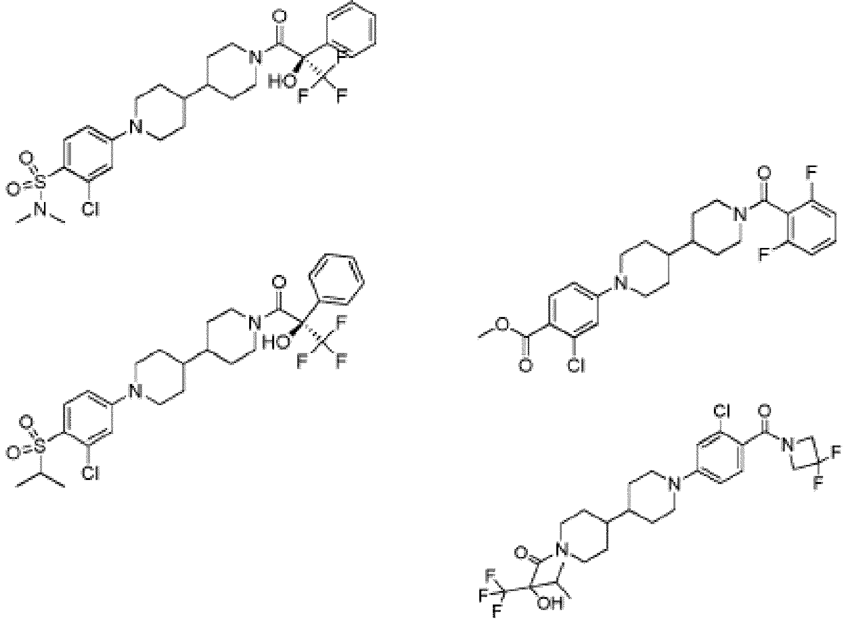

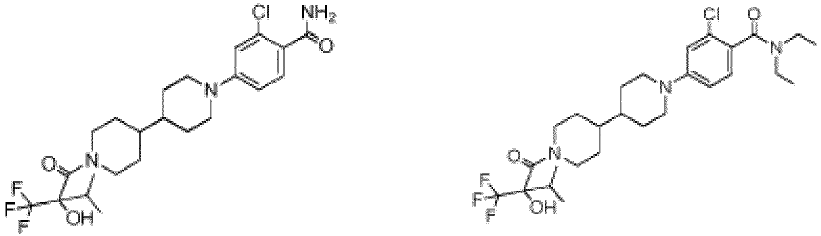

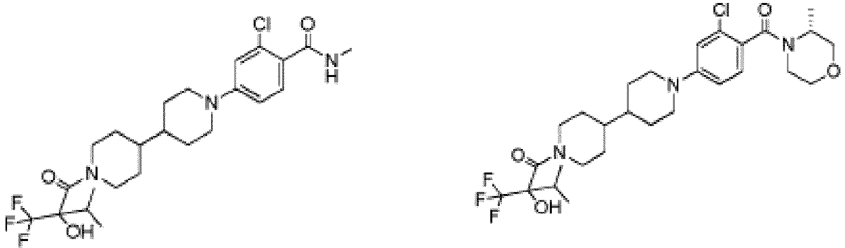

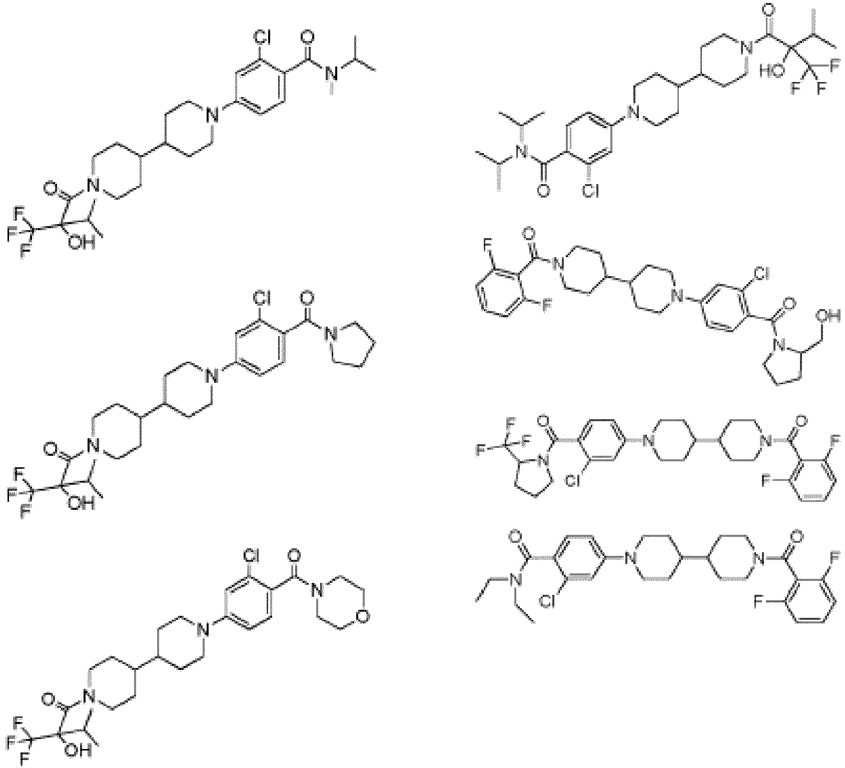

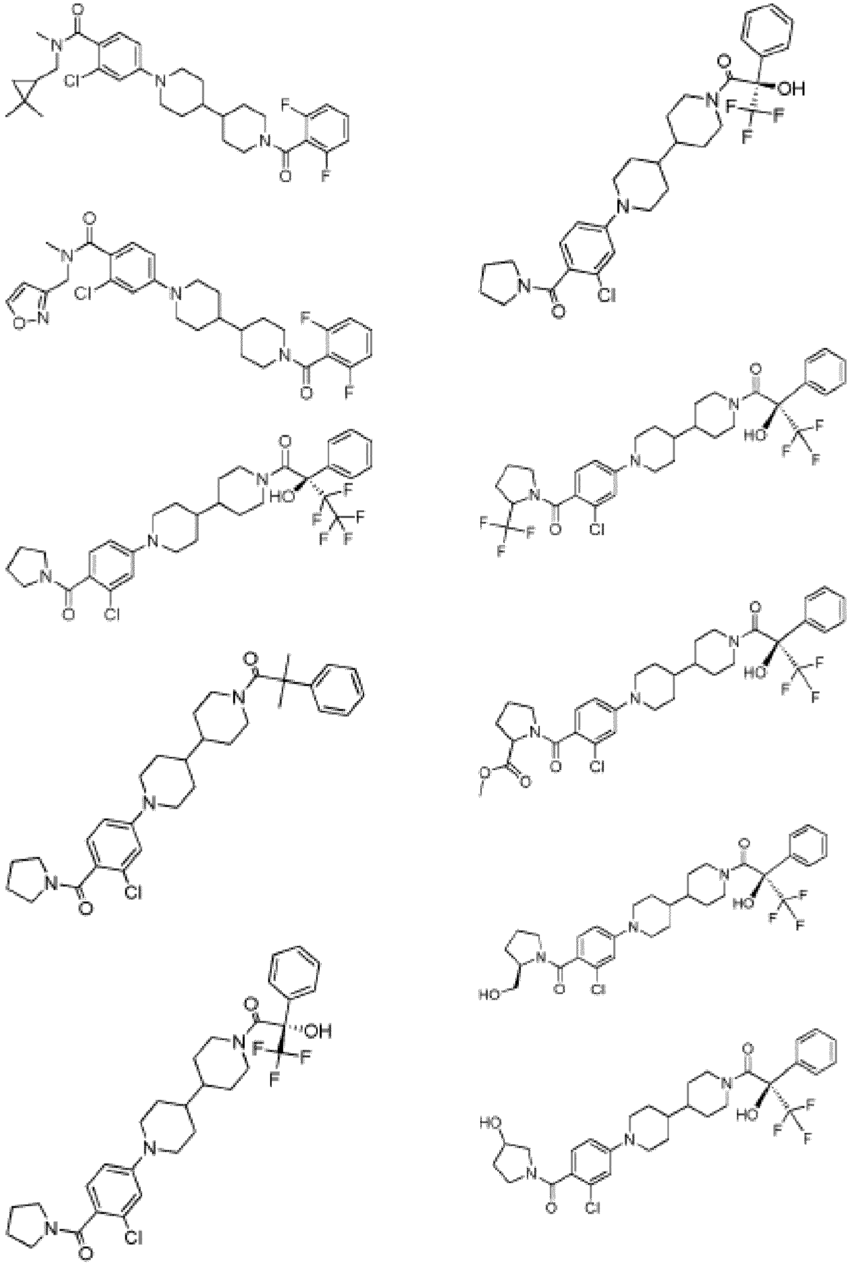

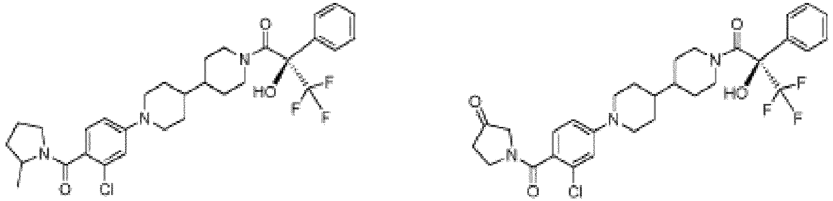

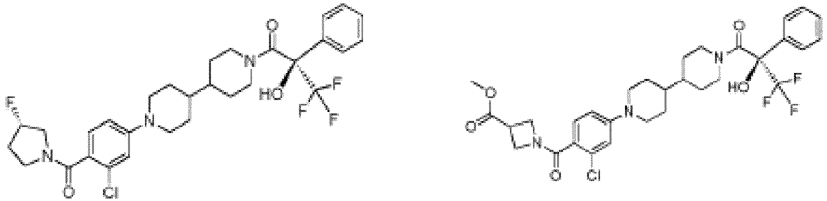

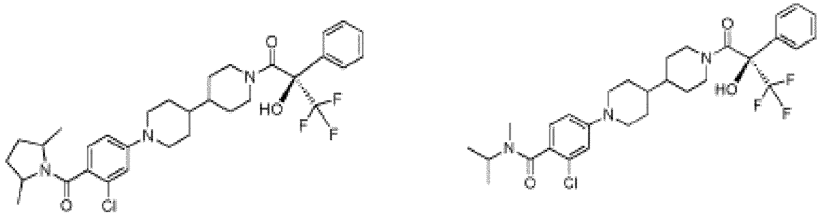

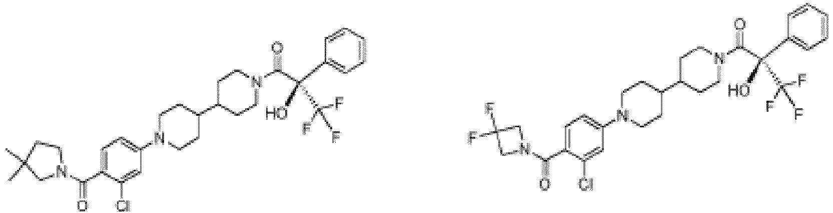

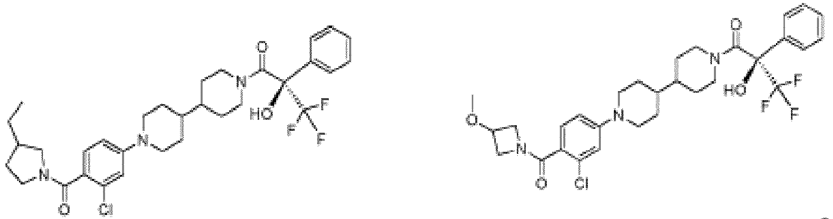

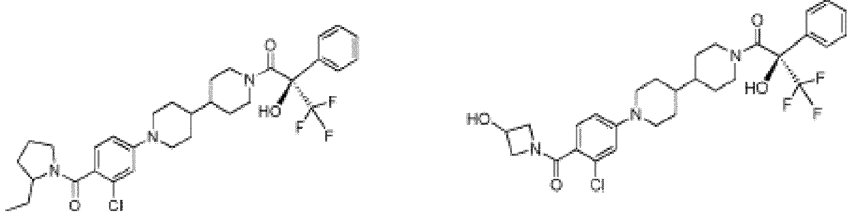

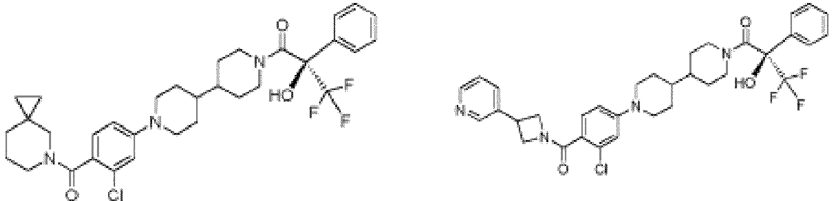

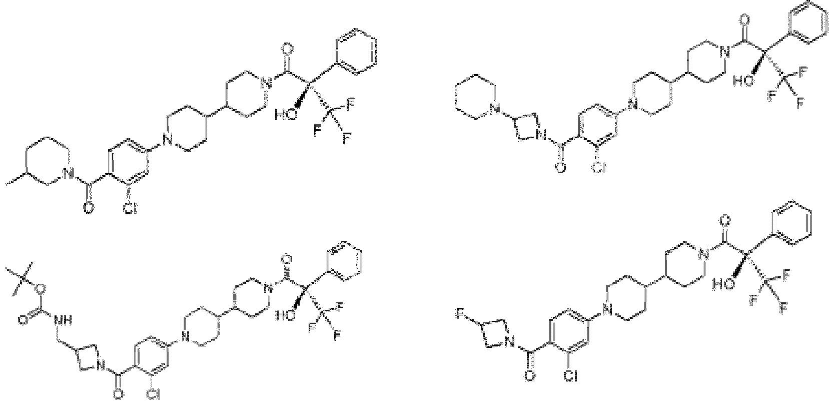

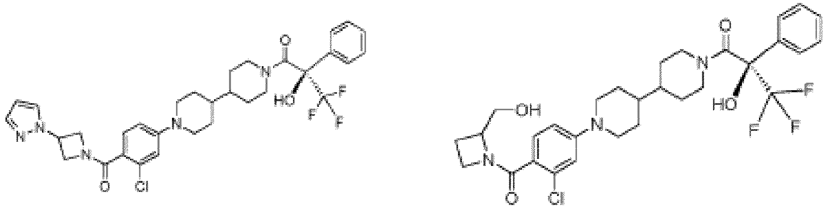

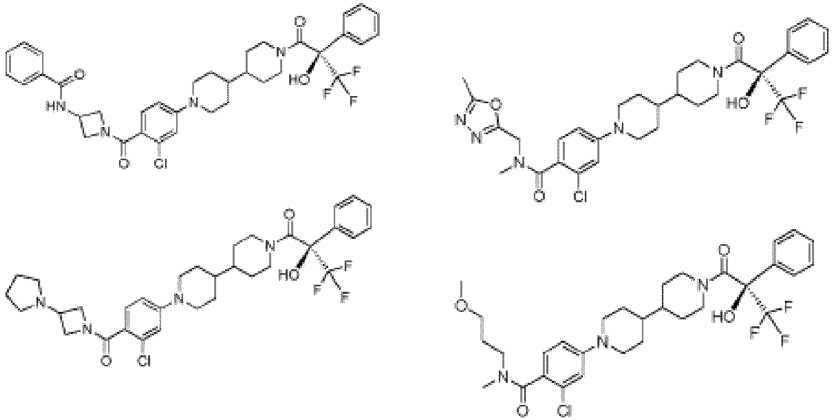

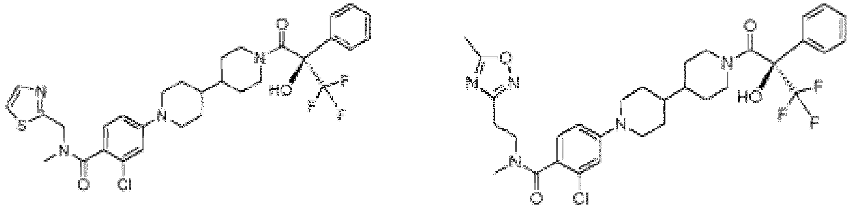

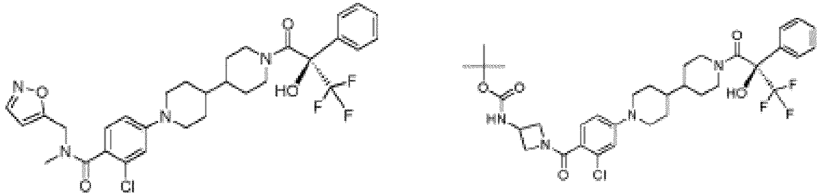

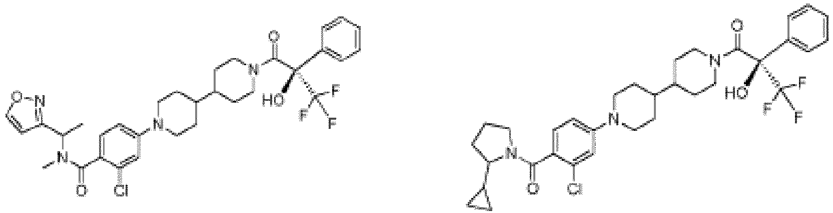

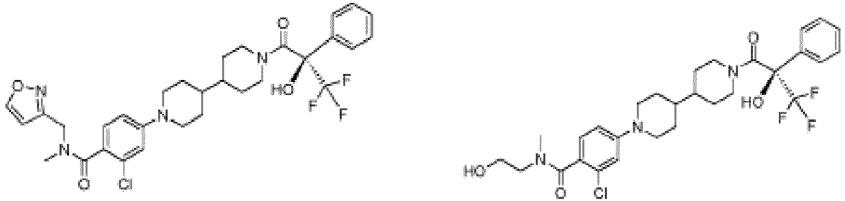

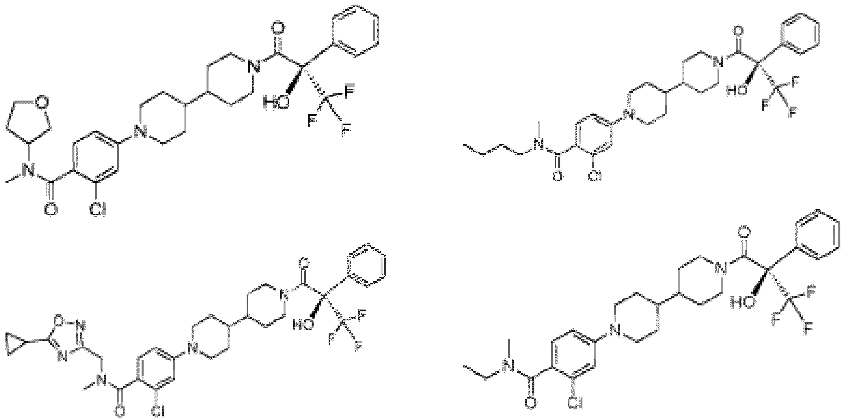

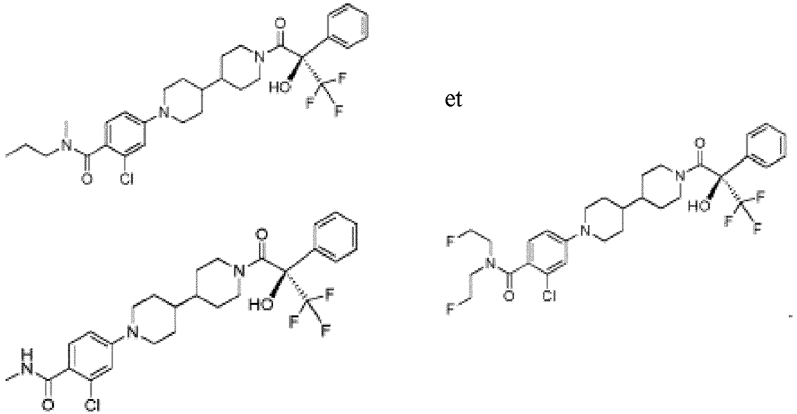

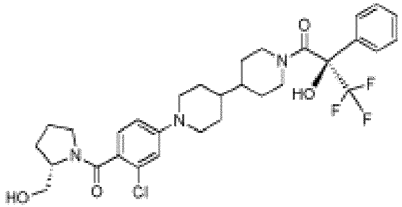

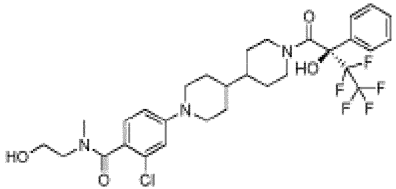

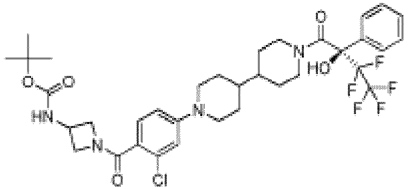

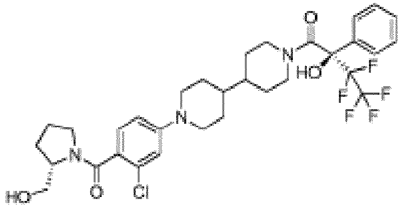

10. Composition pharmaceutique comprenant un composé selon l'une quelconque des revendications 1 à 9, un sel pharmaceutiquement acceptable de celui-ci, et un véhicule ou un diluant pharmaceutiquement acceptable.

11. Composé selon l'une quelconque des revendications 1 à 9, ou un sel pharmaceutiquement acceptable de celui-ci, pour une utilisation en tant que médicament.

12. Composé selon l'une quelconque des revendications 1 à 9, ou un sel pharmaceutiquement acceptable de celui-ci, pour une utilisation dans le traitement de: maladie d'Alzheimer, maladie de Neimann-Pick type Cl, maladie de Parkinson, sclérose latérale amyotrophique, accident vasculaire cérébral, dégénérescence maculaire liée à l'âge, schizophrénie, dépression, maladie cardiovasculaire, obésité ou diabète.

13. Composition pharmaceutique selon la revendication 10 comprenant en outre un ou plusieurs autres ingrédients actifs.
